(19) Europäisches Patentamt
European Patent Office
Office européen des brevets

(11) EP 0 650 485 B1

# (12) EUROPEAN PATENT SPECIFICATION

(45) Date of publication and mention of the grant of the patent: **11.10.2000 Bulletin 2000/41**

(21) Application number: **93918182.2**

(22) Date of filing: **13.07.1993**

(51) Int. Cl.$^7$: **C07D 333/18**, C07D 307/14, C07D 333/20, C07D 333/16, C07D 307/12

(86) International application number: **PCT/US93/06575**

(87) International publication number: **WO 94/01430 (20.01.1994 Gazette 1994/03)**

## (54) 2,5-DIARYL TETRAHYDRO-THIOPHENES, -FURANS AND ANALOGS FOR THE TREATMENT OF INFLAMMATORY AND IMMUNE DISORDERS

2,5-DIARYL TETRAHYDRO-THIOPENE, -FURANE UND ANALOGE ZUR BEHANDLUNG VON ENTZÜNDUNGS-UND IMMUNKRANKHEITEN

2,5-DIARYLE TETRAHYDRO-THIOPHENES, -FURANES ET ANALOGUES UTILISES DANS LE TRAITEMENT DE TROUBLES INFLAMMATOIRES ET IMMUNS

(84) Designated Contracting States:
**AT BE CH DE DK ES FR GB GR IE IT LI LU MC NL PT SE**

(30) Priority: **13.07.1992 US 912788**
**24.08.1992 US 933991**
**12.05.1993 US 62391**

(43) Date of publication of application: **03.05.1995 Bulletin 1995/18**

(73) Proprietor: **CYTOMED, INC.**
**Cambridge, MA 02141 (US)**

(72) Inventors:
- **CAI, Xiong**
  **Framington,MA 01701 Cambridge MA 02139 (US)**
- **HWANG, San-Bao**
  **Wayland, MA 01778 (US)**
- **KILLIAN, David**
  **Cambridge, MA 02141 (US)**
- **SHEN, T., Y.**
  **Charlottesville, VA 22903 (US)**
- **HUSSOIN, Sajjat**
  **Cambridge, MA 02140 (US)**

(74) Representative:
**Bassett, Richard Simon**
**Eric Potter Clarkson,**
**Park View House,**
**58 The Ropewalk**
**Nottingham NG1 5DD (GB)**

(56) References cited:

| | |
|---|---|
| EP-A- 0 144 804 | EP-A- 0 199 324 |
| EP-A- 0 217 204 | EP-A- 0 322 033 |
| EP-A- 0 365 089 | EP-A- 0 402 150 |
| EP-A- 0 402 151 | EP-A- 0 402 155 |
| EP-A- 0 402 156 | WO-A-92/09566 |
| GB-A- 2 197 650 | |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.7 (HRS)/3.6

## Description

## BACKGROUND OF THE INVENTION

This invention is in the area of pharmaceutical compositions and methods for the treatment of inflammatory and immune disorders, and specifically provides novel compounds that reduce damage arising from an inflammatory or immune response. The compounds exhibit this biological activity by acting as PAF receptor antagonists, by inhibiting the enzyme 5-lipoxygenase, or by exhibiting dual activity, i. e., by acting as both a PAF receptor antagonist and inhibitor of 5-lipoxygenase.

Platelet activating factor (PAF, 1-O-alkyl-2-acetyl-*sn*-glycerol-3-phosphorylcholine) is a potent inflammatory phospholipid mediator with a wide variety of biological activities. PAF was initially identified as a water soluble compound released by immunoglobulin E (IgE)-sensitized rabbit basophils. It is now known that PAF is also generated and released by monocytes, macrophages, polymorphonuclear leukocytes (PMNs), eosinophils, neutrophils, natural killer lymphocytes, platelets and endothelial cells, as well as by renal and cardiac tissues under appropriate immunological and non-immunological stimulation. (Hwang, "Specific receptors of platelet-activating factor, receptor heterogeneity, and signal transduction mechanisms", Journal of Lipid Mediators 2. 123 (1990)). PAF causes the aggregation and degranulation of platelets at very low concentrations. The potency (active at $10^{-12}$ to $10^{-9}$M), tissue level (picomoles) and short plasma 1 half life (2-4 minutes) of PAF are similar to those of other lipid mediators such as thromboxane $A_2$, prostaglandins, and leukotrienes.

PAF mediates biological responses by binding to specific PAF receptors found in a wide variety of cells and tissues. Structure-activity studies on PAF and its analogs indicate that the ability of PAF to bind to these receptors is highly structure specific and stereospecific. (Shen, *et al*., "The Chemical and Biological Properties of PAF Agonists, Antagonists, and Biosynthetic Inhibitors", Platelet-Activating Factor and Related Lipid Mediators, F. Snyder. Ed. Plenum Press, New York, NY 153 (1987)).

While PAF mediates essential biological responses, it also appears to play a role in pathological immune and inflammatory responses. Many published studies have provided evidence for the involvement of PAF in human diseases, including arthritis, acute inflammation, asthma, endotoxic shock, pain, psoriasis, ophthalmic inflammation, ischemia, gastrointestinal ulceration, myocardial infarction. inflammatory bowel diseases, and acute respiratory distress syndrome. Animal models also demonstrate that PAF is produced or increased in certain pathological states.

The involvement of PAF in pathological inflammatory and immune states has stimulated a substantial research effort to identify PAF receptor antagonists. In 1983, a phospholipid analog referred to as CV-3988 (*rac*-3-(N-*n*-octadecyl-carbamoyloxy-w-methoxypropy)-2-thiazolioethyl phosphate) was reported to have PAF receptor antagonist properties. (Terashita, *et al*., Life Sciences 32, 1975 (1983)). In other early work in this area, Shen, *et al*., (in Proc. Natl. Acad. Sci. USA 82, 672 (1985)), reported that kadsurenone, a neolignan derivative isolated from Piper futokadsura Sieb et Zucc (a Chinese herbal plant) was a potent, specific and competitive inhibitor of PAF activity at the receptor level. Hwang, *et al*., disclosed in 1985 that *trans*-2,5-*bis*-(3,4,5-trimethoxyphenyl) tetrahydrofuran (L-652,731) inhibits the binding of tritiated PAF to PAF receptor sites. (Hwang, *et al*., "*Trans*-2,5-bis-(3,4,5-trimethoxyphenyl)tetrahydrofuran", Journal of Biological Chemistry 260, 15639 (1985).) L-652,731 was found to be orally active, and to inhibit PAF-induced rat cutaneous vascular permeability at a dosage of 30 mg/kg body weight. The compound was found to have no effect on the enzyme 5-lipoxygenase. Hwang, *et al*., also reported that *trans*-L-652,731, wherein the aryl groups at the 2 and 5 positions are on opposite sides of the plane of the tetrahydrofuran ring, is approximately 1000 times more potent than *cis*-L-652,731, wherein the 2 and 5 aryl substituents are on the same side of the plane of the tetrahydrofuran ring.

In 1988, Hwang, *et al*., reported that L-659,989 (*trans*-2-(3-methoxy-4-propoxyphenyl-5-methylsulfonyl)-5-(3,4,5-trimethoxyphenyl)tetrahydrofuran) is an orally active, potent, competitive PAF receptor antagonist, with an equilibrium inhibition constant 10 times greater than that of *trans*-L-652,731. (Hwang, *et al*., J. Pharmacol. Exp. Ther. 246, 534 (1988).) U.S. Patent Nos. 4,996,203, 5,001,123 and 4,539,332 to Biftu, *et al*. and European Patent Application Nos. 365089, 402151, 402150 disclose that a specific class-of 2,5-diaryl tetrahydrofurans are PAF receptor antagonists.

Leukotrienes, like PAF, are potent local mediators, playing a major role in inflammatory and allergic responses, including arthritis, asthma, psoriasis, and thrombotic disease. Leukotrienes are straight chain eicosanoids produced by the oxidation of arachidonic acid by lipoxygenases. Arachidonic acid is oxidized by 5-lipoxygenase to the hydroperoxide 5-hydroperoxyeicosatetraenoic acid (5-HPETE), which is converted to leukotriene $A_4$, which in turn can be converted to leukotriene $B_4$, $C_4$, or $D_4$. The slow-reacting substance of anaphylaxis is now known to be a mixture of leukotrienes $C_4$, $D_4$, and $E_4$, all of which are potent bronchoconstrictors.

There has been a research effort to develop specific receptor antagonists or inhibitors of leukotriene biosynthesis, to prevent or minimize pathogenic inflammatory responses mediated by these compounds.

Leukotrienes are released simultaneously from leukocytes with PAF, possibly from a common phospholipid precursor such as 1-O-hexadecyl-2-arachidonyl-*sn*-glycero-phosphocholine, and upon cellular activation, act synergistically with PAF in many biological models. Recently, it was reported that the tetrahydrothiophene derivative of L-652,731, *trans*-2,5-*bis*-(3,4,5-trimethoxyphenyl)tetrahydrothiophene (L-653,150), is a potent PAF antagonist and a moderate inhibitor of 5-lipoxygenase. It has been disclosed that certain 2,5-diaryl tetrahydrothiophenes are PAF antagonists and leukotriene synthesis inhibitors. (Biftu, *et al*., Abstr. of 6th Int. Conf. on Prostaglandins and Related Compounds, June 3-6, 1986, Florence, Italy; U.S. Patent No. 4,757,084 to Biftu).

European Patent Application No. 416609 discloses indole, benzofuran, and benzothiophene lipoxygenase inhibiting compounds.

WO-A-92/09566 discloses certain N'- hydroxyureidalkyl and hydroxyamylalkyl derivatives of cycloalkanes as inhibitors of 5- lipoxygenase.

Given the significant number of pathological immune and inflammatory responses that are mediated by PAF and leukotrienes, there remains a need to identify new compounds and compositions that exhibit PAF receptor antagonistic activity or inhibit the enzyme 5-lipoxygenase.

Therefore, it is an object of the present invention to provide compounds that reduce the chemotaxis and respiratory burst leading to the formation of damaging oxygen radicals during an inflammatory or immune response.

It is another object of the present invention to provide pharmaceutical compositions for the treatment of pathological immune or inflammatory disorders mediated by PAF or products of 5-lipoxygenase.

It is another object of the present invention to provide methods for the treatment of pathological immune or inflammatory disorders mediated by PAF or products of 5-lipoxygenase.

## SUMMARY OF THE INVENTION

2,5-Diaryl tetrahydrothiophenes and tetrahydrofurans for the treatment of pathological immune or inflammatory disorders are disclosed of the structure:

**Formula I**

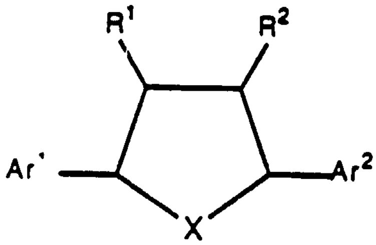

**wherein:**

$Ar^1$ =

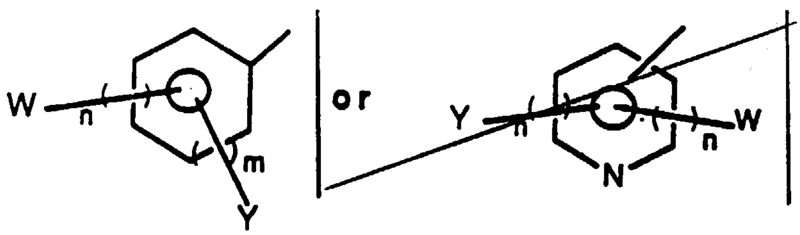

or

$Ar^2$ =

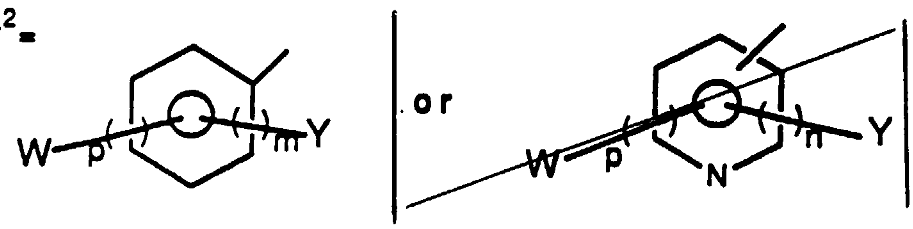

or

wherein:

X is O, S
W is independently:

$-AN(OM)C(O)N(R^3)R^4$, $-AN(R^3)C(O)N(OM)R^4$, $-N(OM)C(O)N(R^3)R^4$ or $-N(R^3)C(O)N(OM)R^4$

n is 1;
m is 2 or 3;
p is 0;
A is $C_{1-10}$ alkylene, $C_{2-10}$ alkenylene or $C_{2-10}$ alkynylene
M is hydrogen;
Y is independently:
$R^1$ and $R^2$ are hydrogen
$R^3$ and $R^4$ are independently $C_{1-10}$ alkyl, $C_{2-10}$ alkenyl or $C_{2-10}$ alkynyl
$R^{11}$ is $C_1$ to $C_{12}$ alkyl or substituted $C_1$ to $C_{12}$ alkyl wherein the substituent is selected from the group consisting of phenyl-$S(O)_g$-$C_{1-6}$ alkylene-; $(R^3O)_d$-phenyl-$S(O)_g$-$C_{1-6}$ alkylene-; $(R^3R^3N)_d$phenyl-$S(O)_g$-$C_{1-6}$ alkylene-; $(CN)_d$-phenyl-$S(O)_g$-$C_{1-6}$ alkylene-; $(halo)_d$-phenyl-$S(O)_g$-$C_{1-6}$ alkylene-; $(R^3COO)_d$-phenyl-$S(O)_g$-$C_{1-6}$ alkylene-; $(R^3OCO)_d$-phenyl-$S(O)_g$-$C_{1-6}$ alkylene-; $(R^3O)_d$-phenyl-O-$C_{1-6}$ alkylene-; $(CN)_d$-phenyl-O-$C_{1-6}$ alkylene-; $(halo)_d$-phenyl-O-$C_{1-6}$ alkylene-; $(R^3COO)_d$-phenyl-O-$C_{1-6}$ alkylene-; $(R^3OCO)_d$-phenyl-O-$C_{1-6}$ alkylene-; or $(R^3CO)_d$-phenyl-O-$C_{1-6}$ alkylene- where d is 1, 2, 3, 4 or 5; and g is 0, 1, or 2.

These compounds in general reduce the chemotaxis and respiratory burst leading to the formation of damaging oxygen radicals of polymorphonuclear leukocytes during an inflammatory or immune response. The compounds exhibit this biological activity by acting as PAF receptor antagonists, by inhibiting the enzyme 5-lipoxygenase, or by exhibiting dual activity, i. e., by acting as both a PAF receptor antagonist and inhibitor of 5-lipoxygenase.

A use of a compound as defined herein in a method to treat disorders mediated by PAF or leukotrienes is also disclosed, that includes administering an effective amount of our or more of the above-identified compounds or a pharmaceutically acceptable salt thereof, optionally in a pharmaceutically acceptable carrier, to reduce formation of oxygen radicals.

The compounds disclosed herein can also be used as research tools to study the structure and location of PAF receptors as well as biological pathways involving leukotrienes.

Where any of the following listed compounds and examples, and/or schemes, tables and figures relating to or including such compounds or examples, do not fall within the scope of the accompanying claims it is to be understood that they are disclosed for the purposes of understanding the invention, and are thus provided in an illustrative sense.

## BRIEF DESCRIPTION OF THE DRAWINGS

Figure 1 is a schematic illustration of a process for a preparation of 3,4,5-trimethoxyphenylvinylketone (compound 106, Fgure 1).

Figure 2 is a schematic illustration of a process for apreparation of *trans*-2-(3,4-dimethoxy-5-aminoethylthiophenyl)-5-(3,4,5-trimethoxyphenyl)-tetrahydrothiophene (compound 1, Figure 2) and *trans*-2-(3,4-dimethoxy-5-aminoethyl sulfonylphenyl)-5-(3,4,5-trimethoxyphenyl)-tetrahydrothiophene (compound 2, Figure 2).

Figure 3 is a schematic illustration of a process for a preparation of *trans*-2-(3-methoxy-4-propoxy-5-aminophenyl)-5-(3,4,5-trimethoxyphenyl)-tetrahydrofuran (compound 122, Figure 3), *trans*-2-(3,4-dimethoxy-5-aminophenyl)-5-(3,4,5-trimethoxyphenyl)-tetrahydrothiophene (compound 123, Figure 3),and *trans*-2-(3-methoxy-4-propoxy-5-aminophenyl)-5-(3,4,5-trimethoxyphenyl) tetrahydrothiophene (compound 124, Figure 3).

Figure 4 is a schematic illustration of a process for the preparation of *trans*-2-(3-methoxy-4-propoxy-5-benzylaminophenyl)-5-(3,4,5-trimethoxyphenyl) tetrahydrofuran (compound 3, Figure 4),*trans*-2-(3-methoxy-4-propoxy-5-hydroxyethylaminophenyl)-5- (3,4,5-trimethoxyphenyl)-tetrahydrofuran (compound 4, Figure 4), *trans*-2-(3-methoxy-4- propoxy-5-N,N-diallylaminophenyl)-5-(3,4,5-trimethoxyphenyl)-tetrahydrofuran (compound 5, Figure 4), *trans*-2-(3,4-dimethoxy-5-benzylaminophenyl)-5-(3,4,5-trimethoxyphenyl)-tetrahydrothiophene (compound 6, Figure 4), *trans*-2-(3-methoxy-4-propoxy-5-benzylaminophenyl)-5-(3,4,5-trimethoxyphenyl)-tetrahydrothiophene

(compound 7, Figure 4), *trans*-2-(3,4-dimethoxy-5-hydroxyethylaminophenyl)-5-(3,4,5-trimethoxyphenyl) tetrahydrothiophene (compound 8, Figure 4), *trans*-2-(3-methoxy-4-propoxy-5-hydroxyethylaminophenyl)-5-(3,4,5-trimethoxyphenyl)-tetrahydrothiophene (compound 9, figure 4), *trans*-2-(3,4-dimethoxy-5-N-diallylaminophenyl)-5-(3,4,5-trimethoxyphenyl)-tetrahydrothiophene (compound 10, Figure 4), and *trans*-2-(3-methoxy-4-propoxy-5- N,N-diallylaminophenyl)-5-(3,4,5-trimethoxyphenyl) tetrahydrothiophene (compound 11, Figure 4), and cis- and *trans*-2-[N'-hydroxyl-N'-(substituted)]-[2-propoxy-3-methoxy-5-{ 5-(3,4,5-trimethoxyphenyl)-(tetrahydrofuran or tetrahydrothiophene)}-phenyl]-urea (compounds 12-32 and 38-41, Figure 4).

Figure 5 is a schematic illustration of a process for the preparation of *trans*-2-(3-methoxy-4-propoxy-5-hydroxyethylsulfonyl)-5-(3,4,5-trimethoxyphenyl)-tetrahydrothiophene (compound 38).

Figure 6 is a schematic illustration of a process for the preparation of *trans*-2-(5-(N-hydroxy-N-(substituted)-aminocarbonyl)-aminomethyl-3-methoxy-4-propoxyphenyl)-5-(3,4,5-trimethoxyphenyl)-tetrahydrofuran (compounds 33-37, Figure 6).

Figure 7 is a schematic illustration of a process for the preparation of 1-(3-nitro-4-propoxy-5-methoxyphenyl)-3-(3,4,5-trimethoxyphenyl)cyclopentane (compound 143, Figure 7), 1-(3-amino-4-propoxy-5-methoxyphenyl)-3-(3,4,5-trimethoxyphenyl)cyclopentane (compound 144, Figure 7), and 2-(N'-hydroxyl-N'-(substituted)-N-(2-propoxy-3-methoxy-5-(5-(3,4,5-trimethoxyphenyl) cyclopentane) phenyl urea (compound 145, Figure 7).

Figure 8 is a schematic illustration of a process for the preparation of 2-(5-(N-hydroxy-N-methylaminocarbonyl)-amino(substituted)-3-methoxy-4-propoxy)-5-(3,4,5-trimethoxyphenyl)-cyclopentane (compound 158, Figure 8).

Figure 9 is a schematic illustration of a process for the preparation of 2-(5-(N-hydroxy-N-methylaminocarbonyl)-amino(substituted)-3-methoxy-4-propoxy)-4-(3,4,5-trimethoxyphenyl)-tetrahydrofuran (compound 167, Figure 9), and 2-(5-(N-hydroxy-N-methylaminocarbonyl)-amino(substituted) -3-methoxy-4-propoxy)-4-(3,4,5-trimethoxyphenyl)-tetrahydrothiophene (compound 168, Figure 9).

Figures 10a and 10b provide a schematic illustration of a process for the preparation of *trans*-2-[5-(N'-methyl-N'-hydroxyureidylmetyl)-3-methoxy-4-p-chlorophenylthioethoxyphenyl]-5-(3,4,5-trimethoxyphenyl)tetrahydrofuran.

## DETAILED DESCRIPTION OF THE INVENTION

### I. Description and Synthesis of the Compounds

#### A. Compounds

The term alkyl, as used herein, unless otherwise specified, refers to a saturated straight, branched, or cyclic hydrocarbon of $C_1$ to $C_{10}$, and specifically includes methyl, ethyl, propyl, isopropyl, butyl, isobutyl, t-butyl, pentyl, cyclopentyl, isopentyl, neopentyl, hexyl, isohexyl, cyclohexyl, 3-methylpentyl, 2,2-dimethylbutyl, and 2,3-dimethylbutyl.

The term lower alkyl, as used herein, and unless otherwise specified, refers to a $C_1$ to $C_6$ saturated straight, branched, or cyclic (in the case of $C_{5-6}$) hydrocarbon, and specifically includes methyl, ethyl, propyl, isopropyl, butyl, isobutyl, t-butyl, pentyl, cyclopentyl, isopentyl, neopentyl, hexyl, isohexyl, cyclohexyl, 3-methylpentyl, 2,2-dimethylbutyl, and 2,3-dimethylbutyl.

The term alkenyl, as referred to herein, and unless otherwise specified, refers to a straight, branched, or cyclic (in the case of $C_{5-6}$) hydrocarbon of $C_2$ to $C_{10}$ with at least one double bond.

The term lower alkenyl, as referred to herein, and unless otherwise specified, refers to an alkenyl group of $C_2$ to $C_6$, and specifically includes vinyl and allyl.

The term lower alkylamino refers to an amino group that has one or two lower alkyl substituents.

The term alkynyl, as referred to herein, and unless otherwise specified, refers to a $C_2$ to $C_{10}$ straight or branched hydrocarbon with at least one triple bond.

The term lower alkynyl, as referred to herein, and unless otherwise specified, refers to a $C_2$ to $C_6$ alkynyl group, specifically including acetylenyl and propynyl.

The term aryl, as used herein, and unless otherwise specified, refers to phenyl or substituted phenyl, wherein the substituent is halo or lower alkyl.

The term halo, as used herein, includes fluoro, chloro, bromo, and iodo.

The term halo (alkyl, alkenyl, or alkynyl) refers to a (alkyl, alkenyl, or alkynyl) group in which at least one of the hydrogens in the group has been replaced with a halogen atom.

The term heterocycle or heteroaromatic, as used herein, refers to an aromatic moiety that includes at least one sulfur, oxygen, or nitrogen in the aromatic ring. Non-limiting examples are pyrryl, furyl, pyridyl, 1,2,4-thiadiazolyl, pyrimidyl, thienyl, isothiazolyl, imidazolyl, tetrazolyl, pyrazinyl, pyrimidyl, quinolyl, isoquinolyl, benzothienyl, isobenzofuryl, pyrazolyl, indolyl, purinyl. carbazolyl, benzimidazolyl, and isoxazolyl.

The term aralkyl refers to an aryl group with an alkyl substituent.

The term alkaryl refers to an alkyl group that has an aryl substituent.

The term organic or inorganic anion refers to an organic or inorganic moiety that carries a negative charge and can be used as the negative portion of a salt.

The term "pharmaceutically acceptable cation" refers to an organic or inorganic moiety that cries a positive charge and that can be administered in association with a pharmaceutical agent, for example, as a countercation in a salt. Pharmaceutically acceptable cations are known to those of skill in the art, and include but are not limited to sodium, potassium, and quaternary amine.

The term "metabolically cleavable leaving group" refers to a moiety that can be cleaved in vivo from the molecule to which it is attached, and includes but is not limited to an organic or inorganic anion, a pharmaceutically acceptable cation, acyl (for example (alkyl)C(O), including acetyl, propionyl, and butyryl), alkyl, phosphate, sulfate and sulfonate.

The term "enantiomerically enriched composition or compound" refers to a composition or compound that includes at least 95% by weight of a single enantiomer of the compound.

The term PAF receptor antagonist refers to a compound that binds to a PAF receptor with a binding constant of 30 μM or lower.

The term 5-lipoxygenase inhibitor refers to a compound that inhibits the enzyme at 30 μM or lower in a broken cell system.

The term pharmaceutically active derivative refers to any compound that upon administration to the recipient, is capable of providing directly or indirectly, the compounds disclosed herein.

Compounds as defined in the accompanying claims exhibit PAF receptor antagonist activity or inhibit the enzyme 5-lipoxygenase, or have dual activity, and are thus useful in the treatment of humans who have immune and allergic disorders that are mediated by PAF or products of 5-lipoxygenase.

*Cis* and *Trans* Isomers of the Following Compounds

**N-Alkyl/arylhydroxyureas:**

2-[5-(N'-Butyl-N'-hydroxyureidyl)-3-methoxy-4-methoxyethoxyphenyl]-5-(3,4,5-trimethoxyphenyl)-tetrahydrothiophene.

2-[5-(N'-Butyl-N'-hydroxyureidyl)-3-methoxy-4-methoxythioethoxyphenyl]-5-(3,4,5-trimethoxyphenyl)-tetrahydrofuran.

2-[5-(N'-Butyl-N'-hydroxyureidyl)-4-hydroxyphenylthioethoxy-3-methoxyphenyl]-5-(3,4,5-trimethoxyphenyl)-tetrahydrofuran.

2-[5-(N'-Butyl-N'-hydroxyureidyl)-4-(N-nicotinoyl-N-phenylaminoethoxy)-3-methoxyphenyl]-5-(3,4,5-trimethoxyphenyl)-tetrahydrofuran.

2-[5-(N'-Butyl-N'-hydroxyureidyl)-4-(N-3-pyridiniumcarbonyl-N-phenylaminoethoxy)-3-methoxyphenyl]-5-(3,4,5-trimethoxyphenyl)-tetrahydrofuran-propyl iodide.

2-[5-(N'-p-Chlorophenyl-N'-hydroxyureidyl)-4-(N-3-pyridiniumcarbonyl-N-phenylaminoethoxy)-3-methoxyphenyl]-5-(3,4,5-trimethoxyphenyl)-tetrahydrofuran-propyl iodide.

2-[5-(N'-Butyl-N'-hydroxyureidyl)-4-(N-3-pyridiniumcarbonyl-N-phenylaminoethoxy)-3-methoxyphenyl]-5-(3,4,5-trimethoxyphenyl)-tetrahydrofuran-ethyl iodide.

2-[5-(N'-p-Chlorophenyl-N'-hydroxyureidyl)-4-(N-3-pyridiniumcarbonyl-N-phenyl aminoethoxy)-3-methoxyphenyl]-5-(3,4,5-trimethoxyphenyl)-tetrahydrofuran-ethyl iodide.

2-[5-(N'-p-Chlorophenyl-N'-hydroxyureidyl)-3-methoxy-4-propoxyphenyl)-5-(3,4,5-trimethoxyphenyl)-tetrahydrofuran.

2-[5-(N'-p-Chlorophenyl-N'-hydroxyureidyl)-4-methoxyethoxy-3-methoxyphenyl]-5-(3,4,5-trimethoxyphenyl)-tetrahydrothiophene.

2-[5-(N'-p-Chlorophenyl-N'-hydroxyureidyl)-4-methylthioethoxy-3-methoxyphenyl]-5-(3,4,5-trimethoxyphenyl)-tetrahydrofuran.

2-[5-(N'-p-Chlorophenyl-N'-hydroxyureidyl)-4-p-hydroxyphenylthioethoxy-3-methoxyphenyl]-5-(3,4,5-trimethoxyphenyl)-tetrahydrofuran.

2-[5-(N'-p-Chlorophenyl-N'-hydroxyureidyl)-4-(N-nicotinoyl-N-phenylaminoethoxy)-3-methoxyphenyl]-5-(3,4,5-trimethoxyphenyl)-tetrahydrofuran.

2-[5-(N'-Butyl-N'-hydroxyureidyl)-4-p-cyanophenylthioethoxy-3-methoxyphenyl]-5-(3,4,5-trimethoxyphenyl)-tetrahydrofuran.

2-[(5-(N'-p-Chlorophenyl-N'-hydroxyureidyl)-4-p-cyanophenylthioethoxy-3-methoxyphenyl]-5-(3,4,5-trimethoxyphenyl)-tetrahydrofuran.

2-[5-(N'-Butyl-N'-hydroxyureidyl)-4-p-methoxyphenylthioethoxy-3-methoxyphenyl]-5-(3,4,5-trimethoxyphenyl)-tetrahydrofuran.

2-[5-(N'-p-Chlorophenyl-N'-hydroxyureidyl)-4-p-methoxyphenylthioethoxy-3-methoxyphenyl]-5-(3,4,5-trimethoxyphenyl)-tetrahydrofuran.

2-[5-(N'-*tert*-Butyl-N'-hydroxyureidyl)-3-methoxy-4-propoxyphenyl]-5-(3, 4,5-trimethoxy phenyl)-tetrahydrothiophene.

2-[5-(N'-n-Butyl-N'-hydroxyureidyl)-3-methoxy-4-propoxyphenyl]-5-(3,4, 5-trimethoxy phenyl)-tetrahydrofuran.

2-[5-(N'-Ethyl-N'-hydroxyureidyl)-3-methoxy-4-propoxyphenyl]-5-(3,4,5-trimethoxy phenyl)-tetrahydrofuran.

2-[5-(N'-Cyclohexyl-N'-hydroxyureidyl)-3-methoxy-4-propoxyphenyl]-5-(3,4,5-trimethoxyphenyl)-tetrahydrofuran.

2-[5-(N'-Benzyl-N'-hydroxyureidyl)-3-methoxy-4-propoxyphenyl]-5-(3,4, 5-trimethoxyphenyl)-tetrahydrofuran.

2-(5-N'-Hydroxyureidyl-3-methoxy-4-propoxyphenyl)-5-(3,4,5-trimethoxyphenyl)-tetrahydrothiophene.

2-[5-(N-Hydroxy-N'-methylureidyl)-3-methoxy-4-propoxyphenyl]-5-(3,4, 5-trimethoxyphenyl)-tetrahydrothiophene.

2-[5-(N'-Hydroxy-N'-i-propylureidyl)-3-methoxy-4-propoxyphenyl]-5-(3, 4,5-trimethoxyphenyl)-tetrahydrothiophene.

2-[5-(N'-*sec*-Butyl-N'-hydroxyureidyl)-3-methoxy-4-propoxyphenyl]-5-(3, 4,5-trimethoxyphenyl)-tetrahydrothiophene.

2-[5-(N'-Hydroxy-N'-propylureidyl)-3-methoxy-4-propoxyphenyl]-5-(3,4, 5-trimethoxyphenyl)-tetrahydrothiophene.

2-[5-(N'-Hydroxy-N'-propylureidyl)-3-methoxy-4-propoxyphenyl]-5-(3,4, 5-trimethoxyphenyl)-tetrahydrofuran.

2-[5-(N'-Hydroxy-N'-n-pentylureidyl)-3-methoxy-4-propoxyphenyl]-5-(3, 4,5-trimethoxyphenyl)-tetrahydrofuran.

2-[5-(N'-Cyclohexyl-N'-hydroxyureidyl)-3-methoxy-4-propoxyphenyl]-5-(3,4,5-trimethoxyphenyl)-tetrahydrofuran.

2-[5-(N'-Hexyl-N'-hydroxyureidyl)-3-methoxy-4-propoxyphenyl]-5-(3,4, 5-trimethoxyphenyl)-tetrahydrofuran.

2-[5-(N'-Benzyl-N'-hydroxyureidyl)-3-methoxy-4-propoxyphenyl]-5-(3,4, 5-trimethoxyphenyl)-tetrahydrofuran.

2-[5-(N'-Hydroxy-N'-octylureidyl)-3-methoxy-4-propoxyphenyl]-5-(3,4,5-trimethoxyphenyl)-tetrahydrofuran.

2-[5-(N'-Hydroxy-N'-methoxyethylureidyl)-3-methoxy-4-propoxyphenyl]-5-(3,4,5-trimethoxyphenyl)-tetrahydrofuran.

2-[5-(N'-Decyl-N'-hydroxyureidyl)-3-methoxy-4-propoxyphenyl]-5-(3,4,5-trimethoxyphenyl)-tetrahydrofuran.

2-[5-(N'-Hydroxy-N'-n-pentylureidyl)-3-methoxy-4-propoxyphenyl]-5-(3, 4,5-trimethoxyphenyl)-tetrahydrothiophene.

2-[5-(N'-Cyclohexyl-N'-hydroxyureidyl)-3-methoxy-4-propoxyphenyl]-5-(3,4,5-trimethoxyphenyl)-tetrahydrothiophene.

2-[5-(N'-Hexyl-N'-hydroxyureidyl)-3-methoxy-4-propoxyphenyl]-5-(3,4, 5-trimethoxyphenyl)-tetrahydrothiophene.

2-[5-(N'-Benzyl-N'-hydroxyureidyl)-3-methoxy-4-propoxyphenyl]-5-(3,4, 5-trimethoxyphenyl)-tetrahydrothiophene.

2-[5-(N'-Hydroxy-N'-octylureidyl)-3-methoxy-4-propoxyphenyl]-5-(3,4,5-trimethoxyphenyl)-tetrahydrothiophene.

2-[5-(N-Hydroxy-N'-methoxyethylureidyl)-3-methoxy-4-propoxyphenyl]-5-(3,4,5-trimethoxyphenyl)-tetrahydrothiophene.

2-[5-(N'-Decyl-N'-hydroxyureidyl)-3-methoxy-4-propoxyphenyl]-5-(3,4,5-trimethoxyphenyl)-tetrahydrothiophene.

2-[5-(N'-Butyl-N'-hydroxyureidyl)-4-(p-bromophenylthioethoxy)-3-methoxyphenyl]-5-(3,4,5-trimethoxyphenyl)-tetrahydrofuran

2-[5-(N'-Butyl-N'-hydroxyureidyl)-4-(2-bromophenylthioethoxy)-3-methoxyphenyl]-5-(3,4,5-trimethoxyphenyl)-tetrahydrofuran

2-[5-(N'-Butyl-N'-hydroxyureidyl)-4-(3-bromophenylthioethoxy)-3-methoxyphenyl]-5-(3,4,5-trimethoxyphenyl)-tetrahydrofuran

2-[5-(N'-Butyl-N'-hydroxyureidyl)-4-(3,4-dichlorophenylthioethoxy)-3-methoxyphenyl]-5-(3,4,5-trimethoxyphenyl)-tetrahydrofuran

2-[5-(N'-Butyl-N'-hydroxyureidyl)-4-(p-chlorophenylthioethoxy)-3-methoxyphenyl]-5-(3,4,5-trimethoxyphenyl)-tetrahydrofuran

2-[5-(N'-Butyl-N'-hydroxyureidyl)-4-(p-fluorophenylthioethoxy)-3-methoxyphenyl]-5-(3,4,5-trimethoxyphenyl)-tetrahydrofuran

2-[5-(N'-Butyl-N'-hydroxyureidyl)-4-(2,3,5,6-tetrafluorophenylthioethoxy)-3-methoxyphenyl]-5-(3,4,5-trimethoxyphenyl)-tetrahydrofuran

2-[5-(N'-Butyl-N'-hydroxyureidyl)-4-(2,3,4,5-tetrafluorophenylethoxy)-3-methoxyphenyl]-5-(3,4,5-trimethoxyphenyl)-tetrahydrofuran

2-[5-(N'-Butyl-N'-hydroxyureidyl)-4-(p-bromophenysulfonylethoxy)-3-methoxyphenyl]-5-(3,4,5-trimethoxyphenyl)-tetrahydrofuran

2-[5-(N'-Butyl-N'-hydroxyureidyl)-4-(2-bromophenylsulfonylethoxy)-3-methoxyphenyl]-5-(3,4,5-trimethoxyphenyl)-tetrahydrofuran

2-[5-(N'-Hydroxy-N'-methylyureidyl)-4-(p-bromophenylthioethoxy)-3-methoxyphenyl]-5-(3,4,5-trimethoxyphenyl)-tetrahydrofuran

2-[5-(N'-Hydroxy-N'-methylureidyl)-4-(2-bromophenylthioethoxy)-3-methoxyphenyl]-5-(3,4,5-trimethoxyphenyl)-tetrahydrofuran

2-[5-(N'-Hydroxy-N'-methylureidyl)-4-(3-bromophenylthioethoxy)-3-methoxyphenyl]-5-(3,4,5-trimethoxyphenyl)-tetrahydrofuran

2-[5-(N'-Hydroxy-N'-methylureidyl)-4-(3,4-dichlorophenylthioethoxy)-3-methoxyphenyl]-5-(3,4,5-trimethoxyphenyl)-tetrahydrofuran

2-[5-(N'-Hydroxy-N'-methylureidyl)-4-(p-chlorophenylthioethoxy)-3-methoxyphenyl]-5-(3,4,5-trimethoxyphenyl)-tetrahydrofuran

2-[5-(N'-Ethyl-N'-hydroxyureidyl)-4-p-fluorophenylthioethoxy)-3-methoxyphenyl]-5-(3,4,5-trimethoxyphenyl)-tetrahydrofuran

2-[5-(N'-Ethyl-N'-hydroxyureidyl)-4-(2,3,5,6-tetrafluorophenylthioethoxy)-3-methoxyphenyl]-5-(3,4,5-trimethoxyphenyl)-tetrahydrofuran

2-[5-(N'-Ethyl-N'-hydroxyureidyl)-4-(2,3,4,5-tetrafluorophenylethoxy)-3-methoxyphenyl]-5-(3,4,5-trimethoxyphenyl)-tetrahydrofuran

2-[5-(N'-Ethyl-N'-hydroxyureidyl)-4-(2,3,4,5-tetrafluorophenylethoxy)-3-methoxyphenyl]-5-(3,4,5-trimethoxyphenyl)-tetrahydrofuran

2-[5-(N'-p-Chlorophenyl-N'-hydroxyureidyl)-4-(2-bromophenylsulfonylethoxy)-3-methoxyphenyl]-5-(3,4,5-trimethoxyphenyl)-tetrahydrofuran

2-[5-(N'-p-Chlorophenyl-N'-hydroxyureidyl)-4-(p-bromophenylthioethoxy)-3-methoxyphenyl]-5-(3,4,5-trimethoxyphenyl)-tetrahydrofuran

2-[5-(N'-*tert*-Butyl-N'-hydroxyureidyl)-4-(p-bromophenylthioethoxy)-3-methoxyphenyl]-5-(3,4,5-trimethoxyphenyl)-tetrahydrofuran

2-[5-(N'-*tert*-Butyl-N'-hydroxyureidyl)-4-(2-bromophenylthioethoxy)-3-methoxyphenyl]-5-(3,4,5-trimethoxyphenyl)-tetrahydrofuran

2-[5-(N'-*tert*-Butyl-N'-hydroxyureidyl)-4-(3-bromophenylthioethoxy)-3-methoxyphenyl]-5-(3,4,5-trimethoxyphenyl)-tetrahydrofuran

2-[5-(N'-Cyclohexyl-N'-hydroxyureidyl)-4-(3,4-dichlorophenylthioethoxy)-3-methoxyphenyl]-5-(3,4,5-trimethoxyphenyl)-tetrahydrofuran

2-[5-(N'-Cyclohexyl-N'-hydroxyureidyl)-4-(p-chlorophenylthioethoxy)-3-methoxyphenyl]-5-(3,4,5-trimethoxyphenyl)-tetrahydrofuran

2-[5-(N'-Cyclohexyl-N'-hydroxyureidyl)-4-(p-fluorophenylthioethoxy)-3-methoxyphenyl]-5-(3,4,5-trimethoxyphenyl)-tetrahydrofuran

2-[5-(N'-Benzyl-N'-hydroxyureidyl)-4-(2,3,5,6-tetrafluorophenylthioethoxy)-3-methoxyphenyl]-5-(3,4,5-trimethoxyphenyl)-tetrahydrofuran

2-[5-(N'-Benzyl-N'-hydroxyureidyl)-4-(2,3,4,5-tetrafluorophenylethoxy)-3-methoxyphenyl]-5-(3,4,5-trimethoxyphenyl)-tetrahydrofuran

2-[5-(N'-Benzyl-N'-hydroxyureidyl)-4-(p-bromophenylsulfonylethoxy)-3-methoxyphenyl]-5-(3,4,5-trimethoxyphenyl)-tetrahydrofuran

2-[5-(N'-Benzyl-N'-hydroxyureidyl)-4-(2-bromophenylsulfonylethoxy)-3-methoxyphenyl]-5-(3,4,5-trimethoxyphenyl)-tetrahydrofuran

2-[5-(N'-Hydroxy-N'-*i*-propylureidyl)-4-(p-bromophenylthioethoxy)-3-methoxyphenyl]-5-(3,4,5-trimethoxyphenyl)-tetrahydrofuran

2-[5-(N'-*sec*-Butyl-N'-hydroxyureidyl)-4-(p-bromophenylthioethoxy)-3-methoxyphenyl]-5-(3,4,5-trimethoxyphenyl)-tetrahydrofuran

2-[5-(N'-*sec*-Butyl-N'-hydroxyureidyl)-4-(2-bromophenylthioethoxy)-3-methoxyphenyl]-5-(3,4,5-trimethoxyphenyl)-tetrahydrofuran

2-[5-(N'-Hydroxy-N'-propylureidyl)-4-(3-bromophenylthioethoxy)-3-methoxyphenyl]-5-(3,4,5-trimethoxyphenyl)-tetrahydrofuran

2-[5-(N'-Hydroxy-N'-*n*-pentylureidyl)-4-(3,4-dichlorophenylthioethoxy)-3-methoxyphenyl]-5-(3,4,5-trimethoxyphenyl)-tetrahydrofuran

2-[5-(N'-Hexyl-N'-hydroxyureidyl)-4-(p-chlorophenylthioethoxy)-3-methoxyphenyl]-5-(3,4,5-trimethoxyphenyl)-tetrahydrofuran

2-[5-(N'-Hydroxy-N'-octylureidyl)-4-(p-fluorophenylthioethoxy)-3-methoxyphenyl]-5-(3,4,5-trimethoxyphenyl)-tetrahydrofuran

2-[5-(N'-Hydroxy-methoxyethylureidyl)-4-(2,3,5,6-tetrafluorophenylthioethoxy)-3-methoxyphenyl]-5-(3,4,5-trimethoxyphenyl)-tetrahydrofuran

2-[5-(N'-Decyl-N'-hydroxyureidyl)-4-(2,3,4,5-tetrafluorophenylethoxy)-3-methoxyphenyl]-5-(3,4,5-trimethoxyphenyl)-tetrahydrofuran

2-[5-(N'-Hydroxy-N'-methylureidylmethyl)-4-(p-chlorophenylthioethoxy)-3-methoxyphenyl]-5-(3,4,5-trimethoxyphenyl)-tetrahydrofuran

2-[5-(N'-Hydroxy-N'-*i*-propylureidylmethyl)-4-(p-chlorophenylthioethoxy)-3-methoxyphenyl]-5-(3,4,5-trimethoxyphenyl)-tetrahydrofuran

2-[5-(N'-Butyl-N'-hydroxyureidylmethy-4-(p-chlorophenylthioethoxy)-3-methoxyphenyl]-5-(3,4,5-trimethoxyphenyl)-tetrahydrofuran

2-[5-(N'-Hydroxy-N'-propylureidylmethyl)-4-(p-chlorophenylthioethoxy)-3-methoxyphenyl]-5-(3,4,5-trimethoxyphenyl)-tetrahydrofuran

2-[5-(N'-Ethyl-N'-hydroxyureidylmethyl)-4-(p-chlorophenylthioethoxy)-3-methoxyphenyl]-5-(3,4,5-trimethoxyphenyl)-tetrahydrofuran

2-[5-(N'-Hydroxy-N'-octylureidylmethyl)-4-(p-chlorophenylthioethoxy)-3-methoxyphenyl]-5-(3,4,5-trimethoxyphenyl)-tetrahydrofuran

2-[5-(N'-Benzyl-N'-hydroxyureidyl)-4-(p-bromophenylsulfonylethoxy)-3-methoxyphenyl]-5-(3,4,5-trimethoxyphenyl)-tetrahydrothiophene

2-[5-(N'-Benzyl-N'-hydroxyureidyl)-4-(2-bromophenylsulfonylethoxy)-3-methoxyphenyl]-5-(3,4,5-trimethoxyphenyl)-tetrahydrothiophene

2-[5-(N'-Hydroxy-N'-*i*-propylureidyl)-4-(p-bromophenylthioethoxy)-3-methoxyphenyl]-5-(3,4,5-trimethoxyphenyl)-tetrahydrothiophene

2-[5-(N'-Hydroxyl-N'-octylureidyl)-4-(p-fluorophenylthioethoxy)-3-methoxyphenyl]-5-(3,4,5-trimethoxyphenyl)-tetrahydrothiophene

2-[5-(N'-Butyl-N'-hydroxyureidyl)-4-(p-bromophenylthioethoxy)-3-methoxyphenyl]-5-(3,4,5-trimethoxyphenyl)-tetrahydrothiophene

2-[5-(N'-Butyl-N'-hydroxyureidyl)-4-(2-bromophenylthioethoxy)-3-methoxyphenyl]-5-(3,4,5-trimethoxyphenyl)-tetrahydrothiophene

2-[5-(N'-Butyl-N'-hydroxyureidyl)-4-(3-bromophenylthioethoxy)-3-methoxyphenyl]-5-(3,4,5-trimethoxyphenyl)-tetrahydrothiophene

2-[5-(N'-Hydroxy-N-methylureidylmethyl)-4-(p-chlorophenylthioethoxy)-3-methoxyphenyl]-5-(3,4,5-trimethoxyphenyl)-tetrahydrothiophene

2-[5-(N'-Hydroxy-N'-*i*-propylureidylmethyl)-4-(p-chlorophenylthioethoxy)-3-methoxyphenyl]-5-(3,4,5-trimethoxyphenyl)-tetrahydrothiophene

2-[5-(N'-Butyl-N'-hydroxyureidylmethyl)-4-(p-chlorophenylthioethoxy)-3-methoxyphenyl]-5-(3,4,5-trimethoxyphenyl)-tetrahydrothiophene

**Triple Bonded Hydroxamates:**

2-[5-[1 (N-Acetyl-N-hydroxyamino)propyn-3-yl]-3-methoxy-4-propoxyphenyl]-5-(3,4,5-trimethoxyphenyl)-tetrahydrofuran.

2-[5-[1-(N-Hydroxy-N-propanoylamino)propyn-3-yl]-3-methoxy-4-propoxyphenyl]-5-(3,4,5-trimethoxyphenyl)-tetrahydrofuran.

2-[5-[1-(N-Butanoyl-N-hydroxyamino)propyn-3-yl]-3-methoxy-4-propoxyphenyl]-5-(3,4,5-trimethoxyphenyl)-tetrahydrofuran.

2-[5-[1-(N-Hydroxy-N-i-propanoylamino)propyn-3-yl] -3-methoxy-4-propoxyphenyl]-5-(3,4,5-trimethoxyphenyl)-tetrahydrofuran.

2-[5-[1-(N-Hydroxy-N-cyclohexanecarbonylamino)propyn-3-yl)-3-methoxy-4-propoxyphenyl]-5-(3,4,5-trimethoxyphenyl)-tetrahydrofuran.

2-[5-[1-(N-Benzoyl-N-hydroxylamino)propyn-3-yl]-3-methoxy-4-propoxyphenyl]-5-(3,4,5-trimethoxyphenyl)-tetrahydrofuran.

2-[5-[1-(N-Hydroxy-N-3-phenoxybenzoylamino)propyn-3-yl]-3-methoxy-4-propoxyphenyl]-5-(3,4,5-trimethoxyphenyl)-tetrahydrofuran.

2-[5-[1-(N-Hydroxy-N-4-methoxybenzoylamnino)propyn-3-yl]-3-methoxy-4-propoxyphenyl]-5-(3,4,5-trimethoxyphenyl)-tetrahydrofuran.

2-[5-[1-(N-3-Benzoylbenzoyl-N-hydroxyamino)propyn-3-yl]-3-methoxy-4-propoxyphenyl]-5-(3,4,5-trimethoxyphenyl)-tetrahydrofuran.

2-[5-[1-(N-Hydroxy-N-4-hydroxybenzoylamino)propyn-3-yl]-3-methoxy-4-propoxyphenyl]-5-(3,4,5-trimethoxypheny)-tetrahydrofuran.

2-[5-[1-(N-Acetyl-N-hydroxyamino)propyn-3-yl)-4-(p-bromophenylthioethoxy)-3-methoxyphenyl]-5-(3,4,5-trimethoxyphenyl)-tetrahydrofuran

2-[5-[1-(N-Hydroxy-N-propanoylamino)propyn-3-yl]-4-(p-chlorophenylthioethoxy)-3-methoxyphenyl]-5-(3,4,5-trimethoxyphenyl)-tetrahydrofuran

2-[5-[1-(N-Butanoyl-N-hydroxyamino)propyn-3-yl)-4-(3,4-dichlorophenylthioethoxy)-3-methoxyphenyl]-5-(3,4,5-trimethoxyphenyl)-tetrahydrofuran

2-[5-[1-(N-Hydroxy-N-cyclohexanecarbonylamino)propyn-3-yl]-4-(p-fluorophenylthioethoxy)-3-methoxyphenyl]-5-(3,4,5-trimethoxyphenyl)-tetrahydrofuran

2-[5-[1-(N-Hydroxy-N-3-phenoxybenzoylamino)propyn-3-yl]-4-(2,3,5,6-tetrafluorophenythioethoxy)-3-methoxyphenyl]-5-(3,4,5-trimethoxyphenyl)-tetrahydrofuran

2-[5-[1-(N-Hydroxy-N-methoxybenzoylamino)propyn-3-yl]-4-(2-bromophenylthioethoxy)-3-methoxyphenyl]-5-(3,4,5-trimethoxyphenyl)-tetrahydrofuran

2-[5-[1-(N-Hydroxy-N-hydroxybenzoylamino)propyn-3-yl]-4-(p-chlorophenylthioethoxy)-3-methoxyphenyl]-5-(3,4,5-trimethoxyphenyl)-tetrahydrofuran

**Triple Bonded Ureas:**

2-[5-[1-(N'-Hydroxy-N-methylureidyl)propyn-3-yl]-3-methoxy-4-propoxyphenyl]-5-(3,4,5-trimethoxyphenyl)-tetrahydrofuran.

2-[5-[1-(N'-Ethyl-N'-hydroxyureidyl)propyn-3-yl]-3-methoxy-4-propoxyphenyl]-5-(3,4,5-trimethoxyphenyl)-tetrahydrofuran.

2-[5-[1-(N'-Hydroxy-N'-propylureidyl)propyn-3-yl)-3-methoxy-4-propoxyphenyl]-5-(3,4,5-trimethoxyphenyl)-tetrahydrofuran.

2-[5-[1(N'-n-Butyl-N'-hydroxyureidyl)propyn-3-yl]-3-methoxy-4-propoxyphenyl]-5-(3,4,5-trimethoxyphenyl)-tetrahydrofuran.

2-[5-[1-(N'-Hydroxy-N'-*i*-propylureidyl)propyn-3-yl]-3-methoxy-4-propoxyphenyl]-5-(3,4,5-trimethoxyphenyl)-tetrahydrofuran.

2-[5-[1-(N'-t-Butyl-N'-hydroxyureidyl)propyn-3-yl]-3-methoxy-4-propoxyphenyl)-5-(3,4,5-trimethoxyphenyl)-tetrahydrofuran.

2-[5-[1-(N'-Benzyl-N'-hydroxyureidyl)propyn-3-yl]-3-methoxy-4-propoxyphenyl]-5-(3,4,5-trimethoxyphenyl)-tetrahydrofuran.

2-[5-[1-(N'-Cyclopropylmethyl-N'-hydroxyureidyl)propyn-3-yl)-3-methoxy-4-propoxyphenyl]-5-(3,4,5-trimethoxyphenyl)-tetrahydrofuran.

2-[5-[1-(N'-Allyl-N'-hydroxyureidyl)propyn-3-yl]-3-methoxy-4-propoxyphenyl]-5-(3,4,5-trimethoxyphenyl)-tetrahydrofuran.

2-[5-[1-(N'-Hydroxy-N'-hydroxyethylureidyl)propyn-3-yl]-3-methoxy-4-propoxyphenyl]-5-(3,4,5-trimethoxyphenyl)-tetrahydrofuran.

2-[5-[1-(N'-Hydroxy-N'-methylureidyl)propyn-3-yl]-4-(p-chlorophenylthioethoxy)-3-methoxyphenyl]-5-(3,4,5-trimethoxyphenyl)-tetrahydrofuran.

2-[5-[1-(N'-Ethyl-N'-hydroxyureidyl)propyn-3-yl)-4-(p-chlorophenylthioethoxy)-3-methoxyphenyl]-5-(3,4,5-trimethoxyphenyl)-tetrahydrofuran.

2-[5-[1-(N'-Hydroxy-N'-propylureidyl)propyn-3-yl]-4-(p-chlorophenylthioethoxy)-3-methoxyphenyl]-5-(3,4,5-trimethoxyphenyl)-tetrahydrofuran.

2-[5-[1-(N'-*n*-Butyl-N'-hydroxyureidyl)propyn-3-yl]-4-(p-chlorophenylthioethoxy)-3-methoxyphenyl]-5-(3,4,5-trimethoxyphenyl)-tetrahydrofuran

2-[5-[1-(N'-Hydroxy-N'-*i*-propylureidyl)propyn-3-yl]-4-(p-chlorophenylthioethoxy)-3-methoxyphenyl]-5-(3,4,5-trimethoxyphenyl)-tetrahydrofuran

2-[5-[1-(N'-*tert*-Butyl-N'-hydroxyureidyl)propyn-3-yl]-4-(p-chlorophenylthioethoxy)-3-methoxyphenyl]-5-(3,4,5-trimethoxyphenyl)-tetrahydrofuran

2-[5-[1-(N'-Benzyl-N'-hydroxyureidyl)propyn-3-yl]-4-(p-chlorophenylthioethoxy)-3-methoxyphenyl]-5-(3,4,5-trimethoxyphenyl)-tetrahydrofuran

2-[5-[1-(N'-Cyclopropylmethyl-N'-hydroxyureidyl)propyn-3-yl]-4-(p-chlorophenylthioethoxy)-3-methoxyphenyl]-5-(3,4,5-trimethoxyphenyl)-tetrahydrofuran

2-[5-[1-(N'-Allyl-N'-hydroxyureidyl)propyn-3-yl]-4-(p-chlorophenylthioethoxy)-3-methoxyphenyl]-5-(3,4,5-trimethoxyphenyl)-tetrahydrofuran

2-[5-[1-(N'-Hydroxy-N'-hydroxyethylureidyl)propyn-3-yl]-4-(p-chlorophenylthioethoxy)-3-methoxyphenyl]-5-(3,4,5-trimethoxyphenyl)-tetrahydrofuran

**Double Bonded Hydroxamates: Both *Cis* and *Trans* Isomers at the Tetrahydrofuran Ring**

2-[5-[*trans*-1-(N-Acetyl-N-hydroxyamino)propen-3-yl]-3-methoxy-4-propoxyphenyl]-5-(3,4,5-trimethoxyphenyl)-tetrahydrofuran.

2-[5-[*trans*-1-(N-Hydroxy-N-propanoylamino)propen-3-yl]-3-methoxy-4-propoxyphenyl]-5-(3,4,5-trimethoxyphenyl)-tetrahydrofuran.

2-[5-[*trans*-1-(N-Butanoyl-N-hydroxyamino)propen-3-yl]-3-methoxy-4-propoxyphenyl]-5-(3,4,5-trimethoxyphenyl)-tetrahydrofuran.

2-[5-[*trans*-1-(N-hydroxy-N-nicotinoylamino)propen-3-yl]-3-methoxy-4-propoxyphenyl]-5-(3,4,5-trimethoxyphenyl)-tetrahydrofuran.

2-[5-[*trans*-1-(N-Hydroxy-N-phenylacetylamino)propen-3-yl)-3-methoxy-4-propoxyphenyl]-5-(3,4,5-trimethoxyphenyl)-tetrahydrofuran.

2-[5-[*trans*-1-(N-3-Phenoxybenzoyl-N-hydroxyamino)propen-3-yl]-3-methoxy-4-propoxyphenyl]-5-(3,4,5-trimethoxyphenyl)-tetrahydrofuran.

2-[5-[*trans*-1-(N-3-Chlorobenzoyl-N-hydroxyamino)propen-3-yl]-3-methoxy-4-propoxyphenyl]-5-(3,4,5-trimethoxyphenyl)-tetrahydrofuran.

2-[5-[*trans*-1-(N-3-Chlorobenzoyl-N-hydroxyamino)propen-3-yl]-3-methoxy-4-propoxyphenyl]-5-(3,4,5-trimethoxyphenyl)-tetrahydrofuran.

2-[5-[*trans*-1-(N-2,4-Difluorobenzoyl-N-hydroxyamino)propen-3-yl]-3-methoxy-4-propoxyphenyl]-5-(3,4,5-trimethoxyphenyl)-tetrahydrofuran.

2-[5-[*trans*-1-(N-3,4-Methylenedioxybenzoyl-N-hydroxyamino)propen-3-yl]-3-methoxy-4-propoxyphenyl]-5-(3,4,5-trimethoxyphenyl)-tetrahydrofuran.

and also the corresponding saturated hydroxamates, e.g.,
2-[5-[1-(N-3,4-Methylenedioxybenzoyl-N-hydroxyamino)propyl]-3-methoxy-4-propoxyphenyl]-5-(3,4,5-trimethoxyphenyl)-tetrahydrofuran.

2-[5-*trans*-1-(N-Acetyl-N-hydroxyamino)propen-3-yl]-4-(p-bromophenylthioethoxy)-3-methoxyphenyl]-5-(3,4,5-trimethoxyphenyl)-tetrahydrofuran

2-[5-[*trans*-1-(N-Hydroxy-N-propanoylamino)propen-3-yl]-4-(p-chlorophenylthioethoxy)-3-methoxyphenyl]-5-(3,4,-trimethoxyphenyl)-tetrahydrofuran

2-[5-[*trans*-1-(N-Butanoyl-N-hydroxyamino)propen-3-yl]-4-(3,4-dichlorophenylthioethoxy)-3-methoxyphenyl]-5-(3,4,5-trimethoxyphenyl)-tetrahydrofuran

2-[5-[*trans*-1-(N-Hydroxy-N-cyclohexanecarbonylamino)propen-3-yl]-4-(p-fluorophenylthioethoxy)-3-methoxyphenyl]-5-(3,4,5-trimethoxyphenyl)-tetrahydrofuran

2-[5-[*trans*-1-(N-Hydroxy-N-phenoxybenzoylamino)propen-3-yl]-4-(2,3,5,6-tetrafluorophenylthioethoxy)-3-methoxyphenyl]-5-(3,4,5-trimethoxyphenyl)-tetrahydrofuran

2-[5-[*trans*-1-(N-Hydroxy-N-methoxybenzoylamino)propen-3-yl]-4-(p-bromophenylthioethoxy)-3-methoxyphenyl]-5-(3,4,5-trimethoxyphenyl)-tetrahydrofuran

2-[5-[*trans*-1-(N-Hydroxy-N-hydroxybenzoylamino)propen-3-yl]-4-(p-chlorophenylthioethoxy)-3-methoxyphenyl]-5-(3,4,5-trimethoxyphenyl)-tetrahydrofuran

**Double Bonded Ureas: Both *Cis* and *Trans* Isomers at the Tetrahydrofuran Ring.**

2-[5-[*trans*-1-(N'-Hydroxy-N'-methylureidyl)propen-3-yl]-3-methoxy-4-propoxyphenyl]-5-(3,4,5-trimethoxyphenyl)-tetahydrofuran.

2-[5-[*trans*-1-(N'-Ethyl-N'-hydroxyureidyl)propen-3-yl]-3-methoxy-4-propoxyphenyl]-5-(3,4,5-trimethoxyphenyl)-tetrahydrofuran.

2-[5-[*trans*-1-(N'-Hydroxy-N'-propylureidyl)propen-3-yl]-3-methoxy-4-propoxyphenyl]-5-(3,4,5-trimethoxyphenyl)-tetrahydrofuran.

2-[5-(*trans*-1-(N'-Butyl-N'-hydroxyureidyl)propen-3-yl]-3-methoxy-4-propoxyphenyl]-5-(3,4,5-trimethoxyphenyl)-tetrahydrofuran.

2-[5-[*trans*-1-(N'-Hydroxy-N'-i-propylureidyl)propen-3-yl)-3-methoxy-4-propoxyphenyl]-5-(3,4,5-trimethoxyphenyl)-tetrahydrofuran.

2-[5-[*trans*-1-(N'-t-Butyl-N'-hydroxyureidyl)propen-3-yl]-3-methoxy-4-propoxyphenyl]-5-(3,4,5-trimethoxyphenyl)-tetrahydrofuran.

2-[5-[*trans*-1-(N'-Benzyl-N'-hydroxyureidyl)propen-3-yl]-N-3-methoxy-4-propoxyphenyl]-5-(3,4,5-trimethoxyphenyl)-terahydrofuran.

2-[5-[trans-1-(N'-Allyl-N'-hydroxyureidyl)propen-3-yl]-3-methoxy-4-propoxyphenyl]-5-(3,4,5-trimethoxyphenyl)-tetrahydrofuran.

2-[5-[*trans*-1-(N'-Cyclohexyl-N'-hydroxyureidyl)propen-3-yl]-3-methoxy-4-propoxyphenyl-5-(3,4,5-trimethoxyphenyl)-tetrahydrofuran.

2-[5-[*trans*-1-(N'-Hydroxy-N'-methylthioethylureidyl)propen-3-yl]-3-methoxy-4-propoxyphenyl]-5-(3,4,5-trimethoxyphenyl)-tetrahydrofuran.

2-[5-[*trans*-1-(N'-Cyclohexyl-N'-hydroxyureidyl)propen-3-yl]-3-methoxy-4-propoxy]-5-(3,4,5-trimethoxyphenyl)-tetrahydrofuran.

and also the corresponding saturated ureas, e.g.,
2-[5-[1(N'-Cyclohexyl-N'-hydroxyureidyl)propyl]-3-methoxy-4-propoxyphenyl]-5-(3,4,5-trimethoxyphenyl)-tetrahydrofuran.

2-[5-[*trans*-1-(N'-Hydroxy-N'-methylureidyl)propen-3-yl]-4-(p-chlorophenylthioethoxy)-3-methoxyphenyl]-5-(3,4,5-trimethoxyphenyl)-tetrahydrofuran

2-[5-[*trans*-1-(N'-Ethyl-N'-hydroxyureidyl)propen-3-yl]-4-(p-chlorophenylthioethoxy)-3-methoxyphenyl]-5-(3,4,5-trimethoxyphenyl)-tetrahydrofuran

2-[5-[*trans*-1-(N'-Hydroxy-N'-propylureidyl)propen-3-yl]-4-(p-chlorophenylthioethoxy)-3-methoxyphenyl]-5-(3,4,5-trimethoxyphenyl)-terahydrofuran

2-[5-[*trans*-1-(N'-*n*-Butyl-N'-hydroxyureidyl)propen-3-yl]-4-(p-chlorophenylthioethoxy)-3-methoxyphenyl]-5-(3,4,5-trimethoxyphenyl)-tetrahydrofuran

2-[5-[*trans*-1-(N'-Hydroxy-N'-*i*-propylureidyl)propen-3-yl]-4-(p-chlorophenylthioethoxy)-3-methoxyphenyl]-5-(3,4,5-trimethoxyphenyl)-tetrahydrofuran

2-[5-[*trans*-1-(N'-*tert*-Butyl-N'-hydroxyureidyl)propen-3-yl]-4-(p-chlorophenylthioethoxy)-3-methoxyphenyl]-5-(3,4,5-trimethoxyphenyl)-tetrahydrofuran

2-[5-[*trans*-1-(N'-Benzyl-N'-hydroxyureidyl)propen-3-yl]-4-(p-chlorophenylthioethoxy)-3-methoxyphenyl]-5-(3,4,5-trimethoxyphenyl)-tetrahydrofuran

2-[5-[*trans*-1-(N'-Cyclopropylmethyl-N'-hydroxyureidyl)propen-3-yl]-4-(p-chlorophenylthioethoxy)-3-methoxyphenyl]-5-(3,4,5-trimethoxyphenyl)-tetrahydrofuran

2-[5-[*trans*-1-(N'-Allyl-N'-hydroxyureidyl)propen-3-yl]-4-(p-chlorophenylthioethoxy)-3-methoxyphenyl]-5-(3,4,5-trimethoxyphenyl)-tetrahydrofuran

2-[5-[*trans*-1-(N'-Hydroxy-N'-hydroxyethylureidyl)propen-3-yl]-4-(p-chlorophenylthioethoxy)-3-methoxyphenyl]-5-(3,4,5-trimethoxyphenyl)-tetrahydrofuran

*Cis* and *Trans* Isomers of the Following Compounds

**N-Alkyl/arylhydroxyureas:**

4-[5-(N'-Butyl-N'-hydroxyureidyl)-3-methoxy-4-methoxyethoxyphenyl]-2-(3,4,5-trimethoxyphenyl)-tetrahydrofuran
4-[5-(N'-Butyl-N'-hydroxyureidyl)-3-methoxy-4-methylthioethoxyphenyl]-2-(3,4,5-trimethoxyphenyl)-tetrahydrofuran
4-[5-(N'-Butyl-N'-hydroxyureidyl)-4-p-hydroxyphenylthioethoxy-3-methoxyphenyl]-2-(3,4,5-trimethoxyphenyl)-tetrahydrofuran
4-[5-(N'-Butyl-N'-hydroxyureidyl)-3-methoxy-4-(N-nicotinoyl-N-phenyaminoethoxy)-phenyl]-2-(3,4,5-trimethoxyphenyl)-tetrahydrofuran
4-[5-(N'-Butyl-N'-hydroxyureidyl)-3-methoxy-4-[(N-(N-propyl-3-pyridinium carbonyl)]-N-phenyaminoethoxy)-phenyl]-2-(3,4,5-trimethoxyphenyl)-tetrahydrofuran iodide
4-[5-(N'-p-Chlorophenyl-N'-hydroxyureidyl)-3-methoxy-4-(N-(N-propyl-3-pyridiniumcarbonyl)-N-phenyaminoethoxy)-phenyl]-2-(3,4,5-trimethoxyphenyl)-tetrahydrofuran iodide
4-[5-(N'-Butyl-N'-hydroxyureidyl)-3-methoxy-4-(N-(N-ethyl-3-pyridinium carbonyl)-N-phenyaminoethoxy)-phenyl]-2-(3,4,5-trimethoxyphenyl)-tetrahydrofuran iodide
4-[5-(N'-p-Chlorophenyl-N'-hydroxyureidyl)-3-methoxy-4-(N-(N-ethyl-3-pyridinium carbonyl)-N-phenyaminoethoxy)-phenyl]-2-(3,4,5-trimethoxyphenyl)-tetrahydrofuran iodide
4-[5-(N'-p-Chlorophenyl-N'-hydroxyureidyl)-3-methoxy-4-propoxyphenyl]-2-(3,4,5-trimethoxyphenyl)-tetrahydrofuran
4-[5-(N'-p-Chlorophenyl-N'-hydroxyureidyl)-3-methoxy-4-methoxyethoxyphenyl]-2-(3,4,5-trimethoxyphenyl)-tetrahydrofuran
4-[5-(N'-p-Chlorophenyl-N'-p-hydroxyureidyl)-3-methoxy-4-methylthioethoxyphenyl]-2-(3,4,5-trimethoxyphenyl)-tetrahydrofuran
4-[5-(N'-p-Chlorophenyl-N'-hydroxyureidyl)-4-p-hydroxyphenylthioethoxy-3-methoxyphenyl]-2-(3,4,5-trimethoxyphenyl)-tetrahydrofuran
4-[5-(N'-p-Chlorophenyl-N'-hydroxyureidyl)-3-methoxy-4-(N-nicotinoyl-N-phenylaminoethoxy)-phenyl]-2-(3,4,5-trimethoxyphenyl)-tetrahydrofuran

4-[5-(N'-Butyl-N'-hydroxyureidyl)-4-p-cyanophenylthioethoxy-3-methoxyphenyl]-2-(3,4,5-trimethoxyphenyl)-tetrahydrofuran

4-[5-(N'-p-Chlorophenyl-N'-hydroxyureidyl)-4-p-cyanophenylthioethoxy-3-methoxyphenyl]-2-(3,4,5-trimethoxyphenyl)-tetrahydrofuran

4-[5-(N'-Butyl-N'-hydroxyureidyl)-3-methoxy-4-p-methoxyphenylthioethoxyphenyl]-2-(3,4,5-trimethoxyphenyl)-tetrahydrofuran

4-[5-(N'-p-Chlorophenyl-N'-hydroxyureidyl)-3-methoxy-4-p-methoxyphenylthioethoxyphenyl)-2-(3,4,5-trimethoxyphenyl)-tetrahydrofuran

4-[5-(N'-*tert*-Butyl-N'-hydroxyureidyl)-3-methoxy-4-propoxyphenyl]-2-(3, 4,5-trimethoxyphenyl)-tetrahydrothiophene

4-[5-(N'-Butyl-N'-hydroxyureidyl)-3-methoxy-4-propoxyphenyl]-2-(3,4,5-trimethoxyphenyl)-tetrahydrofuran

4-[5-(N'-Ethyl-N'-hydroxyureidyl)-3-methoxy-4-propoxyphenyl]-2-(3,4,5-trimethoxyphenyl)-tetrahydrofuran

4-[5-(N'-Cyclohexyl-N'-hydroxyureidyl)-3-methoxy-4-propoxyphenyl]-2-(3,4,5-trimethoxyphenyl)-terahydrofuran

4-[5-(N'-Benzyl-N'-hydroxyureidyl)-3-methoxy-4-propoxyphenyl]-2-(3,4, 5-trimethoxyphenyl)-tetrahydrofuran

4-[5-N'-Hydroxyureidyl-3-methoxy-4-propoxyphenyl]-2-(3,4,5-trimethoxyphenyl)-tetrahydrothiophene

4-[5-(N'-Hydroxy-N'-methylureidyl)-3-methoxy-4-propoxyphenyl]-2-(3,4, 5-trimethoxyphenyl)-tetrahydrothiophene

4-[5-(N'-Hydroxy-N'-i-propylureidyl)-3-methoxy-4-propoxyphenyl]-2-(3, 4,5-trimethoxyphenyl)-tetrahydrothiophene

4-[5-(N'-*sec*-Butyl-N'-hydroxyureidyl)-3-methoxy-4-propoxyphenyl]-2-(3, 4,5-trimethoxyphenyl)-tetrahydrothiophene

4-[5-(N'-Hydroxy-N'-n-propylureidyl)-3-methoxy-4-propoxyphenyl]-2-(3, 4,5-trimethoxyphenyl)-tetrahydrothiophene

4-[5-(N'-Hydroxy-N'-n-propylureidyl)-3-methoxy-4-propoxyphenyl]-2-(3, 4,5-trimethoxyphenyl)-tetrahydrofuran

4-[5-(N-Hydroxy-N'-n-pentylureidyl)-3-methoxy-4-propoxyphenyl]-2-(3, 4,5-trimethoxyphenyl)-tetrahydrofuran

4-[5-(N'-Hydroxy-N'-n-pentylureidyl)-3-methoxy-4-propoxyphenyl]-2-(3, 4,5-trimethoxyphenyl)-tetrahydrofuran

4-[5-(N'-Hydroxy-N'-n-pentylureidyl)-3-methoxy-4-propoxyphenyl]-2-(3, 4,5-trimethoxyphenyl)-tetrahydrofuran

4-[5-(N'-Cyclohexyl-N'-hydroxyureidyl)-3-methoxy-4-propoxyphenyl]-2-(3,4,5-trimethoxyphenyl)-tetrahydro-furan

4-[5-(N'-n-Hexyl-N'-hydroxyureidyl)-3-methoxy-4-propoxyphenyl]-2-(3, 4,5-trimethoxyphenyl)-tetrahydrofuran

4-[5-(N'-Benzyl-N'-hydroxyureidyl)-3-methoxy-4-propoxyphenyl]-2-(3,4, 5-trimethoxyphenyl)-tetrahydrothiophene

4-[5-(N'-Hydroxy-N'-n-octylureidyl)-3-methoxy-4-propoxyphenyl]-2-(3,4, 5-trimethoxyphenyl)-tetrahydrofuran

4-[5-(N'-Hydroxy-N'-methoxyethylureidyl)-3-methoxy-4-propoxyphenyl]-2-(3,4,5-trimethoxyphenyl)-tetrahydro-furan

4-[5-(N'-n-Decyl-N'-hydroxyureidyl)-3-methoxy-4-propoxyphenyl]-2-(3,4, 5-trimethoxyphenyl)-tetrahydrofuran

4-[5-(N'-Hydroxy-N'-n-pentylureidyl)-3-methoxy-4-propoxyphenyl]-2-(3, 4,5-trimethoxyphenyl)-tetrahydrothiophene

4-[5-(N'-Cyclohexyl-N'-hydroxylureidyl)-3-methoxy-4-propoxyphenyl]-2-(3,4,5-trimethoxyphenyl)-tetrahydrothiophene

4-[5-(N'-n-Hexyl-N'-hydroxyureidyl)-3-methoxy-4-propoxyphenyl]-2-(3, 4,5-trimethoxyphenyl)-tetrahydrothiophene

4-[5-(N'-Hydroxy-N'-n-octylureidyl)-3-methoxy-4-propoxyphenyl]-2-(3,4, 5-trimethoxyphenyl)-tetrahydrothiophene

4-[5-(N'-Hydroxy-N'-methoxyethylureidyl)-3-methoxy-4-propoxyphenyl]-2-(3,4,5-trimethoxyphenyl)-tetrahydrothiophene

4-[5-(N'-n-Decyl-N'-hydroxylureidyl)-3-methoxy-4-propoxyphenyl]-2-(3, 4,5-trimethoxyphenyl)-tetrahydrothiophene

4-[5-(N'-Butyl-N'-hydroxyureidyl)-4-(p-bromophenylthioethoxy)-3-methoxyphenyl]-2-(3,4,5-trimethoxyphenyl)-tetrahydrofuran

4-[5-(N'-Butyl-N'-hydroxyureidyl)-4-(2-bromophenylthioethoxy)-3-methoxyphenyl]-2-(3,4,5-trimethoxyphenyl)-tetrahydrofuran

4-[5-(N'-Butyl-N'-hydroxyureidyl)-4-(3-bromophenylthioethoxy)-3-methoxyphenyl]-2-(3,4,5-trimethoxyphenyl)-tetrahydrofuran

4-[5-(N'-Butyl-N'-hydroxyureidyl)-4-(3,4,-dichlorophenylthioethoxy)-3-methoxyphenyl]-2-(3,4,5-trimethoxyphenyl)-tetrahydrofuran

4-[5-(N'-Butyl-N'-hydroxyureidyl)-4-(p-chlorophenylthioethoxy)-3-methoxyphenyl]-2-(3,4,5-trimethoxyphenyl)-tetrahydrofuran

4-[5-(N'-Butyl-N'-hydroxyureidyl)-4-(p-fluorophenylthioethoxy)-3-methoxyphenyl]-2-(3,4,5-trimethoxyphenyl)-tetrahydrofuran

4-[5-(N'-Butyl-N'-hydroxyureidyl)-4-(2,3,5,6-tetrafluorophenylthioethoxy)-3-methoxyphenyl]-2-(3,4,5-trimethoxyphenyl)-tetrahydrofuran

4-[5-(N-Butyl-N-hydroxyureidyl)-4-(2,3,4,5-tetrafluorophenylethoxy)-3-methoxyphenyl]-2-(3,4,5-trimethoxyphenyl)-tetrahydrofuran

4-[5-(N'-Butyl-N'-hydroxyureidyl)-4-(p-bromophenylsulfonylethoxy)-3-methoxyphenyl]-2-(3,4,5-trimethoxyphenyl)-tetrahydrofuran

4-[5-(N'-Butyl-N'-hydroxyureidyl)-4-(2-bromophenylsulfonylethoxy)-3-methoxyphenyl]-2-(3,4,5-trimethoxyphenyl)-tetrahydrofuran

4-[5-(N'-Hydroxy-N-methylureidyl)-4-(p-bromophenylthioethoxy)-3-methoxyphenyl]-2-(3,4,5-trimethoxyphenyl)-tetrahydrofuran

4-[5-(N'-Hydroxy-N'-methylureidyl)-4-(2-bromophenylthioethoxy)-3-methoxyphenyl]-2-(3,4,5-trimethoxyphenyl)-tetrahydrofuran

4-[5-(N'-Hydroxy-N'-methylureidyl)-4-(3-bromophenylthioethoxy)-3-methoxyphenyl]-2-(3,4,5-trimethoxyphenyl)-tetrahydrofuran

4-[5-(N'-Hydroxy-N'-methylureidyl)-4-(3,4-dichlorophenylthioethoxy)-3-methoxyphenyl]-2-(3,4,5-trimethoxyphenyl)-tetrahydrofuran

4-[5-(N'-Hydroxy-N'-methylureidyl)-4-(p-chlorophenylthioethoxy)-3-methoxyphenyl]-2-(3,4,5-trimethoxyphenyl)-tetrahydrofuran

4-[5-(N'-Ethyl-N'-hydroxyureidyl)-4-(p-fluorophenylthioethoxy)-3-methoxyphenyl]-2-(3,4,5-trimethoxyphenyl)-tetrahydrofuran

4-[5-(N'-Ethyl-N'-hydroxyureidyl)-4-(2,3,5,6-tetrafluorophenylthioethoxy)-3-methoxyphenyl]-2-(3,4,5-trimethoxyphenyl)-tetrahydrofuran

4-[5-(N'-Ethyl-N'-hydroxyureidyl)-4-(2,3,4,5-tetrafluorophenylethoxy)-3-methoxyphenyl]-2-(3,4,5-trimethoxyphenyl)-tetrahydrofuran

4-[5-(N'-Ethyl-N'-hydroxyureidyl)-4-(p-bromophenylsulfonylethoxy)-3-methoxyphenyl]-2-(3,4,5-trimethoxyphenyl)-tetrahydrofuran

4-[5-(N'-p-Chlorophenyl-N'-hydroxyureidyl)-4-(2-bromophenylsulfonylethoxy)-3-methoxyphenyl]-2-(3,4,5-trimethoxyphenyl)-tetrahydrofuran

4-[5-(N'-p-Chlorophenyl-N'-hydroxyureidyl)-4-(p-bromophenylthioethoxy)-3-methoxyphenyl]-2-(3,4,5-trimethoxyphenyl)-tetrahydrofuran

4-[5-(N'-*tert*-Butyl-N'-hydroxyureidyl)-4-(p-bromophenylthioethoxy)-3-methoxyphenyl]-2-(3,4,5-trimethoxyphenyl)-tetrahydrofuran

4-[5-(N'-*tert*-Butyl-N'-hydroxyureidyl)-4-(2-bromophenylthioethoxy)-3-methoxyphenyl]-2-(3,4,5-trimethoxyphenyl)-tetrahydrofuran

4-[5-(N'-*tert*-Butyl-N'-hydroxyureidyl)-4-(3-bromophenylthioethoxy)-3-methoxyphenyl]-2-(3,4,5-trimethoxyphenyl)-tetrahydrofuran

4-[5-(N'-Cyclohexyl-N'-hydroxyureidyl)-4-(3,4-dichlorophenylthioethoxy)-3-methoxyphenyl]-2-(3,4,5-trimethoxyphenyl)-tetrahydrofuran

4-[5-(N'-Cyclohexyl-N'-hydroxyureidyl)-4-(p-chlorophenylthioethoxy)-3-methoxyphenyl]-2-(3,4,5-trimethoxyphenyl)-tetrahydrofuran

4-[5-(N'-Cyclohexyl-N'-hydroxyureidyl)-4-(p-fluorophenylthioethoxy-3-methoxyphenyl]-2-(3,4,5-trimethoxyphenyl)-tetrahydrofuran

4-[5-(N'-Benzyl-N'-hydroxyureidyl)-4-(2,3,5,6-tetrafluorophenylthioethoxy)-3-methoxyphenyl]-2-(3,4,5-trimethoxyphenyl)-tetrahydrofuran

4-[5-(N'-Benzyl-N'-hydroxyureidyl)-4-(2,3,4,5-tetrafluorophenylethoxy)-3-methoxyphenyl]-2-(3,4,5-trimethoxyphenyl)-tetrahydrofuran

4-[5-(N'-Benzyl-N'-hydroxyureidyl)-4-(p-bromophenylsulfonylethoxy)-3-methoxyphenyl]-2-(3,4,5-trimethoxyphenyl)-tetrahydrofuran

4-[5-(N'-Benzyl-N'-hydroxyureidyl)-4-(2-bromophenylsulfonylethoxy)-3-methoxyphenyl]-2-(3,4,5-trimethoxyphenyl)-tetrahydrofuran

4-[5-(N'-Hydroxy-N'-*i*-propylureidyl)-4-(p-bromophenylthioethoxy)-3-methoxyphenyl]-2-(3,4,5-trimethoxyphenyl)-tetrahydrofuran

4-[5-(N'-*sec*-Butyl-N'-hydroxyureidyl)-4-(p-bromophenylthioethoxy)-3-methoxyphenyl]-2-(3,4,5-trimethoxyphenyl)-tetrahydrofuran

4-[5-(N'-*sec*-Butyl-N'-hydroxyureidyl)-4-(2-bromophenylthioethoxy)-3-methoxyphenyl]-2-(3,4,5-trimethoxyphenyl)-tetrahydrofuran

4-[5-(N'-Hydroxy-N'-propylureidyl)-4-(3-bromophenylthioethoxy)-3-methoxyphenyl]-2-(3,4,5-trimethoxyphenyl)-tetrahydrofuran

4-[5-(N'-Hydroxy-N'-*n*-pentylureidyl)-4-(3,4-dichlorophenylthioethoxy)-3-methoxyphenyl]-2-(3,4,5-trimethoxyphenyl)-tetrahydrofuran

4-[5-(N'-Hexyl-N'-hydroxyureidyl)-4-(p-chlorophenylthioethoxy)-3-methoxyphenyl]-2-(3,4,5-trimethoxyphenyl)-tetrahydrofuran

4-[5-(N'-Hydroxy-N'-octylureidyl)-4-(p-fluorophenylthioethoxy)-3-methoxyphenyl]-2-(3,4,5-trimethoxyphenyl)-tetrahydrofuran

4-[5-(N'-Hydroxy-N'-methoxyethylureidyl)-4-(2,3,5,6-tetrafluorophenylthioethoxy)-3-methoxyphenyl]-2-(3,4,5-trimethoxyphenyl)-tetrahydrofuran

4-[5-(N'-Decyl-N'-hydroxyureidyl)-4-(2,3,4,5-tetrafluorophenylethoxy)-3-methoxyphenyl]-2-(3,4,5-trimethoxyphenyl)-tetrahydrofuran

4-[5-(N'-Hydroxy-N'-methylureidylmethyl)-4-(p-chlorophenylthioethoxy)-3-methoxyphenyl]-2-(3,4,5-trimethoxyphenyl)-tetrahydrofuran

4-[5-(N'-Hydroxy-N'-*i*-propylureidylmethyl)-4-(p-chlorophenylthioethoxy)-3-methoxyphenyl]-2-(3,4,5-trimethoxyphenyl)-tetrahydrofuran

4-[5-(N'-Butyl-N'-hydroxyureidylmethyl)-4-(p-chlorophenylthioethoxy)-3-methoxyphenyl]-2-(3,4,5-trimethoxyphenyl)-tetrahydrofuran

4-[5-(N'-Hydroxy-N'-propylureidylmethyl)-4-(p-chlorophenylthioethoxy)-3-methoxyphenyl]-2-(3,4,5-trimethoxyphenyl)-tetrahydrofuran

4-[5-(N'-Ethyl-N'-hydroxyureidylmethyl)-4-(p-chlorophenylthioethoxy)-3-methoxyphenyl]-2-(3,4,5-trimethoxyphenyl)-tetrahydrofuran

4-[5-(N'-Hydroxy-N'-octylureidylmethyl)-4-(p-chlorophenylthioethoxy)-3-methoxyphenyl]-2-(3,4,5-trimethoxyphenyl)-tetrahydrofuran

4-[5-(N'-Benzyl-N'-hydroxyureidyl)-4-(p-bromophenylsulfonylethoxy)-3-methoxyphenyl]-2-(3,4,5-trimethoxyphenyl)-tetrahydrofuran

4-[5-(N'-Benzyl-N'-hydroxyureidyl)-4-(2-bromophenylsulfonylethoxy)-3-methoxyphenyl]-2-(3,4,5-trimethoxyphenyl)-tetrahydrofuran

4-[5-(N'-Hydroxy-N'-*i*-propylureidyl)-4-(p-bromophenylthioethoxy)-3-methoxyphenyl]-2-(3,4,5-trimethoxyphenyl)-tetrahydrofuran

4-[5-(N'-Hydroxy-N'-octylureidyl)-4-(p-fluorophenylthioethoxy)-3-methoxyphenyl]-2-(3,4,5-trimethoxyphenyl)-tetrahydrofuran

4-[5-(N'-Butyl-N'-hydroxyureidyl)-4-(p-bromophenylthioethoxy)-3-methoxyphenyl]-2-(3,4,5-trimethoxyphenyl)-tetrahydrofuran

4-[5-(N'-Butyl-N'-hydroxyureidyl-4-(2-bromophenylthioethoxy)-3-methoxyphenyl]-2-(3,4,5-trimethoxyphenyl)-tetrahydrofuran

4-[5-(N'-Butyl-N'-hydroxyureidyl)-4-(3-bromophenylthioethoxy)-3-methoxyphenyl]-2-(3,4,5-trimethoxyphenyl)-tetrahydrofuran

4-[5-(N'-Hydroxy-N'-methylureidylmethyl)-4-(p-chlorophenylthioethoxy)-3-methoxyphenyl]-2-(3,4,5-trimethoxyphenyl)-tetrahydrofuran

4-[5-(N'-Hydroxy-N'-*i*-propylureidylmethyl)-4-(p-chlorophenylthioethoxy)-3-methoxyphenyl]-2-(3,4,5-trimethoxyphenyl)-tetrahydrofuran

4-[5-(N'-Butyl-N'-hydroxyureidylmethyl)-4-(p-chlorophenylthioethoxy)-3-methoxyphenyl]-2-(3,4,5-trimethoxyphenyl)-tetrahydrofuran

**Triple Bonded Hydroxamates:**

4-[5-[1-(N-Acetyl-N-hydroxyamino)propyn-3-yl]-3-methoxy-4-propoxyphenyl]-2-(3,4,5-tri methoxyphenyl)-tetrahydrofuran.

4-[5-[1-(N-Hydroxy-N-propanoylamino)propyn-3-yl]-3-methoxy-4-propoxyphenyl]-2-(3,4,5-trimethoxyphenyl)-tetrahydrofuran.

4-[5-[1-(N-Butanoyl-N-hydroxyamino)propyn-3-yl]-3-methoxy-4-propoxyphenyl]-2-(3,4,5-trimethoxyphenyl)-tetrahydrofuran.

4-[5-[1-(N-Hydroxy-N-i-propanoylamino)propyn-3-yl] -3-methoxy-4-propoxyphenyl]-2-(3,4,5-trimethoxyphenyl)-tetrahydrofuran.

4-[5-[1-(N-Cyclohexanecarbonyl-N-hydroxyamino)propyn-3-yl]-3-methoxy-4-propoxyphenyl]-2-(3,4,5-trimethoxyphenyl)-tetrahydrofuran

4-[5-[1-(N-Benzoyl-N-hydroxylamino)propyn-3-yl]-3-methoxy-4-propoxyphenyl]-2-(3,4,5-trimethoxyphenyl)-tetrahydrofuran.

4-[5-[1-(N-Hydroxy-N-3-phenoxyoxybenzoylamino)propyn-3-yl]-3-methoxy-4-propoxyphenyl]-2-(3,4,5-trimethoxyphenyl)-tetrahydrofuran.

4-[5-[1-(N-Hydroxy-N-4-methoxybenzoylamino)propyn-3-yl]-3-methoxy-4-propoxyphenyl]-2-(3,4,5-trimethoxyphenyl)-tetrahydrofuran.

4-[5-[1-(N-3-Benzoyl-N-hydroxyamino)propyn-3-yl]-3-methoxy-4-propoxyphenyl]-2-(3,4,5-trimethoxyphenyl)-tetrahydrofuran.

4-[5-[1-(N-Hydroxy-N-4-hydroxybenzoylamino)propyn-3-yl]-3-methoxy-4-propoxyphenyl]-2-(3,4,5-trimethoxyphenyl)-tetrahydrofuran.

4-[5-(1-(N-Acetyl-N-hydroxyamino)propyn-3-yl]-4-(p-bromophenylthioethoxy)-3-methoxyphenyl]-2-(3,4,5-trimethoxyphenyl)-tetrahydrofuran

4-[5-[1-(N-Hydroxy-N-propanoylamino)propyn-3-yl]-4-(p-chlorophenylthioethoxy)-3-methoxyphenyl]-2-(3,4,5-trimethoxyphenyl)-tetrahydrofuran

4-[5-[1-(N-Butanoyl-N-hydroxyamino)propyn-3-yl]-4-(3,4-dichlorophenylthioethoxy)-3-methoxyphenyl]-2-(3,4,5-trimethoxyphenyl)-tetrahydrofuran

4-[5-[1-(N-Hydroxy-N-cyclohexanecaxbonylamino)propyn-3-yl]-4-(p-fluorophenylthioethoxy)-3-methoxyphenyl]-2-(3,4,5-trimethoxyphenyl)-tetrahydrofuran

4-[5-[1-(N-Hydroxy-N-phenoxybenzoylamino)propyn-3-yl]-4-(2,3,5,6-tetrafluorophenylthioethoxy) 3-methoxyphenyl]-2-(3,4,5-trimethoxyphenyl)-tetrahydrofuran

4-[5-[1-(N-Hydroxy-N-methoxybenzoylamino)propyn-3-yl]-4-(2-bromophenylthioethoxy)-3-methoxyphenyl]-2-(3,4,5-trimethoxyphenyl)-tetrahydrofuran

4-[5-[1-(N-Hydroxy-N-hydroxybenzoylamino)propyn-3-yl]-4-(p-chlorophenylthioethoxy)-3-methoxyphenyl]-2-(3,4,5-trimethoxyphenyl)-tetrahydrofuran

**Triple Bonded Ureas:**

4-[5-[1-(N'-Hydroxy-N'-methylureidyl)propyn-3-yl]-3-methoxy-4-propoxyphenyl]-2-(3,4,5-trimethoxyphenyl)-tetrahydrofuran.

4-[5-[1-(N'-Ethyl-N'-hydroxyureidyl)propyn-3-yl]-3-methoxy-4-propoxyphenyl]-2-(3,4,5-trimethoxyphenyl)-tetrahydrofuran.

4-[5-[1-(N'-Hydroxy-N'-propylureidyl)propyn-3-yl]-3-methoxy-4-propoxyphenyl]-2-(3,4,5-trimethoxyphenyl)-tetrahydrofuran.

4-[5-[1(N'-n-Butyl-N'-hydroxyureidyl)propyn-3-yl]-3-methoxy-4-propoxyphenyl]-2-(3,4,5-trimethoxyphenyl)-tetrahydrofuran.

4-[5-[1-(N'-Hydroxy-N'-i-propylureidyl)propyn-3-yl]-3-methoxy-4-propoxyphenyl]-2-(3,4,5-trimethoxyphenyl)-tetrahydrofuran.

4-[5-[1-(N'-t-Butyl-N'-hydroxyureidyl)propyn-3-yl]-3-methoxy-4-propoxyphenyl]-2-(3,4,5-trimethoxyphenyl)-tetrahydrofuran.

4-[5-[1-(N'-Benzyl-N'-hydroxyureidyl)propyn-3-yl]-3-methoxy-4-propoxyphenyl]-2-(3,4,5-trimethoxyphenyl)-tetrahydrofuran.

4-[5-[1-(N'-Cyclopropylmethyl-N'-hydroxyureidyl)propyn-3-yl]-3-methoxy-4-propoxyphenyl]-2-(3,4,5-trimethoxyphenyl)-tetrahydrofuran.

4-[5-[1-(N'-Allyl-N'-hydroxyureidyl)propyn-3-yl]-3-methoxy-4-propoxyphenyl]-2-(3,4,5-trimethoxyphenyl)-tetrahydrofuran.

4-[5-[1-(N'-Hydroxy-N'-hydroxyethylureidyl)propyn-3-yl]-3-methoxy-4-propoxyphenyl]-2-(3,4,5-trimethoxyphenyl)-tetrahydrofuran.

4-[5-[1-(N'-Hydroxy-N'-methylureidyl)propyn-3-yl]-4-(p-chlorophenylthioethoxy)-3-methoxyphenyl]-2-(3,4,5-trimethoxyphenyl)-tetrahydrofuran

4-[5-(1-(N'-Ethyl-N'-hydroxyureidyl)propyn-3-yl]-4-(p-chlorophenylthioethoxy)-3-methoxyphenyl]-2-(3,4,5-trimethoxyphenyl)-tetrahydrofuran

4-[5-[1-(N'-Hydroxy-N'-propylureidyl)propyn-3-yl]-4-(p-chlorophenylthioethoxy)-3-methoxyphenyl]-2-(3,4,5-trimethoxyphenyl)-tetrahydrofuran

4-[5-[1-(N'-*n*-Butyl-N'-hydroxyureidyl)propyn-3-yl]-4-(p-chlorophenythioethoxy)-3-methoxyphenyl]-2-(3,4,5-trimethoxyphenyl)-tetrahydrofuran

4-[5-[1-(N'-Hydroxy-N'-*i*-propylureidyl)propyn-3-yl]-4-(p-chlorophenylthioethoxy)-3-methoxyphenyl]-2-(3,4,5-trimethoxyphenyl)-tetrahydrofuran

4-[5-[1-(N'-*tert*-Butyl-N'-hydroxyureidyl)propyn-3-yl]-4-(p-chlorophenylthioethoxy)-3-methoxyphenyl]-2-(3,4,5-trimethoxyphenyl)-tetrahydrofuran

4-[5-[1-(N'-Benzyl-N'-hydroxyureidyl)propyn-3-yl]-4-(p-chlorophenylthioethoxy)-3-methoxyphenyl]-2-(3,4,5-trimethoxyphenyl)-tetrahydrofuran

4-[5-[1-(N'-Cyclopropylmethyl-N'-hydroxyureidyl)propyn-3-yl]-4-(p-chlorophenylthioethoxy)-3-methoxyphenyl]-2-(3,4,5-trimethoxyphenyl)-tetrahydrofuran

4-[5-[1-(N'-Allyl-N'-hydroxyureidyl)propyn-3-yl]-4-(p-chlorophenylthioethoxy)-3-methoxyphenyl]-2-(3,4,5-trimethoxyphenyl)-tetrahydrofuran

4-[5-[1-(N'-Hydroxy-N'-hydroxyethylureidyl)propyn-3-yl]-4-(p-chlorophenythioethoxy)-3-methoxyphenyl]-2-(3,4,5-trimethoxyphenyl)-tetrahydrofuran

**Double Bonds Hydroxamates: Both *Cis* and *Trans* Isomers at the Tetrahydrofuran Ring.**

4-[5-[*trans*-1-(N-Acetyl-N-hydroxyamino)propen-3-yl]-3-methoxy-4-propoxyphenyl]-2-(3,4,5-trimethoxyphenyl)-tetrahydrofuran.

4-[5-(*trans*-1-(N-Hydroxy-N-propanoylamino)propen-3-yl]-3-methoxy-4-propoxyphenyl]-2-(3,4,5-trimethoxyphenyl)-tetrahydrofuran.

4-[5-[*trans*-1-(N-Butyl-N-hydroxyamnino)propen-3-yl]-3-methoxy-4-propoxyphenyl]-2-(3,4,5-trimethoxyphenyl)-tetrahydrofuran.

4-[5-[*trans*-1-(N-hydroxy-N-nicotinoylamino)propen-3-yl]-3-methoxy-4-propoxyphenyl]-2-(3,4,5-trimethoxyphenyl)-tetrahydrofuran.

4-[5-[*trans*-1-(N-Hydroxy-N-phenylacetylamino)propen-3-yl]-3-methoxy-4-propoxyphenyl]-2-(3,4,5-trimethoxyphenyl)-tetrahydrofuran.

4-[5-*[trans*-1-(N-Hydroxy-N-3-phenoxybenzoylamino)propen-3-yl]-3-methoxy-4-propoxy phenyl]-2-(3,4,5-trimethoxyphenyl)-tetrahydrofuran.

4-[5-[*trans*-1-(N-3-Chlorobenzoyl-N-hydroxyamino)propen-3-yl]-3-methoxy-4-propoxyphenyl]-2-(3,4,5-trimethoxyphenyl)-tetrahydrofuran.

4-[5-[*trans*-1-(N-3-Chlorobenzoyl-N-hydroxyamino)propen-3-yl]-3-methoxy-4-propoxyphenyl]-2-(3,4,5-trimethoxyphenyl)-tetrahydrofuran.

4-[5-[*trans*-1-(N-2,4-Difluorobenzoyl-N-hydroxyamino)propen-3-yl]-3-methoxy-4-propoxyphenyl]-2-(3,4,5-trimethoxyphenyl)-tetrahydrofuran.

4-[5-[*trans*-1-(N-Hydroxy-N-3,4-methylenedioxybenzoylamino)propen-3-yl]-3-methoxy-4-pro poxyphenyl]-2-(3,4,5-trimethoxyphenyl)-tetrahydrofuran.

and also the corresponding saturated hydroxamates, e.g., 4-[5-[1-(-N-Hydroxy N-3,4-methylenedioxybenzoyl-N-hydroxyamino)propyl]-3-methoxy-4-propoxyphenyl]-2-(3,4,5-trimethoxyphenyl)tetrahydrofuran.

4-[5-[*trans*-1-(N-Acetyl-N-hydroxyamino)propen-3-yl]-4-(p-bromophenylthioethoxy)-3-methoxyphenyl]-2-(3,4,5-trimethoxyphenyl)-tetrahydrofuran

4-[5-[*trans*-1-(N-Hydroxy-N-propanoylamino)propen-3-yl]-4-(p-chlorophenylthioethoxy)-3-methoxyphenyl]-2-(3,4,5-trimethoxyphenyl)-tetrahydrofuran

4-[5-[*trans*-1-(N-Butanoyl-N-hydroxyamino)propen-3-yl]-4-(3,4-dichlorophenythioethoxy)-3-methoxyphenyl]-2-(3,4,5-trimethoxyphenyl)-tetrahydrofuran

4-[5-[*trans*-1-(N-Hydroxy-N-cyclohexanecarbonylamino)propen-3-yl]-4-(p-fluorophenylthioethoxy)-3-methoxyphenyl]-2-(3,4,5-trimethoxyphenyl)-tetrahydrofuran

4-[5-[*trans*-1-(N-Hydroxy-N-phenoxybenzoylamino)propen-3-yl]-4-(2,3,5,6-tetrafluorophenylthioethoxy)-3-methoxyphenyl]-2-(3,4,5-trimethoxyphenyl)-tetrahydrofuran

4-[5-[*trans*-1-(N-Hydroxy-N-methoxybenzoylamino)propen-3-yl]-4-(2-bromophenylthioethoxy)-3-methoxyphenyl]-2-(3,4,5-trimethoxyphenyl)-tetrahydrofuran

4-(5-[*trans*-1-(N-Hydroxy-N-hydroxybenzoylamino)propen-3-yl]-4-(p-chlorophenylthioethoxy)-3-methoxyphenyl]-2-(3,4,5-trimethoxyphenyl)-tetrahydrofuran

**Double Bonded Ureas: Both *Cis* and *Trans* Isomers at the Tetrahydrofuran Ring.**

4-[5-[*trans*-1-(N'-Hydroxy-N'-methylureidyl)propen-3-yl]-3-methoxy-4-propoxyphenyl]-2-(3,4,5-trimethoxyphenyl)-tetrahydrofuran.

4-[5-[*trans*-1-(N'-Ethyl-N'-hydroxyureidyl)propen-3-yl]-3-methoxy-4-propoxyphenyl]-2-(3,4,5-trimethoxyphenyl)-tetrahydrofuran.

4-[5-[*trans*-1-(N'-Hydroxy-N'-propylureidyl)propen-3-yl]-3-methoxy-4-propoxyphenyl]-2-(3,4,5-trimethoxyphenyl)-tetrahydrofuran.

4-[5-[*trans*-1-(N'-Butyl-N'-hydroxyureidyl)propen-3-yl]-3-methoxy-4-propoxyphenyl]-2-(3,4,5-trimethoxyphenyl)-tetrahydrofuran.

4-[5-[*trans*-1-(N'-Hydroxy-N'-i-propylureidyl)propen-3-yl]-3-methoxy-4-propoxyphenyl]-2-(3,4,5-trimethoxyphenyl)-tetrahydrofuran.

4-[5-[*trans*-1-(N'-t-Butyl-N'-hydroxyureidyl)propen-3-yl]-3-methoxy-4-propoxyphenyl]-2-(3,4,5-trimethoxyphenyl)-tetrahydrofuran.

4-[5-*[trans*-1-(N'-Benzyl-N'-hydroxyureidyl)propen-3-yl]-N-3-methoxy-4-propoxyphenyl]-2-(3,4,5-trimethoxyphenyl)-tetrahydrofuran.

4-[5-[*trans*-1-(N'-Allyl-N'-hydroxyureidyl)propen-3-yl]-3-methoxy-4-propoxyphenyl]-2-(3,4,5-trimethoxyphenyl)-tetrahydrofuran.

4-[5-[*trans*-1-(N'-Cyclohexyl-N'-hydroxyureidyl)propen-3-yl]-3-methoxy-4-propoxyphenyl-2-(3,4,5-trimethoxyphenyl)-tetrahydrofuran.

4-[5-[*trans*-1-(N'-Hydroxy-N'-methylthioethylureidyl)propen-3-yl]-3-methoxy-4-propoxyphenyl]-2-(3,4,5-trimethoxyphenyl)-tetrahydrofuran.

4-[5-[*trans*-1-(N'-Cyclohexyl-N'-hydroxyureidyl)propen-3-yl)-3-methoxy-4-propoxyl-2-(3,4,5-trimethoxyphenyl)-tetrahydrofuran.

and also the corresponding saturated ureas, e.g.,
4-[5-[1(N'-Cyclohexyl-N'-hydroxyureidyl)propyl]-3-methoxy-4-propoxyphenyl]-2-(3,4,5-trimethoxyphenyl)-tetrahydrofuran.

4-[5-[*trans*-1-(N'-Hydroxy-N'-methylureidyl)propen-3-yl]-4-(p-chlorophenylthioethoxy)-3-methoxyphenyl]-2-(3,4,5-trimethoxyphenyl)-tetrahydrofuran

4-[5-[*trans*-1-(N'-Ethyl-N'-hydroxyureidyl)propen-3-yl]-4-(p-chlorophenylthioethoxy)-3-methoxyphenyl]-2-(3,4,5-trimethoxyphenyl)-tetrahydrofuran

4-[5-[*trans*-1-(N'-Hydroxy-N'-propylureidyl)propen-3-yl]-4-(p-chlorophenylthioethoxy)-3-methoxyphenyl]-2-(3,4,5-trimethoxyphenyl)-tetrahydrofuran

4-[5-[*trans*-1-(N-*n*-Butyl-N'-hydroxyureidyl)propen-3-yl]-4-(p-chlorophenylthioethoxy)-3-methoxyphenyl]-2-(3,4,5-trimethoxyphenyl)-tetrahydrofuran

4-[5-[*trans*-1-(N'-Hydroxy-N'-*i*-propylureidyl)propen-3-yl]-4-(p-chlorophenylthioethoxy)-3-methoxyphenyl]-2-(3,4,5-trimethoxyphenyl)-tetrahydrofuran

4-[5-[*trans*-1-(N'-*tert*-Butyl-N'-hydroxyureidyl)propen-3-yl]-4-(p-chlorophenylthioethoxy)-3-methoxyphenyl]-2-(3,4,5-trimethoxyphenyl)-tetrahydrofuran

4-[5-[*trans*-1-(N'-Benzyl-N-hydroxyureidyl)propen-3-yl]-4-(p-chlorophenylthioethoxy)-3-methoxyphenyl]-2-(3,4,5-trimethoxyphenyl)-tetrahydrofuran

4-[5-[*trans*-1-(N'-Cyclopropyl-N'-hydroxyureidyl)propen-3-yl]-4-(p-chlorophenylthioethoxy)-3-methoxyphenyl]-2-(3,4,5-trimethoxyphenyl)-tetrahydrofuran

4-[5-[*trans*-1-(N'-Allyl-N'-hydroxyureidyl)propen-3-yl-4-(p-chlorophenylthioethoxy)-3-methoxyphenyl]-2-(3,4,5-trimethoxyphenyl)-tetrahydrofuran

4-[5-[*trans*-1-(N'-Hydroxy-N'-hydroxyethylureidyl)propen-3-yl]-4-(p-chlorophenylthioethoxy)-3-methoxyphenyl]-2-(3,4,5-trimethoxyphenyl)-tetrahydrofuran

*Cis* and *Trans* Isomers of the Following Compounds:

2-(3-Methoxy-4-methoxyethoxy-5-N-methylaminophenyl)-5-(3,4,5-trimethoxyphenyl)-tetrahydrothiophene.

2-(3-Methoxy-4-methoxyethoxy-5-N-ethylaminophenyl)-5-(3,4,5-trimethoxyphenyl)-tetrahydrothiophene.

2-(3-Methoxy-4-methoxyethoxy-5-N,N-dipropylaminophenyl)-5-(3,4,5-trimethoxyphenyl)-tetrahydrothiophene.

2-(3-Methoxy-4-methylthioethoxy-5-N-methylaminophenyl)-5-(3,4,5-trimethoxyphenyl)-tetrahydrofuran.

2-(4-p -Hydroxyphenylthioethoxy-3-methoxy-5-N-methylaminophenyl)-5-(3,4,5-trimethoxyphenyl)-tetrahydrofuran.

2-[3-Methoxy-4-methoxyethoxy-5-(1-pyrrolidinyl) phenyl]-5-(3,4,5-trimethoxyphenyl)-tetrahydrothiophene.

2-(3-Methoxy-4-methoxyethoxy-5-N, N-diethylaminophenyl)-5-(3,4,5-trimethoxyphenyl)-tetrahydrothiophene.

2-[4-p-Cyanophenylthioethoxy-3-methoxy-5-(1-pyrrolidinyl) phenyl]-5-(3,4,5-trimethoxyphenyl)-tetrahydrofuran.

2-(3-Methoxy-4-p-methoxyphenylthioethoxy-5-N, N-dimethylaminophenyl)-5-(3,4,5-trimethoxyphenyl)-tetrahydrofuran.

2-(3-Methoxy-4-p-cyanophenylthioethoxy-5-N, N-diethylaminophenyl)-5-(3,4,5-trimethoxyphenyl)-tetrahydrothiophene.

2-(4-p-Hydroxyphenythioethoxy-3-methoxy-5-N-methylaminophenyl)-5-(3,4,5-trimethoxy phenyl)-tetrahydrothiophene.

2-(4-p-Cyanophenylthioethoxy-3-methoxy-5-N,N-dimethylaminophenyl)-5-(3,4,5-trimethoxyphenyl)-tetrahydrothiophene.

2-[3-Methoxy-4-p-methoxyphenylthioethoxy-5-(4-morpholinyl)phenyl]-5-(3,4,5-trimethoxyphenyl)-tetrahydrothiophene.

2-(3-Methoxy-4-p-methoxyphenylthioethoxy-5-N,N-dimethylaminophenyl)-5-(3,4,5-trimethoxyphenyl)-tetrahydrothiophene.

2-(4-p-Cyanophenylthioethoxy-3-methoxy-5-N-methylaminophenyl)-5-(3,4, 5-trimethoxyphenyl)-tetrahydrofuran.

2-(4-p-Cyanophenythioethoxy-3-methoxy-5-N-methylaminophenyl)-5-(3,4, 5-trimethoxyphenyl)-tetrahydrothiophene.

2-[4-p-Cyanophenylthioethoxy-3-methoxy-5-(4-morpholinyl) phenyl]-5-(3,4,5-trimethoxyphenyl)-tetrahydrofuran.

2-(3-Methoxy-4-methoxyethoxy-5-N, N-dibutylaminophenyl)-5-(3,4,5-trimethoxyphenyl)-tetrahydrothiophene.

2-(3-Methoxy-4-methylthioethoxy-5-N-methylaminophenyl)-5-(3,4,5-trimethoxyphenyl)-tetrahydrothiophene.

2-(3-Methoxy-4-p-chlorophenylthioethoxy-5-N-methylaminophenyl)-5-(3,4,5-trimethoxyphenyl)-tetrahydrofuran

2-(3-Methoxy-4-p-chlorophenylthioethoxy-5-N-ethylaminophenyl)-5-(3,4,5-trimethoxyphenyl)-tetrahydrofuran

2-(3-Methoxy-4-p-chlorophenythioethoxy-5-N,N-dipropylaminophenyl)-5-(3,4,5-trimethoxyphenyl)-tetrahydrofuran

2-(3-Methoxy-4-p-bromophenylthioethoxy-5-N,N-dipropylaminophenyl)-5-(3,4,5-trimethoxyphenyl)-tetrahydrofuran

2-(3-Methoxy-4-3,4-dichlorophenylthioethoxy-5-N,N-dipropylaminophenyl)-5-(3,4,5-trimethoxyphenyl)-tetrahydrofuran

2-(3-Methoxy-4-p-fluorophenylthioethoxy-5-N,N-dipropylaminophenyl)-5(3,4,5-trimethoxyphenyl)-tetrahydrofuran

2-[3-Methoxy-4-(2,3,5,6-tetrafluorophenylthioethoxy)-5-N,N-dipropylaminophenyl]-5-(3,4,5-trimethoxyyphenyl)-tetrahydrofuran

2-[3-Methoxy-4-(2-bromophenylthioethoxy)-5-N,N-dipropylaminophenyl]-5-(3,4,5-trimethoxyphenyl)-tetrahydrofuran

2-[3-Methoxy-4-p-chlorophenylthioethoxy-5-(1-pyrrolidinyl)phenyl]-5-(3,4,5-trimethoxyphenyl)-tetrahydrofuran

2-(3-Methoxy-4-p-chlorophenylthioethoxy-5-N,N-diethylaminophenyl)-5-(3,4,5-trimethoxyphenyl)-tetrahydrofuran

2-[3-Methoxy-4-p-chlorophenylthioethoxy-5-(4-morpholinyl)phenyl]-5-(3,4,5-trimethoxyphenyl)-tetrahydrofuran

2-(3-Methoxy-4-p-chlorophenylthioethoxy-5-N,N-dibutylaminophenyl)-5-(3,4,5-trimethoxyphenyl)-tetrahydrofuran

### B. Stereochemistry

Compounds as defined in the accompanying claims exhibit a number of stereochemical configurations. Carbon atoms 2 and 5 in the center ring are chiral, and thus the center ring exists at a minimum as a diastereomeric pair. Each diastereomer exists as a set of enantiomers. Therefore, based on the chiral $C_2$ and $C_5$ atoms alone, the compound is a mixture of four enantiomers.

The R groups in the active compounds described herein can likewise include chiral carbons, and thus, optically active centers.

It is sometimes found that one or more enantiomers of a biologically active compound is more active, and perhaps less toxic, than other enantiomers of the same compound. Such enantiomerically enriched compounds are often preferred for pharmaceutical administration to humans. For example, it has been discovered that *trans*-2,5-diaryl tetrahydrothiophene and *trans*-2,5-diaryl tetrahydrofuran are often more active PAF receptor antagonists than their *cis* counterparts.

One of ordinary skill in the art can easily synthesize and separate the enantiomers of the disclosed compounds using chiral reagents and known procedures, and can evaluate the biological activity of the isolated enantiomer using methods disclosed herein or otherwise known. Through the use of chiral NMR shift reagents, polarimetry, or chiral HPLC, the optical enrichment of the compound can be determined.

Classical methods of resolution include a variety of physical and chemical techniques. Often the simplest and most efficient technique is repeated recrystallization. Recrystallization can be performed at any stage in the preparation of the compound, or the final enantiomeric product. If successful, this simple approach represents a method of choice.

When recrystallization fails to provide material of acceptable optical purity, other methods can be evaluated. If the compound is basic, one can use chiral acids that form diastereomeric derivatives that may possess significantly different solubility properties. Nonlimiting examples of chiral acids include malic acid, mandelic acid, dibenzoyl tartaric acid, 3-bromocamphor-8-sulfonic acid, 10-camphorsulfonic acid, and di-*p*-toluoyltartaric acid. Similarly, acylation of a free hydroxyl group with a chiral acid also results in the formation of diastereomeric derivatives whose physical properties may differ sufficiently to permit separation. Enantiomerically pure or enriched compounds can be obtained by passing the racemic mixture through a chromatographic column that has been designed for chiral separations, including cyclodextrin bonded columns marketed by Rainin Corporation.

A variety of chemical reagents and experimental procedures have been developed in recent years to produce enantiomerically pure or enriched products. For example, individual 2S,5S or 2R,5R enantiomers of 2,5-diaryl tetrahydrofurans can be prepared by the method described by Corey *et al*. (Corey, E.J. *et al*, <u>Tetrahedron Letters</u> <u>29</u>, 2899 (1988)).

### C. Syntheses of Active Compounds

The 2,5-diaryl tetrahydrofurans and tetrahydrothiophenes disclosed herein can be prepared in a variety of ways known to those skilled in the art, including by methods disclosed in or obvious in view of methods disclosed in U.S. Patent Nos. 4,539,332, 4,757,084, 4,996,203 and 5,001,123, and European Patent Application Nos. 365089, 402151, and 402150.

1,3-Diaryl cyclopentanes can be prepared using the procedure of Graham, *et al* (1.3-Diaryl Cyclopentanes: A New Class of Potent PAF Receptor Antagonists. 197$^{th}$ ACS National Meeting, Dallas, Texas, April 9-14, 1989, Division of Medicinal Chemistry, poster no. 25 (abstract)), or by other known methods.

2,5-Diaryl pyrrolidines can be prepared by methods known to those skilled in the art, including that described by Boekvall, *et al*. (<u>J.Org. Chem.</u> <u>55</u>, 826 (1990)).

2,4-Diaryl tetrahydrofurans and tetrahydrothiophenes and 2,4-diaryl pyrrolidines can also be prepared by adaptations of methods described herein, or by other known methods.

A general procedure for preparing a hydroxyurea is:

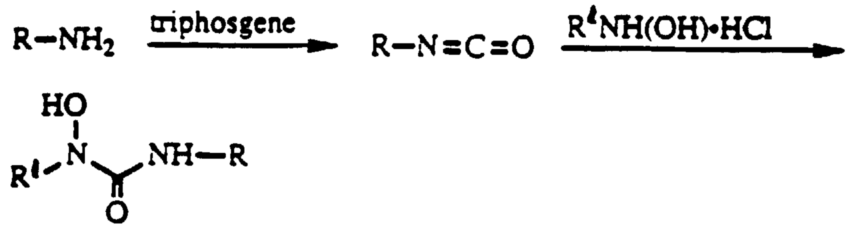


wherein R is a 2,5-diaryl tetrahydrothiophene, tetrahydrofuran, or pyrrolidine; 1,3-diaryl cyclopentane; or 2,4-diaryl tetrahydrothiophene, tetrahydrofuran or pyrrolidine; with or without a linking moiety, and R' is a moiety, and R' is a moiety as defined in detail above.

General procedures for preparing reverse hydroxyureas are:

1. 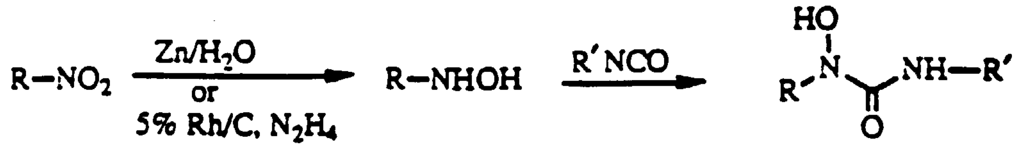


2. 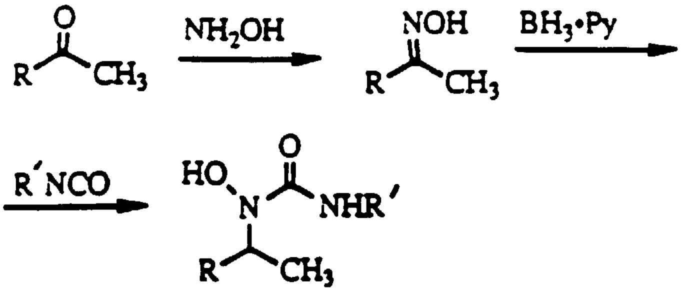


A general procedure for preparing a hydroxamic acid is:

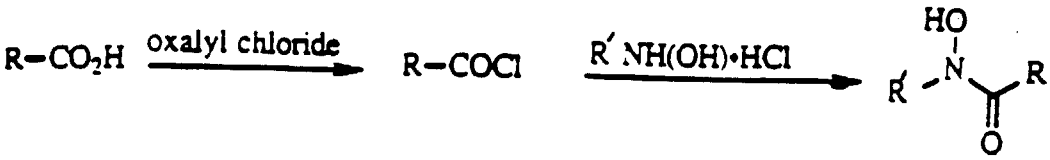


A general procedure for preparing a reverse hydroxamic acid is:

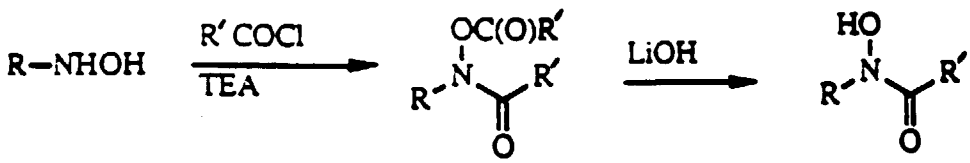


A general procedure for preparing amidohydroxyurea moieties is:

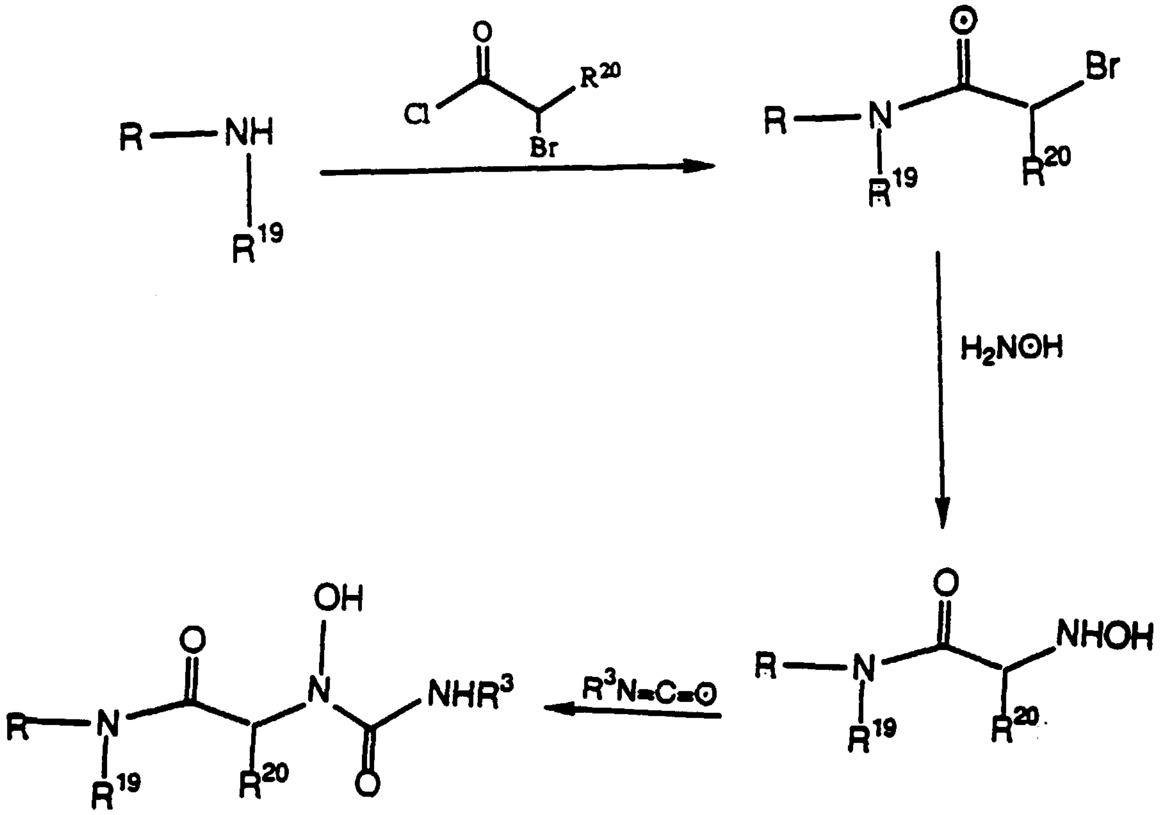


Oxalkanes and thioalkanes can be prepared as described by Crawley, et al, J. Med. Chem., 35, 2600-2609 (1992), and illustrated below, by conversion of the desired moiety into a Grignard reagent or lithium salt, followed by reaction with the appropriate cyclic ketone.

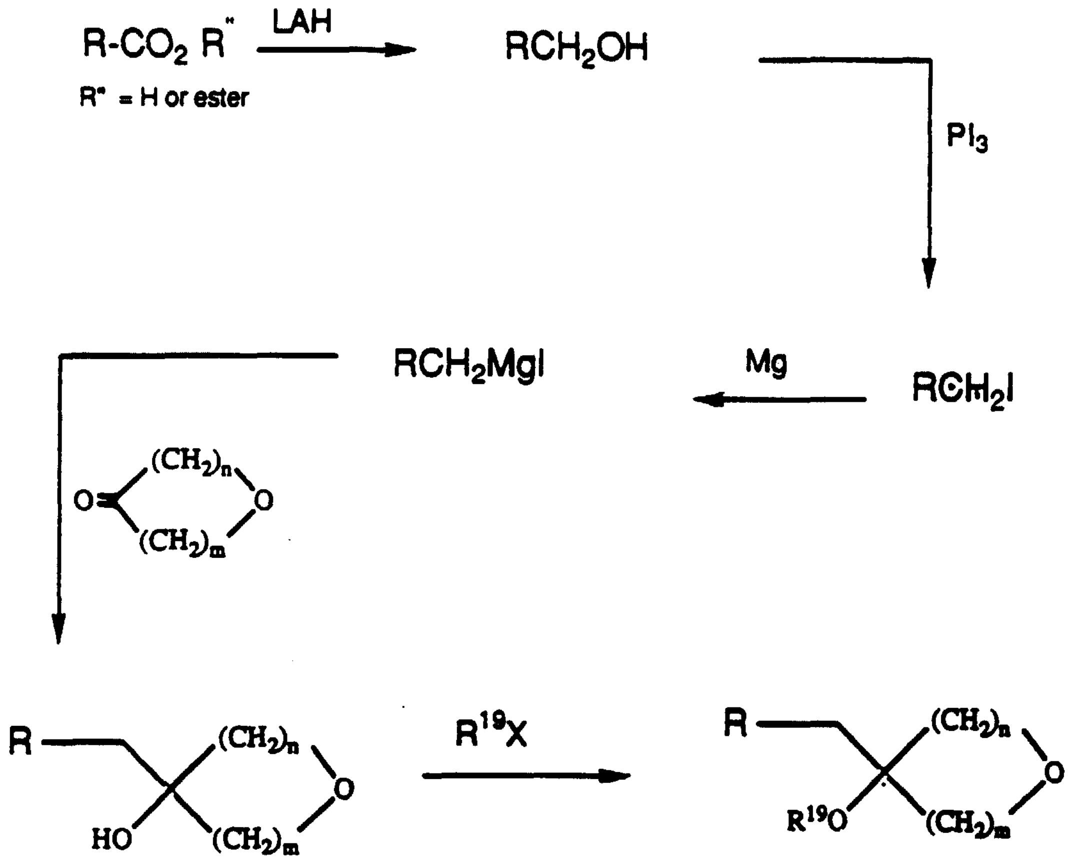
LAH
R" = H or ester
PI3
Mg
R19X

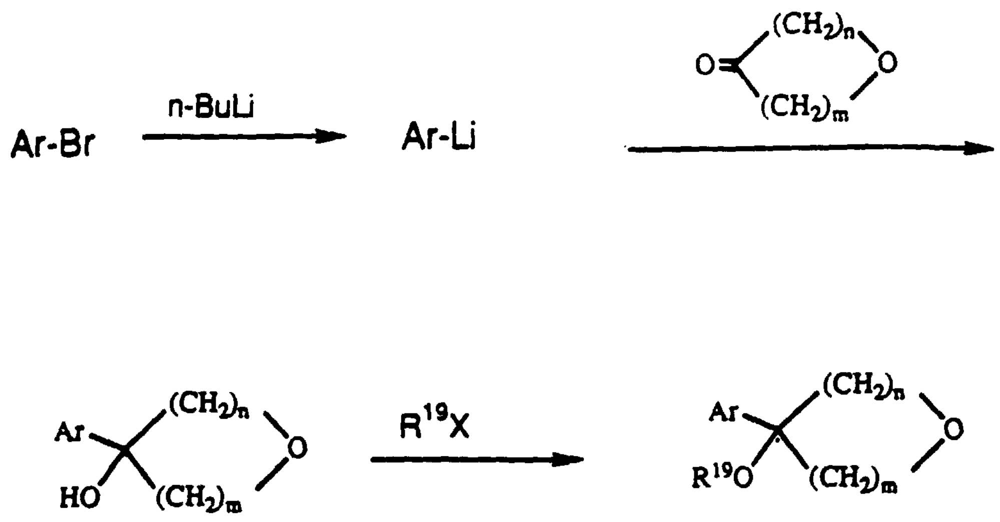
n-BuLi
$R^{19}X$

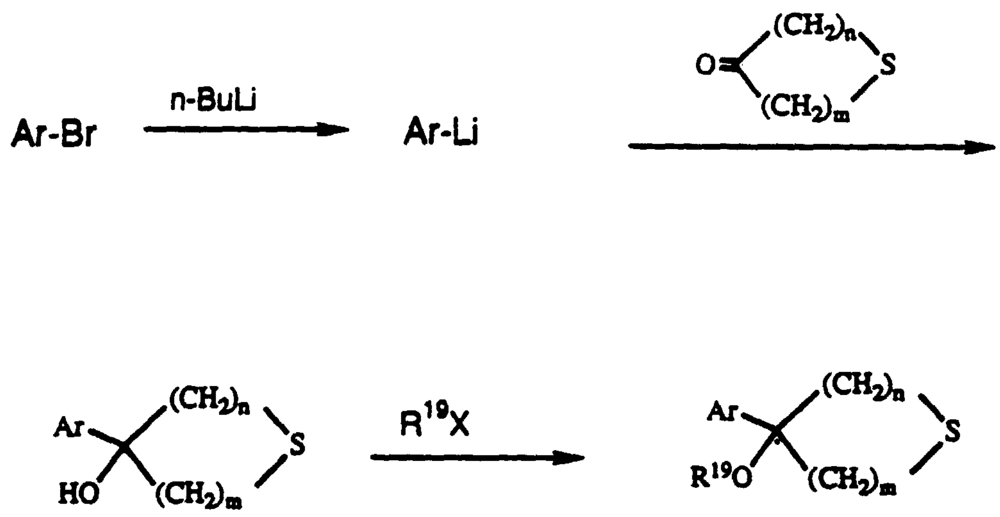
n-BuLi
$R^{19}X$

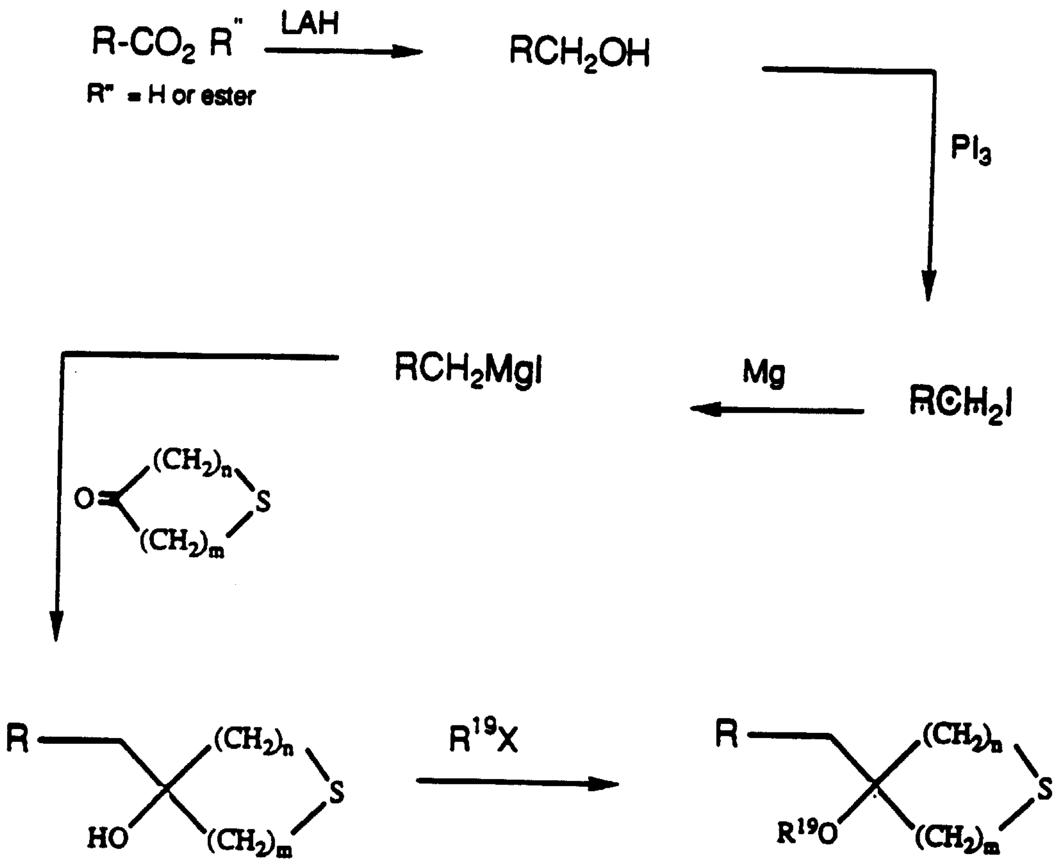


Quinolylmethoxy moieties can be prepared as described by Musser, et al., J. Med. Chem., 35, 2501-2524 (1992), and references cited therein, as illustrated below.

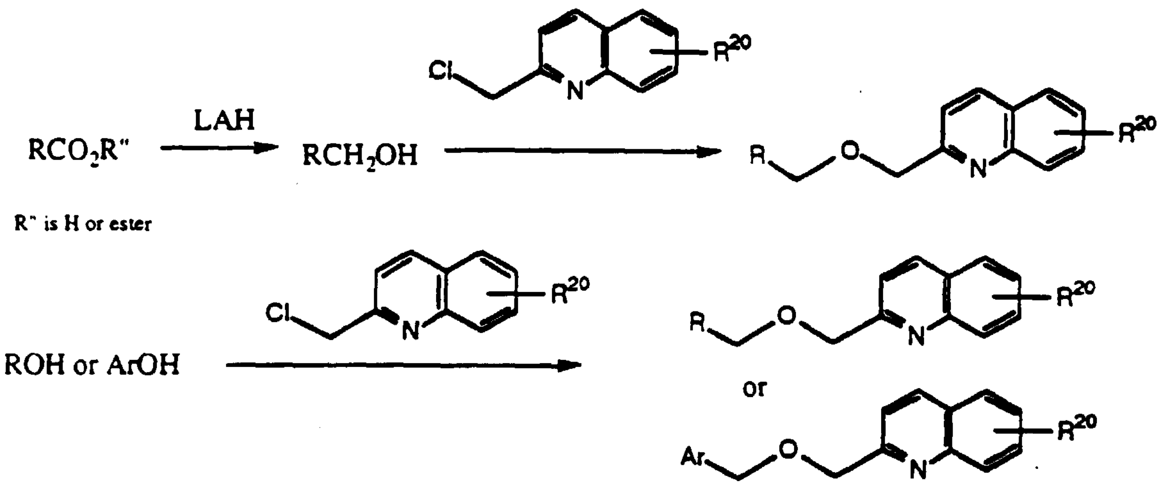


Methods for preparing the disclosed compounds are described in detail in the working examples below.

**Example 1: Preparation of *trans*-2-(3,4-dimethoxy-5-aminoethylthiophenyl)-5-(3,4,5-trimethoxyphenyl)-tetrahydrothiophene (1, Figure 2)**

**N,N-Dibenzyloxycarbonyl cystamine (compound 101, Figure 1).**
Cystamine dichloride (18 g, 79.93 mmole) was dissolved in 180 mL of 2N NaOH (14.4 g, 360 mmole). To this solution was added benzyl chloroformate (29.72g. 174.21 mmole) dropwise at 0°C. A precipitate formed, and the mixture was stirred for 2 hours at 0°C. The precipitate was collected, washed with water and methanol and recrystallized from $CHCl_3$-$CH_3OH$ to obtain a white crystalline solid (30.1 g, 89.6%). $^1H$ NMR ($CDCl_3$: 2.78 (t, 4H), 3.48 (q, 4H); 5.10 (s, 4H); 5.24 (bs, 2H); 7.34 (s, 10H).

**3,4-Dimethoxy-5-iodo-benzaldehyde (compound 102, Figure 1).** A mixture of 5-iodovanillin (7 g, 25.18 mmole), potassium carbonate (8.78 g, 63.53 mmole) and iodomethane (6.43 g, 45.30 mmole) was suspended in 60 mL of DMF and stirred at room temperature for 14 hours. The reaction mixture was quenched with water and extracted with diethyl ether. The organic layer was dried over $MgSO_4$, filtered and evaporated in vacuo to obtain a solid which was recrystallized from hexane/ethyl acetate (2:1) (6.28 g 85.4%).

**3,4-Dimethoxy-5-N-benzyloxycarbonylethylthiobenzaldehyde (compound 103, Figure 1).** A suspension of 3,4-dimethoxy-5-iodo-benzaldehyde (6.18 g. 21.16 mmole) and copper (11.55 g, 181.74 mmole) in 50 mL of DMF was heated at 140°C for 2 hours and N,N-dibenzyloxycarbonyl cystamine (14.19 g, 33.78 mmole) was added. The solution was heated at 140°C for 40 hours, filtered and the residue washed with ethyl acetate. The combined filtrate was evaporated to leave a solid which was recrystallized from ethyl acetate and hexane (7.05 g, 88.5%).

**3-(N,N-Dimethylamino)-1-(3,4,5-trimethoxyphenyl)-1-propanone (compound 104, Figure 1).** 3,4,5-Trimethoxyacetophenone (50 g, 237.8 mmole), paraformaldehyde (9.75 g, 304.7 mmole), dimethylamine hydrochloride (26.42 g, 324.0 mmole) and 5 mL conc. HCl were dissolved in 200 mL absolute ethanol and refluxed for 10 hours. Additional dimethylamine hydrochloride (13.21 g, 162.0 mmole) and paraformaldehyde (9.75 g. 304.7 mmole) were added and the solution returned to reflux. After 54 hours (total reaction time). 80 mL of 10% HCl and 500 mL of water were added and the solution was extracted with ethyl ether. The acidic aqueous layer was adjusted to pH 10 with 10% NaOH. The basic solution was extracted with ethyl acetate, dried over $MgSO_4$, filtered and evaporated in vacuo to provide 57.5 g of a yellow oil (92%). $^1H$ NMR ($CDCl_3$): 2.30 (s, 6H); 2.74 (t, 2H); 3.11 (t, 2H); 3.91 (s, 9H); 7.23 (s, 1H); 7.32 (s, 1H).

**3-(N,N,N-Trimethylamino-1-(3,4,5-trimethoxyphenyl)-1-propanone iodide (compound 105, Figure 1).**
3-(N,N-Dimethylamino-1-(3,4,5-trimethoxyphenyl)-1-propanone (57 g, 213.5 mmole) was dissolved in 200 mL of anhydrous diethyl ether. To this solution was added methyl iodide (57.6 g, 405.7 mmole). A white precipitate formed immediately, and the reaction stirred at room temperature for an additional 2 hours. The product was isolated by suction filtration (83.8 g, 96%).

**3,4,5-Trimethoxyphenylvinylketone (compound 106, Figure 1).**
3-(N,N,N-Trimethylamino-1-(3,4,5-trimethoxyphenyl)-1-propanone iodide (30 g, 73.3 mmole) and potassium carbonate (16.0 g, 115.9 mmole) were suspended in acetone (500 mL). The solution was stirred overnight at room temperature and then heated at reflux for 5 hours. The solution was then filtered, and evaporated to an oil which was purified by flash column chromatography using 1:1 hexane/ethyl acetate as solvent (9.2 g 56.4%). $^1H$ NMR ($CDCl_3$): 3.92 (s, 9H); 5.92 (d, 1H); 6.44 (d, 1H); 7.12 (m, 1H); 7.22 (s, 2H).

**1-(3,4-Dimethoxy-5-N-benzyloxycarbonylethylthiophenyl)-4-(3,4,5-trimethoxyphenyl)-1,4-butanedione (compound 107, Figure 2).**
Freshly prepared 3,4,5-trimethoxyphenylvinylketone (8.46 g, 38.11 mmole), 3,4-dimethoxy-5-N-benzyloxycarbonylethylthiobenzaldehyde (7.05 g, 18.8 mmole), 3-benzyl-5-(2-hydroxyethyl)-4-methyl-thiazolium chloride (2.0 g, 7.41 mmole) and 32 ml of trimethylamine were stirred at 65°C overnight. The reaction was quenched with water, acidified with 10% HCl and extracted with chloroform. The organic layer was washed with saturated aqueous sodium chloride, dried over $MgSO_4$, filtered and evaporated in vacuo to an oil that was purified by flashcolumn chromatography using 1:1 hexane/ethyl acetate as eluent (8.36g, 76.7%). $^1H$ NMR ($CDCl_3$): 3.10 (t, 2H); 3.45 (m, 6H); 3.90 (s, 3H); 3.93 (s, 9H); 3.94 (s, 3H); 5.08 (s, 2H); 5.30 (bt, 1H); 7.29 (s, 2H); 7.33 (s, 5H); 7.46 (d, 1H); 7.72 (d, 1H).

**1-(3,4-Dimethoxyphenyl-5-N-benzyloxy carbonylethylthiophenyl)-4-(3,4,5-trimethoxyphenyl)-1,4-butanediol (compound 109, Figure 2).**

1-(3,4-Dimethoxy-5-N-benzyloxycarbonylethylthiophenyl)-4-(3,4,5-trimethoxyphenyl)-1,4-butanedione (2.26 g, 5.16 mmole) was dissolved in 23 mL of THF and the solution was diluted with 36 mL of methanol. To this solution was added sodium borohydride (264.9 mg, 9.29 mmole) in 10 mL of water dropwise, and the solution stirred at room temperature for 2.5 hours. The reaction mixture was then cooled, quenched with water, and extracted with chloroform. The organic layer was dried over $MgSO_4$, filtered and evaporated in vacuo to provide 2.21 g of diol (97.6%).

**2-(3,4-Dimethoxy-5-N-benzyloxycarbonyl ethylthiophenyl)-5-(3,4,5-trimethoxyphenyl) tetrahydrothiophene (compound 111, Figure 2).** 1-(3,4-Dimethoxy-5-N-benzyloxycarbonylethylthiophenyl-4-(3,4,5-trimethoxyphenyl)-1,4-butanediol (2.66 g, 4.547 mmole) and 2.6 g of $P_4S_{10}$ were dissolved in 30 mL pyridine and heated at 90°C for 16 hours. The solvent was removed by distillation in vacuo and the residue was acidified with 10% HCl and extracted with dichloromethane. The organic layer was washed with 10% HCl, water and saturated aqueous sodium chloride, dried over $MgSO_4$, filtered and evaporated to give a gummy residue that was purified by flash column chromatography using 2:1 hexane/ethyl acetate as solvent and then by HPLC using hexane and ethyl acetate as solvent.

***trans*-2-(3,4-Dimethoxy-5-N-aminoethyl thiophenyl)-5-(3,4,5-trimethoxyphenyl)-tetrahydrothiophene (compound 1, Figure 2).** Compound 1 is prepared from 2-(3,4-dimethoxy-5-N-benzyloxycarbonylethylthiophenyl)-5-(3,4,5-trimethoxyphenyl)tetrahydrothiophene by treatment with KOH (5 equivalents) in ethylene glycol at 100°C for 24 hours. The mixture is quenched with water and extracted wit an organic solvent. The organic layer is dried, and evaporated to leave a solid that is purified by chromatography.

**Example 2: Preparation of *trans*-2-(3,4-Dimethoxy-5-aminoethylsulfonylphenyl)-5-(3,4,5-trimethoxyphenyl)tetrahydrothiophene (compound 2, Figure 2)**

**1-(3,4-Dimethoxy-5-N-benzyloxycarbonyl ethylsulfonylphenyl)-4-(3,4,5-trimethoxyphenyl)-1,4-butanedione (Compound 108, Figure 3).** A solution of magnesium monoperoxyphthalic acid (MMPP, 5.98 g, 9.68 mmole) in water (15 mL) was added to a solution of 1-(3,4-dimethoxy-5-N-benzyloxycarbonylethythiophenyl)-4-(3,4,5-trimethoxyphenyl)-1,4-butanedione (3 g, 5.16 mmole) in 40 mL of acetonitrile at room temperature. The solution was stirred at room temperature for 2 hours, and then water added, and the mixture extracted with dichloromethane. The organic layer was washed with 1N NaOH, water and then saturated aqueous NaCl, filtered and evaporated in vacuo to a solid that was recrystallized in ethyl acetate and hexane to provide 2.97 g of dione (93.7%).

**1-(3,4-Dimethoxy-5-N-benzyloxycarbonylethylsulfonylphenyl)-4-(3,4,5-trimethoxyphenyl)-1,4-butanediol (compound 110, Figure 2).**

1-(3,4-Dimethoxy-5-N-benzyloxycarbonylethylsulfonylphenyl)-1,4-butanedione (2.87 g, 4.68 mmole) was dissolved in 20 mL THF and the solution diluted with 32 mL methanol. To this solution was added sodium borohydride (318.8mg, 8.43 mmole) in 9 mL water dropwise, and the solution stirred at room temperature for 2.5 hours. The solution was cooled and quenched with water, the aqueous layer extracted with dichloromethane and the organic layer dried over MgSO4, filtered and evaporated in vacuo to provide 2.86 g of diol (99%).

***trans*-2-(3,4-Dimethoxy-5-N-benzyloxy carbonylethylsulfonylphenyl)-5-(3,4,5-trimethoxyphenyl) tetrahydrothiophene (compound 112, Figure 2).** 1-(3,4-Dimethoxy-5-N-benzyloxycarbonylethylsulfonylphenyl)-4-(3,4,5-trimethoxyphenyl)-1,4-butanediol (2.8g, 4.54 mmole) was dissolved in 25 mL pyridine and treated with 2.82 g of $P_4S_{10}$ and then heated at 90°C for 16 hours. The solvent was removed by distillation in vacuo, and the residue was acidified with 10% HCl and extracted with dichloromethane. The organic layer was washed with 10% HCl, water, and saturated sodium chloride solution, dried over MgSO4, filtered and then evaporated to give a gummy residue that was purified by flash column chromatography with 1:1 hexane:ethyl acetate, followed by HPLC using hexane and ethyl acetate as solvent (yield 34 mg). $^1H$ NMR ($CDCl_3$): 2.09 (m, 2H); 2.60 (m, 2H); 3.60 (m, 4H); 3.82 (s, 3H); 3.90 (s, 6H); 3.92 (s, 3H); 3.96 (s, 3H); 4.81 (m, 2H); 5.09 (s, 2H); 5.49 (bs, 1H); 6.70 (s, 2H); 7.34 (d, 1H); 7.35 (s, 5H); 7.56 (d, 1H).

***trans*-2-(3,4-Dimethoxy-5-aminoethyl sulfonylphenyl)-5-(3,4,5-trimethoxyphenyl)-tetrahydrothiophene (compound 2, Figure 2).**

*rans*-2-(3,4-Dimethoxy-5-N-benzyloxycarbonylethylsulfonyl-phenyl)-5-(3, 4,5-trimethoxyphenyl)-tetrahydrothiophene (32.6 mg, 0.052 mmole) was dissolved in 3 mL ethanol. To this solution was added 1.2 mL cyclohexene and 46.9 mg 10% Pd-C catalyst The mixture was refluxed for 2 hours, and then the catalyst removed by filtration. The catalyst was washed with ethanol, and the combined filtrate evaporated under reduced pressure to an oil that was purified by flash column chromatography using hexane/ethyl acetate (1:1) followed by chloroform/acetone (4:1) to provide 4.1 mg product $^1$H NMR ($CDCl_3$): 2.09 (m, 2H); 2.60 (m, 2H); 2.68 (t, 2H); 3.10 (bs, 2H); 3.45 (t, 2H); 3.82 (s, 3H); 3.89 (s, 6H); 3.93 (s, 3H); 3.97 (s, 3H); 4.81 (m, 2H); 6.70 (s, 2H); 7.30 (s, 1H); 7.50 (s, 1H).

**Example 3: Preparation of *trans*-2-(3-Methoxy-4-propoxy-5-N-benzylaminophenyl) -5-(3,4,5-tri-methoxyphenyl)-tetrahydrofuran (compound 3, Figure 4)**

**3-Methoxy-4-propoxy-5-nitrobenzaldehyde (compound 114, Figure 3).**
A mixture of sodium hydride (6.09 g, 152.3 mmole) in 20 mL of dry dimethylformamide (DMF) was cooled to 0°C and then 5-nitrovanillin (25 g, 126.8 mmole) in 41 mL of DMF was added dropwise. After 30 minutes, propyl iodide (25.87 g, 152.0 mmole) was added dropwise at 0°C. When the addition was completed, the reaction was maintained at room temperature for 2 hours and then stirred overnight at 70°C. Water was added, and the solution extracted with ethyl ether, washed with 10% NaOH, dried over $MgSO_4$, filtered and then evaporated in vacuo to an oil (19 g, 62.7%).

**1-(3-Methoxy-4-propoxy-5-nitrophenyl)-4-(3,4,5-trimethoxyphenyl)-1,4-butanedione (compound 116, Figure 3).** A solution of 3,4,5-trimethoxyphenylvinylketone (13.25 g, 59.64 mmol), 3-methoxy-4-propoxy-5-nitrobenzaldehyde (11.95, 50 mmol), 3-benzyl-5-(2-hydroxyethyl)-4-methylthiazolium chloride (5.36 g, 19.91 mmol) and triethylamine(50 mL) were stirred at 60°C overnight, and then quenched with water, acidified with 10% HCl and extracted with chloroform. The organic layer was washed with saturated aqueous sodium chloride, dried over magnesium sulfate, filtered and evaporated in vacuo to an yellow oil that was purified by flash column chromatography using 2:1 hexane/ethyl acetate as the solvent to yield a white solid (9.59 g, 41.6%). $^1$H NMR ($CDCl_3$): 1.07 (t, 3H), 1.86 (m, 2H), 3.40 (d, 4H), 3.88 (s, 3H), 3.92 (s, 9H), 4.04 (t, 2H), 7.29 (s, 2H), 7.54 (d, 1H), 8.07 (d, 1H).

**1-(3-Methoxy-4-propoxy-5-nitrophenyl)-4-(3,4,5-trimethoxyphenyl)-1,4-butanediol (compound 118, Figure 3).** To a solution of 1-(3-methoxy-4-propoxy-5-nitrophenyl)-4-(3,4,5-trimethoxyphenyl)-butane-1,4-dione (11.53 g, 25 mmol) in 350 mL of methanol and 250 mL of tetrahydrofuran was added dropwise a solution of sodium borohydride (3.076 g, 81.34 mmol) in 140 mL of water. After the addition was completed, the mixture was stirred at room temperature for 3 hours, quenched with water and extracted with dichloromethane. The organic layer was dried over magnesium sulfate, filtered and evaporated to provide a foam (11.53 g, 99.24%). $^1$H NMR ($CDCl_3$): 1.05 (t, 3H), 1.80-1.95 (m, 6H), 3.80-3.98 (m,12H), 4.1 (t, 2H), 4.72 (m, 2H), 6.6 (s, 2H), 7.15 (s, 1H), 7.35 (s, 1H).

**2-(3-Methoxy-4-propoxy-5-nitrophenyl)-5-(3,4,5-trimethoxyphenyl)-tetrahydrofuran (compound 119, Figure 3).** A suspension of $P_4S_{10}$ (19.25 g, 43.3 mmol) in 250 mL of pyridine was heated at 120°C for 75 minutes. To this suspension was then added a solution of 1-(3-methoxy -4-propoxy-5-nitrophenyl)-4-(3,4,5-trimethoxyphenyl)-butane-1,4-diol (4.65 g, 10 mmol) in 50 mL of pyridine. The temperature was reduced to 90°C and stirring continued for an additional 90 minutes. After cooling, ice and water were added, the mixture was extracted with methylene chloride. The organic layer was washed with water, 5% HCl, water, sodium bicarbonate and saturated aqueous sodium chloride, and then dried over magnesium sulfate, filtered and evaporated.

The mixture of *cis* and *trans* compounds were separated by HPLC using Water's prep Nova-Pak HR silica cartridge (9:1 hexane: ethyl acetate) to provide 300 mg (6.5%) *trans* and 250 mg (5.42%) of *cis* isomer. *Trans* isomer. $^1$H NMR ($CDCl_3$): 1.05 (t, 3H), 1.80-1.90 (m, 2H), 2.10-2.20 (m, 2H), 2.60-2.70 (m, 2H), 3.85-3.95 (m,12H), 4.10 (t, 2H), 4.85 (m, 2H), 6.75 (s, 2H), 7.20 (d, 1H), 7.45 (d, 1H). Cis isomer. $^1$H NMR ($CDCl_3$): 1.05 (t, 3H), 1.80-1.90 (m,2H), 2.10-2.20 (m, 2H). 2.60-2.70 (m, 2H), 3.85-3.95 (m, 12H), 4.10 (t, 2H), 4.70 (m, 2H), 6.75 (s, 2H), 7.20 (d, 1H), 7.45 (d, 1H).

***trans*-2-(3-Methoxy-4-propoxy-5-aminophenyl)-5-(3,4,5-trimethoxyphenyl) tetrahydrofuran (compound 122, Figure 3).**
*trans*-2-(3-Methoxy-4-propoxy-5-nitrophenyl)-5-(3,4,5 trimethoxyphenyl)-tetrahydrofuran (2.235g, 5 mmol) was dissolved in 45 mL of absolute ethanol. To this solution was added calcium chloride (0.5 g, 5 mmol) in 10 mL of water followed by freshly activated zinc dust (7.5 g). The mixture was refluxed for 12 hours, and the solid then removed by vacuum filtration through Celite. The filtrate was washed with water, dried over magnesium sulfate, and

evaporated in vacuo to a white foam (2.085 g, 97.28%).

*trans* isomer. $^1$H NMR: ($CDCl_3$): 1.10 (t, 3H), 1.80-1.90 (m, 2H), 2.00-2.15 (m, 2H), 2.50-2.60 (m, 2H), 3.80-3.97 (m, 12H), 4.10 (t, 2H), 5.20-5.35 (m, 2H), 6.45 (s, 1H), 6.55 (s, 1H), 6.75 (s, 2H).

The *cis* product can be obtained in the same manner as described above usingcis-3-(3,4,5-trimethoxyphenyl)-2-(3-methoxy-4-propoxy-5-nitrophenyl)-tetrahydrofuran as the starting material.

*Cis* isomer. $^1$H NMR: ($CDCl_3$): 1.10 (t, 3H), 1.80-1.90 (m, 2H), 2.00-2.15 (m, 2H), 2.50-2.60 (m, 2H), 3.80-3.97 (m, 12H), 4.10 (t, 2H), 5.10-5.20 (m, 2H), 6.45 (s, 1H), 6.55 (s, 1H), 6.75 (s, 2H).

***trans*-2-(3-Methoxy-4-propoxy-5-benzylaminophenyl)-5-(3,4,5-trimethoxyphenyl)-tetrahydrofuran (compound 3, Figure 4).**

*rans*-2-(3-Methoxy-4-propoxy-5-nitrophenyl)-5-(3,4,5-trimethoxyphenyl)-tetrahydrofuran (50 mg, 0.12 mmole) and potassium carbonate (1.0 g) were suspended in 2 mL of DMF. Benzyl bromide (205.1 mg, 1.2 mmole) was added and the suspension was stared at room temperature for 20 hours. The reaction was quenched with water and extracted with dichloromethane. The organic layer was dried over $MgSO_4$, filtered and evaporated in vacuo to an oil that was purified by column chromatography with hexane/ethyl acetate solvent (yield 50.2 mg, 82.6%). $^1$H NMR ($CDCl_3$): 1.05 (t, 3H); 1.83 (m, 4H); 2.32 (m, 2H); 3.85 (s, 3H); 3.90 (s, 6H); 3.91 (s, 3H); 4.05 (t, 2H); 4.28 (d, 2H); 4.67 (bs, 1H); 5.08 (m, 2H); 6.42 (s, 1H); 6.57 (s, 2H); 6.65 (s, 1H); 7.20 (m, 5H).

**Example 4: Preparation of *trans*-2-(3-Methoxy-4-propoxy-5-hydroxyethylaminophenyl)-5-(3,4,5-trimethoxyphenyl)-tetrahydrofuran (compound 4, Figure 4)**

A mixture of *trans*-2-(3-methoxy-4-propoxy-5-aminophenyl)-5-(3,4,5-trimethoxyphenyl) tetrahydrofuran (20 mg, 0.048 mmol), potassium carbonate (0.4g), and 2-iodoethanol (82.47 mg, 0.48 mmol) was suspended in 2 mL of DMF. The reaction mixture was stirred at room temperature for 20 hours, quenched with water and extracted with dichloromethane. The organic layer was dried over magnesium sulfate, filtered and evaporated in vacuo to an oil which was purified by column using 3:1 hexane/ethyl acetate as solvent (12.8 mg, 57.9%). $^1$H NMR ($CDCl_3$): 1.02 (t, 3H), 1.79 (m, 2H), 1.99 (m, 2H), 2.43 (m, 2H), 3.33 (m, 2H), 3.81 (m, 2H), 3.82 (s, 3H), 3.83 (s, 3H), 3.88 (s, 6H), 3.90 (t, 2H), 5.18 (m, 2H), 6.40 (d, 2H), 6.61 (s, 2H).

**Example 5: Preparation of *trans*-2-(3-Methoxy-4-propoxy-5-N,N-diallylphenyl)-5-(3,4,5-trimethoxyphenyl)-tetrahydrofuran (compound 5, Figure 4)**

A mixture of *trans*-2-(3-methoxy-4-propoxy-5-aminophenyl-5-(3,4,5-trimethoxyphenyl) tetrahydrofuran (20 mg, 0.048 mmol), potassium carbonate (0.4 g), and 2-allyliodide (80.56 mg, 0.48 mmol) was suspended in 2 mL of DMF. The reaction mixture was stirred at room temperature for 20 hours, and then quenched with water and extracted with dichloromethane. The organic layer was dried over magnesium sulfate, filtered and evaporated in vacuo to an oil which was purified by column using 4:1 hexane/ethyl acetate as solvent (5.1 mg, 21.4%). $^1$H NMR ($CDCl_3$): 1.02 (t, 3H),1.79 (m, 2H), 1.99 (m, 2H), 2.43 (m, 2H), 3.81 (m, 2H), 3.85 (s, 3H), 3.87 (s, 6H), 3.89 (s, 3H), 3.90 (m, 2H), 5.10-5.20 (m, 6H), 5.80 (m, 2H), 6.57 (s, 1H), 6.63 (s, 2H), 6.63 (s, 1H).

**Example 6: Preparation of *trans*-2-(3,4-Dimethoxy-5-N-benzylaminophenyl)-5-(3,4,5-trimethoxyphenyl)-tetrahydrothiophene (compound 6, Figure 4)**

**3,4-Dimethoxy-5-nitrobenzaldehyde (compound 113, Figure 3).** A suspension of sodium hydride (4.87 g, 121.7 mmole) in 18 mL dry DMF was cooled to 0°C and then 5-nitrovanillin (20 g, 101.4 mmole) in 30 mL of DMF was added dropwise. After 30 minutes, methyl iodide (43.18 g, 304.2 mmole) was added dropwise at 0°C. When the addition was completed, the mixture was warmed to room temperature and stirred overnight. Water was added and the solution extracted with ethyl ether, the organic layer was washed with 10% NaOH solution, dried over $MgSO_4$, filtered and evaporated in vacuo to an oil (8.7 g, 40.7%).

**1-(3,4-Dimethoxy-5-nitrophenyl)-4-(3,4,5-trimethoxyphenyl)-1,4-butanedione (compound 115, Figure 3).** A mixture of freshly prepared 3,4,5-trimethoxy-phenylvinylketone (7.12 g, 32.08 mmole), 3,4-dimethoxy-5-nitrobenzaldehyde (5.64g, 26.73 mmole), 3-benzyl-5-(2-hydroxyethyl)-4-methylthiazolium chloride (2.79 g, 10.69 mmole) and triethylamine (28 mL) were stirred at 65°C overnight. The reaction was quenched with water, acidified with 10% HCl and extracted with dichloromethane. The organic layer was washed with saturated aqueous sodium chloride,

dried over MgSO4, filtered and evaporated to an oil which was purified by flash column chromatography using 1:1 hexane and ethyl acetate as eluent (4.71 g, 40.7%).

**1-(3,4-Dimethoxy-5-nitrophenyl)-4-(3,4,5-trimethoxyphenyl)-1,4-butanediol (compound 117, Figure 3).**
1-(3,4-Dimethoxy-5-nitrophenyl)-4-(3,4,5-trimethoxyphenyl)-1,4-butanedione (4.65 g, 10.74 mmole) was dissolved in 40 mL THF and the solution was diluted with 75 mL of methanol, To this solution sodium borohydride (0.73 g, 19.33 mmole) in 20 mL of water was added dropwise. The solution was stirred at room temperature for 2.5 hours, and the reaction mixture cooled, quenched with water, and the aqueous layer extracted with dichloromethane. The organic layer was dried over $MgSO_4$, filtered and evaporated in vacuo to provide 4.61 g of product (98.3%).

**2-(3,4-Dimethoxy-5-nitrophenyl)-5-(3,4,5-trimethoxyphenyl)-tetrahydrothiophene (compound 120, Figure 3).**
1-(3,4-Dimethoxy-5-nitrophenyl)-4-(3,4,5-trimethoxyphenyl)-1,4-butanediol (4.61 g, 10.55 mmole) and 6.57 g of $P_4S_{10}$ were suspended in 60 mL of pyridine and heated at 90°C for 16 hours. The solvent was removed by distillation in vacuo. The residue was acidified with 10% HCl and extracted with dichloromethane. The organic layer was washedwith 10% HCl, water and saturated aqueous sodium chloride, dried over $MgSO_4$, filtered and evaporated to give a gummy residue that was purified by flash column chromatography using 3:1 hexane/ethyl acetate as the solvent (335.6 mg, 7.3%). $^1$H NMR ($CDCl_3$): 2.08 (m, 2H); 2.60 (m, 2H); 3.82 (s, 3H); 3.90 (s, 6H); 3.95 (s, 3H); 3.98 (s, 6H); 4.81 (m, 2H); 6.70 (s, 2H); 7.22 (d, 1H); 7.44 (d, 1H).

***trans*-2-(3,4-Dimethoxy-5-aminophenyl)-5-(3,4,5-trimethoxyphenyl)-tetrahydrothiophene (compound 123, Figure 3).**
*rans*-2-(3,4-Dimethoxy-5-nitrophenyl)-5-(3,4,5-trimethoxyphenyl)-tetrahydrothiophene (0.3 g, 0.69 mmole) was suspended in 10 ml of ethanol. To this was added CaCl2 (72.63 mg, 0.65 mmole) in 2 mL water, followed by zinc metal (1.01 g). The suspension was refluxed for 5 hours, filtered, and the residue was washed with ethanol. The combined filtrate was washed with 10% $NaHCO_3$, water and saturated aqueous sodium chloride, dried over MgSO4 and evaporated in vacuo to an oil that was purified by flash column chromatography using 1:1 hexane/ethyl acetate as solvent (30 mg, 10.8%). $^1$H NMR ($CDCl_3$): 2.10 (m, 2H); 2.57 (m, 2H); 3.81 (s, 3H); 3.83 (s, 3H); 3.87 (s, 3H); 3.89 (s, 6H); 4.80 (m, 2H); 6.45 (d, 1H); 6.51 (d, 1H); 6.70 (s, 2H).

***trans*-2-(3,4-Dimethoxy-5-benzylaminophenyl)-5-(3,4,5-trimethoxyphenyl)-tetrahydrothiophene (compound 6, Figure 4).**
*rans*-2-(3,4-Dimethoxy-5-aminophenyl)-5-(3,4,5-trimethoxyphenyl)-tetrahydrofuran (10 mg, 0.025 mmole) and potassium carbonate (0.2g) were suspended in 1 mL of DMF. To this solution was added benzyl bromide (42.23 mg, 0.25 mmole). The mixture was stirred at room temperature for 20 hours, quenched with water and then extracted with dichloromethane. The organic layer was dried over $MgSO_4$ filtered and evaporated in vacuo to an oil that was purified by column chromatography using hexane/ethyl acetate (3:1).

**Example 7: Preparation of *rans*-2-(3-Methoxy-4-propoxy-5-benzylaminophenyl) -5-(3,4,5-trimethoxyphenyl) tetrahydrothiophene (Compound 7, Figure 4)**

3-Methoxy-4-propoxy-5-nitrobenzaldehyde (compound 114, Figure 4), 1-(3-methoxy-4-propoxy-5-nitrophenyl)-4-(3,4,5-trimethoxyphenyl)-1,4-butanedione (compound 116, Figure 4), and 1-(3-methoxy-4-propoxy-5-nitrophenyl)-4-(3,4,5-trimethoxyphenyl)-1,4-butanediol (compound 118, Figure 4) were prepared as described in Example 3.

**2-(3-Methoxy-4-propoxy-5-nitrophenyl)-5-(3,4,5-trimethoxyphenyl)-tetrahydrothiophene (compound 121, Figure 3).**
Compound 121 was prepared in the same way as compound 120 (Example 6), using compound 118 as the starting material.

***trans*-2-(3-Methoxy-4-propoxy-5-aminophenyl)-5-(3,4,5-trimethoxyphenyl)-tetrahydrothiophene (compound 124, Figure 3).**
Compound 124 was prepared in the same way as compound 123 (Example 6), using compound 121 as the starting material.

***trans*-2-(3-Methoxy-4-propoxy-5-benzylaminophenyl)-5-(3,4,5-trimethoxyphenyl)-tetrahydrothiophene (Compound 7, Figure 4).** *trans*-2-(3-Methoxy-4-propoxy-5-aminophenyl)-5-(3,4,5-trimethoxyphenyl)-tetrahydrothiophene (10 mg, 0.023 mmole) and potassium carbonate (0.2 g) were suspended in 1 mL DMF. To this mixture

was added benzyl bromide (39.50 mg, 0.23 mmole). The mixture was stirred at room temperature for 20 hours, quenched with water, and then extracted with dichloromethane. The organic layer was dried over $MgSO_4$, filtered and evaporated in vacuo to an oil that was purified by column chromatography using hexane/ethyl acetate (3:1) as solvent.

**Example 8: *trans*-2-(3,4-Dimethoxy-5-N,N-dihydroxyethylaminophenyl)-5-(3,4,5-tri methoxyphenyl)-tetrahydrothiophene (compound 8, Figure 4)**

Compound 8 was prepared by the method described above for compound 4, using compound 123 as the starting material.

**Example 9: *trans*-2-(3-Methoxy-4-propoxy-5-N-hydroxyethylaminophenyl)-5-(3,4,5-trimethoxyphenyl)-tetrahydrothiophene (Compound 9, Figure 4)**

Compound 9 was prepared in the same manner as compound 4, described above, using compound 122 as the starting material.

**Example 10: *trans* -2-(3,4-Dimethoxy-5-N,N-diallylaminophenyl)-5-(3,4,5-trimethoxyphenyl)-tetrahydrothiophene (Compound 10, Figure 5)**

Compound 10 was prepared by the method described above for compound 5, using compound 123 as the starting material.

**Example 11: Diallylaminophenyl)-5-(3,4,5-trimethoxyphenyl)-tetrahydrothiophene (Compound 11, Figure 4)**

Compound 11 was prepared in the same manner as compound 5, using compound 124 as the starring material.

**Example 12: *cis*- and *trans*-2-[N'-hydroxyl-N'-(substituted)]-N-[2-propoxy-3-methoxy-5-{5-(3,4,5-trimethoxyphenyl)-(tetrahydrofuran or tetrahydrothiophene)-phenyl]-urea (compounds 12-32 and 38-41, Figure 4).**

**3-Methoxy-4-propoxy-5-nitrobenzaldehyde (compound 114, Figure 3).** A mixture of 5-nitrovanillin (19.72 g, 100 mmol), potassium carbonate (35 g, 253.23 mmol) and propyl iodide (32.86 mL, 335.83 mmol) in 160 mL of N,N-dimethylformamide was stirred at 60°C for 12 hours. The mixture was then cooled, quenched with waxer and extracted with methylene chloride. The organic layer was washed several times with water, dried over magnesium sulfate, filtered and evaporated in vacuo to an oil which was purified by flash chromatography on silica gel (230-400 mesh) using 2:1 hexane/ethyl acetate as eluent, to provide 3-methoxy-4-propoxy-5-nitrobenzaldehyde (13.54 g, 57%). $^1H$ NMR ($CDCl_3$): 1.00 (t, 3H), 1.85 (m, 2H), 4.00 (s, 3H), 4.25 (t, 2H), 7.65 (s, 1H), 7.85 (s, 1H), 9.95 (s, 1H).

Compounds 122 and 124 are prepared as described above.

***cis* and *trans* 2-[N'-Hydroxyl]-N-(2-propoxy-methoxy-5-{5-(3,4,5-trimethoxyphenyl)-tetrahydrofuran}-phenyl]urea (compound 12, Figure 4).** *rans*-2-(3-Methoxy-4-propoxy-5-aminophenyl)-5-(3,4,5-trimethoxyphenyl)-tetrahydrofuran (41.7 mg, 0.1 mmol), triethylamine (20 mL), and triphosgene (10 mg, 0.034 mmol) in 5 mL of dry dichloromethane were refluxed for 2 hours under an argon atmosphere. The reaction was monitored with thin layer chromatography. On indication that the amine had been converted to isocyanate, the reaction was cooled to room temperature and hydroxylamine hydrochloride (10.5 mg, 0.15 mmol) in 0.5 mL of THF, 29 mL of triethylamine, and 0.1 mL of water added. The solution was stirred at room temperature overnight under an argon atmosphere. The solvent was then removed in vacuo, and the resulting oil dissolved in dichloromethane. The organic layer was washed with water, dried over magnesium sulfate, filtered and concentrated to an oil in vacuo. The oil was purified by flash chromatography on silica gel with 1:1.5 hexane/ethyl acetate as eluent (38 mg, 79.83%). $^1H$ NMR ($CDCl_3$): 1.00 (t, 3H),1.75 (m, 2H), 2.00 (m, 2H), 2.50 (m, 2H), 3.80-4.00 (m,14H), 5.20 (m, 2H), 6.60 (s, 2H), 6.75 (s, 1H), 7.40 (s, 1H), 7.85 (br, 1H), 8.50 (s, 1H).

***cis* and *trans*-2-(3-Methoxy-4-propoxy-5-nitrophenyl)-5-(3,4,5 trimethoxyphenyl)tetrahydrothiophene (Compound 119, Figure 3).**

Compound 119 was prepared as described in Example 3.

The nitro group in compound 119 can be reduced to provide the corresponding amine (compound 122, Figure 3) in a manner similar to that described above for the reduction of the nitro group in compound 121 to provide compound 124.

A wide variety of hydroxy urea derivatives of 2,5-diaryl tetrahydrofurans and tetrahydrothiophenes can be obtained using the general procedure set out above for the preparation of compound 12, by reaction of the appropriately substituted hydroxy amine with a 2,5-diaryl tetrahydrofuran or tetrahydrothiophene that has an appropriately placed amine function. The following compounds were obtained using this general method.

***trans*-2-[N'-Hydroxyl-N'-methyl]-N-2-propoxy-3-methoxy-5-[5-(3,4,5-trimethoxyphenyl)-tetrahydrofuran] phenyl urea (Compound 13, Figure 4).** $^1$H NMR ($CDCl_3$):1.00 (t, 3H),1.75 (m, 2H), 2.00 (m,2H), 2.50 (m, 2H), 3.2 (s, 3H), 3.80-4.00 (m, 14H), 5.20 (m, 2H), 6.60 (s, 2H), 6.75 (s, 1H), 7.40 (s, 1H), 7.85 (s, H), 8.50 (s, 1H).

***trans*-2-[N'-Hydroxyl-N'-isopropyl]-N-2-propoxy-3-methoxy-5-[5-(3,4,5-trimethoxyphenyl)-tetrahydrofuran] phenylurea (compound 14, Figure 4).** $^1$H NMR ($CDCl_3$): 1.00 (t, 3H), 1.2 (d, 6H), 1.75 (m, 2H), 2.00 (m, 2H), 2.50 (m, 2H), 3.80-4.00 (m, 14H), 4.6 (m, 1H), 5.20 (m,2H), 6.60 (s, 2H), 6.75 (s, 1H), 6.77 (s, 1H), 7.90 (s, 1H), 8.50 (s, 1H).

***trans* -2-[5-(N'-t- Butyl-N'-hydroxyureidyl)-3-methoxy-4-propoxyphenyl]-5-(3,4,5- trimethoxyphenyl) tetrahydrofuran (compound 15, Figure 4).** $^1$H NMR ($CDCl_3$):1.00 (t, 3H), 1.4 (s, 9H), 1.75 (m, 2H), 2.00 (m, 2H), 2.50 (m, 2H), 3.80-4.00 (m,14H), 5.20 (m,2H), 6.60 (s, 2H), 6.75 (s, 1H), 6.77 (s, 1H), 7.90 (s, 1H), 8.50 (s, 1H).

***trans* -2-[5-(N'-Cyclohexyl-N'-hydroxyureidyl)-3-methoxy-4-propoxyphenyl]-5-(3,4,5- trimethoxyphenyl) tetrahydrofuran (compound 16, Figure 4).** $^1$H NMR ($CDCl_3$):1.00 (t, 3H),1.30 (m, 2H), 1.6 (m,4H), 1.75 (m, 5H), 2.00 (m,3H), 2.50 (m, 2H), 3.80-4.00 (m,14H), 5.20 (m,2H), 6.60 (s, 2H), 6.75 (s, 1H), 6.77 (s, 1H), 7.85 (s, 1H), 8.50 (s, 1H).

***trans*-2-[5-(N'-Benzyl-N'-hydroxyureidyl)-3-methoxy-4-propoxyphenyl]-5-(3,4,5- trimethoxyphenyl) tetrahydrofuran (compound 17, Figure 4).** $^1$H NMR ($CDCl_3$):1.00 (t, 3H),1.75 (m, 2H), 2.00 (m, 2H), 2.50 (m, 2H), 3.80-4.00 (m,14H), 4.7 (s, 2H), 5.20 (m,2H), 6.60 (s, 2H), 6.75 (s, 1H), 7.2-7.5 (m, 6H), 7.90 (s, 1H), 8.50 (s, 1H).

***cis* -2-[5-(N'-Hydroxyureidyl)-3-methoxy-4-propoxyphenyl]- 5-(3,4,5- trimethoxyphenyl) tetrahydrofuran (compound 18, Figure 4).** $^1$H NMR ($CDCl_3$):1.00 (t, 3H),1.75 (m,2H), 2.00 (m,2H), 2.50 (m, 2H), 3.80-4.00 (m,14H), 5.00 (m,2H), 6.60 (s, 2H), 6.70 (s, 1H), 7.40 (s, 1H), 7.85 (s, H), 8.50 (s, 1H).

***cis* -2-[ 5-(N'-Hydroxy-N'-methylureidyl)-3-methoxy-4-propoxyphenyl]-5-(3,4,5- trimethoxyphenyl) tetrahydrofuran (compound 19, Figure 4).** $^1$H NMR ($CDCl_3$):1.00 (t, 3H),1.75 (m, 2H), 2.00 (m, 2H), 2.50 (m, 2H), 3.2 (s, 3H), 3.80-4.00 (m, 14H), 5.00 (m, 2H), 6.60 (s, 2H), 6.70 (s, 1H), 7.70 (s, 1H), 7.85 (br, H), 8.50 (s, 1H).

***cis* -2-[5-(N'-Hydroxy-N'-i-propylureidyl)-3-methoxy-4-propoxyphenyl]-5-(3,4,5- trimethoxyphenyl) tetrahydrofuran (compound 20, Figure 4).** $^1$H NMR ($CDCl_3$):1.00 (t, 3H), 1.2 (d, 6H), 1.75 (m, 2H), 2.00 (m, 2H), 2.50 (m, 2H), 3.80-4.00 (m,14H), 4.6 (m, 1H), 5.00 (m,2H), 6.60 (s, 2H), 6.75 (s, 1H), 6.77 (s, 1H), 7.90 (s, 1H), 8.50 (s, 1H).

***cis* -2-[5-(N'-tert-Butyl-N'-hydroxyureidyl)-3-methoxy-4-propoxyphenyl]-5-(3,4,5- trimethoxyphenyl) tetrahydrofuran (compound 21, Figure 4).** $^1$H NMR ($CDCl_3$):1.00 (t, 3H), 1.4 (s, 9H), 1.75 (m, 2H), 2.00 (m, 2H), 2.50 (m, 2H), 3.80-4.00 (m,14H), 5.00 (m,2H), 6.60 (s, 2H), 6.75 (s, 1H), 6.77 (s, 1H), 7.90 (s, 1H), 8.50 (s, 1H).

***cis* -2-[5-(N'-Cyclohexyl-N'-hydroxyureidyl)-3-methoxy-4-propoxyphenyl]-5-(3,4,5- trimethoxyphenyl) tetrahydrofuran (compound 22, Figure 4).** $^1$H NMR ($CDCl_3$):1.00 (t, 3H),1.30 (m, 2H), 1.6 (m,4H), 1.75 (m, 5H), 2.00 (m,3H), 2.50 (m, 2H), 3.80-4.00 (m,14H), 5.00 (m,2H), 6.60 (s, 2H), 6.75 (s, 1H), 6.77 (s, 1H), 7.85 (s, 1H), 8.50 (s, 1H).

***cis* -2-[5-(N'-Benzyl-N'-hydroxyureidyl)-3-methoxy-4-propoxyphenyl]-5-(3,4,5- trimethoxyphenyl) tetrahydrofuran (compound 23, Figure 4).** $^1$H NMR ($CDCl_3$):1.00 (t, 3H),1.75 (m, 2H), 2.00 (m, 2H), 2.50 (m, 2H), 3.80-4.00 (m,14H), 4.7 (s, 2H), 5.0 (m,2H), 6.60 (s, 2H), 6.70 (s, 1H), 7.2-7.5 (m, 6H), 8.00 (s, 1H), 8.50 (s, 1H).

***trans* -2-[5-(N'-Hydroxyureidyl)-3-methoxy-4-propoxyphenyl]-5-(3,4,5- trimethoxyphenyl) tetrahydrothi-**

**ophene (compound 24, Figure 4).** $^1H$ NMR ($CDCl_3$):1.00 (t, 3H),1.80 (m,2H), 2.12 (m,2H), 2.60 (m, 2H), 3.80-4.00 (m,12H), 4.01 t, 2H), 4.75 (m,2H), 6.60 (s, 2H), 6.70 (s, 1H), 7.40 (s, 1H), 7.85 (s, H), 8.50 (s, 1H).

***trans*** **-2-[5-(N'-Hydroxy-N'-methylureidyl)-3-methoxy-4-propoxyphenyl]-5-(3,4,5- trimethoxyphenyl) tetrahydrothiophene (compound 25, Figure 4).** $^1H$ NMR ($CDCl_3$):1.00 (t, 3H),1.75 (m,2H), 2.00 (m,2H), 2.50 (m, 2H), 3.20 (s, 3H), 3.80-4.00 (m,12H), 4.1 (t, 2H), 4.80 (m,2H), 6.60 (s, 2H), 6.70 (s, 1H), 7.70 (s, 1H), 7.85 (s, 1H), 8.50 (s, 1H).

***trans*** **-2-[5-(N'-Hydroxy-N'-i-propylylureidyl)-3-methoxy-4-propoxyphenyl]-5-(3,4,5- trimethoxyphenyl) tetrahydrothiophene (compound 26, Figure 4).** $^1H$ NMR ($CDCl_3$):1.00 (t, 3H), 1.2 (d, 6H), 1.75 (m, 2H), 2.00 (m, 2H), 2.50 (m, 2H), 3.80-4.00 (m,12 H), 4.1 (t, 2H), 4.60 (m, 1H), 4.8 (m, 2H), 6.60 (s, 2H), 6.75 (s, 1H), 6.77 (s, 1H), 7.90 (s, 1H), 8.50 (s, 1H).

***trans*-2-(N'-Hydroxyl-N'-tert-butyl-N-(2-propoxy-3-methoxy-5-(3,4,5-trimethoxyphenyl)-tetrahydrothiophene) phenyl urea (compound 27, Table 2).** $^1H$ NMR ($CDCl_3$):1.00 (t, 3H), 1.4 (s, 9H), 1.75 (m,2H), 2.00 (m,2H), 2.50 (m, 2H), 3.80-4.00 (m,12H), 4.1 (t, 2H), 4.8 (m, 2H), 6.60 (s, 2H), 6.75 (s, 1H), 7.40 (s, 1H), 7.85 (s, H), 8.50 (s, 1H).

***cis*** **-2-[5-(N'-Hydroxyureidyl)-3-methoxy-4-propoxyphenyl]-5-(3,4,5- trimethoxyphenyl) tetrahydrothiophene (compound 28, Figure 4).** $^1H$ NMR ($CDCl_3$):1.00 (t, 3H), 1.75 (m, 2H), 2.00 (m, 2H), 2.50 (m, 2H), 3.20 (s, 3H), 3.80-4.00 (m, 12H), 4.1 (t, 2H), 4.65 (m, 2H), 6.60 (s, 2H), 6.70 (s, 1H), 7.70 (s, 1H), 7.85 (s. 1H), 8.50 (s, 1H).

***cis*-2-[5-(N'-Hydroxy-N'-methylureidyl)-3-methoxy-4-propoxyphenyl]-5-(3,4,5-trimethoxyphenyl) tetrahydrothiophene (compound 29, Figure 4).** $^1H$ NMR ($CDCl_3$):1.00 (t, 3H),1.75 (m,2H), 2.00 (m,2H), 2.50 (m, 2H), 3.20 (s, 3H), 3.80-4.00 (m,12H); 4.1 (t, 2H), 4.65 (m,2H), 6.60 (s, 2H), 6.70 (s, 1H), 7.70 (s, 1H), 7.85 (s, 1H), 8.50 (s, 1H).

***cs-*** **2-(N'-Hydroxyl-N'-isopropyl-N- (2-propoxy-3-methoxy-5-5-(3,4,5-trimethoxyphenyl) tetrahydrothiophene) phenyl urea (compound 30, Table 2).** $^1H$ NMR ($CDCl_3$):1.00 (t, 3H), 1.2 (d, 6H), 1.75 (m, 2H), 2.00 (m, 2H), 2.50 (m, 2H), 3.80-4.00 (m, 14H), 4.60 (m, 1H), 4.65 (m,2H), 6.60 (s, 2H), 6.70 (s, 1H), 7.40 (s, 1H), 7.85 (s, 1H), 8.50 (s, 1H).

***trans-*** **2-(N'-hydroxyl-N'-ethyl-N-(2-propoxy-3-methoxy-5- (5-(3,4,5-trimethoxyphenyl) tetrahydrofuran) phenyl urea (compound 38, Table 2).** $^1H$ NMR ($CDCl_3$):1.00 (t, 3H), 1.2 (t, 3H), 1.75 (m, 2H), 2.00 (m, 2H), 2.50 (m, 2H), 3.75 (q, 2H), 3.80-4.00 (m,14H), 5.20 (m, 2H), 6.60 (s, 2H), 6.75 (s, 1H), 6.77 (s, 1H), 7.90 (s, 1H), 8.50 (s, 1H).

***trans*** **-2-(N'-hydroxyl-N'-n-butyl-N-( 2-propoxy-3-methoxy -5-(5-(3,4,5-trlmethoxyphenyl) tetrahydrofuran) phenyl urea (compound 39, Table 2).** $^1H$ NMR ($CDCl_3$):1.00 (t, 3H), 1.2 (t, 3H), 1.5 (m, 2H), 1.75 (m, 2H), 1.8 (m, 2H), 2.00 (m, 2H), 2.50 (m, 2H), 3.70 (t, 2H), 3.80-4.00 (m,14H), 5.20 (m,2H), 6.60 (s, 2H), 6.75 (s, 1H), 6.77 (s, 1H), 7.90 (s, 1H), 8.50 (s, 1H).

***cis-*** **2-(N'-Hydroxyl-N'-isoprpyl-N- (2-propoxy-3-methoxy-5-5-(3,4,5-trimethoxyphenyl) tetrahydrothiophene) phenyl urea (compound 30, Table 2).** $^1H$ NMR ($CDCl_3$):1.00 (t, 3H), 1.2 (d, 6H), 1.75 (m, 2H), 2.00 (m, 2H), 2.50 (m, 2H), 3.80-4.00 (m, 14H), 4.60 (m, 1H), 4.65 (m,2H), 6.60 (s, 2H), 6.70 (s, 1H), 7.40 (s, 1H), 7.85 (s, 1H), 8.50 (s, 1H).

***cis-*** **2-(N'-Hydroxyl-N'-isoprpyl-N- (2-propoxy-3-methoxy-5-5-(3,4,5-trimethoxyphenyl) tetrahydrothiophene) phenyl urea (compound 30, Table 2)** $^1H$ NMR ($CDCl_3$):1.00 (t, 3H), 1.2 (d, 6H), 1.75 (m, 2H), 2.00 (m, 2H), 2.50 (m, 2H), 3.80-4.00 (m, 14H), 4.60 (m, 1H), 4.65 (m,2H), 6.60 (s, 2H), 6.70 (s, 1H), 7.40 (s, 1H), 7.85 (s, 1H), 8.50 (s, 1H).

***cis*** **-2-(N'-Hydroxyl-N'-tert-butyl-N-(2-propoxy-3-methoxy-5-2-(3,4,5-trimethoxyphenyl) tetrahydrothiophene) phenyl urea (compound 31, Table 2).** $^1H$ NMR ($CDCl_3$):1.00 (t, 3H), 1.4 (s, 9H), 1.75 (m,2H), 2.00 (m,2H), 2.50 (m, 2H), 3.80-4.00 (m,12H), 4.1 (t, 2H), 4.65 (m, 2H), 6.60 (s, 2H), 6.75 (s, 1H), 7.40 (s, 1H), 7.85 (s, 1H), 8.50 (s, 1H).

***cis*** **-2-(N'-Hydroxyl-N'-cyclohexyl-N- (2-propoxy-3-methoxy-5-5-(3,4,5-trimethoxy phenyl) tetrahydrothi-**

**ophene) phenyl urea (compound 32, Table 2).** $^1$H NMR ($CDCl_3$):1.00 (t, 3H), 1.30 (m, 2H), 1.6 (m, 4H), 1.75 (m, 5H), 2.00 (m, 2H), 2.50 (m, 2H), 3.80-4.00 (m,14H), 4.6 (m, 2H), 6.60 (s, 2H), 6.75 (s, 1H), 6.77 (s, 1H), 7.90 (s, 1H), 8.50 (s, 1H).

***cis* -2-(N'-Hydroxyl-N'-tert-butyl-N-(2-propoxy-3-methoxy-5-2-(3,4,5-trimethoxyphenyl) tetrahydrothiophene) phenyl urea (compound 31, Table 2).** $^1$H NMR ($CDCl_3$):1.00 (t, 3H), 1.4 (s, 9H), 1.75 (m,2H), 2.00 (m,2H), 2.50 (m, 2H), 3.80-4.00 (m,12H), 4.1 (t, 2H), 4.65 (m, 2H), 6.60 (s, 2H), 6.75 (s, 1H), 7.40 (s, 1H), 7.85 (s, 1H), 8.50 (s, 1H).

***cis* -2-(N'-Hydroxyl-N'-cyclohexyl-N- (2-propoxy-3-methoxy-5-5-(3,4,5-trimethoxy phenyl) tetrahydrothiophene) phenyl urea (compound 32,Table 2).** $^1$H NMR ($CDCl_3$):1.00 (t, 3H), 1.30 (m, 2H), 1.6 (m, 4H), 1.75 (m, 5H), 2.00 (m, 2H), 2.50 (m, 2H), 3.80-4.00 (m,14H), 4.6 (m, 2H), 6.60 (s, 2H), 6.75 (s, 1H), 6.77 (s, 1H), 7.90 (s, 1H), 8.50 (s, 1H).

***cis* -2-(N'-Hydroxyl-N-ethyl-N-(2-propoxy-3-methoxy-5-(5-(3,4,5-trimethoxyphenyl) tetrahydrofuran) phenyl urea (compound 40,Table 2).** $^1$H NMR ($CDCl_3$):1.00 (t, 3H), 1.2 (t, 3H), 1.75 (m, 2H), 2.00 (m, 2H), 2.50 (m, 2H), 3.75 (q, 2H), 3.80-4.00 (m,14H), 5.00 (m, 2H), 6.60 (s, 2H), 6.75 (s, 1H), 6.77 (s, 1H), 7.90 (s, 1H), 8.50 (s, 1H).

***cis* -2-(N'-Hydroxyl-N'-n-butyl-N-(2-propoxy-3-methoxy-5-(5-(3,4,5-trimethoxyphenyl) tetrahydrofuran) phenyl urea (compound 41, Table 2).** $^1$H NMR ($CDCl_3$):1.00 (t, 3H), 1.2 (t, 3H), 1.5 (m, 2H), 1.75 (m, 2H), 1.8 (m, 2H), 2.00 (m, 2H), 2.50 (m, 2H), 3.70 (t, 2H), 3.80-4.00 (m,14H), 5.00 (m,2H), 6.60 (s, 2H), 6.75 (s, 1H), 6.77 (s, 1H), 7.90 (s, 1H), 8.50 (s, 1H).

**Example 13: *trans*-2-(5-(N-Hydroxy-N-substituted-aminocarbonyl)-aminomethyl-4-propoxy-3-methoxy)-5-(3,4,5-trimethoxyphenyl)-tetrahydrofuran (compounds 33-37, Figure 8)**

**3-Methoxy-4-propoxy-5-iodobenzaldehyde (compound 125, Figure 5)**

5-Iodovanillin (25 g, 0.09 mol) in DMF (100 mL) was treated with potassium carbonate (32 g, 0.23 mol) and n-propyl iodide (52 g, 0.3 mol, 31 mL) and heated for 16 hours. The solution was allowed to cool to room temperature and then poured into water (500 mL) and extracted with ether (3X 250 mL). The organic layers were combined and washed with water and saturated sodium chloride solution, and then dried over magnesium sulfate. The organic layer was evaporated by distillation under diminished pressure to an oil, and then purified by column chromatography (silica, 7:3 petroleum ether-ethyl acetate), to provide an amber colored solid (26.9 g, 93%). $^1$H NMR ($CDCl_3$) 1.07 (t, 3 H), 1.85 (m, 2 H), 3.89 (s, 3H), 4.06 (t, 3 H), 7.39 (s, 1 H), 7.84 (s, 1 H), 9.81 (s, 1 H).

**1-(3-methoxy-4-propoxy-5-iodophenyl)-4-(3,4,5-trimethoxyphenyl) -1,4-butanedione (compound 126, Figure 5)**

3,4,5-Trimethoxyphenylvinylketone (11.6 g, 0.052 mol), 3-methoxy-4-propoxy-5-iodobenzaldehyde (13.8 g, 0.043 mol), and 3-benzyl-5-(2-hydroxyethyl)-4-methythiazolium chloride (4.6 g, 0.017 mol) were stirred in triethylamine (35 mL) at 60°C for 16 hours. The solution was then acidified, poured into chloroform (500 mL) and washed with 10% HCl, water and saturated aqueous sodium chloride. The organic layer was dried over magnesium sulfate, concentrated under reduced pressure, and the product isolated from petroleum ether-ethyl acetate to provide pale yellow microcrystals (27 g, 92%). m.p. 119-121°C. $^1$H NMR ($CDCl_3$) 1.07 (t, 3 H), 1.86 (m, 2 H), 3.40 (dd, 4H), 3.88 (s, 3H), 3.92 (s, 9H), 4.04(t, 2 H), 7.29 (d, 1 H), 8.07 (d, 1 H).

**1-(3-Methoxy-4-propoxy-5-cyano)-4-(3,4,5-trimethoxyphenyl)-1,4-butanedione (compound 132, Figure 6)**

1-(3-Methoxy-4-propoxy-5-iodophenyl)-4-(3,4,5-trimethoxyphenyl)-1,4-butanedione (10.0 g, 18.4 mmol) and CuCN (16.6 g, 0.184 mol) in DMF (100 mL) were heated at 140oC for 2 hours. The solution was cooled and poured onto water (500 mL). The aqueous phase was extracted with chloroform (3 x 250 mL). The organic layers were combined, washed with water and saturated aqueous sodium chloride, and then dried over magnesium sulfate. The resulting material was concentrated, and the product isolated from hexane-ethyl acetate as pale yellow microcrystals (7.75 g, 95%).

**1-(3-Methoxy-4-propoxy-5-cyano)-4-(3,4,5-trimethoxyphenyl)-1,4-butanediol (compound 133, Figure 6)**

To 1-(3-methoxy-4-propoxy-5-cyano)-4-(3,4,5-trimethoxyphenyl)-1,4-butanedione (4.0 g, 9.06 mmol) in 5:1 methanol/THF (125 mL) was added sodium borohydride (617 mg, 16.3 mmol), and the solution stirred overnight.

Solvent was removed under reduced pressure and the crude residue was dissolved in dichloromethane (250 mL). The solution was washed with water and brine, dried over magnesium sulfate, and then concentrated under reduced pressure. It was used without further purification.

***trans* -2-(3-Methoxy-4-propoxy-5- Cyano)-5-(3,4,5-trimethoxyphenyl)-tetrahydrofuran (compound 134, Figure 6)**

To 1-(3-methoxy-4-propoxy-5-cyano)-4-(3,4,5-trimethoxyphenyl)-1,4-butanediol (9.0 mmol) in chloroform (100 mL) at 0°C was added dropwise trifluoroacetic acid (36 mmol, 2.8 mL) in chloroform (50 mL) over 30 minutes. The solution was then stirred at 0°C overnight. The solution was washed with 10% potassium carbonate, water, and saturated aqueous sodium chloride, and then dried over magnesium sulfate. The solution was then concentrated under reduced pressure and the *trans* isomer isolated as colorless needles from petroleum ether-ethyl acetate (3.06 g, 79%).

***trans*-2-(3-Methoxy-4-propoxy-5-aminomethyl)-5-(3,4,5-trimethoxyphenyl)-tetrahydrofuran (compound 135, Figure 6)**

To *trans*-2-(3-Methoxy-4-propoxy-5-cyano)-5-(3,4,5-trimethoxyphenyl)-tetrahydrofuran (500 mg, 1.17 mmol) in THF (25 mL) was added sodium borohydride (80 mg, 2.1 mmol) and boron trifluoride etherate (400 mg, 2.8 mmol) dropwise over thirty minutes. The solution was then refluxed for four hours, cooled, treated with a few drops of 10% HCl, poured into water and extracted with dichloromethane. The organic layers were combined, washed with water and saturated aqueous sodium chloride, and then dried over magnesium sulfate. The dried solution was concentrated, and then purified by column chromatography (silica, 9:1 dichloromethane-methanol). The product was isolated as an amber oil (207 mg, 41%).

***trans*-2-[3-Methoxy-4-propoxy-5-(4-nitrophenoxy carbonyl)-aminomethyl] -5-(3,4,5-trimethoxyphenyl)-tetrahydrofuran (compound 136, Figure 6).**

*trans*-2-(3-Methoxy-4-propoxy-5-aminomethyl)-5-(3,4,5-trimethoxyphenyl)-tetrahydrofuran (207 mg, 0.048 mmol), nitrophenylchloroformate (106 mg, 0.53 mmol), and diisopropylethylamine (69 mg, 0.53 mmol) were stirred in dichloromethane (3 mL) for 12 hours. The solution was then concentrated and purified by column chromatography (silica. 85:15 dichloromethane-ether). The product was isolated as a yellow oil (251 mg, 88%).

***trans*-2-[3-Methoxy-4-propoxy-5-(N-hydroxyaminocarbonyl)-aminomethyl] -5-(3,4,5-trimethoxyphenyl)-tetrahydrofuran (compound 33, Figure 6).**

*rans*-2-[3-Methoxy-4-propoxy-5-(4-nitrophenoxy carbonyl) aminomethyl] -5-(3,4,5-trimethoxyphenyl)-tetrahydrofuran (57 mg, 0.096 mmol), hydroxylamine hydrochloride (10 mg, 0.15 mmol), and diisopropylethylamine (40 mg, 0.3 mmol) were stirred in dichloromethane (3 mL) for 24 hours. The solution was then diluted with dichlorormethane and washed with 10% sodium bicarbonate, water, and saturated aqueous sodium chloride, and ten dried over magnesium sulfate. The solution was then concentrated, and purified by preparative TLC (silica. 95:5 dichloromethane-methanol). The pure material was isolated as a colorless oil (16 mg, 34%). $^1$H NMR ($CDCl_3$) 1.0 (t, 3 H), 1.75 (m, 2 H), 2.0 (m, 2H), 2.44 (m, 2 H), 3.86 (m, 12 H), 3.91 (t, 2 H), 4.42 (d, 2 H), 5.13 (t, 2 H), 6.3-7.1 (m, 4H).

***trans*-2-3-Methoxy-4-propoxy-5-(N-hydroxy N-methylaminocarbonyl) -5-(3,4,5-trimethoxyphenyl)-tetrahydro-furan (compound 34, Figure 6).** *trans*-2-3-methoxy-4-propoxy-5-(N-hydroxyaminocarbonyl) aminomethyl -5-(3,4,5-trimethoxyphenyl)-tetrahydrofuran (42 mg, 0.070 mmol), N-methyl hydroxylamine hydrochloride (9 mg, 0.11 mmol), and diisopropylethylamine (0.33 mmol) in dichloromethane (3 mL) were stirred for 24 hours. The solution was diluted with dichloromethane and washed with 10% sodium bicarbonate, water and saturated sodium chloride, and then dried over magnesium sulfate. The solution was then concentrated, and purified by preparative TLC (silica, 95:5 dichloromethane-methanol). The product was isolated as a colorless oil weighing (14 mg, 40%). $^1$H NMR ($CDCl_3$) 1.0 (t, 3 H), 1.75 (m, 2 H), 2.0 (m, 2H), 2.44 (m, 2 H), 3.04 (s, 3 H), 3.86 (m, 12 H), 3.91 (t, 2 H), 4.42 (d, 2 H), 5.13 (m, 2 H), 6.3-7.1 (m, 4H).

***trans*-2-3-Methoxy-4-propoxy-5-(N-Hydroxy-N-isopropylaminocarbonyl)-5-(3,4,5- trimethoxyphenyl)-tetrahydrofuran (compound 35, Figure 6).**

*rans*-2-3-methoxy-4-propoxy-5-(N-hydroxyaminocarbonyl) aminomethyl -5-(3,4,5-trimethoxyphenyl)-tetrahydrofuran (40 mg, 0.067 mmol), N-isopropyl hydroxylamine hydrochloride (11 mg, 0.1 mmol), and diisopropylethylamine (0.2 mmol) were stirred in dichloromethane (3 mL) for 24 hours. The solution was diluted with dichloromethane, washed with 10% sodium bicarbonate, water and saturated aqueous sodium chloride, and then dried over magnesium sulfate. The solution was concentrated, and then purified by preparative TLC (silica, 95:5 dichloromethane-methanol). The product was isolated as a colorless solid (16 mg, 45%). $^1$H NMR ($CDCl_3$) 1.0 (t, 3 H), 1.1 (d, 6 H),

1.75 (m, 2 H), 2.0 (m, 2H), 2.44 (m, 2 H), 3.04 (s, 3 H), 3.86 (m, 12 H), 3.91 (t, 2 H), 4.40 (m, 1 H), 4.42 (d, 2 H), 5.13 (m, 2 H), 6.3-7.1 (m, 4H).

***trans*-2-3-methoxy-4-propoxy-5-(N-hydroxy-N-cyclohexylaminocarbonyl) aminomethyl-5-(3,4,5-trimethoxyphenyl)-tetrahydrofuran (Compound 36, Figure 6).**
*trans*-2-3-methoxy-4-propoxy-5-(N-Hydroxy-N-isopropylaminocarbonyl)-5-(3,4,5-trimethoxyphenyl)-tetrahydrofuran (59 mg, 0.1 mmol), N-cyclohexyl hydroxylamine hydrochloride (23 mg, 0.15 mmol, and diisopropylethylamine (0.2 mmol) were stirred in dichloromethane (3 mL) for 24 hours. The solution was diluted with dichloromethane, washed with 10% sodium bicarbonate, water and saturated sodium chloride, and then dried over magnesium sulfate. The solution was then concentrated and purified by preparative TLC (silica, 95:5 dichloromethane-methanol), to provide the product a colorless solid (24 mg.,42%). $^1$H NMR ($CDCl_3$) 1.0 (t, 3 H), 1.1-1.8 (m, 10 H), 1.75 (m, 2 H), 2.0 (m, 2H), 2.44 (m, 2 H), 3.04 (s, 3 H), 3.86 (m, 13 H), 3.91 (t, 2 H), 4.42 (d, 2 H), 5.13 (m, 2 H), 6.3-7.1 (m, 4H).

***trans*-2-3-methoxy-4-propoxy-5-(N-hydroxy-N-benzylaminocarbonyl)-5-(3,4,5-trimethoxyphenyl)-tetrahydro-furan (compound 37, Figure 6).** *trans*-2-3-methoxy-4-propoxy-5-(N-hydroxy-N-cyclohexylaminocarbonyl) aminomethyl-5-(3,4,5-trimethoxyphenyl)-t etrahydrofuran (53 mg, 0.089 mmol), N-benzyl hydroxylamine hydrochloride (21 mg, 0.133 mmol), diisopropylethylamine (0.27 mmol), and stirred in dichloromethane (3 mL) for 24 hours. The reaction was diluted with dichloromethane and the organic layer was washed with 10% sodium bicarbonate, water, and saturated aqueous sodium chloride, and then dried over magnesium sulfate. The solution was then concentrated and purified by preparative TLC (silica, 95:5 dichlorormethane-methanol), and the product isolated as a colorless oil (22 mg.,42%). $^1$H NMR ($CDCl_3$) 1.0 (t, 3 H), 1.75 (m, 2 H), 2.0 (m, 2H), 2.44 (m, 2 H), 3.86 (m, 12 H), 3.91 (t, 2 H), 4.42 (d, 2 H), 4.65 (s, 2 H), 5.13 (t, 2 H), 6.3-7.1 (m, 4H), 7.3-7.5 (m, 5 H).

**Example 14: Cis and trans-5-(3,4,5-Trimethoxyphenyl)-2-(3-methoxy-4-propoxy-5-hydroxyethylsulfonyl)-tetrahydrothiophene (Compound 42, Figure 5)**

Compounds 125 and 126 were prepared as described above.

**1-(3-Methoxy-4-propoxy-5-hydroxyethylthiophenyl)-4-(3,4,5-trimethoxyphenyl)-butane-1,4-dione (Compound 127, Figure 5).** To a solution of 1-(3-methoxy-4-propoxy-5-iodophenyl)-4-(3,4,5-trimethoxyphenyl)-butane-1,4-dione (13.55 g, 25 mmol) in 100 mL of DMF was added 13.5 g (212.43 mmol) of copper powder. The slurry was heated at 130°C for 2 hours and a solution of 2-hydroxyethyldisulfide (4.88 mL, 40 mmol) in 25 mL of DMF added. Heating was continued for an additional 20 hours. The mixture was then cooled, filtered and washed with ethyl acetate. Water (125 m L) was added and the mixture extracted with ethyl acetate. The organic layer was washed several times with water, dried over magnesium sulfate and evaporated. Thecrude product was purified by flash chromatography on silica gel using 1:1 hexane/ethyl acetate as eluent (7.5 g, 61%). 1H NMR (CDCl3): 1.07 (t, 3H), 1.85 (m, 2H), 2.38 (t, 1H), 3.13 (t, 2H), 3.41 (s, 4H), 3.72 (q, 2H), 3.90 (s, 3H), 3.93 (s, 9H), 4.07 (t, 2H), 7.29 (s, 2H), 7.49 (d, 1H), 7.73 (d, 1H).

**1-(3-Methoxy-4-propoxy-5-hydroxyethylsulfonylphenyl)-4-(3, 4,5-trimethoxyphenyl)-butane-1,4-dione (compound 128, Figure 5).** A suspension of magnesium monoperoxyphthalic acid (18.51 g, 37.42 mmol) in 60 mL of water was added to 1-(3-methoxy-4-propoxy-5-hydroxyethylthiophenyl)-4-(3,4,5-trimethoxyphenyl)-butane-1,4-dione (9.84 g. 20 mmol) in 180 mL of acetonitrile. The solution was stirred for 2 hours, and then 600 mL of water added, and the mixture extracted with methylene chloride. The organic layer was washed with 5% sodium hydroxide solution, water, and saturated aqueous sodium chloride and then dried with magnesium sulfate and evaporated to a white solid. The solid was purified by flash column chromatography using 2:1 ethyl acetate/hexane (7.98 g, 76%). $^1$H NMR ($CDCl_3$): 1.04 (t, 3H), 1.88 (m, 2H), 2.77 (bs, 1H), 3.43 (s, 4H), 3.66 (t, 2H), 3.91 (s, 9H), 3.94 (s, 3H), 3.99 (m, 2H), 4.21 (t, 2H), 7.27 (s, 2H), 7.83 (d, 1H), 8.21 (d, 1H).

**1-(3-methoxy-4-propoxy-5-dimethyl-*tert*-butylsiloxyethylsulfonylphenyl)-4-(3,4,5-tri methoxyphenyl)-butane-1,4-dione (compound 129, Figure 5).** A suspension of 5.24 g (10 mmol) of 1-(3-methoxy-4-propoxy-5-hydroxyethylsulfonylphenyl)-4-(3,4,5-trimethoxyphenyl)-butane-1,4-dione, *tert*-butyldimethylsilyl chloride (1.81 g, 12 mmol), and imidazole (0.82 g, 12.05 mmol) in 55 mL of methylene chloride was stirred at room temperature for 12 hours. The solution was filtered, evaporated to an oil, and then purified by flash chromatography on silica gel using 1:1 hexane/ethyl acetate (5.42 g, 85%). $^1$H NMR ($CDCl_3$): -0.07 (s, 6H), 0.75 (s, 9H), 1.06 (t, 3H), 1.90(m,2H), 3.43 (s, 4H), 3.68 (t, 2H), 4.00 (t, 2H), 4.22 (t, 2H), 7.29 (s, 2H), 7.80 (d, 1H), 8.20 (d, 1H).

**1-(3-Methoxy-4-propoxy-5-dimethyl-tert-butylsiloxyethylsulfonylphenyl)-4-(3,4,5-trimethoxyphenyl)-butane-1,4-diol (compound 130, Figure 5).** To a solution of 1-(3-methoxy-4-propoxy-5-dimethyl-*tert*-butylsiloxyethylsulfonylphenyl)-4-(3,4,5-trimethoxyphenyl)-butane-1,4-dione (6.38 g, 10 mmol) in 90 mL of methanol and 25 mL tetrahydrofuran was added dropwise sodium borohydride (903 mg, 23.88 mmol) in 40 mL of water. After the addition was completed, the reaction mixture was stirred at room temperature for 3 hours, quenched with water and extracted with dichloromethane. The organic layer was dried over magnesium sulfate, filtered and evaporated to a foam (4.57 g, 71%). $^1$H NMR ($CDCl_3$): -0.05 (s, 6H), 0.77 (s, 9H), 1.04 (t, 3H), 1.86 (m, 6H), 3.65 (m, 2H), 3.74 (m, 2H), 3.84 (s, 3H), 3.87 (s, 6H), 3.89 (s, 3H), 3.97 (t, 2H), 4.11 (t, 2H), 4.72 (m, 2H), 6.57 (s, 1H), 7.22 (s, 1H), 7.43 (s, 1H).

***trans* -2-(3-Methoxy-4-propoxy-5-dimethyl-tert-butylsiloxyethylsulfonyl)-5-(3,4,5-trimethoxyphenyl)-tetrahydrothiophene (compound 131, Figure 5).** A suspension of $P_4S_{10}$ (2.90 g, 6.53 mmol) in 48 mL of pyridine was heated at 120°C for 75 minutes. To the suspension was added a solution of 1-(3-methoxy-4-propoxy- 5-dimethyl-*tert*-butylsiloxyethylsulfonylphenyl)-4-(3,4,5-trimethoxyphenyl)-butane-1,4-diol (3.21 g, 5 mmol) in 20 mL of pyridine. The temperature was reduced to 90°C and stirring continued for an additional 90 minutes. Ice and water were then added, and the mixture extracted with methylene chloride. The organic layer was washed with water, 5% HCl, sodium bicarbonate and saturated sodium chloride solution, and then dried with magnesium sulfate, filtered and evaporated. The resulting *cis* and *trans* thiophenes were separated by HPLC using Water's prep Nova-Pak HR silica cartridge (9:1 hexane: ethyl acetate) to provide 580 mg (20%) of the transisomer and 145 mg (5%) of the *cis* isomer. Trans isomer. $^1$H NMR ($CDCl_3$): -0.05 (s, 6H), 0.80 (s, 9H), 1.10 (t, 3H), 1.95 (m, 2H), 2.05 (m, 2H), 2.6 (m, 2H), 3.7 (t, 3H), 3. 95 (m, 12H), 4.00 (t, 3H), 4.2 (t, 3H), 4.9 (m, 2H), 6.8 (s, 2H), 7.40 (d, 1H), 7.60 (d, 1H).

***cis* and *trans*-5-(3,4,5-Trimethoxyphenyl)-2-(3-methoxy-4-propoxy-5-hydroxyethylsulfonyl)-tetrahydrothiophene (compound 38, Figure 5).** To an ice-water cooled solution of trans-2-(3,4,5-trimethoxyphenyl)-5-(3-methoxy-4-propoxy-5-dimethyl-tert-butylsiloxyethylsulfonyl)-tetrahydrothiophene (80 mg, 0.125 mmol) in 4 mL of dry tetrahydrofuran was added dropwise a solution of tetrabutylammonium fluoride in 2 mL of tetrahydrofuran. The solution was stirred at room temperature for two hours, and then the solvent evaporated under vacuum and passed through a small column of silica gel using 1:1 hexane:ethyl acetate to give a white solid (60mg, 91%). $^1$H NMR ($CDCl_3$): 1.10 (t, 3H), 1.95 (m, 2H), 2.05 (m, 2H), 2.6 (m, 2H), 3.7 (t, 3H), 3.95 (m, 12H), 4.00 (t, 3H), 4.15 (t, 3H), 4.9 (m, 2H), 6.8 (s, 2H), 7.40 (d, 1H), 7.60 (d, 1H).

**Example 15: 1-(3-Nitro-4-propoxy-5-methoxyphenyl)-3-(3,4,5-trimethoxyphenyl)-cyclopentane (compound 143, Figure 7).**

This compound is synthesized as set out in detail in Figure 7, following the procedure as outlined by Graham, D. W. et al. ("1,3-Diaryl Cyclopentanes: A New Class of Potent PAF Receptor Antagonists", 197th ACS National Meeting, Dallas, Texas, April 9-14, 1989, Division of Medicinal Chemistry, poster No. 25) starting from compound 114 (Figure 7). Reduction and functionalization of this compound is carried out similar to that described for compounds 119-121 (Figure 3).

**Example 16: 2-(5-(N-hydroxy-N-methylaminocarbonyl)-amino (substituted)-3-methoxy-4-propoxy)-5-(3,4,5-trimethoxyphenyl)-cyclopentane (compound 158, Figure 8)**

3,4,5-Trimethoxyacetophenone is converted to the corresponding acetylene (compound 146, Figure 8) using the procedure outlined by Negishi et al., J. Org. Chem. 45, 2526 (1980), using diethylchlorophosphate and lithium diisopropylamide. 5-Iodovanillin is alkylated with n-propyl iodide as described in Example 6. The resulting alkylated aldehyde (compound 125, Figure 8) is converted to the corresponding nitrile (compound 148, Figure 8) as described in Example 13. Compound 148 is converted to the terminal alkyne (compound 149, Figure 8) via the Corey-Fuchs procedure (Corey, E.J. et al., Tetra. Lett. 3769 (1972)). The E-vinyl iodide (compound 150, Figure 8) is synthesized from compound 149 using catechol borane and iodine. Heat acetylene and catechol borate at 60°C for 3 hours. Stir resulting boronate ester with water for 2 hours. Filter boronic acid to remove catechol. Dissolve the boronic acid in THF and sodium hydroxide (3 equivalent). Treat with iodine (1 equivalent) and stir for 30 minutes. The product was isolated by standard extractive workup and column chromatography. The iodide (compound 150) and the vinyl alane (compound 147) are coupled using a palladium catalyst to yield compound 151. Compound 151 is converted to the allylic chloroacetate (compound 152) via palladium catalysis. Compound 152 is converted to the BOC protected amine, and the acetate removed via saponification to yield compound 153. Compound 153 is saturated by hydrogenation and the alcohol moieties converted to mesylates (compound 154). Cyclization of compound 154 to the pyrrole (compound 155) is accomplished with mild base. Compound 155 is then reduced and the resultant amine (156) converted to the hydroxyurea (157) (*vide infra*). Finally, the pyrrole is deprotected using trifluoroacetic acid and anisole in dichloromethane at zero degrees to yield the compound 158.

**Example 17: Synthesis of 2,4-Diaryl Tetrahydrofuran and Tetrahydrothiophene (Compounds 167 and 168, Figure 9).**

3,4,5-Trimethoxyacetophenone is oxidized to the corresponding hydroxyketone (compound 159, Figure 9) and then protected as the silyl ether (compound 160, Figure 9). Compound 160 is converted to the corresponding olefin (compound 161, Figure 9) using a Wittig reaction. Compound 161 is converted to the primary bromide compound 162 with 9-BBN (9-borabicyclo[3.3.1]nonane). Compound 162 is then converted to the Grignard reagent and treated with the compound 148 to yield compound 163. The alcohol protecting group in compound 163 is removed with TBAF (tetrabutylammonium fluoride) and the resulting diol (compound 164) converted to the tetrahydrofuran (165) or tetrahydrothiophene (166) using procedures described above. Compounds 167 and 168 are converted to the hydroxyureas (167) and (169) by procedures described above.

**Example 18: Preparation of *trans*-2-[5-(N'-methyl-N'-hydroxyureidylmethyl)-3-methoxy-4-p-chlorophenylthioethoxyphenyl]-5-(3,4,5-trimethoxyphenyl)tetrahydrofuran (229, Figures).**

**3-(N,N-Dimethylamino)-1-(3,4,5-trimethoxyphenyl)-1-propanone (compound 301, Figure 1).** 3,4,5-Trimethoxyacetophenone (50 g, 237.8 mmole), paraformaldehyde (9.75 g, 304.7 mmole), dimethylamine hydrochloride (26.42 g, 324.0 mmole) and 5 mL conc. HCl were dissolved in 200 mL absolute ethanol and refluxed for 10 hours. Additional dimethylamine hydrochloride (13.21 g, 162.0 mmole) and paraformaldehyde (9.75 g, 304.7 mmole) were added and the solution returned no reflux. After 54 hours (tonal reaction time), 80 mL of 10% HCl and 500 mL of water were added and the solution was extracted with ethyl ether. The acidic aqueous layer was adjusted no pH 10 with 10% NaOH. The basic solution was extracted with ethyl acetate, dried over $MgSO_4$, filtered and evaporated <u>in vacuo</u> to provide 57.5 g of a yellow oil (92%). $^1$H NMR ($CDCl_3$): 2.30 (s, 6H); 2.74 (t, 2H); 3.11 (t, 3H); 3.91 (s, 9H); 7.23 (s, 1H); 7.32 (s, 1H).

**3-(N,N,N-Trimethylamino)-1-(3,4,5-trimethoxyphenyl)-1-propanone iodide (compound 302, Figure 1).** 3-(N,N-Dimethylamino)-1-(3,4,5-trimethoxyphenyl)-1-propanone (57 g, 213.5 mmole) was dissolved in 200 mL of anhydrous diethyl ether. To this solution was added methyl iodide (57.6 g, 405.7 mmole). A white precipitate formed immediately, and the reaction mixture was stirred at room temperature for an additional 2 hours. This product was isolated by suction filtration (83.8 g, 96%)

**3,4,5-Trimethoxyphenylvinylketone (compound 303, Figure 1).** 3-(N,N,N-Trimethylamino)-1-(3,4,5-trimethoxyphenyl)-1-propanone iodide (50 g, 120 mmole) was dissolved in $H_20$ (500 mL) and ethyl acetate (500 mL) was added. The mixture was vigorously stirred at reflux for 3 hours. The reaction mixture was cooled and the layers were separated. To the aqueous phase was added ethyl acetate (400 mL). This was brought to reflux for 1.5 hours. The reaction mixture was cooled and separated. The combined organic layers were washed with saturated NaCl solution, dried over $Na_2S0_4$, filtered and concentrated <u>in vacuo</u> to an oil which was purified by flash column chromatography using 3:1 hexane/ethyl acetate as solvent (14.7 g, 54%). $^1$H NMR ($CDCl_3$): 3.92 (s, 9H); 5.92 (d, 1H); 6.44 (d, 1H); 7.12 (m, 1H); 7.22 (s, 2H).

**3-Methoxy-4-hydoxyethoxy-5-iodobenzaldehyde (compound 304, Figure 1).** 5-Iodovanillin (25 g, 90 mmol) in DMF (100 mL) was added to potassium carbonate (18.6 g, 135 mmol). The mixture was heated at 40°C for 16 hours. The reaction mixture was allowed to cool to room temperature and quenched with water (500 mL) and extracted with ethyl acetate. The organic layer was washed with water and saturated NaCl solution, and dried over $MgSO_4$, filtered and evaporated <u>in vacuo</u> to an oil, and then purified by column chromatography (silica, 2:1 hexane/ethyl acetate), to provide the product (16.6 g, 57%). $^1$H NMR ($CDCl_3$): 2.70 (t, 1H); 3.92 (t, 2H); 3.92 (s, 3H); 3.94 (s, 3H); 4.29 (t, 2H); 7.44 (s,1H); 7.87 (s, 1H); 9.85 (s, 1H).

**1-(3-Methoxy-4-hydroxyethoxy-5-iodophenyl)-4-(3,4,5-trimethoxyphenyl)-1,4-butanedione (compound 305, Figure 1).** 3,4,5-Trimethoxyphenylvinylketone (4.8 g, 21.6 mmol), 3-methoxy-4-hydroxyethoxy-5-iodobenzaldehyde (5.7 g, 17.8 mmol), and 3-benzyl-5-(2-hydroxyethyl)-4-methylthiazolium chloride (1.9 g, 7.0 mmol) were stirred in triethylamine (20 mL) at 60°C for 16 hours. The reaction mixture was then acidified with 10% HCl, and extracted with dichloromethane. The organic layer was dried over $MgSO_4$, filtered and evaporated <u>in vacuo</u>. The

product was purified in column chromatography (silica, 1:1 hexane/ethyl acetate) as a solid (9.7 g, 51%). $^1$H NMR ($CDCl_3$): 3.41 (m, 4H); 3.90 (m, 2H); 3.92 (s, 3H); 3.93 (s, 9H); 4.26 (t, 2H); 7.29 (s, 2H); 7.57 (d, 1H); 8.08 (d, 1H).

**1-(3-Methoxy-4-hydroxyethoxy-5-iodophenyl)-4-(3,4,5-trimethoxyphenyl)-1,4-butanediol (compound 306, Figure 1).** 1-(3-Methoxy-4-hydroxyethoxy-5-iodophenyl)-4-(3,4,5-trimethoxyphenyl)-1,4-butanedione (11.6 g, 21.3 mmol), was added to 120 mL tetrahydrofuran and 240 mL methanol. To this solution was added dropwise sodium borohydride (1.45 g, 38.4 mmol), in 60 mL water. The reaction mixture was stirred at room temperature for 2.5 hours, and then cooled, quenched with water, and the aqueous layer extracted with ethyl acetate. The organic layer was dried over $MgSO_4$, filtered and evaporated in vacuo to provide the product (11.8 g, 98.8%). $^1$H NMR ($CDCl_3$): 1.84 (m, 4H); 3.84 (m, 2H); 3.86 (s, 3H); 3.87 (s, 9H); 4.15 (t, 2H); 4.68 (m, 2H); 6.57 (s, 2H); 6.91 (s, 1H); 7.32 (s, 1H).

***trans*-2-(3-Methoxy-4-hydroxyethoxy-5-iodophenyl)-5-(3,4,5-trimethoxyphenyl)tetrahydrofuran (compound 307, FIgure 1).** To 1-(3-methoxy-4-hydroxyethoxy-5-iodophenyl)-4-(3,4,5-trimethoxyphenyl)-1,4-butanediol (11.8 g, 21.5 mmol) in chloroform (100 mL) at 0°C was added dropwise trifluoroacetic acid (9.82 g, 86.1 mmol) in chloroform (100 mL) over 30 minutes. The solution was stirred at 0°C for 2 hours and then at room temperature for 1 hour. The reaction mixture was quenched with 1N NaOH and chloroform (100 mL) was added. The organic layer was washed with 1N NaOH solution, water and saturated NaCl solution, and then dried over $MgSO_4$, filtered and evaporated in vacuo to an oil which was a *cis* and *trans* mixture. The *trans* isomer was isolated by column chromatography (silica, 1:1 hexane/ethyl acetate) (4.7 g, 41.4%) as the faster eluting isomer. $^1$H NMR ($CDCl_3$): 1.99 (m, 2H); 2.47 (m, 2H); 3.83 (t, 2H); 3.84 (s, 3H); 3.87 (s, 3H); 3.89 (s, 6H); 4.16 (t, 2H); 5.18 (m, 2H); 6.62 (s, 2H); 6.96 (d, 1H); 7.39 (d, 1H).

***trans*-2-(3-Methoxy-4-methylsulfoxyethoxy-5-iodophenyl)-5-(3,4,5-trimethoxyphenyl)tetrahydrofuran (compound 308, Figure 1).** To the solution of *trans*-2-(3-methoxy-4-hydroxyethoxy-5-iodophenyl)-5-(3,4,5-trimethoxyphenyl) tetrahydrofuran (4.7 g, 8.87 mmol) in dichloromethane (50 mL) at 0°C was added methylsulfonyl chloride (3.05 g, 26.6 mmole) and triethylamine (2.69 g, 26.60 mmol). The reaction mixture was stirred at 0°C for 2 hours and room temperature overnight. The solvent was evaporated in vacuo and the residue purified by column chromatography (silica. 1:1 hexane/ethyl acetate) (4.17 g, 77.3%). $^1$H NMR ($CDCl_3$): 1.98 (m, 2H); 2.45 (m, 2H); 3.15 (s, 3H); 3.84 (s, 3H); 3.88 (s, 9H); 4.26 (t, 2H); 4.61 (t, 2H); 5.17 (m, 2H); 6.62 (s, 2H); 6.96 (d, 1H); 7.38 (d, 1H).

***trans*-2-(3-Methoxy-4-p-chlorophenylthioethoxy-5-iodophenyl)-5-(3,4,5-trimethoxyphenyl)tetrahydrofuran (compound 309, Figure 1).** *trans*-2-(3-Methoxy-4-methylsulfoxyethoxy-5-iodophenyl)-5-(3,4,5-trimethoxyphenyl) tetrahydrofuran (2.5 g, 4.11 mmol) was dissolved in 50 mL ethanol. To this solution was added 4-chlorothiophenol (1.19 g, 8.22 mmol) and triethylamine (0.831 g, 8.22 mmol). The reaction mixture was refluxed for 16 hours and then the solvent was removed in vacuo. The residue was purified by column chromatography (silica, 3:1 hexane/ethyl acetate)(2.35 g, 87%). $^1$H NMR ($CDCl_3$): 1.97 (m, 2H); 2.45 (m, 2H); 3.35 (t, 2H); 3.82 (s, 3H); 3.84 (s, 3H); 3.88 (s, 6H); 4.11 (t, 2H); 5.17 (m, 2H); 6.61 (s, 2H); 6.92 (s, 1H); 7.26 (d, 2H); 7.33 (d, 2H); 7.35 (s, 1H).

***trans*-2-(3-Methoxy-4-p-chlorophenylthioethoxy-5-cyanophenyl)-5-(3,4,5-trimethoxyphenyl)tetrahydrofuran (compound 310, Figure 1).**
*trans*-2-(3-Methoxy-4-p-chlorophenylthioethoxy-5-iodophenyl)-5-(3,4,5-trimethoxyphenyl) tetrahydrofuran (2.35 g, 3.58 mmole) and CuCN (0.358 g, 4.30 mmole) in DMF (20 mL) were heated at 140°C for 16 hours. The reaction mixture was cooled and quenched with water and extracted with ethyl acetate. The organic layer was washed with water and saturated NaCl solution, dried over $MgSO_4$, filtered and evaporated in vacuo to oil which was purified by column chromatography (silica, 2:1 hexane/ethyl acetate) (1.79 g, 90.0%). $^1$H NMR ($CDCl_3$): 1.99 (m, 2H); 2.47 (m, 2H); 3.32 (t, 2H); 3.85 (s, 6H), 3.89 (s, 6H); 4.27 (t, 2H); 5.17 (m, 2H); 6.61 (s, 2H); 7.16 (s, 2H); 7.28 (d, 2H); 7.32 (d, 2H).

***trans*-2-(3-Methoxy-4-p-chlorophenylthioethoxy-5-aminomethylphenyl)-5-(3,4,5-trimethoxyphenyl)tetrahydrofuran (compound 311, Figure 1).**
To *trans*-2-(3-methoxy-4-p-chlorophenylthioethoxy-5-cyanophenyl)-5-(3,4,5-trimethoxyphenyl tetrahydrofuran (300 mg, 0.5405 mmol) in THF (10 mL) was added sodium borohydride (36.8 mg, 0.9729 mmol) and boron trifluoride etherate (191.8 mg, 1.3512 mmol) dropwise. The reaction mixture was refluxed for 1 hour, cooled, and then treated with a few drops of 10% HCl. The reaction mixture was poured into 10% $K_2CO_3$ and extracted with ethyl acetate. The organic layer was washed with water and saturated NaCl solution, dried over $MgSO_4$, filtered and evaporated in vacuo to an oil which was purified by column chromatography (silica, 93:7 $CH_2Cl_2$/MeOH) 64 mg, 21.2%). $^1$H NMR ($CDCl_3$): 1.99 (m, 2H); 2.46 (m, 2H); 3.28 (t, 2H); 3.84 (s, 6H); 3.88 (s, 6H); 4.26 (t, 2H); 5.19 (m, 2H); 6.71

(s, 2H); 6.90 (s, 2H); 7.25 (d, 2H); 7.32 (d, 2H).

***trans*-2-[5-(N'-Methyl-N'-hydroxyureidylmethyl)-3-methoxy-4-p-chlorophenylthioethoxypenyl]-5-(3, 4, 5-trimethoxyphenyl)tetrahydrofuran (229, Figure 1).** *trans*-2-(3-methoxy-4-p-chlorophenylthioethoxy-5-aminomethylphenyl)-5-(3,4,5-trimethoxyphenyl) tetrahydrofuran (54 mg, 0.0966 mmol) was dissolved in 4 mL dry dichloromethane. To this solution was added triphosgene (9.46 mg, 0.0319 mmol) and triethylamine (9.77 mg, 0.0966 mmol). The reaction mixture was refluxed for 2 hours and then cooled to room temperature. To this solution was then added triethylamine (35.2 mg, 0.3478 mmol) and methylhydroxyamine hydrochloride (24.2 mg. 0.2898 mmol). The reaction mixture was stirred at room temperature overnight, and then quenched with water and extracted with dichloromethane. The organic layer was washed with water and saturated NaCl solution, dried over $MgSO_4$, filtered and evaporated in vacuo. The product was purified by column chromatography (silica, ethyl acetate) (49 mg, 80.1%). $^1H$ NMR ($CDCl_3$): 1.97 (m, 2H); 2.43 (m, 2H); 3.08 (s, 3H); 3.27 (t, 2H); 3.82 (s, 3H); 3.83 (s, 3H); 3.87 (s, 6H); 4.15 (t, 2H); 4.39 (d, 2H); 5.17 (m, 2H); 6.41 (t, 1H); 6.51 (s, 2H); 6.78 (broad s, 1 H); 6.90 (s, 2H); 7.24 (d, 2H); 7.31 (d, 2H).

## II. Pharmaceutical Compositions

Humans, equine, canine, bovine and other animals, and in particular, mammals, suffering from inflammatory diseases, and in particular, disorders mediated by PAF or products of 5-lipoxygenase can be treated by administering to the patient an effective amount of one or more of the above-identified compounds or a pharmaceutically acceptable derivative or salt thereof in a pharmaceutically acceptable carrier or diluent to reduce formation of oxygen radicals. The active materials can be administered by any appropriate route, for example, orally, parenterally, intravenously, intradermally, subcutaneously, or topically, in liquid, cream, gel or solid form.

As used herein, the term pharmaceutically acceptable salts or complexes refers to salts or complexes that retain the desired biological activity of the above-identified compounds and exhibit minimal undesired toxicological effects. Nonlimiting examples of such salts are (a) acid addition salts formed with inorganic acids (for example, hydrochloric acid, hydrobromic acid, sulfuric acid, phosphoric acid, nitric acid, and the like), and salts formed with organic acids such as acetic acid, oxalic acid, tartaric acid, succinic acid, malic acid, ascorbic acid, benzoic acid, tannic acid, pamoic acid, alginic acid, polyglutamic acid, naphthalenesulfonic acid, naphthalenedisulfoinc acid, and polygalacturonic acid; (b) base addition salts formed with metal cations such as zinc, calcium, bismuth, barium, magnesium, aluminum, copper, cobalt, nickel, cadmium, sodium, potassium, and the like, or with a cation formed from ammonia, N,N-dibenzylethylene-diamine, D-glucosamine, tetraethylammonium, or ethylenediamine; or (c) combinations of (a) and (b); e.g., a zinc tannate salt or the like.

The compounds can also be administered as pharmaceutically acceptable quaternary salts known by those skilled in the art, which specifically include the quaternary ammonium salt of the formula -NR+Z-, wherein R is alkyl or benzyl, and Z is a counterion, including chloride, bromide, iodide, -O-alkyl, toluenesulfonate, methylsulfonate, sulfonate, phosphate, or carboxylate (such as benzoate, succinate, acetate, glycolate, maleate, malate, citrate, tartrate, ascorbate, benzoate, cinnamoate, mandeloate, benzyloate, and diphenylacetate.

The active compound is included in the pharmaceutically acceptable carrier or diluent in an amount sufficient to deliver to a patient a therapeutically effective amount without causing serious toxic effects in the patient treated. A preferred dose of the active compound for all of the above-mentioned conditions is in the range from about 0.01 to 300 mg/kg, preferably 0.1 to 100 mg/kg per day, more generally 0.5 to about 25 mg per kilogram body weight of the recipient per day. A typical topical dosage will range from 0.01 -3% wt/wt in a suitable carrier. The effective dosage range of the pharmaceutically acceptable derivatives can be calculated based on the weight of the parent compound to be delivered. If the derivative exhibits activity in itself, the effective dosage can be estimated as above using the weight of the derivative, or by other means known to those skilled in the art.

The compound is conveniently administered in any suitable unit dosage form, including but not limited to one containing 1 to 3000 mg, preferably 5 to 500 mg of active ingredient per unit dosage form. A oral dosage of 25-250 mg is usually convenient.

The active ingredient should be administered to achieve peak plasma concentrations of the active compound of about 0.01-30 mM, preferably about 0.1-10 mM. This may be achieved, for example, by the intravenous injection of a solution or formulation of the active ingredient optionally in saline, or an aqueous medium or administered as a bolus of the active ingredient.

The concentration of active compound in the drug composition will depend on absorption, distribution, inactivation, and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed composition. The active ingredient may be administered at once, or may be divided into a number of smaller doses to be administered at varying intervals of time.

Oral compositions will generally include an inert diluent or an edible carrier. They may be enclosed in gelatin capsules or compressed into tablets. For the purpose of oral therapeutic administration, the active compound can be incorporated with excipients and used in the form of tablets, troches, or capsules. Pharmaceutically compatible binding agents, and/or adjuvant materials can be included as part of the composition.

The tablets, pills, capsules, troches and the like can contain any of the following ingredients, or compounds of a similar nature: a binder such as microcrystalline cellulose, gum tragacanth or gelatin; an excipient such as starch or lactose, a dispersing agent such as alginic acid. Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. When the dosage unit form is a capsule, it can contain, in addition to material of the above type, a liquid carrier such as a fatty oil. In addition, dosage unit forms can contain various other materials which modify the physical form of the dosage unit, for example, coatings of sugar, shellac, or enteric agents.

The active compound or pharmaceutically acceptable salt or derivative thereof can be administered as a component of an elixir, suspension, syrup, wafer, chewing gum or the like. A syrup may contain, in addition to the active compounds, sucrose as a sweetening agent and certain preservatives, dyes and colorings and flavors.

The active compound or pharmaceutically acceptable derivatives or salts thereof can also be mixed with other active materials that do not impair the desired action, or with materials that supplement the desired action, such as antibiotics, antifungals, other antiinflammatories, or antiviral compounds.

Solutions or suspensions used for parenteral, intradermal, subcutaneous, or topical application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parental preparation can be enclosed in ampoules, disposable syringes or multiple dose vials made of glass or plastic.

If administered intravenously, preferred carriers are physiological saline or phosphate buffered saline (PBS).

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation (CA) and Scios Nova (Baltimore, MD).

Liposomal suspensions may also be pharmaceutically acceptable carriers. These may be prepared according to methods known to those skilled in the art, for example, as described in U.S. Patent No. 4,522,811 (which is incorporated herein by reference in its entirety). For example, liposome formulations may be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the active compound or its monophosphate, diphosphate, and/or triphosphate derivatives are then introduced into the container. The container is then swirled by hand to free lipid material from the sides of the container and to disperse lipid aggregates, thereby forming the liposomal suspension.

## III. Biological Activity

A wide variety of biological assays have been used to evaluate the ability of a compound to act as a PAF receptor antagonist, including the ability of the compound to bind to PAF receptors, and the effect of the compound on various PAF mediated pathways. Any of these known assays can be used to evaluate the ability of the compounds disclosed herein to act as PAF receptor antagonists.

For example, PAF is known to induce hemoconcentration and increased permeability of microcirculation leading to a decrease in plasma volume. PAF mediated acute circulatory collapse can be used as the basis of an assay to evaluate the ability of a compound to act as a PAF antagonist, by analyzing the effect of the compound on PAF induced decreased plasma volume in an animal model such as mouse.

Endotoxemia causes the release of chemical mediators including eicosanoids, PAF, and tumor necrosis factor (TNF) that stimulate a variety of physiologic responses including fever, hypotension, leukocytosis, and disturbances in glucose and lipid metabolism. Endotoxemia can result in severe shock and death. Endotoxin-induced mouse mortality is a useful animal model to evaluate the pharmacological effect of compounds on endotoxic shock.

Two other common assays used to evaluate the ability of a compound to act as a PAF receptor antagonist are platelet aggregation in vitro and hypotension in rats (Shen, *et al*., "The Chemical and Biological Properties of PAF Agonists, Antagonists, and Biosynthetic Inhibitors", Platelet-Activating Factor and Related Lipid Mediators, F. Snyder, Ed. Plenum Press, New York, NY 153 (1987)).

A wide variety of biological assays have also been used to evaluate the ability of a compound to inhibit the enzyme 5-lipoxygenase. For example, a cytosol 5-lipoxygenase of rat basophilic leukemia cells (RBL) has been widely utilized in studies on leukotriene biosynthesis. Compounds that inhibit 5-lipoxygenase decrease the levels of leukotrienes.

Another biological assay used to evaluate the ability of a compound to inhibit the enzyme 5-lipoxygenase is based on the classic pharmacological model of inflammation induced by the topical application of arachidonic acid to the mouse ear. On application, arachidonic acid is converted by 5-lipoxygenase to various leukotrienes (and other mediators), which induce changes in blood flow, erythema, and increase vasodilation and vasopermeability. The resulting edema is measured by comparing the thickness of the treated ear to a control ear. Agents that inhibit 5-lipoxygenase reduce the edematous response, by lowering the amounts of biochemical mediators formed from arachidonic acid.

### Example 19: Ability of Compound to Bind to PAF Receptors

a) Preparation of Human Platelet Membranes:

Human platelet membranes were prepared from platelet concentrates obtained from the American Red Cross Blood Services (Dedham, MA). After several washes with platelet wash solution (150 mM NaCl, 10 mM Tris, and 2 mM EDTA, pH 7.5), the platelet pellets were resuspended in 5 mM $MgCl_2$, 10 mM Tris, and 2 mM EDTA at pH 7.0. The cells were then quickly frozen with liquid nitrogen and thawed slowly at room temperature. The freezing and thawing procedure was repeated at least three times. For further fractionation of membrane fragments, the lysed membrane suspension was layered over the top of a discontinuous sucrose density gradient of 0.25, 1.03, and 1.5 M sucrose prepared in 10 mM $MgCl_2$, 10 mM Tris and 2 mM EDTA, pH 7.0, and centrifuged at 63,500 x g for 2 hr. The membrane fractions banding between 0.25 and 1.03 M (membrane A) and between 1.03 and 1.5 M (membrane B) were collected separately. The protein concentration of the membrane preparations was determined by Lowry's method with bovine serum albumin (BSA) as the standard. The membranes were then separated into smaller fractions (4 ml each) and stored at -80°C and thawed before use.

b) [$^3$H]PAF Binding inhibition:

The ability of [$^3$H]PAF to bind to specific receptors on human platelet membranes was evaluated at optimal conditions at pH 7.0 and in the presence of 10 mM $MgCl_2$. Membrane protein (100 μg) was added to a final 0.5 ml solution containing 0.15 pmol (0.3 nM concentration) of [$^3$H]PAF and a known amount of unlabeled PAF or PAF receptor antagonist in 10 mM $MgCl_2$, 10 mM Tris and 0.25% BSA at pH 7.0. After incubation for four hours at 0°C, the bound and unbound [$^3$H]PAF were separated through a Whatman GF/C glass fiber filter under vacuum. No degradation of filter bound [$^3$H]PAF has been detected under this assay condition. The nonspecific binding was defined as the total binding in the presence of excess unlabeled PAF (1 mM) where no further displacement was found with higher concentrations of either unlabeled PAF or PAF analogs or PAF receptor antagonists. The specific binding was defined as the difference between total binding and nonspecific binding.

To determine the relative potency of tested compounds, [$^3$H]PAF binding in the presence of inhibitors was normalized in terms of percent inhibition by assigning the total binding in the absence of inhibitors as 0% inhibition and the total binding in the presence of 1 mM unlabeled PAF as 100%. The percent inhibition by the compound can be calculated by the formula expressed below:

% inhibition = [(Total binding - total binding in the presence of compound)/nonspecific binding] x 100%

The $IC_{50}$ was calculated as the concentration of the inhibitor necessary to obtain 50% inhibition of the specific [$^3$H]PAF binding and was calculated by a nonlinear regression computer software program, GraphPad Inplot, version 3.0 (GraphPad software, San Diego, CA). Tables 1-6 provide $IC_{50}$ values for a number of the disclosed compounds.

**Table 1**

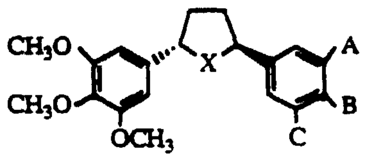

| Cmpd No | X | A | B | C | PAF IC50 | *5-LO |
|---|---|---|---|---|---|---|
| 1 | S | $SCH_2CH_2NH_2$ | $OCH_3$ | $OCH_3$ | | |
| 2 | S | $O_2CH_2CH_2NH_2$ | $OCH_3$ | $OCH_3$ | >1000 | 0.0% |
| 3 | O | $HCH_2Ph$ | $OCH_2CH_2CH_3$ | $OCH_3$ | | 21.5% |
| 4 | O | $NHCH_2CH_2OH$ | $OCH_2CH_2CH_3$ | $OCH_3$ | 136 | 0.0% |
| 5 | O | $N(CH_2CH{=}CH_2)_2$ | $OCH_3CH_2CH3$ | $OCH_3$ | 98 | 10.0 |
| 6 | S | $NHCH_2Ph$ | $OCH_3$ | $OCH_3$ | >1000 | 4.1% |
| 7 | S | $NHCH_2Ph$ | $OCH_2CH_2CH_3$ | $OCH_3$ | >1000 | 16.2% |
| 8 | S | $NHCH_2CH_2OH$ | $OCH_3$ | $OCH_3$ | 157 | 6.0% |
| 9 | S | $NCH_2CH_2OH_2$ | $OCH_2CH_2CH_3$ | $OCH_3$ | 91.5 | 18.8% |
| 10 | S | $N(CH_2CH{=}CH_2)_2$ | $OCH_3$ | $OCH_3$ | | |
| 11 | S | $N(CH_2CH{=}CH_2)_2$ | $OCH_2CH_2CH_3$ | $OCH_3$ | | |

*Inhibitions shown by percentage are at 10μM

Table 2

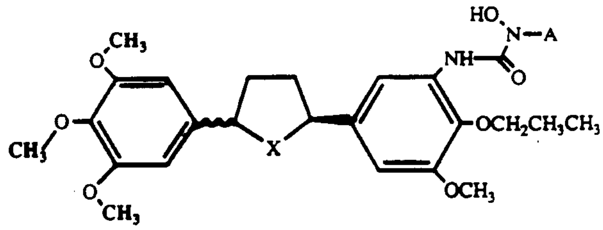

| No. | Isomer | X | A | IC50 PAF (nM) | IC50 5-LO (µM) | 5-LO inhibition* |
|---|---|---|---|---|---|---|
| 12. | trans | O | H | 30 | | 22% |
| 13. | trans | O | $CH_3$ | 17 | 6.9 | |
| 38. | cis | O | $CH_3CH_2$ | 5.1 | 0.48 | |
| 14. | trans | O | $CH(CH_3)_2$ | 57 | | 40% |
| 39. | trans | O | $CH_3(CH_2)_3$ | 34.5 | 0.3 | |
| 15. | trans | O | $C(CH_3)_3$ | 25 | 2.9 | |
| 16. | trans | O | $C_6H_{11}$ | 278 | | 24% |
| 17. | trans | O | $CH_2\phi$ | 423 | 2.24 | |
| 18. | cis | O | H | 300 | | 28% |
| 19. | cis | O | $CH_3$ | 514 | | 56% |
| 40. | cis | O | $CH_3CH_2$ | 382 | 3 | |
| 20. | cis | O | $CH(CH_3)_2$ | 858 | 2.83 | |
| 41. | cis | O | $CH_3(CH_2)_3$ | 1313 | 0.5 | |
| 21. | cis | O | $C(CH_3)_3$ | 456 | | 54% |
| 22. | cis | O | $C_6H_{11}$ | >1000 | 2.5 | |
| 23. | cis | O | $CH_2\phi$ | 585 | 8 | |
| 24. | trans | S | H | 14 | 1 | |
| 25. | trans | S | $CH_3$ | 46 | 1 | |
| 26. | trans | S | $CH(CH_3)_2$ | 96.5 | 0.5 | |
| 27. | trans | S | $C(CH_3)_3$ | 46 | 0.5 | |
| 28. | cis | S | H | 496 | 3 | |
| 29. | cis | S | $CH_3$ | 300 | 0.64 | |
| 30. | cis | S | $CH(CH_3)_2$ | 828 | 2 | |
| 31. | cis | S | $C(CH_3)_3$ | 334 | 1.5 | |
| 32. | cis | S | $C_6H_{11}$ | 1287 | 2 | |

*All inhibitions shown by percentage are at 10 µM

Table 3

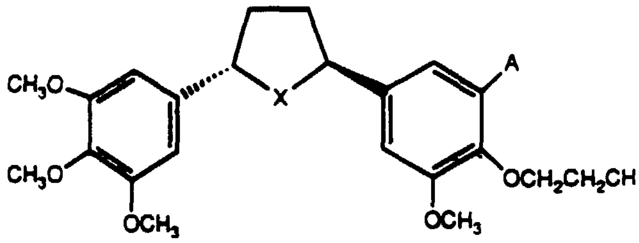

| Cmpd No | X | A | PAF | 5-LO | inhibition* |
|---|---|---|---|---|---|
| | | | IC50 (nM) | IC50 µM | |
| 33 | O | $CH_2NHCONH(OH)$ | 280 | | 32.9% |
| 34 | O | $CH_2NHCONCH_3(OH)$ | 16.7 | 1.6 | |
| 35 | O | $CH_2NHCONi\text{-}Pr(OH)$ | 284 | 2.5 | |
| 36 | O | $CH_2NHCONC_6H_{11}(OH)$ | 420 | 1.7 | |
| 37 | O | $CH_2NHCOBn(OH)$ | 73.6 | 0.4 | |

*All inhibition shown in percentage is at 10 µM

Table 4

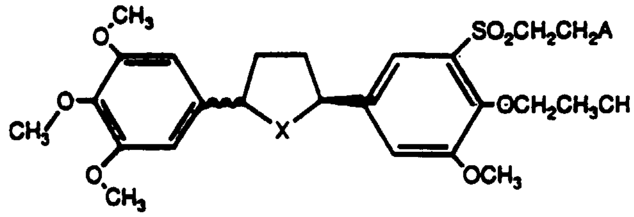

| No. | Isomer | X | A | PAF $IC_{50}$ (nM) | 5-LO inhibition* |
|---|---|---|---|---|---|
| 42. | trans | S | CH | 30 | 10.5% |

* Inhibition shown by percentage is at 10 μM

Table 5

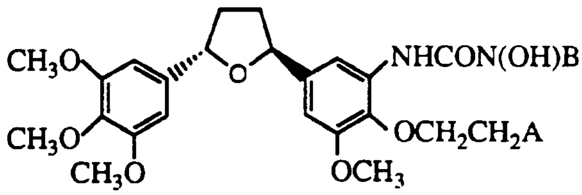

| Compounds | A | B | IC50(nM) PAF | 5-LO |
|---|---|---|---|---|
| 201 | S-Ph-pBr* | $CH_2CH_2CH_2CH_3$ | 20.9 | 18.7 |
| 202 | $SO_2$-Ph-p-Br | $CH_2CH_2CH_2CH_3$ | 38.3 | |
| 203 | S-Ph-2-Br | $CH_2CH_2CH_2CH_3$ | 23.0 | 33.0 |
| 204 | $SO_2$-Ph-2-Br | $CH_2CH_2CH_2CH_3$ | 25.0 | 161.0 |
| 205 | S-Ph-3-Br | $CH_2CH_2CH_2CH_3$ | 16.0 | 43.3 |
| 206 | S-Ph-p-F | $CH_2CH_2CH_2CH_3$ | 45.0 | 63.8 |
| 207 | S-Ph-2,3,5,6-F | $CH_2CH_2CH_2CH_3$ | 2.2 | 118.4 |
| 208 | $SO_2$-Ph-2,3,5,6-F | $CH_2CH_2CH_2CH_3$ | 285.3 | 520.2 |
| 209 | O-Ph-2,3,5,6-F | $CH_2CH_2CH_2CH_3$ | 55.3 | 132.8 |
| 210 | S-Ph-p-Cl | $CH_2CH_2CH_2CH_3$ | 10.0 | 58.5 |
| 211 | S-Ph-3,4-Cl | $CH_2CH_2CH_2CH_3$ | 45.0 | 17.4 |
| 212 | S-Ph-p-OH | $CH_2CH_2CH_2CH_3$ | 5.53 | 180.0 |
| 213 | S-Ph-p-$OCH_3$ | $CH_2CH_2CH_2CH_3$ | 39.2 | 71.2 |
| 214 | S-Ph-p-CN | $CH_2CH_2CH_2CH_3$ | 62.6 | 62.3 |

* Ph = phenyl

| | | | | |
|---|---|---|---|---|
| 215 | $SCH_3$ | $CH_2CH_2CH_2CH_3$ | 13.5 | 190.0 |
| 216 | $OCH_3$ | $CH_2CH_2CH_2CH_3$ | | 195.2 |
| 217 | 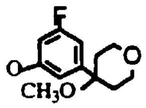 | $CH_2CH_2CH_2CH_3$ | 281.0 | 87.0 |
| 218 | $OCH_2$- 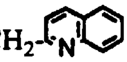 .HCl | $CH_2CH_2CH_2CH_3$ | | 390.6 |
| 219 | 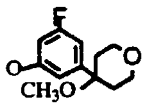 | $CH_3$ | 321.2 | 719.0 |
| 220 | 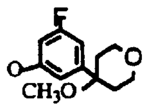 | $CH_2Ph$ | 622.7 | 900.9 |
| 221 | 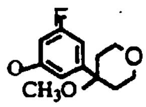 | $CH_2CH_2CH_2CH_3$ | 321.8 | 366.3 |
| 222 | N(Ph)CO- 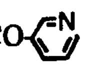 | $CH_2CH_2CH_2CH_3$ | 16.3 | 479.0 |
| 223 | N(Ph)CO- 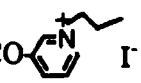 $I^-$ | $CH_2CH_2CH_2CH_3$ | 197.4 | |
| 224 | N(Ph)CO- 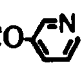 | Ph-p-Cl | 84.2 | |
| 225 | N(Ph)CO- 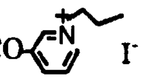 $I^-$ | Ph-p-Cl | 6285 | 670.0 |
| 226 | $CH_3$ | Ph-p-Cl | 217.6 | 533.0 |

| | | | | |
|---|---|---|---|---|
| 227 | S-Ph-p-OH | Ph-p-Cl | 26.9 | 3000 |
| 228 | $SCH_3$ | Ph-p-Cl | 317.7 | 3000 |

**Table 6**

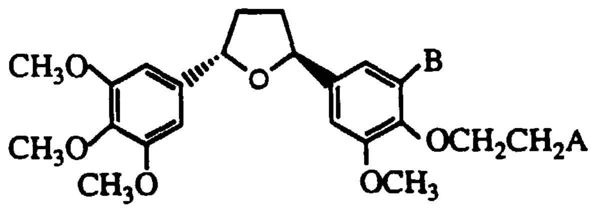

| Compounds | A | B | $IC_{50}$(nM) PAF | 5-LO |
|---|---|---|---|---|
| 229 | S-Ph-p-Cl | $CH_2NHCON(OH)CH_3$ | 7.60 | 22.2 |
| 230 | S-Ph-p-Cl | $CH_2N(CH_2CH_2CH_3)CON(OH)CH_3$ | 7.40 | |
| 231 | S-Ph-p-Cl | $CH_2N(OH)CONH_2$ | 33.2 | 34.2 |
| 232 | S-Ph-p-Cl | $CH_2N(OH)CONHCH_3$ | 7.06 | 185.0 |
| 233 | S-Ph-p-Cl | $NHCOCH_2N(OH)CONH_2$ | 47.5 | 318.0 |
| 234 | S-Ph-p-Cl | $NHCOCH_2N(OH)CONHCH_3$ | 3318.8 | |
| 235 | O-Ph-p-F | $\equiv-CH_2N(OH)CONH_2$ | 73.9 | 828.2 |
| 236 | S-Ph-p-Cl | $\equiv-CH_2N(OH)CONH_2$ | | 11.3 |

**Example 20: Effect of Compound on PAF-induced Hemoconcentration**

a) Animals

Female CD-1 mice, weighing 16-20 grams, were obtained from Charles River Laboratory (Wilmington, MA). Tap water and rodent laboratory chow (5001, Purina Mills, St. Louis, MO) were provided ad libitum. The mice were housed for an average of four days prior to use.

b) Hematocrit measurement

PAF (1-O-alkyl-2-acetyl-*sn*-glyceryl-3-phosphorylcholine, Sigma Chemical Co.) was dissolved in 0.25% bovine serum albumin (BSA) in 0.9% NaCl solution. Except for dose-response studies, 10 μg (10 ml/kg) of PAF solution was injected into the tail vein. All test compounds were dissolved in 0.5 DMSO saline solution and intravenously injected at 3 mg/kg body weight 15 minutes prior to PAF challenge. Thirty to fifty μL blood was collected by cutting the tail end into a heparinized micro-hematocrit tube (O.D. 1.50 mm) 15 minutes after PAF administration. Table 7 provides the mouse hematocrit response to varying concentration of PAF at 15 minutes after injection of PAF. Tables 8-10 provide the effect of various test compounds on PAF-induced mouse hemoconcentration; the reference compound MK287 is *trans*-2-(3,4,5-trimethoxy)-5-(3-methoxy-4-oxyallyl-(2-hydroxyethylsulfonyl))-tetrahydrofuran. (Sahoo, *et al*., Bioorganic Medicinal Chem. Letters, (1991), 1, 327.)

Table 7

| Mouse Hematocrit Response to Varying Concentration of PAF at 15 Minutes After Injection of PAF | | | |
|---|---|---|---|
| Doses of PAF (μg /kg) | Animal number | Hematocrit (%) | |
| | | Mean | SEM |
| 0 | 5 | 45.4 | 0.5 |
| 0.049 | 5 | 45.2 | 0.3 |
| 0.195 | 5 | 48.2 | 0.6 |
| 0.781 | 5 | 52.0 | 2.5 |
| 3.125 | 5 | 62.0 | 1.8 |
| 12.5 | 5 | 68.0 | 1.2 |
| 50 | 5 | 72.4 | 1.2 |
| 200 | 5 | 75.8 | 1.2 |

Table 8

| Effect of Test Compounds on PAF-Induced Mouse Hemoconcentration | | | |
|---|---|---|---|
| Compound | Animal number | Hematocrit(%) | |
| | | Mean | SEM |
| Vehicle | 11 | 66.5 | 1.5 |
| Compound | | | |
| 42 | 5 | 45.2 | 0.6 |
| 14 | 5 | 54.4 | 2.4 |
| 12 | 5 | 61.1 | 1.2 |
| 13 | 5 | 62.3 | 2.1 |
| 15 | 5 | 64.6 | 1.5 |

Table 9

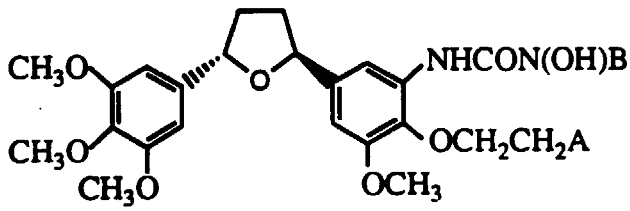

| Compounds | A | B | InH (%)* | |
|---|---|---|---|---|
| | | | PAF-Htc | AA-Ed |
| 201 | S-Ph-p-Br | $CH_2CH_2CH_2CH_3$ | -10.5 | -2.7 |
| 202 | $SO_2$-Ph-p-Br | $CH_2CH_2CH_2CH_3$ | 29.3 | 34.5 |
| 203 | S-Ph-2-Br | $CH_2CH_2CH_2CH_3$ | 34.2 | 26.3 |
| 204 | $SO_2$-Ph-2-Br | $CH_2CH_2CH_2CH_3$ | 60.4 | -9.1 |
| 205 | S-Ph-3-Br | $CH_2CH_2CH_2CH_3$ | 28.2 | 40.2 |
| 206 | S-Ph-p-F | $CH_2CH_2CH_2CH_3$ | 33.6 | |
| 207 | S-Ph-2,3,5,6-F | $CH_2CH_2CH_2CH_3$ | 58.8 | 30.4 |
| 208 | $SO_2$-Ph-2,3,5,6-F | $CH_2CH_2CH_2CH_3$ | 50.4 | 11.9 |
| 209 | O-Ph-2,3,5,6-F | $CH_2CH_2CH_2CH_3$ | 59.1 | 29.2 |
| 210 | S-Ph-p-Cl | $CH_2CH_2CH_2CH_3$ | 25.3 | 39.2 |
| 211 | S-Ph-3,4-Cl | $CH_2CH_2CH_2CH_3$ | 26.1 | 26.3 |
| 212 | S-Ph-p-OH | $CH_2CH_2CH_2CH_3$ | 33.5 | 49.9 |
| 213 | S-Ph-p-$OCH_3$ | $CH_2CH_2CH_2CH_3$ | 23.6 | 2.7 |
| 214 | S-Ph-p-CN | $CH_2CH_2CH_2CH_3$ | -12.4 | 46.5 |
| 215 | $SCH_3$ | $CH_2CH_2CH_2CH_3$ | 11.1 | 41.1 |

| | | | | |
|---|---|---|---|---|
| 216 | $OCH_3$ | $CH_2CH_2CH_2CH_3$ | 11.2 | |
| 217 | 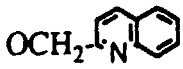 | $CH_2CH_2CH_2CH_3$ | 26.1 | 57.0 |
| 219 | 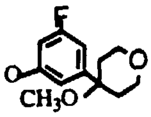 | $CH_3$ | 49.6 | 47.8 |
| 220 | 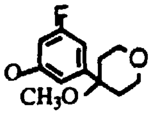 | $CH_2Ph$ | 63.1 | 49.9 |
| 221 | 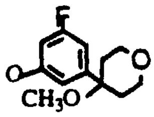 | $CH_2CH_2CH_2CH_3$ | 70.4 | 57.0 |
| 226 | $CH_3$ | Ph-p-Cl | | 23.7 |

* All test compounds were given intravenously at 3 mg/kg 15 minutes before PAF (10 ug/kg, intravenously) or AA (0.5 mg/ear) in mice.

## Table 10

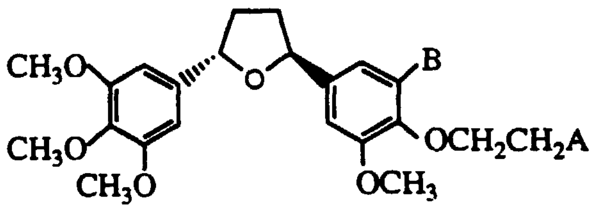

| Compounds | A | B | InH (%) * | |
|---|---|---|---|---|
| | | | PAF-Htc | AA |
| Ed | | | | |
| 229 | S-Ph-p-Cl | $CH_2NHCON(OH)CH_3$ | 55.7 | 45. |
| 231 | S-Ph-p-Cl | $CH_2N(OH)CONH_2$ | 57.9 | 23. |
| 232 | S-Ph-p-Cl | $CH_2N(OH)CONHCH_3$ | 41.1 | 10. |
| 233 | S-Ph-p-Cl | $NHCOCH_2N(OH)CONH_2$ | 2.1 | 51. |
| 234 | S-Ph-p-Cl | $NHCOCH_2N(OH)CONHCH_3$ | -9.8 | 31.0 |
| 235 | O-Ph-p-F | $\equiv - CH_2N(OH)CONH_2$ | 99.5 | 24.5 |

* All test compounds were given intravenously at 3 mg/kg 15 minutes before PA (10 ug/kg, intravenously) or AA (0.5 mg/ear) in mice.
Ph = phenyl

**Example 21: Effect of 2,5-Diaryl Tetrahydrothiophenes and Tetrahydrofurans on Arachidonic Acid-induced-Mouse Ear Edema**

a) Animals

The animals were obtained and treated as in Example 20 above.

b) Edema measurement

Arachidonic add was applied to both ears of mice in 0.025 ml of freshly prepared vehicle (acetone:pyridine:water) (97:2:1 v/v/v) and dried under a Sun-Lite Hitensity bulb. Except for dose-response studies, 0.5 mg of arachidonic acid was used for all applications. All test compounds were dissolved in 0.5% DMSO saline solution and intravenously injected at 3 mg/kg body weight 15 minutes prior to arachidonic acid treatment. Animals were sacrificed by cervical dislocation at 1 hour after topical application of arachidonic acid. A 7 mm-diameter disc of tissue was removed from each ear by means of a metal punch. Edema was measured by the average wet weight of the both ear tissues.

Tables 9,10, and 12 provide the effect of various test compounds on arachidonic acid induced mouse ear edema. Table 11 provides the mouse ear edematous response to varying concentrations of arichidonic acid at 1 hour after topical application.

Table 11

| Mouse Ear Edema in Response to Varying Concentration of Arachidonic Acid at 1 Hour After Topical Application | | | |
|---|---|---|---|
| Doses of AA (mg/ear) | Ear number | Ear tissue weight (mg) | |
| | | Mean | SEM |
| 0 | 4 | 12.4 | 0.08 |
| 0.125 | 4 | 15.8 | 0.63 |
| 0.25 | 4 | 14.9 | 1.05 |
| 0.5 | 4 | 25.1 | 2.88 |
| 1.0 | 4 | 29.8 | 2.56 |
| 2.0 | 4 | 30.3 | 1.67 |

Table 12

| Effect of Test Compounds on Arachidonic Acid-Induced Mouse Ear Edema | | |
|---|---|---|
| Compound | Animal number | Inhibition (%) |
| 15 | 8 | 54.5 |
| 13 | 8 | 29.0 |
| 42 | 4 | 18.0 |

**Example 22: Effect of 2,5-Diaryl Tetrahydrothiophenes and Tetrahydrofurans on Endotoxin-induced Mouse Mortality**

a) Animals

The mice are obtained and treated as in Example 20 above.

b) Mortality Measurement

Endotoxin (*E. coli* serotype 0127:B8, lipopolysaccharide, Sigma Chemical Co. (St. Louis, MO) are freshly dissolved in 0.9% NaCl solution. Except for dose-response studies, endotoxin at 50 mg/kg is injected into the tail vein. All test compounds are dissolved in 0.5% DMSO saline solution and intravenously injected at 3 mg/kg body weight 15 minutes prior to PAF challenge. Death occurs typically within 12-36 hours. Mortality is recorded 48 hours after endotoxin

challenge, as death rarely occurs after 48 hours. The results of these evaluations are provided in Tables 13 and 14.

**Example 23: Effect of Compounds on Cytosol 5-Lipoxygenase of Rat Basophile Leukemia Cells**

a) Enzyme preparation

Washed rat RBL cells (4x108) are suspended in 20 ml of 50 M potassium phosphate buffer at pH 7.4 containing 10% ethylene glycol/1 mM EDTA (Buffer A). The cell suspension is sonicated at 20 KHz for 30 seconds, and the sonicate is centrifuged at 10,000 x g for 10 minutes, followed by further centrifugation at 105,000 x g for 1 hr. The supernatant solution (cytosol fraction) containing 5-lipoxygenase is stored at - 70°C. Protein concentration is determined according to the procedure of Bradford (Bradford Dye Reagent) with bovine serum albumin as a standard.

b) Enzyme assay

For routine assay of 5-LO the mixture contains 50 mM potassium phosphate buffer at pH 7.4, 2 mM $CaCl_2$, 2 mM ATP, 25 M arachidonic acid (0.1 Ci) and enzyme (50-100 mg of protein) in a final volume of 200 L. The reaction is carried out at 24°C for 3 minutes. The mixture is extracted with 0.2 ml of an ice-cold mixture of ethyl ether:methanol: 0.2 M citric acid (30:4:1). The extract is subjected to thin-layer chromatography at -10°C in a solvent system of petroleum ether:ethyl ether:acetic acid (15:85:0.1). The silica gel zones corresponding to authentic arachidonic acid and its metabolites are scraped into scintillation vials for counting. The enzyme activity is expressed in terms of the amount of arachidonic acid oxygenated for 3 minutes.

Table 13

| Mouse Mortality in Response to Varying Concentration of Endotoxin Within 48 Hours After I.V. Injection of Endotoxin | | |
|---|---|---|
| Doses of endotoxin (mg /kg) | Animal number | Survival (%) |
| 12.5 | 10 | 100 |
| 25 | 10 | 80 |
| 37.5 | 10 | 50 |
| 50 | 10 | 0 |
| 75 | 10 | 0 |

Table 14

| Effect of Test Compounds on Endotoxin-Induced Mouse Mortality | | |
|---|---|---|
| Compound | Animal number | Survival (%) |
| 13 | 6 | 83 |
| 15 | 5 | 17 |

## Claims

**1.** A compound of the formula:

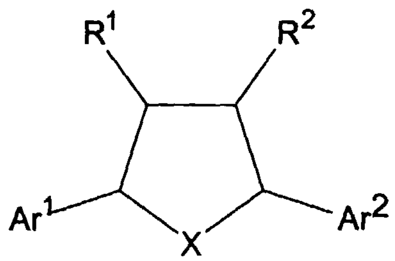

wherein

$Ar^1$ =

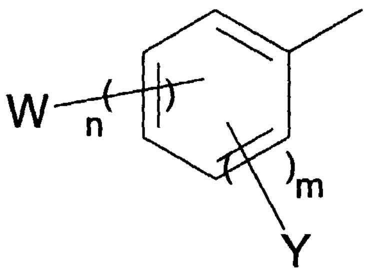

$Ar^2$ =

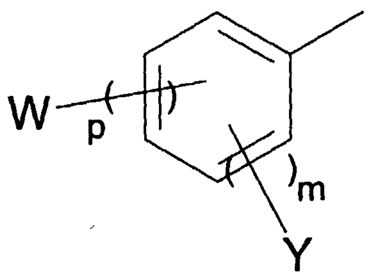

wherein:

X is O, S;
W is independently :

$-AN(OM)C(O)N(R^3)R^4$, $-AN(R^3)C(O)N(OM)R^4$, $-N(OM)C(O)N(R^3)R^4$ or $-N(R^3)C(O)N(OM)R^4$;

n is 1;
m is 2 or 3;
p is 0;
A is $C_{1-10}$ alkylene, $C_{2-10}$ alkenylene or $C_{2-10}$ alkynylene;
M is hydrogen;
Y is independently $-OR^{11}$
$R^1$ and $R^2$ are hydrogen;
$R^3$ and $R^4$ are independently $C_{1-10}$ alkyl, $C_{2-10}$ alkenyl or $C_{2-10}$ alkynyl;
$R^{11}$ is $C_1$ to $C_{12}$ alkyl or substituted $C_1$ to $C_{12}$ alkyl wherein the substituent is selected from the group consisting of

phenyl-S(O)$_g$-C$_{1-6}$ alkylene-; (R$^3$O)$_d$-phenyl-S(O)$_g$-C$_{1-6}$ alkylene-; (CN)$_d$-phenyl-S(O)$_g$-C$_{1-6}$ alkylene-; (halo)$_d$-phenyl-S(O)$_g$-C$_{1-6}$ alkylene-; (R$^3$COO)$_d$-phenyl-S(O)$_g$-C$_{1-6}$ alkylene-; (R$^3$OCO)$_d$-phenyl-S(O)$_g$-C$_{1-6}$ alkylene-; (R$^3$CO)$_d$-phenyl-S(O)$_g$-C$_{1-6}$ alkylene; phenyl-O-C$_{1-6}$ alkylene-; (R$^3$O)$_d$-phenyl-O-C$_{1-6}$ alkylene-; (CN)$_d$-phenyl-O-C$_{1-6}$ alkylene-; (halo)$_d$-phenyl-O-C$_{1-6}$ alkylene-; (R$^3$COO)$_d$-phenyl-O-C$_{1-6}$ alkylene-; (R$^3$OCO)$_d$-phenyl-O-C$_{1-6}$ alkylene-; (R$^3$R$^3$N)$_d$phenyl-S(O)$_g$-C$_{1-6}$ alkylene; or (R$^3$CO)$_d$-phenyl-O-C$_{1-6}$ alkylene- where d is 1, 2, 3, 4 or 5; and g is 0, 1, or 2;

**2.** A compound which is

2-[5-(N'-Butyl-N'-hydroxyureidyl)-3-methoxy-4-methoxyethoxyphenyl]-5-(3,4,5-trimethoxyphenyl)-tetrahydrothiophene.

2-[5-(N'-Butyl-N'-hydroxyureidyl)-3-methoxy-4-methoxythioethoxyphenyl]-5-(3,4,5-trimethoxyphenyl)-tetrahydrofuran.

2-[5-(N'Butyl-N'-hydroxyureidyl)-4-hydroxyphenylthioethoxy-3-methoxyphenyl]-5-(3,4,5-trimethoxyphenyl)-tetrahydrofuran.

2-[5-(N'-Butyl-N'-hydroxyureidyl)-4-(N-nicotinoyl-N-phenylaminoethoxy)-3-methoxyphenyl]-5-(3,4,5-trimethoxyphenyl)-tetrahydrofuran.

2-[5(N'-Butyl-N'-hydroxyureidyl)-4-(N-3-pyridiniumcarbonyl-N-phenylaminoethoxy)-3-methoxyphenyl]-5-(3,4,5-trimethoxyphenyl)-tetrahydrofuran-propyl iodide.

2-[5-(N'-p-Chlorophenyl-N'-hydroxyureidyl)-4-(N-3-pyridiniumcarbonyl-N-phenylaminoethoxy)-3-methoxyphenyl]-5-(3,4,5-trimethoxyphenyl)-tetrahydrofuran-propyl iodide.

2-[5-(N'Butyl-N'-hydroxyureidyl)-4-(N-3-pyrdiniumcarbonyl-N-phenylaminoethoxy)-3-methoxyphenyl]-5-(3,4,5-trimethoxyphenyl)-tetrahydrofuran-ethyl iodide.

2-[5-(N'-p-Chlorophenyl-N'-hydroxyureidyl)-4-(N-3-pyridiniumcarbonyl-N-phenylaminoethoxy)-3-methoxyphenyl]-5-(3,4,5-trimethoxyphenyl)-tetrahydrofuran-ethyl iodide.

2-[5-(N'-p-Chlorophenyl-N'-hydroxyureidyl)-3-methoxy-4-propoxyphenyl)-5-(3,4,5-trimethoxyphenyl)-tetrahydrofuran.

2-[5-(N'-p-Chlorophenyl-N'-hydroxyureidyl)-4-methoxyethoxy-3-methoxyphenyl]-5-(3,4,5-trimethoxyphenyl)-tetrahydrothiophene.

2-[5-(N'-p-Chlorophenyl-N'-hydroxyureidyl)-4-methylthioethoxy-3-methoxyphenyl]-5-(3,4,5-trimethoxyphenyl)-tetrahydrofuran.

2-[5-(N'-p-Chlorophenyl-N'-hydroxyureidyl)-4-p-hydroxyphenylthioethoxy-3-methoxyphenyl]-5-(3,4,5-triethoxyphenyl)-tetrahydrofuran.

2-[5-(N'-p-Chlorophenyl-N'-hydroxyureidyl)-4-(N-nicotinoyl-N-phenylaminoethoxy)-3-methoxyphenyl]-5-(3,4,5-trimethoxyphenyl)-tetrahydrofuran.

2-[5-(N'-Butyl-N'-hydroxyureidyl)-4-p-cyanophenylthioethoxy-3-methoxyphenyl]-5-(3,4,5-trimethoxyphenyl)-tetrahydrofuran.

2-[5-(N'-p-Chlorophenyl-N'-hydroxyureidyl)-4-p-cyanophenylthioethoxy-3-methoxyphenyl]-5-(3,4,5-trimethoxyphenyl)-tetrahydrofuran.

2-[5-(N'-Butyl-N'-hydroxyureidyl)-4-p-methoxyphenylthioethoxy-3-methoxyphenyl]-5-(3,4,5-trimethoxyphenyl)-tetrahydrofuran.

2-[5-(N'-p-Chlorophenyl-N'-hydroxyureidyl)-4-p-methoxyphenylthioethoxy-3-methoxyphenyl]-5-(3,4,5-trimethoxyphenyl)-tetrahydrofuran.

2-[5-(N'-*tert*-Butyl-N'-hydroxyureidyl)-3-methoxy-4-propoxyphenyl]-5-(3,4,5-trimethoxyphenyl)- tetrahydrothiophene.

2-[5-(N'-n-Butyl-N'-hydroxyureidyl)-3-methoxy-4-propoxyphenyl]-5-(3,4,5-trimethoxyphenyl)-tetrahydrofuran.

2-[5(N'-Ethyl-N'-hydroxyureidyl)-3-methoxy-4-propoxyphenyl]-5-(3,4,5-trimethoxyphenyl)-tetrahydrofuran.

2-[5-(N'-Cyclohexyl-N'-hydroxyureidyl)-3-methoxy-4-propoxyphenyl]-5-(3,4,5-trimethoxyphenyl-tetrahydrofuran.

2-[5-N'-Benzyl-N'-hydroxyureidyl)-3-methoxy-4-propoxyphenyl]-5-(3,4,5-trimethoxyphenyl)-tetrahydrofuran.

2-(5-N'-Hydroxyureidyl-3-methoxy-4-propoxyphenyl)-5-(3,4,5-trimethoxyphenyl)-tetrahydrothiophene.

2-[5-(N'-Hydroxy-N'-methylureidyl)-3-methoxy-4-propoxyphenyl]-5-(3,4,5-trimethoxyphenyl)-tetrahydrothiophene.

2-[5-(N'-Hydroxy-N'-i-propylureidyl)-3-methoxy-4-propoxyphenyl]-5-(3,4,5-trimethoxyphenyl)-tetrahydrothiophene.

2-[5-(N'-sec-Butyl-N'-hydroxyureidyl)-3-methoxy-4-propoxyphenyl]-5-(3,4,5-trimethoxyphenyl)-tetrahydrothiophene.

2-[5-(N'-Hydroxy-N'-propylureidyl)-3-methoxy-4-propoxyphenyl]-5-(3,4,5-trimethoxyphenyl)-tetrahydrothiophene.

2-[5-(N'-Hydroxy-N'-propylureidyl)-3-methoxy-4-propoxyphenyl]-5-(3,4,5-trimethoxyphenyl)-tetrahydrofuran.

2-[5-(N'-Hydroxy-N'-n-pentylureidyl)-3-methoxy-4-propoxyphenyl]-5-(3,4,5-trimethoxyphenyl)-tetrahydrofuran.

2-[5-(N'-Cyclohexyl-N'-hydroxyureidyl)-3-methoxy-4-propoxyphenyl]-5-(3,4,5-trimethoxyphenyl)-tetrahydrofuran.

2-[5-(N'-Hexyl-N'-hydroxyureidyl)-3-methoxy-4-propoxyphenyl]-5-(3,4,5-trimethoxyphenyl)-tetrahydrofuran.

2-[5-(N'-Benzyl-N'-hydroxyureidyl)-3-methoxy-4-propoxyphenyl]-5-(3,4,5-trimethoxyphenyl)-tetrahydrofuran.

2-[5-(N'-Hydroxy-N'-octylureidyl)-3-methoxy-4-propoxyphenyl]-5-(3,4,5-trimethoxyphenyl)-tetrahydrofuran.

2-[5-(N'-Hydroxy-N'-methoxyethylureidyl)-3-methoxy-4-propoxyphenyl]-5-(3,4,5-trimethoxyphenyl)-tetrahydrofuran.

2-[5-(N'-Decyl-N'-hydroxyureidyl)-3-methoxy-4-propoxyphenyl]-5-(3,4,5-trimethoxyphenyl)-tetrahydrofuran.

2-[5-(N'-Hydroxy-N'-n-pentylureidyl)-3-methoxy-4-propoxyphenyl]-5-(3,4,5-trimethoxyphenyl)-tetrahydrothiophene.

2-[5-(N'-Cyclohexyl-N'-hydroxyureidyl)-3-methoxy-4-propoxyphenyl]-5-(3,4,5-trimethoxyphenyl)-tetrahydrothiophene.

2-[5-(N'-Hexyl-N'-hydroxyureidyl)-3-methoxy-4-propoxyphenyl]-5-(3,4,5-trimethoxyphenyl)-tetrahydrothiophene.

2-[5-(N'-Benzyl-N'-hydroxyureidyl)-3-methoxy-4-propoxyphenyl]-5-(3,4,5-trimethoxyphenyl)-tetrahydrothiophene.

2-[5-(N'-Hydroxy-N'-octylureidyl)-3-methoxy-4-propoxyphenyl]-5-(3,4,5-trimethoxyphenyl)-tetrahydrothiophene.

2-[5-(N'-Hydroxy-N'-methoxyethylureidyl)-3-methoxy-4-propoxyphenyl]-5-(3,4,5-trimethoxyphenyl)-tetrahydrothiophene.

2-[5-(N'-Decyl-N'-hydroxyureidyl)-3-methoxy-4-propoxyphenyl]-5-(3,4,5-trimethoxyphenyl)-tetrahydrothiophene.

2-[5-(N'-Butyl-N'-hydroxyureidyl)-4-(p-bromophenylthioethoxy)-3-methoxyphenyl]-5-(3,4,5-trimethoxyphenyl)-tetrahydrofuran

2-[5-(N'-Butyl-N'-hydroxyureidyl)-4-(2-bromophenylthioethoxy)-3-methoxyphenyl]-5-(3,4,5-trimethoxyphenyl)-tetrahydrofuran

2-[5-(N'-Butyl-N'-hydroxyureidyl)-4-(3-bromophenylthioethoxy)-3-methoxyphenyl]-5-(3,4,5-trimethoxyphenyl)-tetrahydrofuran

2-[5-(N'-Butyl-N'-hydroxyureidyl)-4-(3,4-dichlorophenylthioethoxy)-3-methoxyphenyl]-5-(3,4,5-trimethoxyphenyl)-tetrahydrofuran

2-[5-(N'-Butyl-N-hydroxyureidyl)-4-(p-chlorophenylthioethoxy)-3-methoxyphenyl]-5-(3,4,5-trimethoxyphenyl)-tetrahydrofuran

2-[5-(N'-Butyl-N'-hydroxyureidyl)-4-(p-fluorophenylthioethoxy)-3-methoxyphenyl]-5-(3,4,5-trimethoxyphenyl)-tetrahydrofuran

2-[5-N'-Butyl-N'-hydroxyureidyl)-4-(2,3,5,6-tetrafluorophenylthioethoxy)-3-methoxyphenyl]-5-(3,4,5-trimethoxyphenyl)-tetrahydrofuran

2-[5-(N'-Butyl-N'-hydroxyureidyl)-4-(2,3,4,5-tetrafluorophenylethoxy)-3-methoxyphenyl]-5-(3,4,5-trimethoxyphenyl)-tetrahydrofuran

2-[5-(N'-Butyl-N'-hydroxyureidyl)-4-(p-bromophenysulfonylethoxy)-3-methoxyphenyl]-5-(3,4,5-trimethoxyphenyl)-tetrahydrofuran

2-[5-(N'-Butyl-N'-hydroxyureidyl)-4-(2-bromophenylsulfonylethoxy)-3-methoxyphenyl]-5-(3,4,5-trimethoxyphenyl)-tetrahydrofuran

2-[5-(N'-Hydroxy-N'-methylyureidyl)-4-(p-bromophenylthioethoxy)-3-methoxyphenyl]-5-(3,4,5-trimethoxyphenyl)-tetrahydrofuran

2-[5-(N'-Hydroxy-N'-methylureidyl)-4-(2-bromophenylthioethoxy)-3-methoxyphenyl]-5-(3,4,5-trimethoxyphenyl)-tetrahydrofuran

2-[5-(N'-Hydroxy-N'-methylureidyl-4-(3-bromophenylthioethoxy)-3-methoxyphenyl]-5-(3,4,5-trimethoxyphenyl)-tetrahydrofuran

2-[5-(N'-Hydroxy-N'-methylureidyl)-4-(3,4-dichlorophenylthioethoxy)-3-methoxyphenyl]-5-(3,4,5-trimethoxyphenyl)-tetrahydrofuran

2-[5-(N'-Hydroxy-N'-methylureidyl)-4-(p-chlorophenylthioethoxy)-3-methoxyphenyl]-5-(3,4,5-trimethoxyphenyl)-tetrahydrofuran

2-[5-(N'-Ethyl-N'-hydroxyureidyl)-4-p-fluorophenylthioethoxy)-3-methoxyphenyl]-5-(3,4,5-trimethoxyphenyl)-

tetrahydrofuran

2-[5-(N'-Ethyl-N'-hydroxyureidyl)-4-(2,3,5,6-tetrafluorophenylthioethoxy)-3-methoxyphenyl]-5-(3,4,5-trimethoxyphenyl)-tetrahydrofuran

2-[5-(N'-Ethyl-N'-hydroxyureidyl)-4-(2,3,4,5-tetrafluorophenylethoxy)-3-methoxyphenyl]-5-(3,4,5-trimethoxyphenyl)-tetrahydrofuran

2-[5-(N'-Ethyl-N'-hydroxyureidyl)-4-(2,3,4,5-tetrafluorophenylethoxy)-3-methoxyphenyl]-5-(3,4,5-trimethoxyphenyl)-tetrahydrofuran

2-[5-(N'-p-Chlorophenyl-N'-hydroxyureidyl)-4-(2-bromophenylsulfonylethoxy)-3-methoxyphenyl]-5-(3,4,5-trimethoxyphenyl)-tetrahydrofuran

2-[5-(N'-p-Chlorophenyl-N'-hydroxyureidyl-4-(p-bromophenylthioethoxy)-3-methoxyphenyl]-5-(3,4,5-trimethoxyphenyl)-tetrahydrofuran

2-[5-(N'-*tert*-Butyl-N'-hydroxyureidyl)-4-(p-bromophenylthioethoxy)-3-methoxyphenyl]-5-(3,4,5-trimethoxyphenyl)-tetrahydrofuran

2-[5-(N'-*tert*-Butyl-N'-hydroxyureidyl)-4-(2-bromophenylthioethoxy)-3-methoxyphenyl]-5-(3,4,5-trimethoxyphenyl)-tetrahydrofuran

2-[5-(N'-*tert*-Butyl-N'-hydroxyureidyl)-4-(3-bromophenythioethoxy)-3-methoxyphenyl]-5-(3,4,5-trimethoxyphenyl)-tetrahydrofuran

2-[5-(N'-Cyclohexyl-N'-hydroxyureidyl-4-(3,4-dichlorophenylthioethoxy)-3-methoxyphenyl]-5-(3,4,5-trimethoxyphenyl)-tetrahydrofuran

2-[5-(N'-Cyclohexyl-N'-hydroxyureidyl)-4-(p-chlorophenylthioethoxy)-3-methoxyphenyl]-5-(3,4,5-trimethoxyphenyl)-tetrahydrofuran

2-[5-(N'-Cyclohexyl-N'-hydroxyureidyl)-4-(p-fluorophenylthioethoxy)-3-methoxyphenyl)-5-(3,4,5-trimethoxyphenyl)-tetrahydrofuran

2-[5-(N'-Benzyl-N'-hydroxyureidyl)-4-(2,3,5,6-tetrafluorophenylthioethoxy)-3-methoxyphenyl]-5-(3,4,5-trimethoxyphenyl)-tetrahydrofuran

2-[5-(N'-Benzyl-N'-hydroxyureidyl-4-(2,3,4,5-tetrafluorophenylethoxy)-3-methoxyphenyl]-5-(3,4,5-trimethoxyphenyl)-tetrahydrofuran

2-[5-(N'-Benzyl-N'-hydroxyureidyl)-4-(p-bromophenylsulfonylethoxy)-3-methoxyphenyl]-5-(3,4,5-trimethoxyphenyl)-tetrahydrofuran

2-[5-(N'-Benzyl-N'-hydroxyureidyl)-4-(2-bromophenylsulfonylethoxy)-3-methoxyphenyl]-5-(3,4,5-trimethoxyphenyl)-tetrahydrofuran

2-[5-(N'-Hydroxy-N'-*i*-propylureidyl)-4-(p-bromophenylthioethoxy)-3-methoxyphenyl]-5-(3,4,5-trimethoxyphenyl)-tetrahydrofuran

2-[5-(N'-*sec*-Butyl-N'-hydroxyureidyl)-4-(p-bromophenylthioethoxy)-3-methoxyphenyl]-5-(3,4,5-trimethoxyphenyl)-tetrahydrofuran

2-[5-(N'-*sec*-Butyl-N'-hydroxyureidyl-4-(2-bromophenylthioethoxy)-3-methoxyphenyl]-5-(3,4,5-trimethoxyphenyl)-tetrahydrofuran

2-[5-(N'-Hydroxy-N'-propylureidyl)-4-(3-bromophenylthioethoxy)-3-methoxyphenyl]-5-(3,4,5-trimethoxyphenyl)-tetrahydrofuran

2-[5-(N'-Hydroxy-N'-*n*-pentylureidyl)-4-(3,4-dichlorophenylthioethoxy)-3-methoxyphenyl]-5-(3,4,5-trimethoxyphenyl)-tetrahydrofuran

2-[5-(N'-Hexyl-N'-hydroxyureidyl-4-(p-chlorophenylthioethoxy)-3-methoxyphenyl]-5-(3,4,5-trimethoxyphenyl)-tetrahydrofuran

2-[5-(N'-Hydroxy-N'-octylureidyl)-4-(p-fluorophenylthioethoxy)-3-methoxyphenyl]-5-(3,4,5-trimethoxyphenyl)-tetrahydrofuran

2-[5-(N'-Hydroxy-methoxyethylureidyl)-4-(2,3,5,6-tetrafluorophenylthioethoxy)-3-methoxyphenyl]-5-(3,4,5-trimethoxyphenyl)-tetrahydrofuran

2-[5-(N'-Decyl-N'-hydroxyureidyl-4-(2,3,4,5-tetrafluorophenylethoxy)-3-methoxyphenyl]-5-(3,4,5-trimethoxyphenyl)-tetrahydrofuran

2-[5-(N'-Hydroxy-N'-methylureidylmethyl)-4-(p-chlorophenylthioethoxy)-3-methoxyphenyl]-5-(3,4,5-trimethoxyphenyl)-tetrahydrofuran

2-[5-(N'-Hydroxy-N'-*i*-propylureidylmethyl)-4-(p-chlorophenylthioethoxy)-3-methoxyphenyl]-5-(3,4,5-trimethoxyphenyl)-tetrahydrofuran

2-[5-(N'-Butyl-N'-hydroxyureidylmethyl)-4-(p-chlorophenylthioethoxy)-3-methoxyphenyl]-5-(3,4,5-trimethoxyphenyl)-tetrahydrofuran

2-[5-(N'-Hydroxy-N'-propylureidylmethyl)-4-(p-chlorophenylthioethoxy)-3-methoxyphenyl]-5-(3,4,5-trimethoxyphenyl)-tetrahydrofuran

2-(5-(N'-Ethyl-N'-hydroxyureidylmethyl-4-(p-chlorophenylthioethoxy-3-methoxyphenyl]-5-(3,4,5-trimethoxyphenyl)-tetrahydrofuran

2-[5-(N'-Hydroxy-N'-octylureidylmethyl-4-(p-chlorophenylthioethoxy)-3-methoxyphenyl]-5-(3,4,5-trimethoxyphenyl)-tetrahydrofuran

2-[5-(N'-Benzyl-N'-hydroxyureidyl-4-(p-bromophenylsulfonylethoxy)-3-methoxyphenyl]-5-(3,4,5-trimethoxyphenyl)-tetrahydrothiophene

2-[5-(N-Benzyl-N'-hydroxyureidyl)-4-(2-bromophenylsulfonylethoxy)-3-methoxyphenyl]-5-(3,4,5-trimethoxyphenyl)-tetrahydrothiophene

2-[5-(N'-Hydroxy-N'-*i*-propylureidyl)-4-(p-bromophenylthioethoxy)-3-methoxyphenyl]-5-(3,4,5-trimethoxyphenyl)-tetrahydrothiophene

2-[5-(N'-Hydroxyl-N'-octylureidyl)-4-(p-fluorophenylthioethoxy)-3-methoxyphenyl]-5-(3,4,5-trimethoxyphenyl)-tetrahydrothiophene

2-[5-N'-Butyl-N'-hydroxyureidyl)-4-(p-bromophenylthioethoxy)-3-methoxyphenyl]-5-(3,4,5-trimethoxyphenyl)-tetrahydrothiophene

2-[5-(N'-Butyl-N'-hydroxyureidyl)-4-(2-bromophenylthioethoxy)-3-methoxyphenyl]-5-(3,4,5-trimethoxyphenyl)-tetrahydrothiophene

2-[5-(N'-Butyl-N'-hydroxyureidyl)-4-(3-bromophenylthioethoxy)-3-methoxyphenyl]-5-(3,4,5--trimethoxyphenyl)-tetrahydrothiophene

2-(5-(N'-Hydroxy-N'-methylureidylmethyl)-4-(p-chlorophenylthioethoxy)-3-methoxyphenyl]-5-(3,4,5-trimethoxyphenyl)-tetrahydrothiophene

2-[5-(N'-Hydroxy-N'-*i*-propylureidylmethyl)-4-(p-chlorophenylthlioethoxy)-3-methoxyphenyl]-5-(3,4,5-trimethoxyphenyl)-tetrahydrothiophene

2-[5-(N'-Butyl-N'-hydroxyureidylmethyl-4-(p-chlorophenylthioethoxy)-3-methoxyphenyl]-5-(3,4,5-trimethoxyphenyl)-tetrahydrothiophene

2-[5-[1-(N'-Hydroxy-N'-methylureidyl)propyn-3-yl]-3-methoxy-4-propoxyphenyl]-5-(3,4,5-trimethoxyphenyl)-tetrahydrofuran.

2-[5-[1-(N'-Ethyl-N'-hydroxyureidyl)propyn-3-yl]-3-methoxy-4-propoxyphenyl]-5-(3,4,5-trimethoxyphenyl)-tetrahydrofuran.

2-[5-[1-(N'-Hydroxy-N'-propylureidyl)propyn-3-yl]-3-methoxy-4-propoxyphenyl]-5-(3,4,5-trimethoxyphenyl)-tetrahydrofuran.

2-[5-[1(N'-n-Butyl-N'-hydroxyureidyl)propyn-3-yl]-3-methoxy-4-propoxyphenyl]-5-(3,4,5-trimethoxyphenyl)-tetrahydrofuran.

2-[5-[1-(N'-Hydroxy-N'-*i*-propylureidyl)propyn-3-yl]-3-methoxy-4-propoxyphenyl]-5-(3,4,5-trimethoxyphenyl)-tetrahydrofuran.

2-[5-[1-(N'-t-Butyl-N'-hydroxyureidyl)propyn-3-yl]-3-methoxy-4-propoxyphenyl]-5-(3,4,5-trimethoxyphenyl)-tetrahydrofuran.

2-[5-[1-(N'-Benzyl-N'-hydroxyureidyl)propyn-3-yl]-3-methoxy-4-propoxyphenyl]-5-(3,4,5-trimethoxyphenyl)-tetrahydrofuran.

2-[5-[1-(N'-Cyclopropylmethyl-N'-hydroxyureidyl)propyn-3-yl]-3-methoxy-4-propoxyphenyl]-5-(3,4,5-trimethoxyphenyl)-tetrahydrofuran.

2-[5-[1-(N'-Allyl-N'-hydroxyureidyl)propyn-3-yl]-3-methoxy-4-propoxyphenyl]-5-(3,4,5-trimethoxyphenyl)-tetrahydrofuran.

2-[5-[1-(N'-Hydroxy-N'-hydroxyethylureidyl)propyn-3-yl]-3-methoxy-4-propoxyphenyl]-5-(3,4,5-trimethoxyphenyl)-tetrahydrofuran.

2-[5-(1-(N'-Hydroxy-N'-methylureidyl)propyn-3-yl]-4-(p-chlorophenylthioethoxy)-3-methoxyphenyl]-5-(3,4,5-trimethoxyphenyl)-tetrahydrofuran.

2-[5-[1-(N'-Ethyl-N'-hydroxyureidyl)propyn-3-yl]-4-(p-chlorophenylthioethoxy)-3-methoxyphenyl]-5-(3,4,5-trimethoxyphenyl)-tetrahydrofuran.

2-[5-[1-(N'-Hydroxy-N'-propylureidyl)propyn-3-yl)-4-(p-chlorophenylthioethoxy)-3-methoxyphenyl]-5-(3,4,5-trimethoxyphenyl)-tetrahydrofuran.

2-[5-[1-(N'-*n*-Butyl-N'-hydroxyureidyl)propyn-3-yl]-4-(p-chlorophenylthioethoxy)-3-methoxyphenyl]-5-(3,4,5-trimethoxyphenyl)-tetrahydrofuran

2-[5-[1-(N'-Hydroxy-N'-*i*-propylureidyl)propyn-3-yl]-4-(p-chlorophenylthioethoxy)-3-methoxyphenyl)-5-(3,4,5-trimethoxyphenyl)-tetrahydrofuran

2-[5-[1-(N'-*tert*-Butyl-N'-hydroxyureidyl)propyn-3-yl]-4-(p-chlorophenylthioethoxy)-3-methoxyphenyl]-5-(3,4,5-trimethoxyphenyl)-tetrahydrofuran

2-[5-[1-(N'-Benzyl-N'-hydroxyureidyl)propyn-3-yl]-4-(p-chlorophenylthioethoxy)-3-methoxyphenyl]-5-(3,4,5-trimethoxyphenyl)-tetrahydrofuran

2-[5-[1-(N'-Cyclopropylmethyl-N'-hydroxyureidyl)propyn-3-yl]-4-(p-chlorophenylthioethoxy)-3-methoxyphenyl]-5-(3,4,5-trimethoxyphenyl)-tetrahydrofuran

2-[5-[1-(N'-Allyl-N'-hydroxyureidyl)propyn-3-yl]-4-(p-chlorophenylthioethoxy)-3-methoxyphenyl]-5-(3,4,5-trimethoxyphenyl)-tetrahydrofuran

2-[5-[1-(N'-Hydroxy-N'-hydroxyethylureidyl)propyn-3-yl]-4-(p-chlorophenylthioethoxy)-3-methoxyphenyl]-5-(3,4,5-trimethoxyphenyl)-tetrahydrofuran

2-[5-[*trans*-1-(N'-Hydroxy-N'-methylureidyl)propen-3-yl]-3-methoxy-4-propoxyphenyl]-5-(3,4,5-trimethoxyphenyl)-tetrahydrofuran.

2-[5-[*trans*-1-(N'-Ethyl-N'-hydroxyureidyl)propen-3-yl]-3-methoxy-4-propoxyphenyl]-5-(3,4,5-trimethoxyphenyl)-tetrahydrofuran.

2-[5-[*trans*-1-(N'-Hydroxy-N'-propylureidyl)propen-3-yl]-3-methoxy-4-propoxyphenyl]-5-(3,4,5-trimethoxyphenyl)-tetrahydrofuran.

2-[5-[*trans*-1-(N'-Butyl-N'-hydroxyureidyl)propen-3-yl]-3-methoxy-4-propoxyphenyl]-5-(3,4,5-trimethoxyphenyl)-tetrahydrofuran.

2-[5-[*trans*-1-(N'-Hydroxy-N'-i-propylureidyl)propen-3-yl]-3-methoxy-4-propoxyphenyl]-5-(3,4,5-trimethoxyphenyl)-tetrahydrofuran.

2-[-[5-[*trans*-1-(N'-t-Butyl-N'-hydroxyureidyl)propen-3-yl]-3-methoxy-4-propoxyphenyl]-5-(3,4,5-trimethoxyphenyl)-tetrahydrofuran.

2-[-[5-[*trans*-1-(N'-Benzyl-N'-hydroxyureidyl)propen-3-yl]-N-3-methoxy-4-propoxyphenyl]-5-(3,4,5-trimethoxyphenyl)-tetrahydrofuran.

2-[5-[*trans*-1-(N'-Allyl-N'-hydroxyureidyl)propen-3-yl]-3-methoxy-4-propoxyphenyl]-5-(3,4,5-trimethoxyphenyl)-tetrahydrofuran.

2-[5-[*trans*-1-(N'-Cyclohexyl-N'-hydroxyureidyl)propen-3-yl]-3-methoxy-4-propoxyphenyl-5-(3,4,5-trimethoxyphenyl)-tetrahydrofuran.

2-[5-[*trans*-1-(N'-Hydroxy-N'-methylthioethylureidyl)propen-3-yl]-3-methoxy-4-propoxyphenyl]-5-(3,4,5-trimethoxyphenyl)-tetrahydrofuran.

2-[5-*trans*-1-(N'-Cyclohexyl-N'-hydroxyureidyl)propen-3-yl]-3-methoxy-4-propoxy]-5-(3,4,5-trimethoxyphenyl)-tetrahydrofuran.

2-[5-[1-(N'-Cyclohexyl-N'-hydroxyureidyl)propyl]-3-methoxy-4-propoxyphenyl]-5-(3,4,5-trimethoxyphenyl)-tetrahydrofuran.

2-[5-[*trans*-1-(N'-Hydroxy-N'-methylureidyl)propen-3-yl]-4-(p-chlorophenylthioethoxy)-3-methoxyphenyl]-5-(3,4,5-trimethoxyphenyl)-tetrahydrofuran

2-[5-[*trans*-1-(N'-Ethyl-N'-hydroxyureidyl)propen-3-yl]-4-(p-chlorophenylthioethoxy)-3-methoxyphenyl]-5-(3,4,5-trimethoxyphenyl)-tetrahydrofuran

2-[5-[*trans*-1-(N'-Hydroxy-N'-propylureidyl)propen-3-y]-4-(p-chlorophenylthioethoxy)-3-methoxyphenyl]-5-(3,4,5-trimethoxyphenyl)-tetrahydrofuran

2-[5-[*trans*-1-(N'-*n*-Butyl-N'-hydroxyureidyl)propen-3-yl]-4-(p-chlorophenythioethoxy)-3-methoxyphenyl]-5-(3,4,5-trimethoxyphenyl)-tetrahydrofuran

2-[5-*trans*-1-(N'-Hydroxy-N'-*i*-propylureidyl)propen-3-yl]-4-(p-chlorophenylthioethoxy)-3-methoxyphenyl]-5-(3,4,5-trimethoxyphenyl)tetrahydrofuran

2-[5-[*trans*-1-(N'-*tert*-Butyl-N'-hydroxyureidyl)propen-3-yl]-4-(p-chlorophenylthioethoxy)-3-methoxyphenyl]-5-(3,4,5-trimethoxyphenyl)-tetrahydrofuran

2-[5-[*trans*-1-(N'-Benzyl-N'-hydroxyureidyl)propen-3-yl]-4-(p-chlorophenylthioethoxy)-3-methoxyphenyl]-5-(3,4,5-trimethoxyphenyl)tetrahydrofuran

2-[5-(*trans*-1-(N'-Cyclopropylmethyl-N'-hydroxyureidyl)propen-3-yl]-4-(p-chlorophenylthioethoxy)-3-methoxyphenyl]-5-(3,4,5-trimethoxyphenyl)-tetrahydrofuran

2-[5-[*trans*-1-(N'-Allyl-N'-hydroxyureidyl)propen-3-yl]-4-(p-chlorophenylthioethoxy)-3-methoxyphenyl]-5-(3,4,5-trimethoxyphenyl)-tetrahydrofuran
Or

2-[5-(*trans*-1-(N'-Hydroxy-N'-hydroxyethylureidyl)propen-3-yl]-4-(p-chlorophenylthioethoxy)-3-methoxyphenyl]-5-(3,4,5-trimethoxyphenyl)- tetrahydrofuran

**3.** *trans*-2-[5-N'-Methyl-N'-hydroxyureidylmethyl)-3-methoxy-4-*p*-chlorophenylthioethoxyphenyl]-5-(3,4,5-trimethoxyphenyl)tetrahydrofuran.

**4.** A pharmaceutical composition comprising an effective amount of a compound according to any one of claims 1 to 3 in a pharmaceutically acceptable carrier.

**5.** A topical pharmaceutical composition comprising an effective amount of a compound according to any one of claims 1 to 3 in a pharmaceutically acceptable carrier.

**6.** A compound according to any one of claims 1 to 3 for use as a pharmaceutical.

**7.** The use of a compound according to any one of claims 1 to 3 in the manufacture of a medicament for use in the treatment of an inflammatory disorder.

**8.** The use of a compound according to any one of claims 1 to 3 in the manufacture of a medicament for use in the treatment of a patient in need of a PAF receptor antagonist.

**9.** The use of a compound according to any one of claims 1 to 3 in the manufacture of a medicament for use in the treatment of a patient in need of an inhibitor of 5-lipoxygenase activity.

**Patentansprüche**

**1.** Verbindung der Formel:

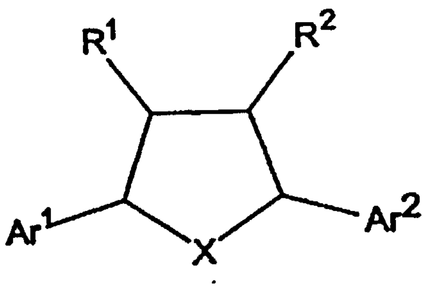

worin

$Ar^1$ =

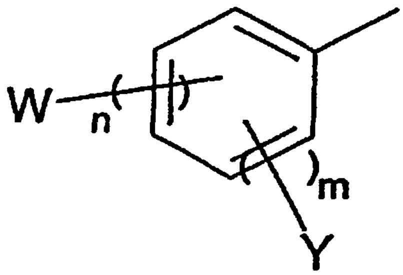

$Ar^2$ =

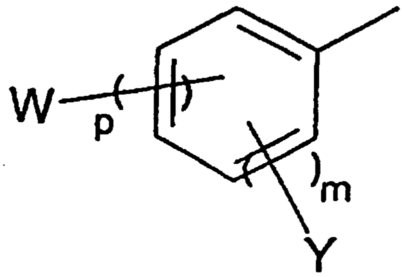

worin:

X O, S ist;

W unabhängig voneinander:

-AN(OM)C(O)N($R^3$)$R^4$, -AN($R^3$)C(O)N(OM)$R^4$, -N(OM)C(O)N($R^3$)$R^4$ oder -N($R^3$)C(O)N(OM)$R^4$ ist;

n 1 ist;

m 2 oder 3 ist;

p O ist;

A $C_{1-10}$-Alkylen, $C_{2-10}$-Alkenylen oder $C_{2-10}$-Alkynylen ist;

M Wasserstoff ist;

Y unabhängig -$OR^{11}$ ist;

$R^1$ und $R^2$ Wasserstoff sind;

$R^3$ und $R^4$ unabhängig voneinander $C_{1-10}$-Alkyl, $C_{2-10}$-Alkenyl oder $C_{2-10}$-Alkynyl sind;

$R^{11}$ $C_1$-$C_{12}$-Alkyl oder substituiertes $C_1$-$C_{12}$-Alkyl ist, worin der Substituent ausgewählt ist aus der Gruppe, die besteht aus

Phenyl-S$(O)_g$-$C_{1-6}$-Alkylen-; $(R^3O)_d$-Phenyl-S$(O)_g$-$C_{1-6}$-Alkylen-;

$(CN)_d$-Phenyl-$S(O)_g$-$C_{1-6}$-Alkylen-; $(Halogen)_d$-Phenyl-$S(O)_g$-$C_{1-6}$-Alkylen-; $(R^3COO)_d$-Phenyl-$S(O)_g$-$C_{1-16}$-Alkylen-; $(R^3OCO)_d$-Phenyl-$S(O)_g$-$C_{1-6}$-Alkylen-; $(R^3O)_d$-Phenyl-$S(O)_g$-$C_{1-6}$-Alkylen; Phenyl-O-$C_{1-6}$-Alkylen-; $(R^3O)_d$-Phenyl-O-$C_{1-6}$-Alkylen-; $(CN)_d$-Phenyl-O-$C_{1-6}$-Alkylen-; $(Halogen)_d$-Phenyl-O-$C_{1-6}$-Alkylen-, $(R^3COO)_d$-Phenyl-O-$C_{1-6}$-Alkylen; $(R^3OCO)_d$-Phenyl-O-$C_{1-6}$-Alkylen-; $(R^3R^3N)_d$-Phenyl-$S(O)_g$-$C_{1-6}$-Alkylen; oder $(R^3CO)_d$-Phenyl-O-$C_{1-6}$-Alkylen-, wobei d 1,2,3,4 oder 5 ist; und g 0, 1 oder 2 ist.

**2.** Verbindung, die

2-[5-(N'-Butyl-N'-hydroxyureido)-3-methoxy-4-methoxyethoxyphenyl]-5-(3,4,5-trimethoxyphenyl)-tetrahydrothiophen,

2-[5-(N'-Butyl-N'-hydroxyureido)-3-methoxy-4-methoxythioethoxyphenyl]-5-(3,4,5-trimethoxyphenyl)-tetrahydrofuran,

2-[5-(N'-Butyl-N'-hydroxyureido)-4-hydroxyphenylthioethoxy-3-methoxyphenyl]-5-(3,4,5-trimethoxyphenyl)-tetrahydrofuran,

2-[5-(N'-Butyl-N'-hydroxyureido)-4-(N-nicotinoyl-N-phenylaminoethoxy)-3-methoxyphenyl]-5-(3,4,5-trimethoxyphenyl)-tetrahydrofuran,

2-[5-(N'-Butyl-N'-hydroxyureido)-4-(N-3-pyridiniumcarbonyl-N-phenylaminoethoxy)-3-methoxyphenyl]-5-(3,4,5-trimethoxyphenyl)-tetrahydrofuran-propyliodid,

2-[5-(N'-p-Chlorphenyl-N'-hydroxyureido)-4-(N-3-pyridiniumcarbonyl-N-phenylaminoethoxy)-3-methoxyphenyl]-5-(3,4,5-trimethoxyphenyl)-tetrahydrofuran-propyliodid,

2-[5-(N'-Butyl-N'-hydroxyureido)-4-(N-3-pyridiniumcarbonyl-N-phenylaminoethoxy)-3-methoxyphenyl]-5-(3,4,5-trimethoxyphenyl)-tetrahydrofuran-ethyliodid,

2-[5-(N'-p-Chlorphenyl-N'-hydroxyureido)-4-(N-3-pyridiniumcarbonyl-N-phenylaminoethoxy)-3-methoxyphenyl]-5-(3,4,5-trimethoxyphenyl)-tetrahydrofuran-ethyliodid,

2-[5-(N'-p-Chlorphenyl-N'-hydroxyureido)-3-methoxy-4-propoxyphenyl)-5-(3,4,5-trimethoxyphenyl)-tetrahydrofuran,

2-[5-(N'-p-Chlorphenyl-N'-hydroxyureido)-4-methoxyethoxy-3-methoxyphenyl]-5-(3,4,5-trimethoxyphenyl)-tetrahydrothiophen,

2-[5-(N'-p-Chlorphenyl-N'-hydroxyureido)-4-methylthioethoxy-3-methoxyphenyl]-5-(3,4,5-trimethoxyphenyl)-tetrahydrofuran,

2-[5-(N'-p-Chlorphenyl-N'-hydroxyureido)-4-p-hydroxyphenylthioethoxy-3-methoxyphenyl]-5-(3,4,5-triethoxyphenyl)-tetrahydrofuran,

2-[5-(N'-p-Chlorphenyl-N'-hydroxyureido)-4-(N-nicotinoyl-N-phenylaminoethoxy)-3-methoxyphenyl]-5-(3,4,5-trimethoxy phenyl)-tetrahydrofuran,

2-[5-(N'-Butyl-N'-hydroxyureido)-4-p-cyanophenylthioethoxy-3-methoxyphenyl]-5-(3,4,5-trimethoxyphenyl)-tetrahydrofuran,

2-[5-(N'-p-Chlorphenyl-N'-hydroxyureido)-4-p-cyanophenylthioethoxy-3-methoxyphenyl]-5-(3,4,5-trimethoxyphenyl)-tetrahydrofuran,

2-[5-(N'-Butyl-N'-hydroxyureido)-4-p-methoxyphenylthioethoxy-3-methoxyphenyl]-5-(3,4,5-trimethoxyphenyl)-tetrahydrofuran,

2-[5-(N'-p-Chlorphenyl-N'-hydroxyureido)-4-p-methoxyphenylthioethoxy-3-methoxyphenyl]-5-(3,4,5-trimethoxyphenyl)-tetrahydrofuran,

2-[5-(N'-tert-Butyl-N'-hydroxyureido)-3-methoxy-4-propoxyphenyl]-5-(3,4,5-trimethoxyphenyl)-tetrahydrothiophen,

2-[5-(N'-n-Butyl-N'-hydroxyureido)-3-methoxy-4-propoxyphenyl]-5-(3,4,5-trimethoxyphenyl)-tetrahydrofuran,

2-[5-(N'-Ethyl-N'-hydroxyureido)-3-methoxy-4-propoxyphenyl]-5-(3,4,5-trimethoxyphenyl)-tetrahydrofuran,

2-[5-(N'-Cyclohexyl-N'-hydroxyureido)-3-methoxy-4-propoxyphenyl]-5-(3,4,5-trimethoxyphenyl)-tetrahydrofuran,

2-[5-(N'-Benzyl-N'-hydroxyureido)-3-methoxy-4-propoxyphenyl]-5-(3,4,5-trimethoxyphenyl)-tetrahydrofuran,

2-[5-N'-Hydroxyureido-3-methoxy-4-propoxyphenyl]-5-(3,4,5-trimethoxyphenyl)-tetrahydrothiophen,

2-[5-(N'-Hydroxy-N'-methylureido)-3-methoxy-4-propoxyphenyl]-5-(3,4,5-trimethoxyphenyl)-tetrahydrothiophen,

2-[5-(N'-Hydroxy-N'-i-proplureido)-3-methoxy-4-propoxyphenyl]-5-(3,4,5-trimethoxyphenyl)-tetrahydrothiophen,

2-[5-(N'-sec-Butyl-N'-hydroxyureido)-3-methoxy-4-propoxyphenyl]-5-(3,4,5-trimethoxyphenyl)-tetrahydrothiophen,

2-[5-(N'-Hydroxy-N'-propylureido)-3-methoxy-4-propoxyphenyl]-5-(3,4,5-trimethoxyphenyl)-tetrahydrothiophen,

2-[5-(N'-Hydroxy-N'-propylureido-3-methoxy-4-propoxyphenyl]-5-(3,4,5-trimethoxyphenyl)-tetrahydrofuran,

2-[5-(N'-Hydroxy-N'-n-pentylureido)-3-methoxy-4-propoxyphenyl]-5-(3,4,5-trimethoxyphenyl)-tetrahydrofuran,

2-[5-(N'-Cyclohexyl-N'-hydroxyureido)-3-methoxy-4-propoxyphenyl]-5-(3,4,5-trimethoxyphenyl)-tetrahydrofuran,

2-[5-(N'-Hexyl-N'-hydroxyureido)-3-methoxy-4-propoxyphenyl]-5-(3,4,5-trimethoxyphenyl)-tetrahydrofuran,

2-[5-(N'-Benzyl-N'-hydroxyureido)-3-methoxy-4-propoxyphenyl]-5-(3,4,5-trimethoxyphenyl)-tetrahydrofuran,

2-[5-(N'-Hydroxy-N'-octylureido)-3-methoxy-4-propoxyphenyl]-5-(3,4,5-trimethoxyphenyl)-tetrahydrofuran,

2-[5-(N'-Hydroxy-N'-methoxyethylureido)-3-methoxy-4-propoxyphenyl]-5-(3,4,5-trimethoxyphenyl)-tetrahydrofuran,

2-[5-(N'-Decyl-N'-hydroxyureido)-3-methoxy-4-propoxyphenyl]-5-(3,4,5-trimethoxyphenyl)-tetrahydrofuran,

2-[5-(N'-Hydroxy-N'-n-pentylureido)-3-methoxy-4-propoxyphenyl]-5-(3,4,5-trimethoxyphenyl)-tetrahydrothiophen,

2-[5-(N'-Cyclohexyl-N'-hydroxyureido)-3-methoxy-4-propoxyphenyl]-5-(3,4,5-trimethoxyphenyl)-tetrahydrothiophen,

2-[5-(N'-Hexyl-N'-hydroxyureido)-3-methoxy-4-propoxyphenyl]-5-(3,4,5-trimethoxyphenyl)-tetrahydrothiophen,

2-[5-(N'-Benzyl-N'-hydroxyureido)-3-methoxy-4-propoxyphenyl]-5-(3,4,5-trimethoxyphenyl)-tetrahydrothiophen,

2-[5-(N'-Hydroxy-N'-octylureido)-3-methoxy-4-propoxyphenyl]-5-(3,4,5-trimethoxyphenyl)-tetrahydrothiophen,

2-[5-(N'-Hydroxy-N'-methoxyethylureido)-3-methoxy-4-propoxyphenyl]-5-(3,4,5-trimethoxyphenyl)-tetrahydrothiophen,

2-[5-(N'-Decyl-N'-hydroxyureido)-3-methoxy-4-propoxyphenyl]-5-(3,4,5-trimethoxyphenyl)-tetrahydrothiophen,

2-[5-(N'-Butyl-N'-hydroxyureido)-4-(p-bromphenylthioethoxy)-3-methoxyphenyl]-5-(3,4,5-trimethoxyphenyl)-tetrahydrofuran,

2-[5-(N'-Butyl-N'-hydroxyureido)-4-(2-bromphenylthioethoxy)-3-methoxyphenyl]-5-(3,4,5-trimethoxyphenyl)-tetrahydrofuran,

2-[5-(N'-Butyl-N'-hydroxyureido)-4-(3-bromphenylthioethoxy)-3-methoxyphenyl]-5-(3,4,5-trimethoxyphenyl)-tetra-tetrahydrofuran,

2-[5-(N'-Butyl-N'-hydroxyureido)-4-(3,4-dichlorphenylthioethoxy)-3-methoxyphenyl]-5-(3,4,5-trimethoxyphenyl)-tetrahydrofuran,

2-[5-(N'-Butyl-N'-hydroxyureido)-4-(p-chlorphenylthioethoxy)-3-methoxyphenyl]-5-(3,4,5-trimethoxyphenyl)-tetrahydrofuran,

2-[5-(N'-Butyl-N'-hydroxyureido)-4-(p-fluorphenylthioethoxy)-3-methoxyphenyl]-5-(3,4,5-trimethoxyphenyl)-tetrahydrofuran,

2-[5-(N'-Butyl-N'-hydroxyureido)-4-(2,3,5,6-tetrafluorphenylthioethoxy)-3-methoxyphenyl]-5-(3,4,5-trimethoxyphenyl)-tetrahydrofuran,

2-[5-(N'-Butyl-N'-hydroxyureido)-4-(2,3,4,5,-tetrafluorphenylethoxy)-3-methoxyphenyl]-5-(3,4,5-trimehthoxyphenyl)- - tetrahydrofuran,

2-[5-(N'-Butyl-N'-hydroxyureido)-4- (p-bromphenylsulfonylethoxy)-3-methoxyphenyl-5-(3,4,5-trimethoxyphenyl)-tetrahydrofuran,

2-[5-(N'-Butyl-N'-hydroxyureido)-4-(2-bromphenylsulfonylethoxy)-3-methoxyphenyl]-5-(3,4,5-trimethoxyphenyl)-tetrahydrofuran,

2-[5-(N'-Hydroxy-N'-methylureido)-4-(p-bromphenylthioethoxy)-3-methoxyphenyl]-5-(3,4,5-trimethoxyphenyl)-tetrahydrofuran,

2-[5-(N'-Hydroxy-N'-methylureido)-4-(2-bromphenylthioethoxy)-3-methoxyphenyl]-5-(3,4,5-trimethoxyphenyl)-tetrahydrofuran,

2-[5-(N'-Hydroxy-N'-methylureido)-4-(3-bromphenylthioethoxy)-3-methoxyphenyl]-5-(3,4,5-trimethoxyphenyl)-tetrahydrofuran,

2-[5-(N'-Hydroxy-N'-methylureido)-4-(3,4-dichlorphenylthioethoxy)-3-methoxyphenyl]-5-(3,4,5-trimethoxyphenyl)-tetrahydrofuran,

2-[5-(N'-Hydroxy-N'-methylureido)-4-(p-chlorphenylthioethoxy)-3-methoxyphenyl]-5-(3,4,5-trimethoxyphenyl)-tetra hydrofuran,

2-[5-(N'-Ethyl-N'-hydroxyureido)-4-(p-fluorphenylthioethoxy)-3-methoxyphenyl]-5-(3,4,5-trimethoxyphenyl)-tetrahydrofuran,

2-[5-(N'-Ethyl-N'-hydroxyureido)-4-(2,3,5,6-tetrafluorphenylthioethoxy)-3-methoxyphenyl]-5-(3,4,5-trimethoxyphenyl)-tetrahydrofuran,

2-[5-(N'-Ethyl-N'-hydroxyureido)-4-(2,3,4,5-tetrafluorphenylethoxyl-3-methoxyphenyl]-5-(3,4,5-trimethoxyphenyl)- - tetrahydroturan,

2-[5-(N'-Ethyl-N'-hydroxyureido)-4-(2,3,4,5-tetrafluorphenylethoxy)-3-methoxyphenyl]-5-(3,4,5-trimethoxyphenyl)- - tetrahydrofuran,

2-[5-(N'-p-Chlorphenyl-N'hydroxyureido)-4-(2-bromphenylsulfonylethoxy)-3-methoxyphenyl]-5-(3,4,5-trimethoxyphenyl)-tetrahydrofuran,

2-[5-(N'-p-Chlorphenyl-N'-hydroxyureido)-4-(p-bromphenylthioethoxy)-3-methoxyphenyl]-5-(3,4,5-trimethoxyphenyl)- - tetrahydrofuran,

2-[5-(N'-tert-Butyl-N'-hydroxyureido)-4-(p-bromphenylthioethoxy)-3-methoxyphenyl]-5-(3,4,5-trimethoxyphenyl)-tetrahydrofuran,

2-[5-(N'-tert-Butyl-N'-hydroxyureido)-4-(2-bromphenylthioethoxy)-3-methoxyphenyl]-5-(3,4,5-trimethoxyphenyl)-tetrahydrofuran,

2-[5-(N'-tert-Butyl-N'-hydroxyureido)-4-(3-bromphenylthioethoxy)-3-methoxyphenyl]-5-(3,4,5-trimethoxyphenyl)-tetrahydrofuran,

2-[5-(N'-Cyclohexyl-N'-hydroxyureido)-4-(3,4-dichlorphenylthioethoxy)-3-methoxyphenyl]-5-(3,4,5-trimethoxyphenyl)- - tetrahydrofuran,

2-[5-(N'-Cyclohexyl-N'-hydroxyureido)-4-(p-chlorphenylthioethoxy)-3-methoxyphenyl]-5-(3,4,5-trimethoxyphenyl)-tetrahydrofuran,

2-[5-(N'-Cyclohexyl-N'-hydroxyureido)-4-(p-fluorphenylthioethoxy)-3-methoxyphenyl]-5-(3,4,5-trimethoxyphenyl)-tetra-tetrahydrofuran,

2-[5-(N'-Benzyl-N'-hydroxyureido)-4-(2,3,5,6-tetrafluorphenylthioethoxy)-3-methoxyphenyl]-5-(3,4,5-trimethoxyphenyl)-tetrahydrofuran,

2-[5-(N'-Benzyl-N'-hydroxyureido)-4-(2,3,4,5,-tetrafluorphenylethoxy)-3-methoxyphenyl]-5-(3,4,5-trimethoxyphenyl)- - tetrahydrofuran,

2-[5-(N'-Benzyl-N'-hydroxyureido)-4-(p-bromphenylsulfonylethoxy)-3-methoxyphenyl]-5-(3,4,5-trimethoxyphenyl)-tetrahydrofuran,

2-[5-(N'-Benzyl-N'-hydroxyureido)-4-(2-bromphenylsulfonylethoxy)-3-methoxyphenyl]-5-(3,4,5-trimethoxyphenyl)-tetrahydrofuran,

2-[5-(N'-Hydroxy-N'-i-propylureido)-4-(p-bromphenylthioethoxy)-3-methoxyphenyl]-5-(3,4,5-trimethoxyphenyl)-tetrahydrofuran,

2-[5-(N'-sec-Butyl-N'-hydroxyureido)-4-(p-bromphenylthioethoxy)-3-methoxyphenyl]-5-(3,4,5-trimethoxyphenyl)-tetrahydrofuran,

2-[5-(N'-sec-Butyl-N'-hydroxyureido)-4-(2-bromphenylthioethoxy)-3-methoxyphenyl]-5-(3,4,5-trimethoxyphenyl)-tetrahydrofuran,

2-[5-(N'-Hydroxy-N'-propylureido)-4-(3-bromphenylthioethoxy)-3-methoxyphenyl]-5-(3,4,5-trimethoxyphenyl)-tetrahydrofuran,

2-[5-(N'-Hydroxy-N'-n-pentylureido)-4-(3,4-dichlorphenylthioethoxy)-3-methoxyphenyl]-5-(3,4,5-trimethoxyphenyl)-tetrahydrofuran,

2-[5-(N'-Hexyl-N'-hydroxyureido)-4-(p-chlorphenylthioethoxy)-3-methoxyphenyl]-5-(3,4,5-trimethoxyphenyl)-

tetrahydrofuran,

2-[5-(N'-Hydroxy-N'-octylureido)-4-(p-fluorphenylthioethoxy)-3-methoxyphenyl]-5-(3,4,5-trimethoxyphenyl)-tetrahydrofuran,

2-[5-(N'-Hydroxy-methoxyethylureido)-4-(2,3,5,6-tetrafluorphenylthioethoxy)-3-methoxyphenyl]-5-(3,4,5-trimethoxyphenyl)-tetrahydrofuran,

2-[5-(N'-Decyl-N'-hydroxyureido)-4-(2,3,4,5-tetrafluorphenylethoxy)-3-methoxyphenyl]-5-(3,4,5-trimethoxyphenyl)-tetrahydrofuran,

2-[5-(N'-Hydroxy-N'-methylureidomethyl)-4-(p-chlorphenylthioethoxy)-3-methoxyphenyl]-5-(3,4,5-trimethoxyphenyl)-tetrahydrofuran,

2-[5-(N'-Hydroxy-N'-i-propylureidomethyl)-4-(p-chlorphenylthioethoxy)-3-methoxyphenyl]-5-(3,4,5-trimethoxyphenyl)- - tetrahydrofuran,

2-[5-(N'-Butyl-N'-hydroxyureidomethyl)-4-(p-chlorphenylthioethoxy)-3-methoxyphenyl)-5-(3,4,5-trimethoxyphenyl)- - tetrahydrofuran,

2-[5-(N'-Hydroxy-N'-propylureidomethyl)-4-(p-chlorphenylthioethoxy)-3-methoxyphenyl]-5-(3,4,5-trimethoxyphenyl)- - tetrahydrofuran,

2-[5-(N'-Ethyl-N'-hydroxyureidomethyl)-4-(p-chlorphenylthioethoxy)-3-methoxyphenyl]-5-(3,4,5-trimethoxyphenyl)-tetrahydrofuran,

2-[5-(N'-Hydroxy-N'-octylureidomethyl)-4-(p-chlorphenylthioethoxy)-3-methoxyphenyl]-5-(3,4,5-trimethoxyphenyl)- - tetrahydrofuran,

2-[5-(N'-Benzyl-N'-hydroxyureido)-4-(p-bromphenylsulfonylethoxy)-3-methoxyphenyl]-5-(3,4,5-trimethoxyphenyl)-tetrahydrothiophen,

2-[5-(N'-Benzyl-N'-hydroxyureido)-4-(2-bromphenylsulfonylethoxy)-3-methoxyphenyl]-5-(3,4,5-trimethoxyphenyl)-tetrahydrothiophen,

2-[5-(N'-Hydroxy-N'-i-propylureido)-4-(p-bromphenylthioethoxy)-3-methoxyphenyl]-5-(3,4,5-trimethoxyphenyl)-tetrahydrothiophen,

2-[5-(N'-Hydroxy-N'-octylureido)-4-(p-fluorphenylthioethoxy)-3-methoxyphenyl]-5-(3,4,5-trimethoxyphenyl)-tetrahydrothiophen,

2-[5-(N'-Hydroxy-N'-hydroxyureido)-4-(p-bromphenylthioethoxy)-3-methoxyphenyl]-5-(3,4,5-trimethoxyphenyl)-tetra hydrothiophen,

2-[5-(N'-Butyl-N'-hydroxyureido)-4-(2-bromphenylthioethoxy)-3-methoxyphenyl]-5-(3,4,5-trimethoxyphenyl)-tetrahydrothiophen,

2-[5-(N'-Butyl-N'-hydroxyureido)-4-(3-bromphenylthioethoxy)-3-methoxyphenyl]-5-(3,4,5-trimethoxyphenyl)-tetrahydrothiophen,

2-[5-(N'-Hydroxy-N'-methylureidomethyl)-4-(p-chlorphenylthioethoxy)-3-methoxyphenyl]-5-(3,4,5-trimethoxyphenyl)-tetrahydrothiophen,

2-[5-(N'-Hydroxy-N'-i-propylureidomethyl)-4-(p-chlorphenylthioethoxy)-3-methoxyphenyl]-5-(3,4,5-trimethoxyphenyl)-tetrahydrothiophen,

2-[5-(N'-Butyl-N'-hydroxyureidomethyl)-4-(p-chlorphenylthioethoxy)-3-methoxyphenyl]-5-(3,4,5-trimethoxyphenyl)-tetrahydrothiophen,

2-[5-[1-(N'-Hydroxy-N'-methylureido)propin-3-yl]-3-methoxy-4-propoxyphenyl]-5-(3,4,5-trimethoxyphenyl)-tetrahydrofuran,

2-[5-[1-(N'-Ethyl-N'-hydroxyureido)propin-3-yl]-3-methoxy-4-propoxyphenyl]-5-(3,4,5-trimethoxyphenyl)-tetrahydrofuran,

2-[5-[1-(N'-Hydroxy-N'-propylureido)propin-3-yl]-3-methoxy-4-propoxyphenyl]-5-(3,4,5-trimethoxyphenyl)-tetrahydrofuran,

2-[5-[1-(N'-n-Butyl-N'-hydroxyureido)propin-3-yl]-3-methoxy-4-propoxyphenyl]-5-(3,4,5-trimethoxyphenyl)-tetrahydro furan,

2-[5-[1-(N'-Hydroxy-N'-i-propylureido)propin-3-yl]-3-methoxy-4-propoxyphenyl]-5-(3,4,5-trimethoxyphenyl)-tetrahydrofuran,

2-[5-[1-(N'-t-Butyl-N'-hydroxyureido)propin-3-yl]-3-methoxy-4-propoxyphenyl]-5-(3,4,5-trimethoxyphenyl)-tetrahydrofuran,

2-[5-[1-(N'-Benzyl-N'-hydroxyureido)propin-3-yl]-3-methoxy-4-propoxyphenyl]-5-(3,4,5-trimethoxyphenyl)-tetrahydrofuran,

2-[5-[1-(N'-Cyclopropylmethyl-N'-hydroxyureido)propin-3-yl]-3-methoxy-4-propoxyphenyl]-5-(3,4,5-trimethoxyphenyl)- - tetrahydrofuran,

2-[5-[1-(N'-Allyl-N'-hydroxyureido)propin-3-yl]-3-methoxy-4-propoxyphenyl]-5-(3,4,5-trimethoxyphenyl)-tetrahydrofuran,

2-[5-[1-(N'-Hydroxy-N'-hydroxyethylureido)propin-3-yl]-3-methoxy-4-propoxyphenyl]-5-(3,4,5-trimethoxyphenyl)-tetrahydrofuran,

2-[5-[1-(N'-Hydroxy-N'-methylureido)propin-3-yl]-4-(p-chlorphenylthioethoxy)-3-methoxyphenyl]-5-(3,4,5-trimethoxyphenyl)-tetrahydrofuran,

2-[5-[1-(N'-Ethyl-N'-hydroxyureido)propin-3-yl]-4-(p-chlorphenylthioethoxy)-3-methoxyphenyl]-5-(3,4,5-trimethoxyphenyl)-tetrahydrofuran,

2-[5-[1-(N'-Hydroxy-N'-propylureido)propin-3-yl-]-4-(p-chlorphenylthioethoxy)-3-methoxyphenyl]-5-(3,4,5-trimethoxyphenyl)-tetrahydrofuran,

2-[5-[1-(N'-n-Butyl-N'-hydroxyureido)propin-3-yl]-4-(p-chlorphenylthioethoxy)-3-methoxyphenyl]-5-(3,4,5-trimethoxyphenyl)-tetrahydrofuran,

2-[5-[1-(N'-Hydroxy-N'-i-propylureido)propin-3-yl]-4-(p-chlorphenylthioethoxy)-3-methoxyphenyl]-5-(3,4,5-trimethoxyphenyl)-tetrahydrofuran,

2-[5-[1-(N'-tert-Butyl-N'-hydroxyureido)propin-3-yl]-4-(p-chlorphenylthioethoxy)-3-methoxyphenyl]-5-(3,4,5-trimethoxyphenyl)-tetrahydrofuran,

2-[5-[1-(N'-Benzyl-N'-hydroxyureido)propin-3-yl]-4-(p-chlorphenylthioethoxy)-3-methoxyphenyl]-5-(3,4,5-trimethoxyphenyl)-tetrahydrofuran,

2-[5-[1-(N'-Cyclopropylmethyl-N'-hydroxyureido)propin-3-yl]-4-(p-chlorphenylthioethoxy)-3-methoxyphenyl]-5-(3,4,5-trimethoxyphenyl)-tetrahydrofuran,

2-[5-[1-(N'-Allyl-N'-hydroxyureido)propin-3-yl]-4-(p-chlorphenylthioethoxy)-3-methoxyphenyl]-5-(3,4,5-trimethoxyphenyl)-tetrahydrofuran,

2-[5-[1-(N'-Hydroxy-N'-hydroxyethylureido)propin-3-yl]-4-(p-chlorphenylthioethoxy)-3-methoxyphenyl]-5-(3,4,5-trimethoxyphenyl)-tetrahydrofuran,

2-[5-[trans-1-(N'-Hydroxy-N'-methylureido)propen-3-yl]-3-methoxy-4-propoxyphenyl]-5-(3,4,5-trimethoxyphenyl)-tetrahydrofuran,

2-[5-[trans-1-(N'-Ethyl-N'-hydroxyureido)propen-3-yl]-3-methoxy-4-propoxyphenyl]-5-(3,4,5-trimethoxyphenyl)-tetrahydrofuran,

2-[5-[trans-1-(N'-Hydroxy-N'-propylureido)propen-3-yl]-3-methoxy-4-propoxyphenyl]-5-(3,4,5-trimethoxyphenyl)-tetra hydrofuran,

2-[5-[trans-1-(N'-Butyl-N'-hydroxyureido)propen-3-yl]-3-methoxy-4-propoxyphenyl]-5-(3,4,5-trimethoxyphenyl)-tetrahydrofuran,

2-[5-[trans-1-(N'-Hydroxy-N'-i-propylureido)propen-3-yl]-3-methoxy-4-propoxyphenyl]-5-(3,4,5-trimethoxyphenyl)-tetrahydrofuran,

2-[5-[trans-1-(N'-t-Butyl-N'-hydroxyureido)propen-3-yl]-3-methoxy-4-propoxyphenyl]-5-(3,4,5-trimethoxyphenyl)-tetrahydrofuran,

2-[5-[trans-1-(N'-Benzyl-N'-hydroxyureido)propen-3-yl]-N-3-methoxy-4-propoxyphenyl]-5-(3,4,5-trimethoxyphenyl)-tetrahydrofuran,

2-[5-[trans-1-(N'-Allyl-N'-hydroxyureido)propen-3-yl]-3-methoxy-4-propoxyphenyl]-5-(3,4,5-trimethoxyphenyl)-tetra- hydrofuran,

2-[5-[trans-1-(N'-Cyclohexyl-N'-hydroxyureido)propen-3-y]-3-methoxy-4-propoxyphenyl]-5-(3,4,5-trimethoxyphenyl)-tetrahydrofuran,

2-[5-[trans-1-(N'-Hydroxy-N'-methylthioethylureido)propen3-yl]-3-methoxy-4-propoxyphenyl]-5-(3,4,5-trimethoxyphenyl)-tetrahydrofuran,

2-[5-[trans-1-(N'-Cyclohexyl-N'-hydroxyureido)propen-3-yl]-3-methoxy-4-propoxyphenyl]-5-(3,4,5-trimethoxyphenyl)-tetrahydrofuran,

2-[5-[1-(N'-Cyclohexyl-N'-hydroxyureido)propyl]-3-methoxy-4-propoxyphenyl]-5-(3,4,5-trimethoxyphenyl)-tetrahydrofuran,

2-[5-[trans-1-(N'-Hydroxy-N'-methylureido)propen-3-yl]-4-(p-chlorphenylthioethoxy)-3-methoxyphenyl]-5-(3,4,5-trimethoxyphenyl)-tetrahydrofuran,

2-[5-[trans-1-(N'-Ethyl-N'-hydroxyureido)propen-3-yl]-4-(p-chlorphenylthioethoxy)-3-methoxyphenyl]-5-(3,4,5-trimethoxyphenyl)-tetrahydrofuran,

2-[5-[trans-1-(N'-Hydroxy-N'-propylureido)propen-3-yl]-4-(p-chlorphenylthioethoxy)-3-methoxyphenyl]-5-(3,4,5-trimethoxyphenyl)-tetrahydrofuran,

2-[5-[trans-1-(N'-Butyl-N'-hydroxyureido)propen-3-yl]-4-(p-chlorphenylthioethoxy)-3-methoxyphenyl]-5-(3,4,5-trimethoxyphenyl)-tetrahydrofuran,

2-[5-[trans-1-(N'-Hydroxy-N'-i-propylureido)propen-3-yl]-4-(p-chlorphenylthioethoxy)-3-methoxyphenyl]-5-(3,4,5-trimethoxyphenyl)-tetrahydrofuran,

2-[5-[trans-1-(N'-tert-Butyl-N'-hydroxyureido)propen-3-yl]-4-(p-chlorphenylthioethoxy)-3-methoxyphenyl]-5-(3,4,5-trimethoxyphenyl)-tetrahydrofuran,

2-[5-[trans-1-(N'-Benzyl-N'-hydroxyureido)propen-3-yl]-4-(p-chlorphenylthioethoxy)-3-methoxyphenyl]-5-(3,4,5-trimethoxyphenyl)-tetrahydrofuran,

2-[5-[trans-1-(N'-Cyclopropylmethyl-N'-hydroxyureido)-propen-3-yl]-4-(p-chlorphenylthioethoxy)-3-methoxyphenyl]-5-(3,4,5-trimethoxyphenyl)-tetrahydrofuran,

2-[5-[trans-1-(N'-Allyl-N'-hydroxyureido)propen-3-yl]-4-(p-chlorphenylthioethoxy)-3-methoxyphenyl-5-(3,4,5-trimethoxyphenyl)-tetrahydrofuran,
oder

2-[5-[trans-1-(N'-Hydroxy-N'-hydroxyethylureido)propen-3-yl]-4-(p-chlorphenylthioethoxy)-3-methoxyphenyl]-5-(3,4,5-trimethoxyphenyl)-tetrahydrofuran ist.

**3.** Trans-2-[5-N'-Methyl-N'-hydroxyureidomethyl)-3-methoxy-4-p-chlorphenylthioethoxyphenyl]-5-(3,4,5-trimethoxyphenyl)-tetrahydrofuran.

**4.** Pharmazeutische Zusammensetzung, die eine wirksame Menge einer Verbindung nach irgendeinem der Ansprüche 1 bis 3 in einem pharmazeutisch zulässigen Träger umfaßt.

**5.** Topische pharmazeutische Zusammensetzung, die eine wirksame Menge einer Verbindung nach irgendeinem der Ansprüche 1 bis 3 in einem pharmazeutisch zulässigen Träger umfaßt.

**6.** Verbindung nach irgendeinem der Ansprüche 1 bis 3 für die Verwendung als ein pharmazeutisches Mittel.

**7.** Verwendung einer Verbindung nach irgendeinem der Ansprüche 1 bis 3 bei der Herstellung eines Medikaments für die Verwendung bei der Behandlung einer entzündlichen Störung.

**8.** Verwendung einer Verbindung nach irgendeinem der Ansprüche 1 bis 3 bei der Herstellung eines Medikaments für die Verwendung bei der Behandlung eines Patienten, der einen PAF-Rezeptorantagonisten benötigt.

**9.** Verwendung einer Verbindung nach irgendeinem der Ansprüche 1 bis 3 bei der Herstellung eines Medikaments für die Verwendung bei der Behandlung eines Patienten, der einen Inhibitor der 5-Lipoxygenaseaktivität benötigt.

**Revendications**

**1.** Composé de formule :

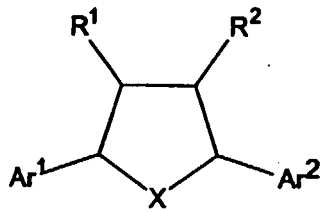

dans laquelle

$Ar^1$ =

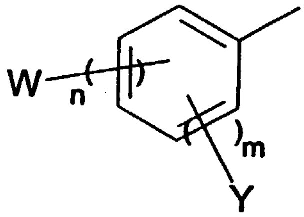

$Ar^2$ =

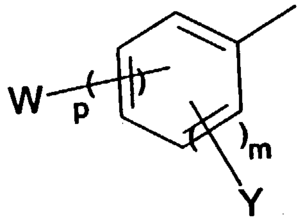

où :

X est 0, S ;
W représente, indépendamment :

-AN(OM)C(O)N($R^3$)$R^4$, -AN($R^3$)C(O)N(OM)$R^4$, -N(OM)C(O)N($R^3$)$R^4$ ou -N($R^3$)C(O)N(OM)$R^4$ ;

n = 1 ;
m est 2 ou 3 ;
p est 0 ;
A est un groupe alkylène en $C_{1-10}$, alcénylène en $C_{2-10}$ ou alcynylène en $C_{2-10}$ ;
M est un atome d'hydrogène ;
Y est indépendamment -$OR^{11}$ ;
$R^1$ et $R^2$ représentent des atomes d'hydrogène ;
$R^3$ et $R^4$ représentent indépendamment un groupe alkyle en $C_{1-10}$ alcényle en $C_{2-10}$ ou alcynyle en $C_{2-10}$ ;
$R^2$ est un groupe alkyle en $C_1$-$C_{12}$ ou un groupe alkyle en $C_1$-$C_{12}$ substitué dans lequel le substituant est choisi dans l'ensemble constitué par les groupes :

phényl-S$(O)_g$-alkylène($C_{1-6}$)-,
($R^3$O)$_d$-phényl-S$(O)_g$-alkylène-($C_{1-6}$)-,
(CN)$_d$-phényl-S$(O)_g$-alkylène($C_{1-6}$)-,
(halogéno)$_d$-phényl-S$(O)_g$-alkylène($C_{1-6}$)-,
($R^3$COO)$_d$-phényl-S$(O)_g$-alkylène($C_{1-6}$)-,
($R^3$OCO)$_d$-phényl-S$(O)_g$-alkylène-($C_{1-6}$)-,
($R^3$CO)$_d$-phényl-S$(O)_g$-alkylène($C_{1-6}$)-,
phényl-O-alkylène($C_{1-6}$)-,
($R^3$O)$_d$-phényl-O-alkylène($C_{1-6}$)-,
(CN)$_d$-phényl-O-alkylène($C_{1-6}$)-,
(halogéno)$_d$-phényl-O-alkylène($C_{1-6}$)-,
($R^3$COO)$_d$-phényl-O-alkylène($C_{1-6}$)-,
($R^3$OCO)$_d$-phényl-O-alkylène($C_{1-6}$)-,
($R^3R^3$N)$_d$-phényl-S$(O)_g$-alkylène($C_{1-6}$)- et
($R^3$CO)$_d$-phényl-O-alkylène-($C_{1-6}$)-, d étant 1, 2, 3, 4 ou 5 ; et g étant 0, 1 ou 2.

**2.** Composé, qui est le

2-[5-(N'-butyl-N'-hydroxyuréidyl)-3-méthoxy-4-méthoxyéthoxyphényl)-5-(3,4,5-triméthoxyphényl)tétrahydrothiophène,

2-[5-(N'-butyl-N'-hydroxyuréidyl)-3-méthoxy-4-méthoxythioéthoxyphényl]-5-(3,4,5-triméthoxyphényl)-tétrahydrofuranne,

2-[5-(N'-butyl-N'-hydroxyuréidyl)-4-hydroxyphénylthioéthoxy-3-méthoxyphényl]tétrahydrofuranne,

2-[5-(N'-butyl-N'-hydroxyuréidyl)-4-(N-nicotinoyl-N-phénylaminoéthoxy)-3-méthoxyphényl]-5-(3,4,5-triméthoxyphényl)tétrahydrofuranne,

iodure de 2-[5-(N'-butyl-N'-hydroxyuréidyl)-4-(N-3-pyridiniumcarbonyl-N-phénylaminoéthoxy)-3-méthoxyphényl]-5-(3,4,5-triméthoxyphényl)tétrahydrofurannepropyle,

iodure de 2-[5-(N'-p-chlorophényl-N'-hydroxyuréidyl)-4-(N-3-pyridiniumcarbonyl-N-phénylaminoéthoxy)-3-méthoxyphényl]-5-(3,4,5-triméthoxyphényl)tétrahydrofurannepropyle,

iodure de 2-[5-(N'-butyl-N'-hydroxyuréidyl)-4-(N-3-pyridiniumcarbonyl-N-phénylaminoéthoxy)-3-méthoxyphényl]-5-(3,4,5-triméthoxyphényl)tétrahydrofuranneéthyle,

iodure de 2-[5-(N'-p-chlorophényl-N'-hydroxyuréidyl)-4-(N-3-pyridiniumcarbonyl-N-phénylaminoéthoxy)-3-méthoxyphényl]-5-(3,4,5-triméthoxyphényl)tétrahydrofuranne-éthyle,

2-[5-(N'-p-chlorophényl-N'-hydroxyuréidyl)-3-méthoxy-4-propoxyphényl]-5-(3,4,5-triméthoxyphényl)tétrahydrofuranne,

2-[5-(N'-p-chlorophényl-N'-hydroxyuréidyl)-4-méthoxyéthoxy-3-méthoxyphényl]-5-(3,4,5-triméthoxyphényl)-tétrahydrothiophène,

2-[5-(N'-p-chlorophényl-N'-hydroxyuréidyl)-4-méthylthioéthoxy-3-méthoxyphényl]-5-(3,4,5-triméthoxyphényl)tétrahydrofuranne,

2-[5-(N'-p-chlorophényl-N'-hydroxyuréidyl)-4-p-hydroxyphénylthioéthoxy-3-méthoxyphényl]-5-(3,4,5-triéthoxyphényl)tétrahydrofuranne,

2-[5-(N'-p-chlorophényl-N'-hydroxyuréidyl)-4-(N-nicotinoyl-N-phénylaminoéthoxy)-3-méthoxyphényl]-5-(3,4,5-triméthoxyphényl)tétrahydrofuranne,

2-[5-(N'-butyl-N'-hydroxyuréidyl)-4-cyanophénylthioéthoxy-3-méthoxyphényl]-5-(3,4,5-triméthoxyphényl)-tétrahydrofuranne,

2-[5-(N'-p-chlorophényl-N'-hydroxyuréidyl)-4-p-cyanophénylthioéthoxy-3-méthoxyphényl]-5-(3,4,5-triméthoxyphényl)tétrahydrofuranne,

2-[5-(N'-butyl-N'-hydroxyuréidyl)-4-p-méthoxyphénylthioéthoxy-3-méthoxyphényl]-5-(3,4,5-triméthoxyphényl)tétrahydrofuranne,

2-[5-(N'-p-chlorophényl-N'-hydroxyuréidyl)-4-p-méthoxyphénylthioéthoxy-3-méthoxyphényl]-5-(3,4,5-triméthoxyphényl)tétrahydrofuranne,

2-[5-(N'-tert-butyl-N'-hydroxyuréidyl)-3-méthoxy-4-propoxyphényl]-5-(3,4,5-triméthoxyphényl)tétrahydrothiophène,

2-[5-(N'-n-butyl-N'-hydroxyuréidyl)-3-méthoxy-4-propoxyphényl]-5-(3,4,5-triméthoxyphényl)tétrahydrofuranne,

2-[5-(N'-éthyl-N'-hydroxyuréidyl)-3-méthoxy-4-propoxyphényl]-5-(3,4,5-triméthoxyphényl)tétrahydrofuranne,

2-[5-(N'-cyclohexyl-N'-hydroxyuréidyl)-3-méthoxy-4-propoxyphényl]-5-(3,4,5-triméthoxyphényl)tétrahydrofuranne,

2-[5-(N'-benzyl-N'-hydroxyuréidyl)-3-méthoxy-4-propoxyphényl]-5-(3,4,5-triméthoxyphényl)tétrahydrofuranne,

2-(5-N'-hydroxyuréidyl-3-méthoxy-4-propoxyphényl)-5-(3,4,5-triméthoxyphényl)tétrahydrothiophène,

2-[5-(N'-hydroxy-N'-méthyluréidyl)-3-méthoxy-4-propoxyphényl]-5-(3,4,5-triméthoxyphényl)tétrahydrothiophène,

2-[5-(N'-hydroxy-N'-isopropyluréidyl)-3-méthoxy-4-propoxyphényl]-5-(3,4,5-triméthoxyphényl)tétrahydrothiophène,

2-[5-(N'-sec-butyl-N'-hydroxyuréidyl)-3-méthoxy-4-propoxyphényl]-5-(3,4,5-triméthoxyphényl)tétrahydrothiophène,

2-[5-(N'-hydroxy-N'-propyluréidyl)-3-méthoxy-4-propoxyphényl]-5-(3,4,5-triméthoxyphényl)tétrahydrothiophène,

2-[5-(N'-hydroxy-N'propyluréidyl)-3-méthoxy-4-propoxyphényl]-5-(3,4,5-triméthoxyphényl)tétrahydrofuranne,

2-[5-(N'-hydroxy-N'-n-pentyluréidyl)-3-méthoxy-4-propoxyphényl]-5-(3,4,5-triméthoxyphényl)tétrahydrofuranne,

2-[5-(N'-cyclohexyl-N'-hydroxyuréidyl)-3-méthoxy-4-propoxyphényl]-5-(3,4,5-triméthoxyphényl)tétrahydrofuranne,

2-[5-(N'-hexyl-N'-hydroxyuréidyl)-3-méthoxy-4-propoxyphényl]-5-(3,4,5-triméthoxyphényl)tétrahydrofuranne,

2-[5-(N'-benzyl-N'-hydroxyuréidyl)-3-méthoxy-4-propoxyphényl]-5-(3,4,5-triméthoxyphényl)tétrahydrofuranne,

2-[5-(N'-hydroxy-N'-octyluréidyl)-3-méthoxy-4-propoxyphényl]-5-(3,4,5-triméthoxyphényl)tétrahydrofuranne,

2-[5-(N'-hydroxy-N'-méthoxyéthyluréidyl)-3-méthoxy-4-propoxyphényl]-5-(3,4,5-triméthoxyphényl)tétrahydrofuranne,

2-[5-(N'-décyl-N'-hydroxyuréidyl)-3-méthoxy-4-propoxyphényl]-5-(3,4,5-triméthoxyphényl)tétrahydrofuranne,
2-[5-(N'-hydroxy-N'-n-pentyluréidyl)-3-méthoxy-4-propoxyphényl]-5-(3,4,5-triméthoxyphényl)tétrahydrothiophène,
2-[5-(N'-cyclohexyl-N'-hydroxyuréidyl)-3-méthoxy-4-propoxyphényl]-5-(3,4,5-triméthoxyphényl)tétrahydrothiophène,
2-[5-(N'-hexyl-N'-hydroxyuréidyl)-3-méthoxy-4-propoxyphényl]-5-(3,4,5-triméthoxyphényl)tétrahydrothiophène,
2-[5-(N'-benzyl-N'-hydroxyuréidyl)-3-méthoxy-4-propoxyphényl]-5-(3,4,5-triméthoxyphényl)tétrahydrothiophène,
2-[5-(N'-hydroxy-N'-octyluréidyl)-3-méthoxy-4-propoxyphényl]-5-(3,4,5-triméthoxyphényl)tétrahydrothiophène,
2-[5-(N'-hydroxy-N'-méthoxyéthyluréidyl)-3-méthoxy-4-propoxyphényl]-5-(3,4,5-triméthoxyphényl)tétrahydrothiophène,
2-[5-(N'-décyl-N'-hydroxyuréidyl)-3-méthoxy-4-propoxyphényl]-5-(3,4,5-triméthoxyphényl)tétrahydrothiophène,
2-[5-(N'-butyl-N'-hydroxyuréidyl)-4-(p-bromophénylthioéthoxy)-3-méthoxyphényl]-5-(3,4,5-triméthoxyphényl)-tétrahydrofuranne,
2-[5-(N'-butyl-N'-hydroxyuréidyl)-4-(2-bromophénylthioéthoxy)-3-méthoxyphényl]-5-(3,4,5-triméthoxyphényl)-tétrahydrofuranne,
2-[5-(N'-butyl-N'-hydroxyuréidyl)-4-(3-bromophénylthioéthoxy)-3-méthoxyphényl]-5-(3,4,5-triméthoxyphényl)-tétrahydrofuranne,
2-[5-(N'-butyl-N'-hydroxyuréidyl-4-(3,4-dichlorophénylthioéthoxy)-3-méthoxyphényl]-5-(3,4,5-triméthoxyphényl)tétrahydrofuranne,
2-[5-(N'-butyl-N-hydroxyuréidyl)-4-(p-chlorophénylthioéthoxy)-3-méthoxyphényl]-5-(3,4,5-triméthoxyphényl)-tétrahydrofuranne,
2-[5-(N'-butyl-N'-hydroxyuréidyl)-4-(p-fluorophénylthioéthoxy)-3-méthoxyphényl]-5-(3,4,5-triméthoxyphényl)tétrahydrofuranne,
2-[5-(N'-butyl-N'-hydroxyuréidyl)-4-(2,3,5,6-tétrafluorophénylthioéthoxy)-3-méthoxyphényl]-5-(3,4,5-triméthoxyphényl)tétrahydrofuranne,
2-[5-(N'-butyl-N'-hydroxyuréidyl)-4-(2,3,4,5-tétrafluorophényléthoxy)-3-méthoxyphényl]-5-(3,4,5-triméthoxyphényl)tétrahydrofuranne,
2-[5-(N'-butyl-N'-hydroxyuréidyl)-4-(p-bromophénylsulfonyléthoxy)-3-méthoxyphényl]-5-(3,4,5-triméthoxyphényl)tétrahydrofuranne,
2-[5-(N'-butyl-N'-hydroxyuréidyl)-4-(2-bromophénylsulfonyléthoxy)-3-méthoxyphényl]-5-(3,4,5-triméthoxyphényl)tétrahydrofuranne,
2-[5-(N'-hydroxy-N'-méthyluréidyl)-4-(p-bromophénylthioéthoxy)-3-méthoxyphényl]-5-(3,4,5-triméthoxyphényl)tétrahydrofuranne,
2-[5-(N'-hydroxy-N'-méthyluréidyl)-4-(2-bromophénylthioéthoxy)-3-méthoxyphényl]-5-(3,4,5-triméthoxyphényl)tétrahydrofuranne,
2-[5-(N'-hydroxy-N'-méthyluréidyl)-4-(3-bromophénylthioéthoxy)-3-méthoxyphényl]-5-(3,4,5-triméthoxyphényl)tétrahydrofuranne,
2-[5-(N'-hydroxy-N'-méthyluréidyl)-4-(3,4-dichlorophénylthioéthoxy)-3-méthoxyphényl]-5-(3,4,5-triméthoxyphényl)tétrahydrofuranne,
2-[5-(N'-hydroxy-N'-méthyluréidyl)-4-(p-chlorophénylthioéthoxy)-3-méthoxyphényl]-5-(3,4,5-triméthoxyphényl)tétrahydrofuranne,
2-[5-(N'-éthyl-N'-hydroxyuréidyl)-4-p-fluorophénylthioéthoxy-3-méthoxyphényl]-5-(3,4,5-triméthoxyphényl)-tétrahydrofuranne,
2-[5-(N'-éthyl-N'-hydroxyuréidyl)-4-(2,3,5,6-tétrafluorophénylthioéthoxy)-3-méthoxyphényl]-5-(3,4,5-triméthoxyphényl)tétrahydrofuranne,
2-[5-(N'-éthyl-N'-hydroxyuréidyl)-4-(2,3,4,5-tétrafluorophényléthoxy)-3-méthoxyphényl]-5-(3,4,5-triméthoxyphényl)tétrahydrofuranne,
2-[5-(N'-p-chlorophényl-N'-hydroxyuréidyl)-4-(2-bromophénylsulfonyléthoxy)-3-méthoxyphényl]-5-(3,4,5-triméthoxyphényl)tétrahydrofuranne,
2-[5-(N'-p-chlorophényl-N'-hydroxyuréidyl)-4-(p-bromophénylthioéthoxy)-3-méthoxyphényl]-5-(3,4,5-triméthoxyphényl)tétrahydrofuranne,
2-[5-(N'-tert-butyl-N'-hydroxyuréidyl)-4-(p-bromophénylthioéthoxy)-3-méthoxyphényl]-5-(3,4,5-triméthoxyphényl)tétrahydrofuranne,
2-[5-(N'-tert-butyl-N'-hydroxyuréidyl)-4-(2-bromophénylthioéthoxy)-3-méthoxyphényl]-5-(3,4,5-triméthoxyphényl)tétrahydrofuranne,

2-[5-(N'-tert-butyl-N'-hydroxyuréidyl)-4-(3-bromophénylthioéthoxy)-3-méthoxyphényl)-5-(3,4,5-triméthoxyphényl)tétrahydrofuranne,

2-[5-(N'-cyclohexyl-N'-hydroxyuréidyl)-4-(3,4-dichlorophénylthioéthoxy)-3-méthoxyphényl]-5-(3,4,5-triméthoxyphényl)tétrahydrofuranne,

2-[5-(N'-cyclohexyl-N'-hydroxyuréidyl)-4-(p-chlorophénylthioéthoxy)-3-méthoxyphényl]-5-(3,4,5-triméthoxyphényl)tétrahydrofuranne,

2-[5-(N'-cyclohexyl-N'-hydroxyuréidyl)-4-(p-fluorophénylthioéthoxy)-3-méthoxyphényl]-5-(3,4,5-triméthoxyphényl)tétrahydrofuranne,

2-[5-(N'-benzyl-N'-hydroxyuréidyl)-4-(2,3,5,6-tétrafluorophénylthioéthoxy)-3-méthoxyphényl]-5-(3,4,5-triméthoxyphényl)tétrahydrofuranne,

2-[5-(N'-benzyl-N'-hydroxyuréidyl)-4-(2,3,4,5-tétrafluorophényléthoxy)-3-méthoxyphényl]-5-(3,4,5-triméthoxyphényl)tétrahydrofuranne,

2-[5-(N'-benzyl-N'-hydroxyuréidyl)-4-(p-bromophénylsulfonyléthoxy)-3-méthoxyphényl]-5-(3,4,5-triméthoxyphényl)tétrahydrofuranne,

2-[5-(N'-benzyl-N'-hydroxyuréidyl)-4-(2-bromophénylsulfonyléthoxy)-3-méthoxyphényl]-5-(3,4,5-triméthoxyphényl)tétrahydrofuranne,

2-[5-(N'-hydroxy-N'-isopropyluréidyl)-4-(p-bromophénylthioéthoxy)-3-méthoxyphényl]-5-(3,4,5-triméthoxyphényl)tétrahydrofuranne,

2-[5-(N'-sec-butyl-N'-hydroxyuréidyl)-4-(p-bromophénylthioéthoxy)-3-méthoxyphényl]-5-(3,4,5-triméthoxyphényl)tétrahydrofuranne,

2-[5-(N'-sec-butyl-N'-hydroxyuréidyl)-4-(2-bromophénylthioéthoxy)-3-méthoxyphényl]-5-(3,4,5-triméthoxyphényl)tétrahydrofuranne,

2-[5-(N'-hydroxy-N'-propyluréidyl)-4-(2-bromophénylthioéthoxy)-3-méthoxyphényl]-5-(3,4,5-triméthoxyphényl)tétrahydrofuranne,

2-[5-(N'-hydroxy-N'-n-pentyluréidyl)-4-(3,4-dichlorophénylthioéthoxy)-3-méthoxyphényl]-5-(3,4,5-triméthoxyphényl)tétrahydrofuranne,

2-[5-(N'-hexyl-N'-hydroxyuréidyl)-4-(p-chlorophénylthioéthoxy)-3-méthoxyphényl]-5-(3,4,5-triméthoxyphényl)tétrahydrofuranne,

2-[5-(N'-hydroxy-N'-octyluréidyl)-4-(p-fluorophénylthioéthoxy)-3-méthoxyphényl]-5-(3,4,5-triméthoxyphényl)tétrahydrofuranne,

2-[5-(N'-hydroxyméthoxyéthyluréidyl)-4-(2,3,5,6-tétrafluorophénylthioéthoxy)-3-méthoxyphényl]-5-(3,4,5-triméthoxyphényl)tétrahydrofuranne,

2-[5-(N'-décyl-N'-hydroxyuréidyl)-4-(2,3,4,5-tétrafluorophényléthoxy)-3-méthoxyphényl]-5-(3,4,5-triméthoxyphényl)tétrahydrofuranne,

2-[5-(N'-hydroxy-N'-méthyluréidyl)-4-(p-chlorophénylthioéthoxy)-3-méthoxyphényl]-5-(3,4,5-triméthoxyphényl)tétrahydrofuranne,

2-[5-(N'-hydroxy-N'-isopropyluréidylméthyl)-4-(p-chlorophénylthioéthoxy)-3-méthoxyphényl]-5-(3,4,5-triméthoxyphényl)tétrahydrofuranne,

2-[5-(N'-butyl-N'-hydroxyuréidylméthyl)-4-(p-chlorophénylthioéthoxy)-3-méthoxyphényl]-5-(3,4,5-triméthoxyphényl)tétrahydrofuranne,

2-[5-(N'-hydroxy-N'-propyluréidylméthyl)-4-(p-chlorophénylthioéthoxy)-3-méthoxyphényl]-5-(3,4,5-triméthoxyphényl)tétrahydrofuranne,

2-[5-(N'-éthyl-N'-hydroxyuréidyméthyl)-4-(p-chlorophénylthioéthoxy)-3-méthoxyphényl]-5-(3,4,5-triméthoxyphényl)tétrahydrofuranne,

2-[5-(N'-hydroxy-N'-octyluréidylméthyl)-4-(p-chlorophénylthioéthoxy)-3-méthoxyphényl]-5-(3,4,5-triméthoxyphényl)tétrahydrofuranne,

2-[5-(N'-benzyl-N'-hydroxyuréidyl)-4-(p-bromophénylsulfonyléthoxy)-3-méthoxyphényl]-5-(3,4,5-triméthoxyphényl)tétrahydrothiophène,

2-[5-(N'-benzyl-N'-hydroxyuréidyl)-4-(2-bromophénylsulfonyléthoxy)-3-méthoxyphényl]-5-(3,4,5-triméthoxyphényl)tétrahydrothiophène,

2-[5-(N'-hydroxy-N'-isopropyluréidyl)-4-(p-bromophénylthioéthoxy)-3-méthoxyphényl]-5-(3,4,5-triméthoxyphényl)tétrahydrothiophène,

2-[5-(N'-hydroxy-N'-octyluréidyl)-4-(p-fluorophénylthioéthoxy)-3-méthoxyphényl]-5-(3,4,5-triméthoxyphényl)tétrahydrothiophène,

2-[5-(N'-butyl-N'-hydroxyuréidyl)-4-(p-bromophénylthioéthoxy)-3-méthoxyphényl]-5-(3,4,5-triméthoxyphényl)-tétrahydrothiophène,

2-[5-(N'-butyl-N'-hydroxyuréidyl)-4-(2-bromophénylthioéthoxy)-3-méthoxyphényl]-5-(3,4,5-triméthoxyphényl)tétrahydrothiophène,

2-[5-(N'-butyl-N'-hydroxyuréidyl)-4-(3-bromophénylthioéthoxy)-3-méthoxyphényl]-5-(3,4,5-triméthoxyphényl)tétrahydrothiophène,

2-[5-(N'-hydroxy-N'-méthyluréidylméthyl)-4-(p-chlorophénylthioéthoxy)-3-méthoxyphényl)-5-(3,4,5-triméthoxyphényl)tétrahydrothiophène,
2-[5-(N'-hydroxy-N'-isopropyluréidylméthyl)-4-(p-chlorophénylthioéthoxy)-3-méthoxyphényl]-5-(3,4,5-triméthoxyphényl)tétrahydrothiophène,
2-[5-(N'-butyl-N'-hydroxyuréidylméthyl)-4-(p-chlorophénylthioéthoxy)-3-méthoxyphényl]-5-(3,4,5-triméthoxyphényl)tétrahydrothiophène,
2-[5-[1-(N'-hydroxy-N'-méthyluréidyl)propyn-3-yl]-3-méthoxy-4-propoxyphényl]-5-(3,4,5-triméthoxyphényl)-tétrahydrofuranne,
2-[5-[1-(N'-éthyl-N'-hydroxyuréidyl)propyn-3-yl]-3-méthoxy-4-propoxyphényl]-5-(3,4,5-triméthoxyphényl)-tétrahydrofuranne,
2-[5-[1-(N'-hydroxy-N'-propyluréidyl)propyn-3-yl]-3-méthoxy-4-propoxyphényl]-5-(3,4,5-triméthoxyphényl)-tétrahydrofuranne,
2-[5-[1-(N'-n-butyl-N'-hydroxyuréidyl)propyn-3-yl]-3-méthoxy-4-propoxyphényl]-5-(3,4,5-triméthoxyphényl)-tétrahydrofuranne,
2-[5-[1-(N'-hydroxy-N'-isopropyluréidyl)propyn-3-yl]-3-méthoxy-4-propoxyphényl]-5-(3,4,5-triméthoxyphényl)-tétrahydrofuranne,
2-[5-[1-(N'-tert-butyl-N'-hydroxyuréidyl)propyn-3-yl]-3-méthoxy-4-propoxyphényl]-5-(3,4,5-triméthoxyphényl)tétrahydrofuranne,
2-[5-[1-(N'-benzyl-N'-hydroxyuréidyl)propyn-3-yl]-3-méthoxy-4-propoxyphényl]-5-(3,4,5-triméthoxyphényl)-tétrahydrofuranne,
2-[5-[1-(N'-cyclopropylméthyl-N'-hydroxyuréidyl)propyn3-yl]-3-méthoxy-4-propoxyphényl]-5-(3,4,5-triméthoxyphényl)tétrahydrofuranne,
2-[5-[1-(N'-allyl-N'-hydroxyuréidyl)propyn-3-yl]-3-méthoxy-4-propoxyphényl]-5-(3,4,5-triméthoxyphényl)-tétrahydrofuranne,
2-[5-[1-(N'-hydroxy-N'-hydroxyéthyluréidyl)propyn-3-yl]-3-méthoxy-4-propoxyphényl]-5-(3,4,5-triméthoxyphényl)tétrahydrofuranne,
2-[5-[1-(N'-hydroxy-N'-méthyluréidyl)propyn-3-yl]-4-(p-chlorophénylthioéthoxy)-3-méthoxyphényl]-5-(3,4,5-triméthoxyphényl)tétrahydrofuranne,
2-[5-[1-(N'-éthyl-N'-hydroxyuréidyl)propyn-3-yl]-4-(p-chlorophénylthioéthoxy)-3-méthoxyphényl]-5-(3,4,5-triméthoxyphényl)tétrahydrofuranne,
2-[5-[1-(N'-hydroxy-N'-propyluréidyl)propyn-3-yl]-4-(p-chlorophénylthioéthoxy)-3-méthoxyphényl]-5-(3,4,5-triméthoxyphényl)tétrahydrofuranne,
2-[5-[1-(N'-n-butyl-N'-hydroxyuréidyl)propyn-3-yl]-4-(p-chlorophénylthioéthoxy)-3-méthoxyphényl]-5-(3,4,5-triméthoxyphényl)tétrahydrofuranne,
2-[5-[1-(N'-hydroxy-N'-isopropyluréidyl)propyn-3-yl]-4-(p-chlorophénylthioéthoxy)-3-méthoxyphényl]-5-(3,4,5-triméthoxyphényl)tétrahydrofuranne,
2-[5-[1-(N'-tert-butyl-N'-hydroxyuréidyl)propyn-3-yl]-4-(p-chlorophénylthioéthoxy)-3-méthoxyphényl]-5-(3,4,5-triméthoxyphényl)tétrahydrofuranne,
2-[5-[1-[N'-benzyl-N'-hydroxyuréidyl)propyn-3-yl]-4-(p-chlorophénylthioéthoxy)-3-méthoxyphényl]-5-(3,4,5-triméthoxyphényl)tétrahydrofuranne,
2-[5-[1-(N'-cyclopropylméthyl-N'-hydroxyuréidyl)propyn-3-yl]-4-(p-chlorophénylthioéthoxy)-3-méthoxyphényl]-5-(3,4,5-triméthoxyphényl)tétrahydrofuranne,
2-[5-[1-(N'-allyl-N'-hydroxyuréidyl)propyn-3-yl]-4-(p-chlorophénylthioéthoxy)-3-méthoxyphényl]-5-(3,4,5-triméthoxyphényl)tétrahydrofuranne,
2-[5-[1-(N'-hydroxy-N'-hydroxyéthyluréidyl)propyn-3-yl]-4-(p-chlorophénylthioéthoxy)-3-méthoxyphényl]-5-(3,4,5-triméthoxyphényl)tétrahydrofuranne,
2-[5-[*trans*-1-(N'-hydroxy-N'-méthyluréidyl)propén-3-yl]-3-méthoxy-4-propoxyphényl]-5-(3,4,5-triméthoxyphényl)tétrahydrofuranne,
2-[5-[*trans*-1-(N'-éthyl-N'-hydroxyuréidyl)propén-3-yl]-3-méthoxy-4-propoxyphényl]-5-(3,4,5-triméthoxyphényl)tétrahydrofuranne,
2-[5-[*trans*-1-(N'-hydroxy-N'-propyluréidyl)propén-3-yl]-3-méthoxy-4-propoxyphényl]-5-(3,4,5-triméthoxyphényl)tétrahydrofuranne,
2-[5-[*trans*-1-(N'-butyl-N'-hydroxyuréidyl)propén-3-yl]-3-méthoxy-4-propoxyphényl]-5-(3,4,5-triméthoxyphényl)tétrahydrofuranne,
2-[5-[*trans*-1-(N'-hydroxy-N'-isopropyluréidyl)propén-3-yl]-3-méthoxy-4-propoxyphényl]-5-(3,4,5-triméthoxyphényl)tétrahydrofuranne,

2-[5-[*trans*-1-(N'-tert-butyl-N'-hydroxyuréidyl)propén3-yl]-3-méthoxy-4-propoxyphényl]-5-(3,4,5-triméthoxyphényl)tétrahydrofuranne,

2-[5-[*trans*-1-(N'-benzyl-N'-hydroxyuréidyl)propén-3-yl]-3-méthoxy-4-propoxyphényl]-5-(3,4,5-triméthoxyphényl)tétrahydrofuranne,

2-[5-[*trans*-1-(N'-allyl-N'-hydroxyuréidyl)propén-3-yl]-3-méthoxy-4-propoxyphényl]-5-(3,4,5-triméthoxyphényl)tétrahydrofuranne,

2-[5-[*trans*-1-(N'-cyclohexyl-N'-hydroxyuréidyl)propén-3-yl]-3-méthoxy-4-propoxyphényl]-5-(3,4,5-triméthoxyphényl)tétrahydrofuranne,

2-[5-[*trans*-1-(N'-hydroxy-N'-méthylthioéthyluréidyl)-propén-3-yl]-3-méthoxy-4-propoxyphényl]-5-(3,4,5-triméthoxyphényl)tétrahydrofuranne,

2-[5-[*trans*-1-(N'-cyclohexyl-N'-hydroxyuréidyl)propén-3-yl]-3-méthoxy-4-propoxyphényl]-5-(3,4,5-triméthoxyphényl)tétrahydrofuranne,

2-[5-[1-(N'-cyclohexyl-N'-hydroxyuréidyl)propyl]-3-méthoxy-4-propoxyphényl]-5-(3,4,5-triméthoxyphényl)-tétrahydrofuranne,

2-[5-[*trans*-1-(N'-hydroxy-N'-méthyluréidyl)propén-3-yl]-4-(p-chlorophénylthioéthoxy)-3-méthoxyphényl]-5-(3,4,5-triméthoxyphényl)tétrahydrofuranne,

2-[5-[*trans*-1-(N'-éthyl-N'-hydroxyuréidyl)propén-3-yl]-4-(p-chlorophénylthioéthoxy)-3-méthoxyphényl]-5-(3,4,5-triméthoxyphényl)tétrahydrofuranne,

2-[5-[*trans*-1-(N'-hydroxy-N'-propyluréidyl)propén-3-yl]-4-(p-chlorophénylthioéthoxy)-3-méthoxyphényl]-5-(3,4,5-triméthoxyphényl)tétrahydrofuranne,

2-[5-[*trans*-1-(N'-n-butyl-N'-hydroxyuréidyl)propén-3-yl]-4-(p-chlorophénylthioéthoxy)-3-méthoxyphényl]-5-(3,4,5-triméthoxyphényl)tétrahydrofuranne,

2-[5-[*trans*-1-(N'-hydroxy-N'-isopropyluréidyl)propén-3-yl]-4-(p-chlorophénylthioéthoxy)-3-méthoxyphényl]-5-(3,4,5-triméthoxyphényl)tétrahydrofuranne,

2-[5-[*trans*-1-(N'-tert-butyl-N'-hydroxyuréidyl)propén-3-yl]-4-(p-chlorophénylthioéthoxy)-3-méthoxyphényl]-5-(3,4,5-triméthoxyphényl)tétrahydrofuranne,

2-[5-[*trans*-1-(N'-benzyl-N'-hydroxyuréidyl)propén-3-yl]-4-(p-chlorophénylthioéthoxy)-3-méthoxyphényl]-5-(3,4,5-triméthoxyphényl)tétrahydrofuranne,

2-[5-[*trans*-1-(N'-cyclopropylméthyl-N'-hydroxyuréidyl)-propén-3-yl]-4-(p-chlorophénylthioéthoxy)-3-méthoxyphényl]-5-(3,4,5-triméthoxyphényl)tétrahydrofuranne,

2-[5-[*trans*-1-(N'-allyl-N'-hydroxyuréidyl)propén-3-yl]-4-(p-chlorophénylthioéthoxy)-3-méthoxyphényl]-5-(3,4,5-triméthoxyphényl)tétrahydrofuranne

ou

2-[5-[*trans*-1-(N'-hydroxy-N'-hydroxyéthyluréidyl)-propén-3-yl]-4-(p-chlorophénylthioéthoxy)-3-méthoxyphényl]-5-(3,4,5-triméthoxyphényl)tétrahydrofuranne.

**3.** *trans*-2-[5-(N'-méthyl-N'-hydroxyuréidylméthyl)-3-méthoxy-4-p-chlorophénylthioéthoxyphényl]-5-(3,4,5-triméthoxyphényl)tétrahydrofuranne.

**4.** Composition pharmaceutique comprenant une quantité efficace d'un composé selon l'une quelconque des revendications 1 à 3, dans un véhicule pharmaceutiquement acceptable.

**5.** Composition pharmaceutique topique comprenant une quantité efficace d'un composé selon l'une quelconque des revendications 1 à 3, dans un véhicule pharmaceutiquement acceptable.

**6.** Composé selon l'une quelconque des revendications 1 à 3, pour utilisation en tant que produit pharmaceutique.

**7.** Utilisation d'un composé selon l'une quelconque des revendications 1 à 3, pour la fabrication d'un médicament destiné à être utilisé dans le traitement d'un trouble inflammatoire.

**8.** Utilisation d'un composé selon l'une quelconque des revendications 1 à 3, pour la fabrication d'un médicament destiné à être utilisé dans le traitement d'un patient nécessitant un antagoniste de récepteur de PAF (facteur d'activation des plaquettes).

**9.** Utilisation d'un composé selon l'une quelconque des revendications 1 à 3, pour la fabrication d'un médicament destiné à être utilisé dans le traitement d'un patient nécessitant un inhibiteur de l'activité 5-lipoxygénase.

**Figure 1**

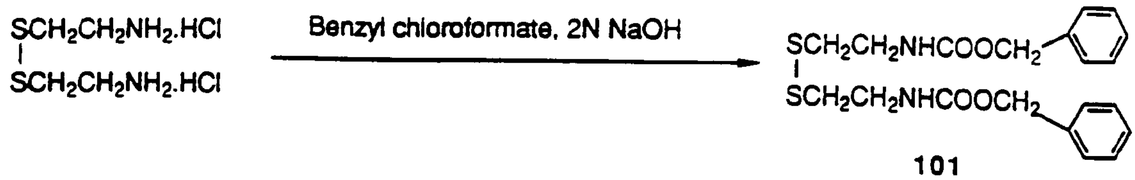


101

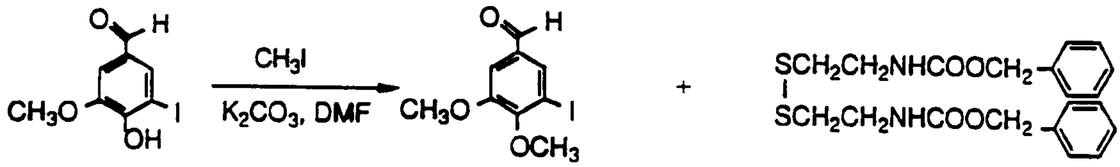


102 101

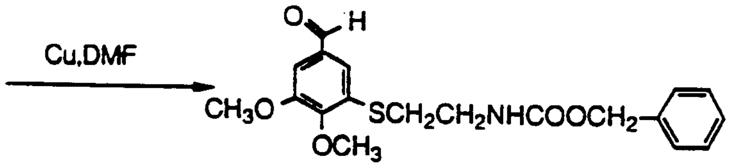


103

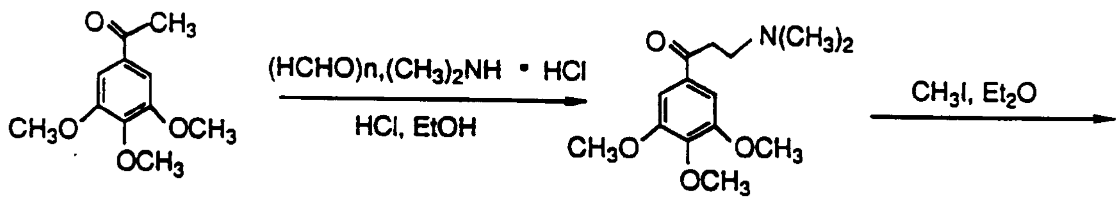


104

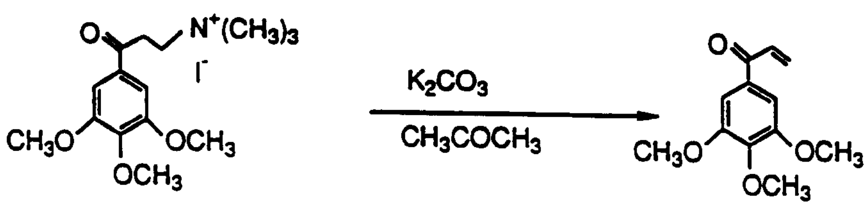


105 106

**Figure 2**

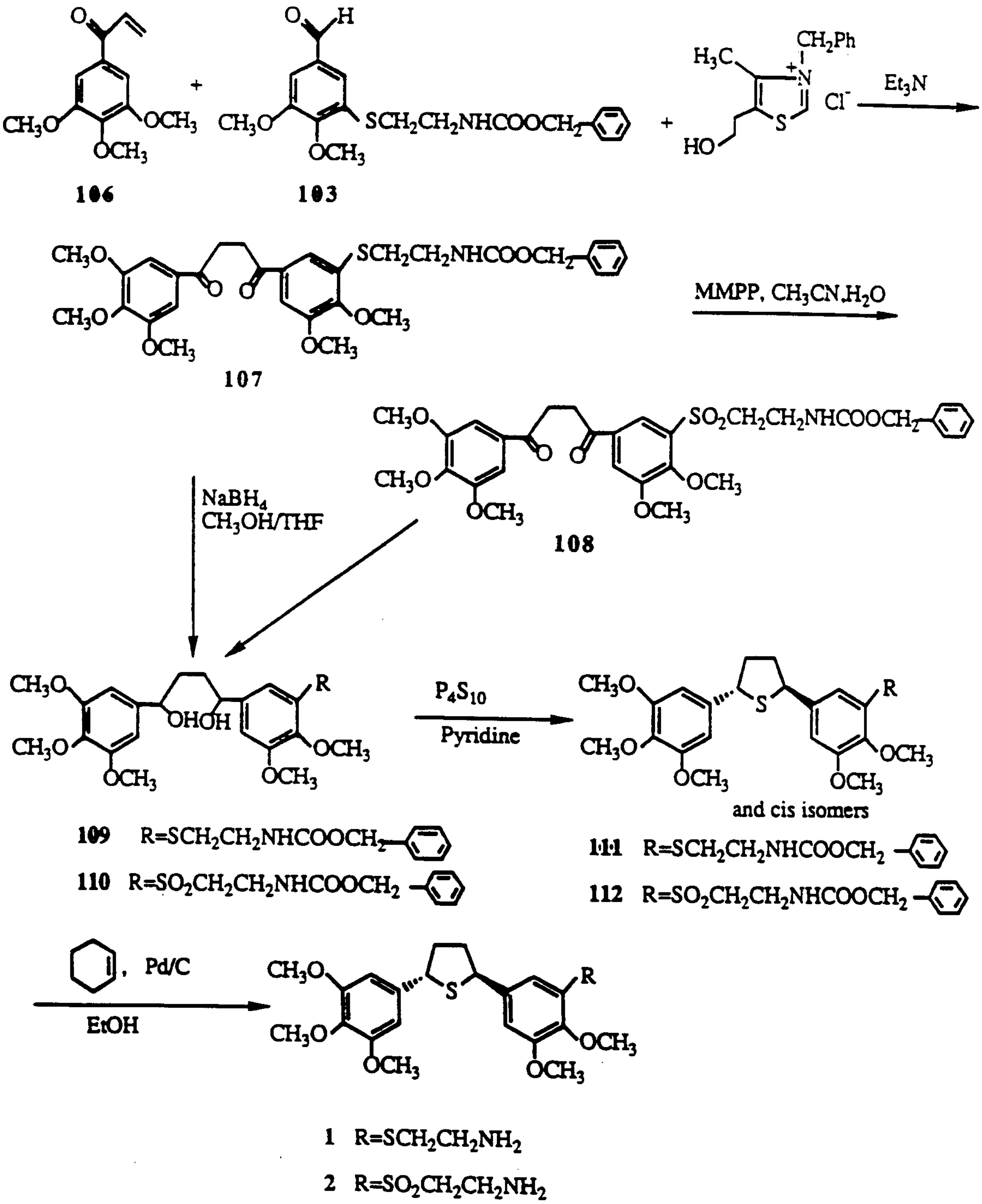


106

103

107

108

109 R=$SCH_2CH_2NHCOOCH_2$-

110 R=$SO_2CH_2CH_2NHCOOCH_2$-

111 R=$SCH_2CH_2NHCOOCH_2$-

112 R=$SO_2CH_2CH_2NHCOOCH_2$-

1 R=$SCH_2CH_2NH_2$

2 R=$SO_2CH_2CH_2NH_2$

**Figure 3**

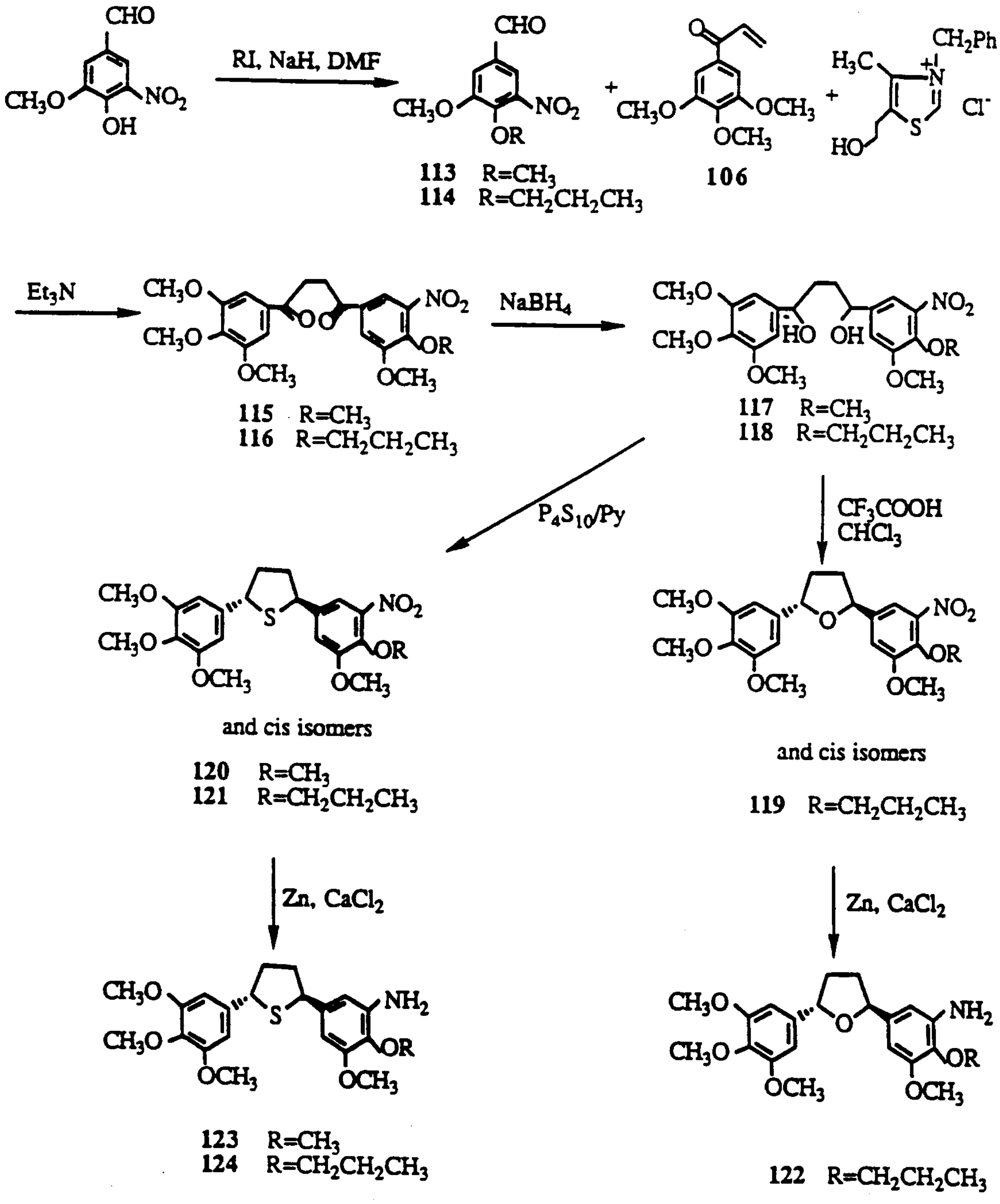


113 R=$CH_3$
114 R=$CH_2CH_2CH_3$
106

115 R=$CH_3$
116 R=$CH_2CH_2CH_3$

117 R=$CH_3$
118 R=$CH_2CH_2CH_3$

and cis isomers

120 R=$CH_3$
121 R=$CH_2CH_2CH_3$

and cis isomers

119 R=$CH_2CH_2CH_3$

123 R=$CH_3$
124 R=$CH_2CH_2CH_3$

122 R=$CH_2CH_2CH_3$

## Figure 4

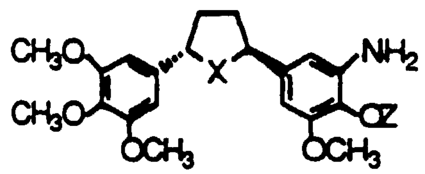

X=O, Z=$CH_2CH_2CH_3$ 122
X=S, Z=$CH_3$ 123
X=S, Z=$CH_2CH_2CH_3$ 124

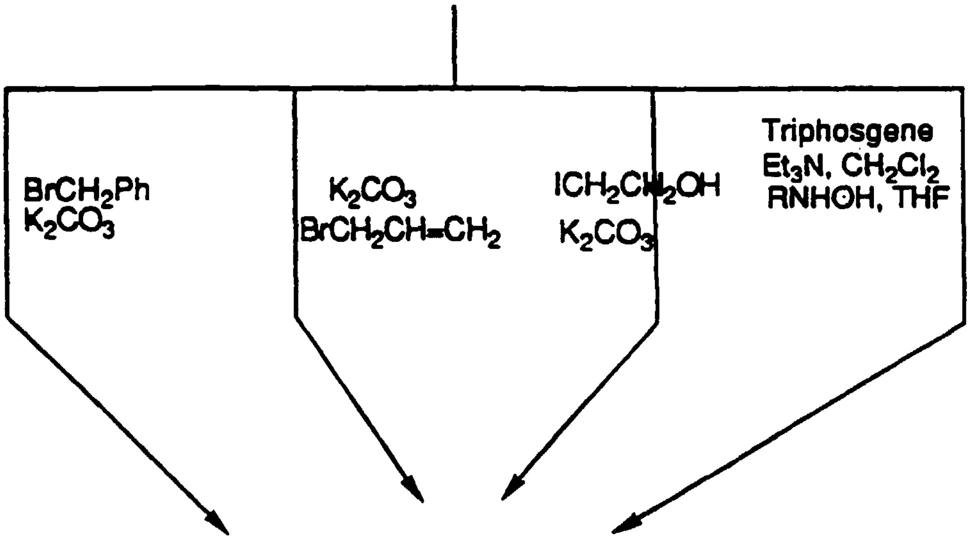


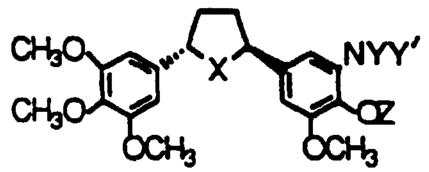

| | | | |
|---|---|---|---|
| X=O, Z=$CH_2CH_2CH_3$ | Y=$CH_2Ph$ | Y'=H | 3 |
| X=O, Z=$CH_2CH_2CH_3$ | Y=$CH_2CH_2OH$ | Y'=H | 4 |
| X=O, Z=$CH_2CH_2CH_3$ | Y=CH2CH=CH2 | Y'=$CH_2CH=CH_2$ | 5 |
| X=S, Z=$CH_2CH_2CH_3$ | Y=$CH_2Ph$ | Y'=H | 7 |
| X=S, Z=$CH_2CH_2CH_3$ | Y=$CH_2CH_2OH$ | Y'=H | 9 |
| X=S, Z=$CH_2CH_2CH_3$ | Y=$CH_2CH=CH_2$ | Y'=$CH_2CH=CH_2$ | 11 |
| X=S, Z=$CH_3$ | Y=$CH_2Ph$ | Y'=H | 6 |
| X=S, Z=$CH_3$ | Y=$CH_2CH_2OH$ | Y'=H | 8 |
| X=S, Z=$CH_3$ | Y=CH2CH=$CH_2$ | Y'=$CH_2CH=CH_2$ | 10 |
| X=O,S; Z=$CH_2CH_2CH_3$ | Y=CONAOH | Y'=H A=H, alkyls | 12-32 38-41 |

Figure 5

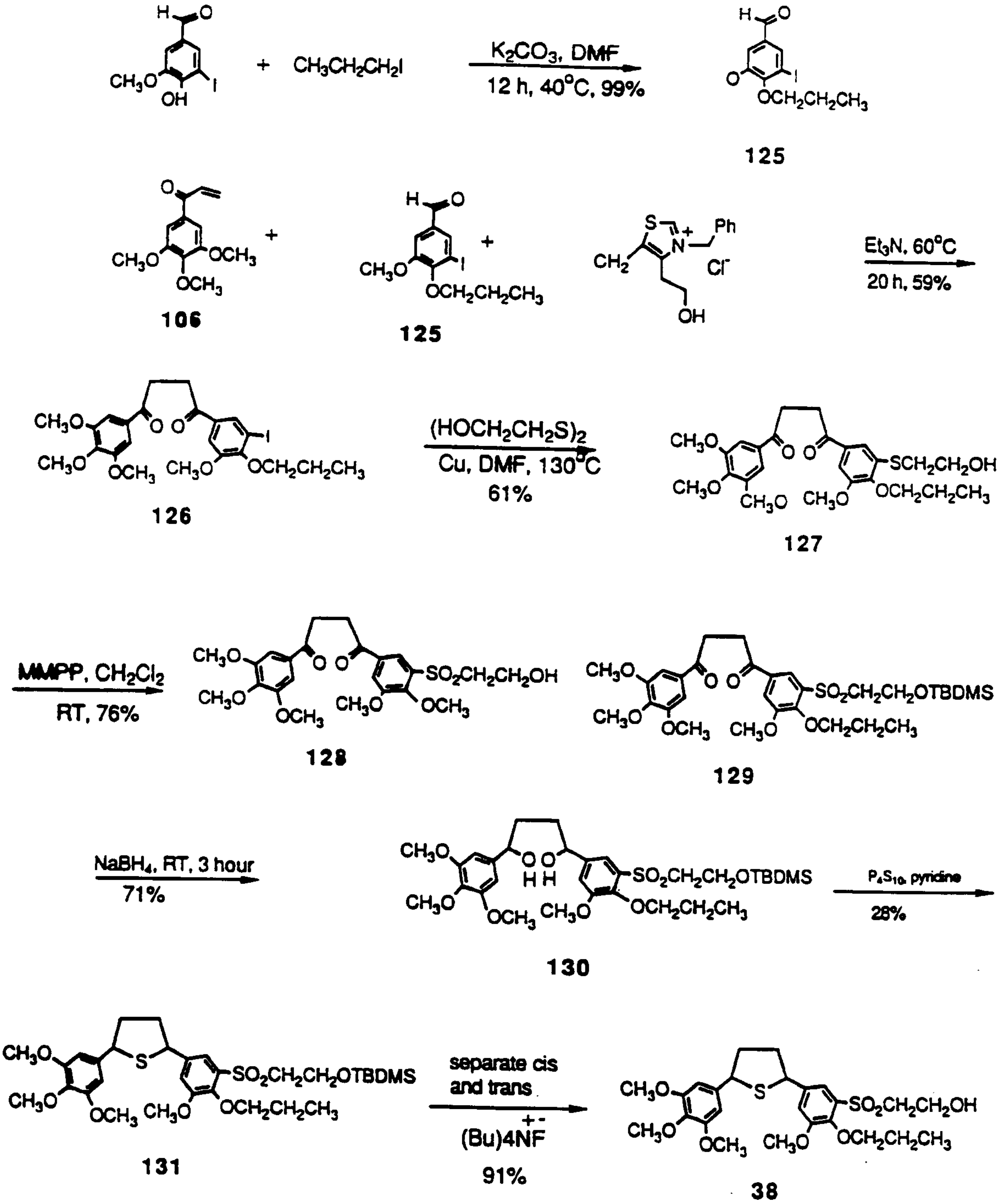


125

106

125

126

127

128

129

130

131

38

**Figure 6**

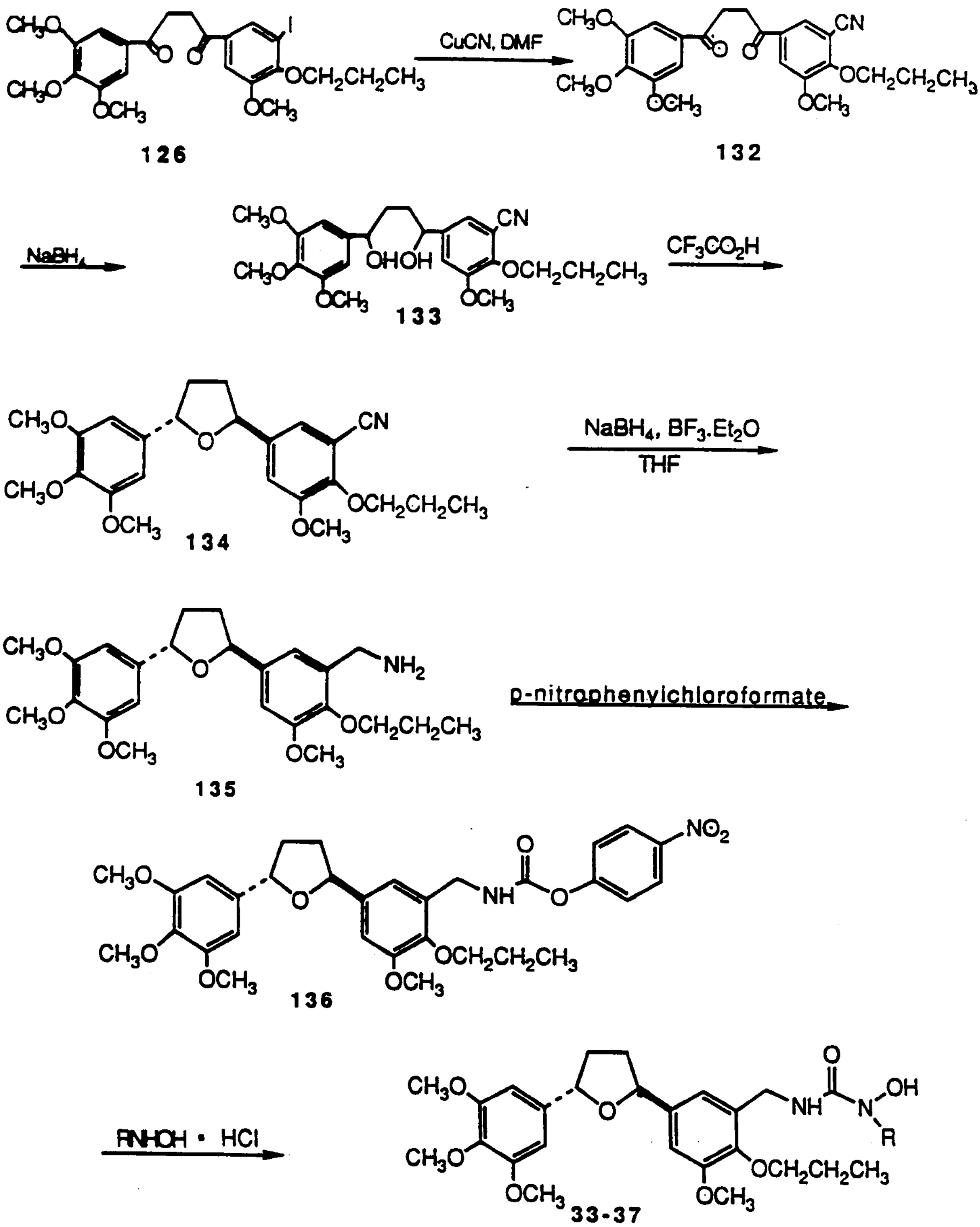


126

132

133

134

135

136

33-37

Figure 7

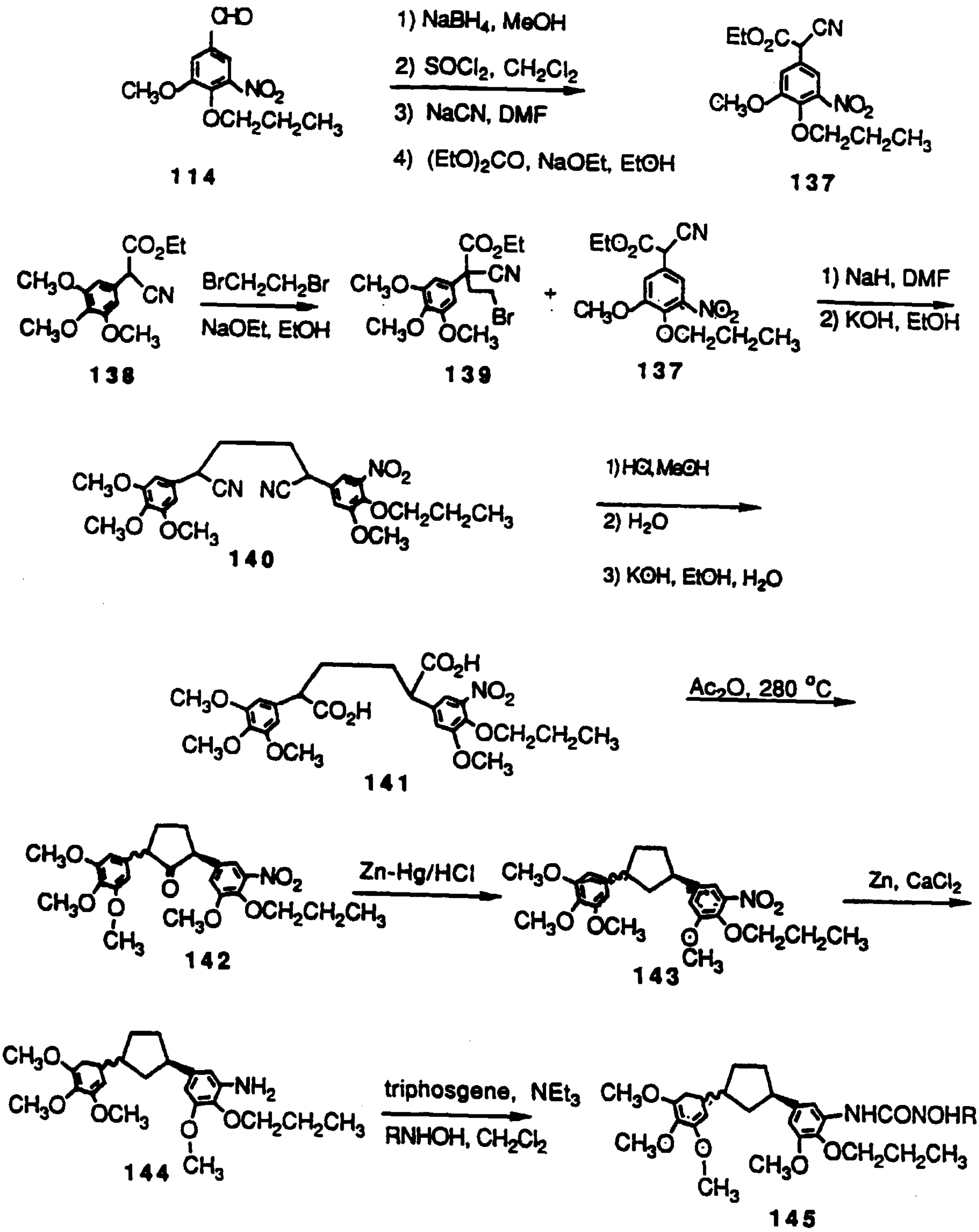


114, 137, 138, 139, 137, 140, 141, 142, 143, 144, 145

Figure 8

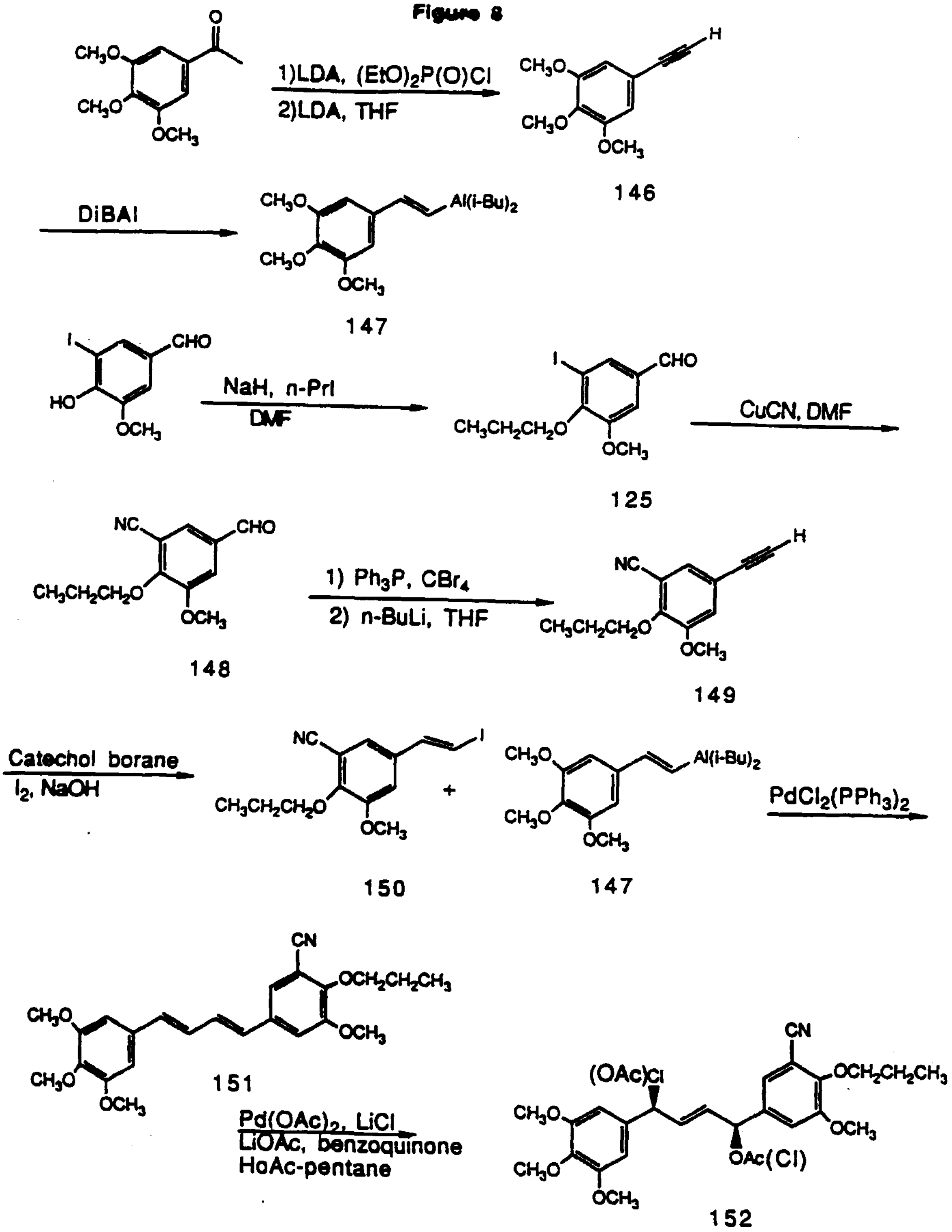
1)LDA, $(EtO)_2P(O)Cl$
2)LDA, THF
146
DiBAl
147
NaH, n-PrI
DMF
125
CuCN, DMF
148
1) $Ph_3P$, $CBr_4$
2) n-BuLi, THF
149
Catechol borane
$I_2$, NaOH
+
150
147
$PdCl_2(PPh_3)_2$
151
$Pd(OAc)_2$, LiCl
LiOAc, benzoquinone
HoAc-pentane
152

**Figure 8 (continue)**

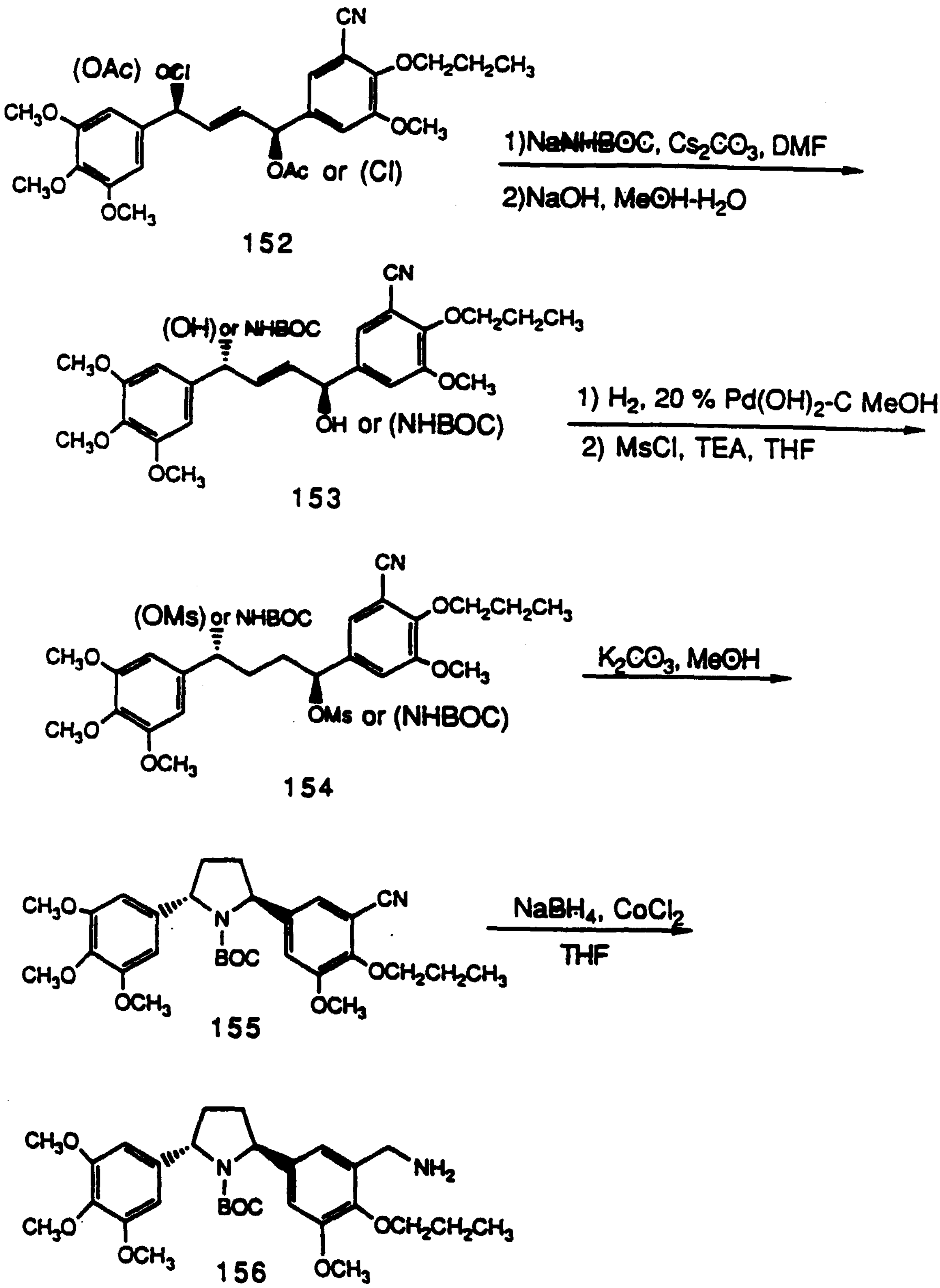

**Figure 8 (continue)**

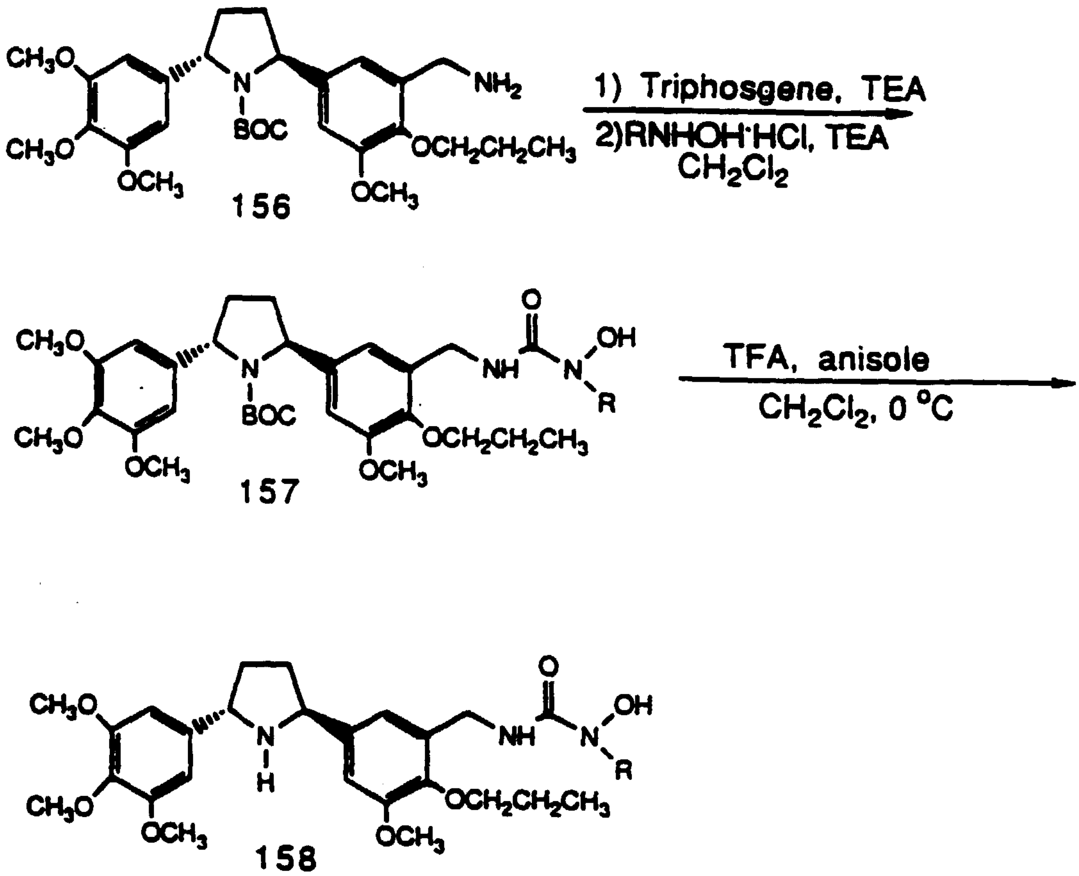


156

157

158

**Figure 9**

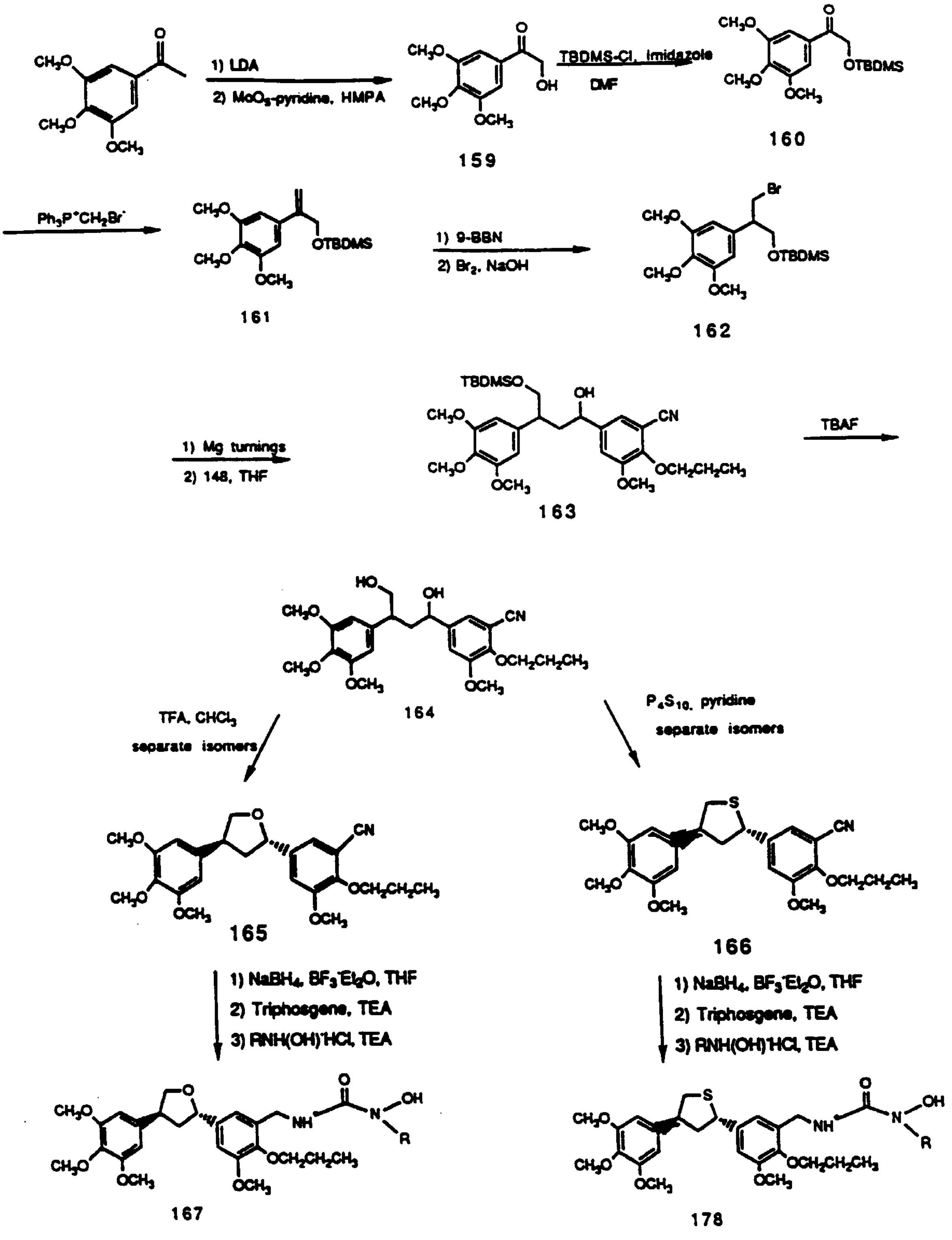
1) LDA
2) MoO$_5$-pyridine, HMPA
159
TBDMS-Cl, Imidazole
DMF
160
Ph$_3$P$^+$CH$_2$Br$^-$
161
1) 9-BBN
2) Br$_2$, NaOH
162
1) Mg turnings
2) 148, THF
163
TBAF
164
TFA, CHCl$_3$
separate isomers
P$_4$S$_{10}$, pyridine
separate isomers
165
166
1) NaBH$_4$, BF$_3$·Et$_2$O, THF
2) Triphosgene, TEA
3) RNH(OH)·HCl, TEA
1) NaBH$_4$, BF$_3$·Et$_2$O, THF
2) Triphosgene, TEA
3) RNH(OH)·HCl, TEA
167
178

Figure 9 (continue)

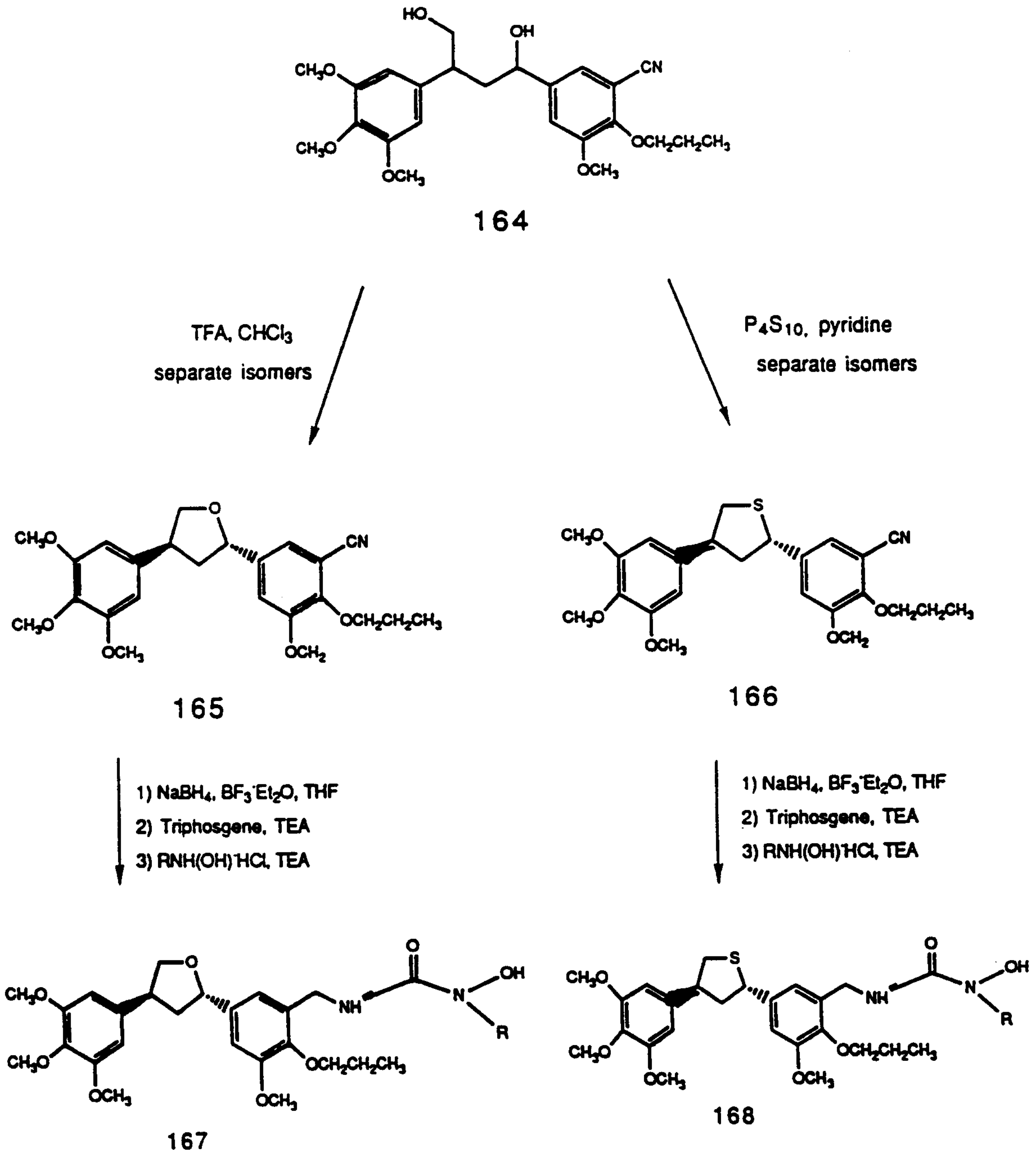


164

165

166

167

168

FIGURE 10a

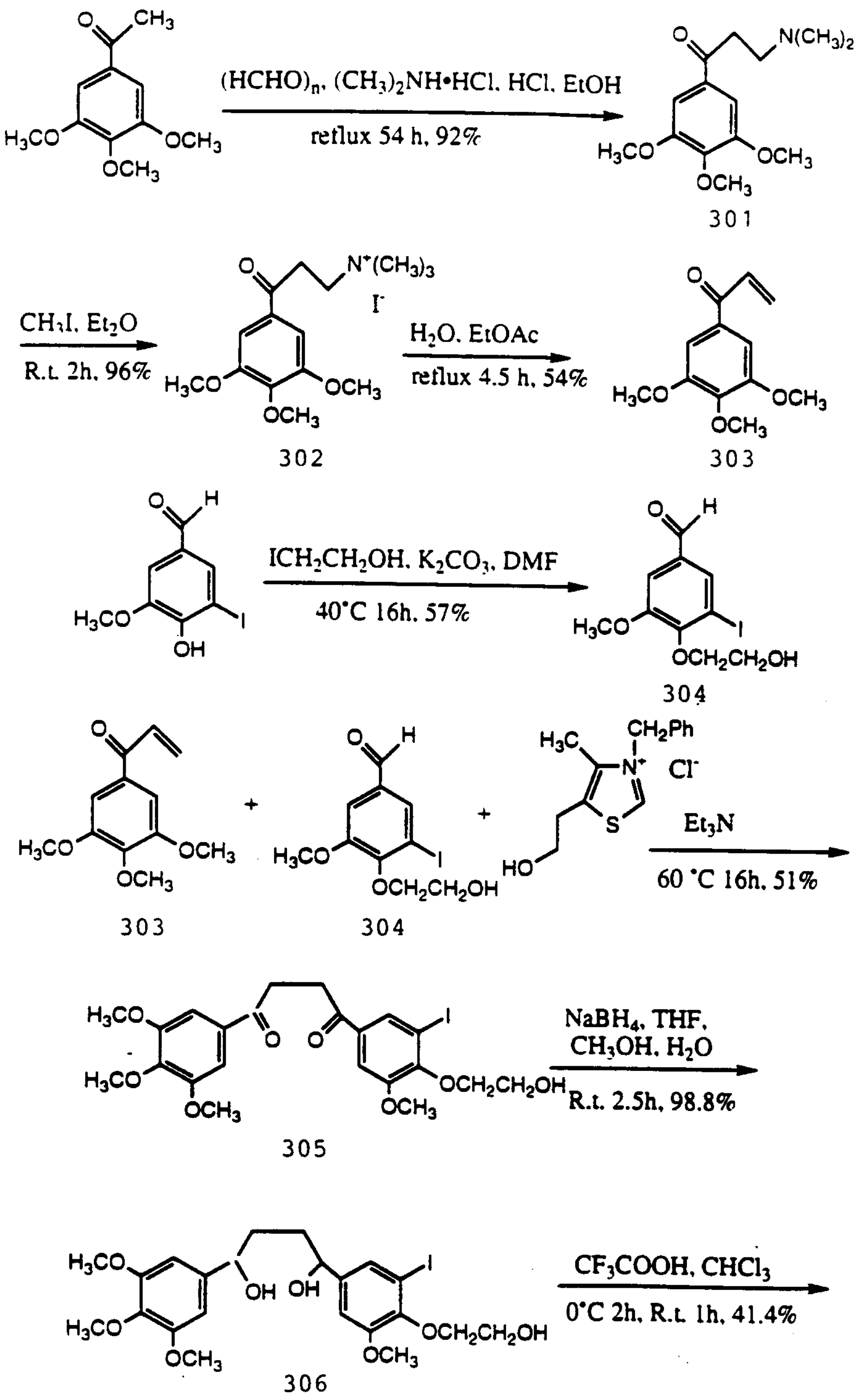
$(HCHO)_n$, $(CH_3)_2NH$•HCl, HCl, EtOH
reflux 54 h, 92%
301
$CH_3I$, $Et_2O$
R.t. 2h, 96%
302
$H_2O$, EtOAc
reflux 4.5 h, 54%
303
$ICH_2CH_2OH$, $K_2CO_3$, DMF
40°C 16h, 57%
304
303
304
$Et_3N$
60 °C 16h, 51%
305
$NaBH_4$, THF, $CH_3OH$, $H_2O$
R.t. 2.5h, 98.8%
306
$CF_3COOH$, $CHCl_3$
0°C 2h, R.t. 1h, 41.4%

FIGURE 10b

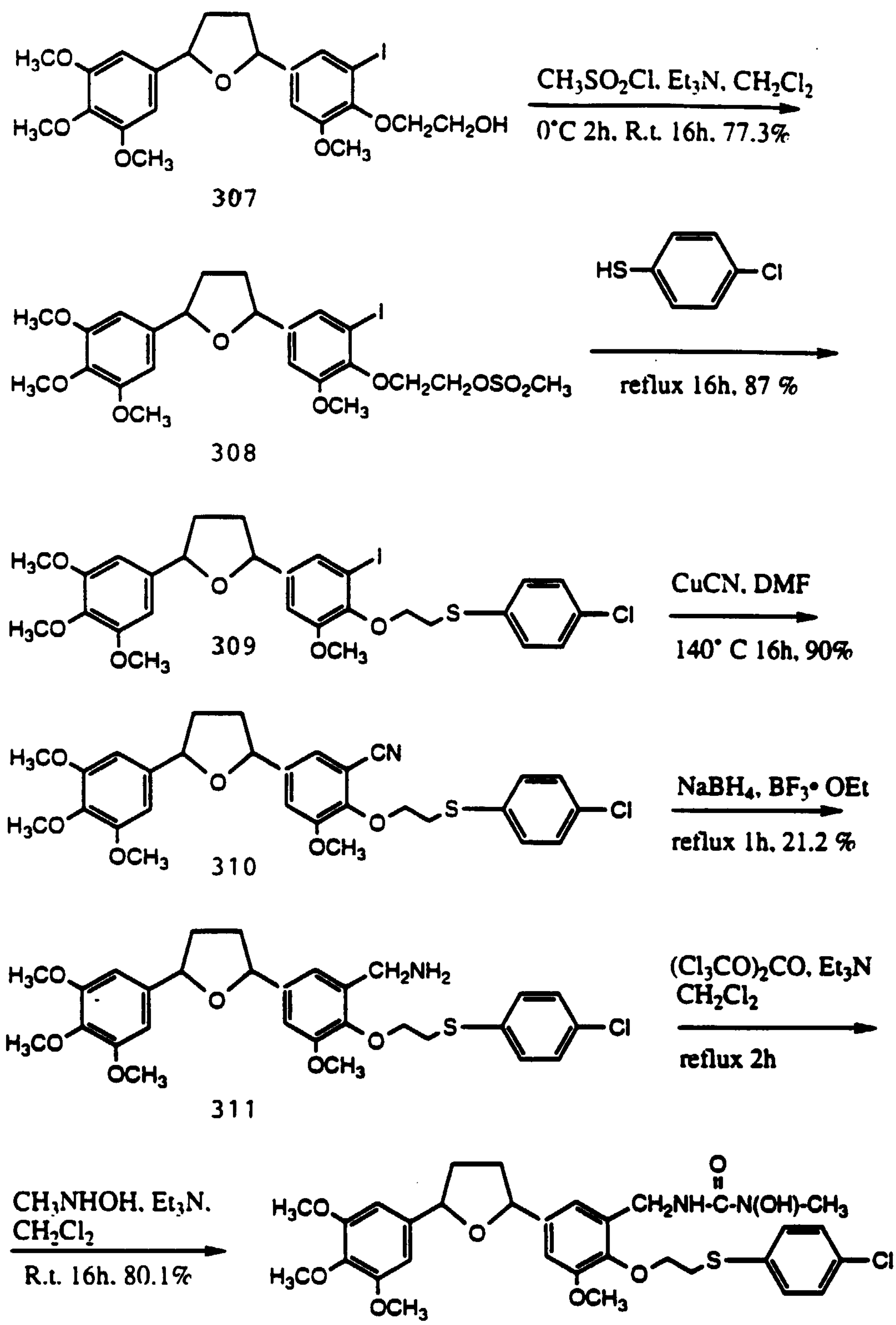


307

308

309

310

311

229